(19) Europäisches Patentamt
European Patent Office
Office européen des brevets

(11) **EP 4 506 002 A1**

(12) **EUROPEAN PATENT APPLICATION**
published in accordance with Art. 153(4) EPC

(43) Date of publication:
**12.02.2025 Bulletin 2025/07**

(21) Application number: **23790996.5**

(22) Date of filing: **29.03.2023**

(51) International Patent Classification (IPC):
***A61K 31/4545*** *(2006.01)* ***A61K 31/4439*** *(2006.01)*
***A61K 31/496*** *(2006.01)* ***A61K 31/551*** *(2006.01)*
***C07D 417/14*** *(2006.01)* ***C07D 417/04*** *(2006.01)*
***C07D 401/14*** *(2006.01)* ***C07D 401/04*** *(2006.01)*
***C07D 421/14*** *(2006.01)* ***A61P 31/00*** *(2006.01)*
***A61P 31/04*** *(2006.01)* ***A61P 31/10*** *(2006.01)*
***A61P 31/12*** *(2006.01)* ***A61P 31/14*** *(2006.01)*
***A61P 31/20*** *(2006.01)* ***A61P 33/00*** *(2006.01)*
***A61P 35/00*** *(2006.01)* ***A61P 35/02*** *(2006.01)*

(86) International application number:
**PCT/CN2023/084751**

(87) International publication number:
**WO 2023/202336 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.04.2022 CN 202210427491**

(71) Applicant: **Zhuhai Yufan Biotechnologies Co., Ltd**
**Zhuhai, Guangdong 519031 (CN)**

(72) Inventor: **LIN, Xingyu**
**Zhuhai, Guangdong 519031 (CN)**

(74) Representative: **Michalski Hüttermann & Partner Patentanwälte mbB**
**Kaistraße 16A**
**40221 Düsseldorf (DE)**

(54) **USE OF HPK1 INHIBITOR IN PREVENTION AND/OR TREATMENT OF HUMAN PATHOGEN INFECTION**

(57) Provided in the present invention are the uses of an HPK1 inhibitor in preparation of drugs for preventing and/or treating human diseases or symptoms caused by pathogen infection or related to pathogen infection, and in preparation of drugs for preventing and/or treating tumors. The HPK1 inhibitor is a compound represented by following general formula I, or pharmaceutically acceptable salts, stereoisomers, esters, prodrugs, solvates and deuterated compounds thereof <img file= "PCTCN2023084751-isre-I000001.jpg" he="58.34" img-content= "drawing" img-format="jpg" inline="yes" orientation="portrait" wi="131.93"/>.

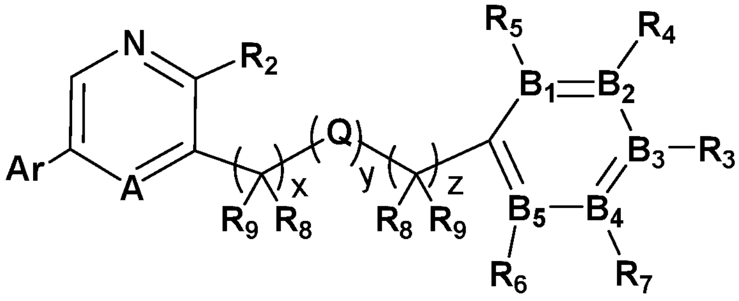

(I)

EP 4 506 002 A1

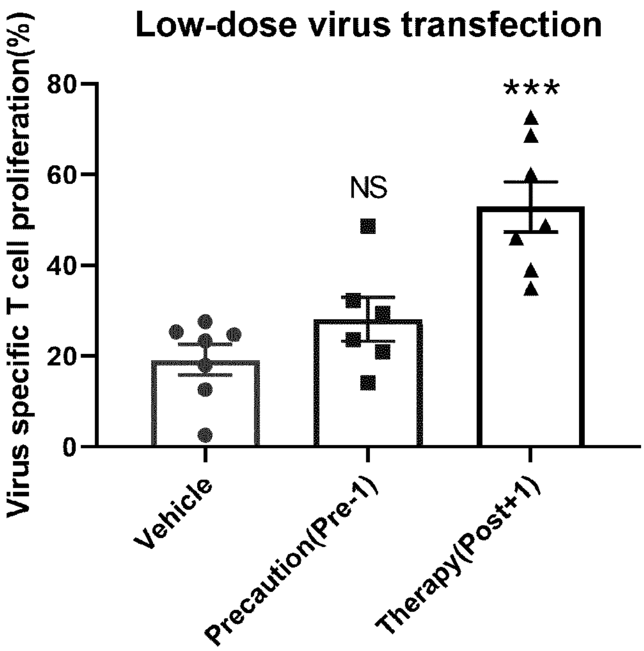
Low-dose virus transfection
Virus specific T cell proliferation(%)
80
60
40
20
0
NS
***
Vehicle
Precaution(Pre-1)
Therapy(Post+1)

FIG. 21

## Description

## FIELD

The present invention relates to the field of medical technology, in particular to the use of an HPK1 inhibitor in preparation of drugs for preventing and/or treating human diseases or symptoms caused by pathogen infection or related to pathogen infection. The present invention also relates to a crystalline form of the above HPK1 inhibitor, preparation method thereof and use thereof.

## BACKGROUND

The prevention and treatment of viral diseases is a major concern in the world today. Although vaccines have been used to some extent to prevent viral infectious diseases, the existing drugs are far from meeting the needs due to the high variability of the virus itself. There is still a long way to develop new and effective antiviral drugs.

HPK1 (hematopoietic progenitor kinase 1), a member of the mitogen-activated protein kinase kinase kinase kinase (MAP4K) family, plays an important role in regulating cell survival, cell migration, apoptosis and autophagy. HPK1 kinase is involved in many signaling cascades, including growth factor signaling, MAPK signaling, cytokine signaling, apoptosis signaling, growth factor signaling, antigen receptor signaling, etc. HPK1 is a negative regulator of T cell, B cell and dendritic cell activation. Inhibiting the function of HPK1 can enhance immune cells and thus enhance immunological function. Therefore, it is of great significance to develop an HPK1 inhibitor.

Patent document CN113861188A discloses a pyrazolo[3,4-b]pyridine derivative having the following general formula:

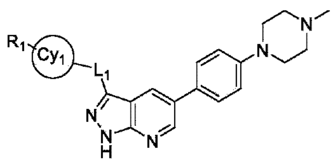

(I)

, preparation method thereof and uses as an HPK1 inhibitor. Patent document CN113316576A discloses an HPK1 compound having the following general formula:

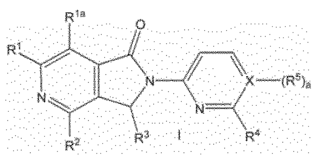

.

Patent document WO2021213317A1 discloses an HPK1 inhibitor, preparation method thereof and use thereof, the compound having the following structural formula:

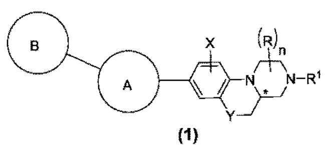

(1)

.

However, there are few reports in the related art on the use of small molecule HPK1 inhibitors in therapeutic drugs against viral infections. Therefore, the application of HPK1 inhibitors in the treatment of viral infections has a very broad prospect.

In view of this, the present invention is hereby proposed.

## SUMMARY

Provided in the present invention is the use of an HPK1 inhibitor in preparation of drugs for preventing and/or treating human diseases or symptoms caused by pathogen infection or related to pathogen infection.

Further, the pathogens may be microorganisms, parasites or other agents;
In particular, the parasites are protozoa, helminths or other agents.

Preferably, the microorganisms are selected from one or more of viruses, chlamydias, rickettsias, mycoplasmas, bacteria, spirochetes, fungi, etc.;

More preferably, the pathogens are viruses, including, but not limited to, adenoviridae, herpesviridae, HSV2, VZV, EBV, CMV, poxviridae, papovaviridae, parvoviridae, hepadnaviridae, polyomaviridae, reoviridae, picornaviridae, caliciviridae, togaviridae, arenaviridae, retroviridae, flaviviridae, orthomyxoviridae, paramyxoviridae, bunyaviridae, coronaviridae, astroviridae and bornaviridae;
In a specific embodiment of the present invention, the pathogens are viruses, for example, but not limited to, adenoviridae (e.g., adenovirus), herpesviridae (e.g., HSV1 (oral herpes), HSV2 (genital herpes), VZV (chickenpox), EBV (Epstein-Barr virus), CMV (cytomegalovirus)), poxviridae (e.g., smallpox virus, vaccinia virus), papovaviridae (e.g., human papilloma virus (HPV)), parvoviridae (e.g., B19 virus), hepadnaviridae (e.g., Hepatitis B Virus (HBV)), polyomaviridae (e.g., polyomavirus), reoviridae (e.g., reovirus, rotavirus), picornaviridae (e.g., enterovirus, foot and mouth disease virus), caliciviridae (e.g., Norwalk virus, hepatitis E virus), togaviridae (e.g., rubella virus), arenaviridae (e.g., lymphocytic choriomeningitis virus), retroviridae (HIV), flaviviridae (e.g., dengue virus, zika virus, encephalitis B virus, chikungunya virus, yellow fever virus, hepatitis C virus (HCV), West Nile virus, etc.), orthomyxoviridae (e.g., influenza viruses (such as influenza A virus, influenza B virus, influenza C virus, etc.)), paramyxoviridae (e.g., human parainfluenza virus type 1 (HPV), HPV type 2, HPV type 3, HPV type 4, sendai virus, mumps virus, measles virus, respiratory syncytial virus, newcastle disease virus, etc.), bunyaviridae (e.g., California encephalitis virus, hantaan virus), rhabdoviridae (e.g., rabies virus), filoviridae (e.g., Ebola virus, Marburg virus), coronaviridae (e.g., HCoV-229E, HCoV-OC43, HCoV-NL63, HCoV-HKU1, SARS-CoV, MERS-CoV, SARS-CoV-2, etc.), astroviridae (e.g., astrovirus) and bornaviridae (e.g., Bornavirus).

More preferably, the viruses are HBV, HIV, HCV, HPV, Ebola virus, Marburg virus, influenza virus, parainfluenza virus, dengue virus and human coronavirus;
More preferably, the human coronavirus includes SARS-CoV, SARS-CoV-2, MERS-CoV, HCoV-229E, HCoV-NL63, HCoV-OC43 and HCoV-HKUl.
More preferably, the diseases caused by pathogen infection or related to pathogen infection include influenza, SARS, MERS, COVID-19, viral hepatitis, AIDS, dengue fever, Ebola virus disease and Marburg virus disease;
More preferably, the viral hepatitis includes hepatitis B and hepatitis C.
It can be seen from the related art that the T cell immune response is an important part of the body's immune response. Activation of CD4+ T cells can help B cells produce neutralizing antibodies and activate CD8+ T cells capable of destroying virus-infected cells (Grifoni A, Weiskopf D, Ramirez S I, Mateus J, Dan J M, Moderbacher C R, Rawlings S A, Sutherland A, Premkumar L, Jadi R S, Marrama D, De Silva A M, Frazier A, Carlin A F, Greenbaum J A, Peters B, Krammer F, Smith D M, Crotty S, Sette A. Targets of T Cell Responses to SARS-CoV-2 Coronavirus in Humans with COVID-19 Disease and Unexposed Individuals[J]. Cell, 2020, 181(7): 1489-1501.e15.). At the site of infection, the activated virus-specific effector T cells produce antiviral cytokines (e.g., IL-2) and thus help the body fight the virus (Liao W, Lin J-X, Leonard W J. IL-2 Family Cytokines: New Insights into the Complex Roles of IL-2 as a Broad Regulator of T helper Cell Differentiation[J]. Current Opinion in Immunology, 2011, 23(5): 598-604, Channappanavar R, Zhao J, Perlman S. T cell-mediated immune response to respiratory coronaviruses[J]. Immunologic Research, 2014, 59(1): 118-128). Applicants have made inventive efforts to discover that the HPK1 inhibitor of the present invention can enhance T cell proliferation, resulting in a sustained elevation of TCR-induced calcium mobilization and pro-inflammatory cytokine (e.g., IL-2) levels. Moreover, the inhibitor can induce B cell activation and significantly enhance the immune function of T cells and dendritic cells. The HPK1 inhibitor of the disclosure can be used not only as an antitumor drug but also as an antiviral drug.
Further, the inhibitor is a compound represented by following general formula I, or pharmaceutically acceptable salts, stereoisomers, esters, prodrugs, solvates and deuterated compounds thereof:

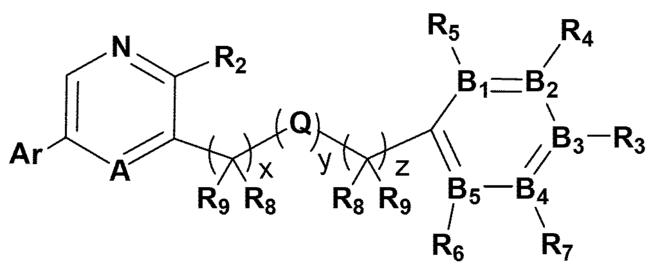

(I)

wherein

A is selected from $CR_{10}$ or N;
Q is selected from O or S;
x and z are independently selected from integers between 0 and 6 (e.g., 0, 1, 2, 3, 4, 5 and 6);
y is 0 or 1;
Ar is selected from an aromatic five-membered heterocyclic group, an aromatic six-membered heterocyclic group or phenyl, wherein the aromatic five-membered heterocyclic group is selected from furyl, thienyl, pyrrolyl, pyrazolyl, imidazolyl, oxazolyl, thiazolyl or selenothiazolyl; the aromatic six-membered heterocyclic group is selected from pyridinyl, pyridazinyl, pyrimidinyl or pyrazinyl; optionally, the H atoms in the aromatic five-membered heterocyclic group, the aromatic six-membered heterocyclic group or the phenyl may be substituted with the following groups: -D, $-SO_2$, $-SO_2N(C_{0-10}$ alkyl)($C_{0-10}$ alkyl), $-N(C_{0-10}$ alkyl)$SO_2(C_{0-10}$ alkyl), -CON($C_{0-10}$ alkyl)($C_{0-10}$ alkyl), -N($C_{0-10}$ alkyl) CO($C_{0-10}$ alkyl), -N($C_{0-10}$ alkyl)COO($C_{0-10}$ alkyl), -OCON($C_{0-10}$ alkyl)($C_{0-10}$ alkyl), halogen, -CN, $-OCH_2F$, $-OCHF_2$, $-OCF_3$, $C_{1-10}$ straight-chain/branched alkyl, -N($C_{0-10}$ alkyl)($C_{0-10}$ alkyl), $-OC_{0-10}$ alkyl, $C_{3-10}$ cycloalkyl, -O-containing heterocycloalkyl, -N-containing heterocycloalkyl, -N-containing heteroaryl, -O-containing heteroaryl or -S-containing heteroaryl, wherein the alkyl moiety may be optionally substituted with one or more of the following groups: $-SO_2$, $-SO_2N(C_{0-10}$ alkyl)($C_{0-10}$ alkyl), -N($C_{0-10}$ alkyl)$SO_2(C_{0-10}$ alkyl), -CON($C_{0-10}$ alkyl)($C_{0-10}$ alkyl), -N($C_{0-10}$ alkyl) CO($C_{0-10}$alkyl), -N($C_{0-10}$ alkyl)COO($C_{0-10}$ alkyl), -OCON($C_{0-10}$ alkyl)($C_{0-10}$ alkyl), halogen, -CN, $-OCH_2F$, $-OCHF_2$, $-OCF_3$, -N($C_{0-10}$ alkyl)($C_{0-10}$ alkyl), $-OC_{0-10}$ alkyl, -CO($C_{0-10}$ alkyl), -COO($C_{0-10}$ alkyl), -N-containing heteroaryl, -O-containing heteroaryl or -S-containing heteroaryl;
$R_2$ is selected from -H, -D, halogen, $-NO_2$, -CN, $C_{1-10}$ straight-chain/branched alkyl, $C_{3-10}$ cycloalkyl, -N($C_{0-10}$ alkyl) ($C_{0-10}$ alkyl), $-CF_3$, $-OCF_3$, $-OCHF_2$, $-OCH_2F$ or $-OC_{0-10}$ alkyl;
$B_1$, $B_2$, $B_3$, $B_4$ and $B_5$ are independently selected from C or N (when $B_1$, $B_2$, $B_3$, $B_4$ or $B_5$ is N, its corresponding $R_3$, $R_4$, $R_5$, $R_6$ and $R_7$ are absent);
$R_3$, $R_4$, $R_5$, $R_6$ and $R_7$, when present, are independently selected from -H, -D, halogen, -CN, $-OC_{0-10}$ alkyl, -CO($C_{0-10}$ alkyl), -CON($C_{0-10}$ alkyl)($C_{0-10}$ alkyl), $C_{1-10}$ straight-chain/branched alkyl, O- or N-containing heteroalkyl, -N($C_{0-10}$ alkyl)($C_{0-10}$ alkyl), $C_{3-10}$ cycloalkyl, $-C{\equiv}C-R_{10}$, -O-containing heterocycloalkyl and -N-containing heterocycloalkyl, or $R_5$ and $R_4$, $R_4$ and $R_3$, $R_3$ and $R_7$, $R_7$ and $R_6$, together with the carbon atoms between them, form $C_{3-8}$ cycloalkyl or -O- and -S-containing $C_{3-8}$ heterocycloalkyl, -N-containing heteroaryl, -O-containing heteroaryl or -S-containing heteroaryl and phenyl, wherein the alkyl moiety may be optionally substituted with one or more of the following groups: $-SO_2$, $-SO_2N(C_{0-10}$ alkyl)($C_{0-10}$ alkyl), -N($C_{0-10}$ alkyl)$SO_2(C_{0-10}$ alkyl), -CON($C_{0-10}$ alkyl)($C_{0-10}$ alkyl), -N($C_{0-10}$ alkyl) CO($C_{0-10}$ alkyl), -N($C_{0-10}$ alkyl)COO($C_{0-10}$ alkyl), -OCON($C_{0-10}$ alkyl)($C_{0-10}$ alkyl), halogen, -CN, $-OCH_2F$, $-OCHF_2$, $-OCF_3$, $C_{1-10}$ straight-chain/branched alkyl, -N($C_{0-10}$ alkyl)($C_{0-10}$ alkyl), $-OC_{0-10}$ alkyl, $C_{3-10}$ cycloalkyl, -O-containing heterocycloalkyl, -N-containing heterocycloalkyl, -N-containing heteroaryl, -O-containing heteroaryl or -S-containing heteroaryl;
$R_8$ and $R_9$ are independently selected from -H, -D, halogen and $C_{1-10}$ straight-chain/branched alkyl;
$R_{10}$ is selected from H, -D, $C_{1-5}$ straight-chain/branched alkyl, $C_{3-10}$ cycloalkyl and

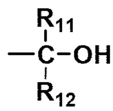
;

and
$R_{11}$ and $R_{12}$ are independently selected from -H, -D, $-CF_3$, $-CHF_2H$, $-CH_2F$, $C_{1-10}$ straight-chain/branched alkyl, -CH=C($C_{0-10}$ alkyl)($C_{0-10}$ alkyl), $-C{\equiv}C(C_{0-10}$ alkyl), $C_{3-10}$ cycloalkyl, an aromatic five-membered ring group or an aromatic six-membered ring group, or $R_{11}$ and $R_{12}$, together with the carbon atom between $R_{11}$ and $R_{12}$, form $C_{3-8}$ cycloalkyl or -O- and -S-containing $C_{3-8}$ heterocycloalkyl, $C_{4-9}$ fused cycloalkyl, $C_{5-10}$ spirocycloalkyl, $C_{4-9}$ bridged cycloalkyl, $C_{3-7}$ cyclic lactam, $C_{3-7}$ cyclic lactone and $C_{3-7}$ cyclic ketone, wherein the alkyl moiety may be optionally substituted with one or more of the following groups: $-SO_2$, $-SO_2N(C_{0-10}$ alkyl)($C_{0-10}$ alkyl), -N($C_{0-10}$ alkyl)$SO_2(C_{0-10}$ alkyl), -CON($C_{0-10}$ alkyl)($C_{0-10}$ alkyl), -N($C_{0-10}$ alkyl)CO($C_{0-10}$ alkyl), -N($C_{0-10}$ alkyl)COO($C_{0-10}$ alkyl), -OCON($C_{0-10}$ alkyl)($C_{0-10}$ alkyl), halogen, -CN, $-OCH_2F$, $-OCHF_2$, $-OCF_3$, -N($C_{0-10}$ alkyl)($C_{0-10}$ alkyl), $-OC_{0-10}$ alkyl, -N-containing heteroaryl, -O-containing heteroaryl or -S-containing heteroaryl;
Further, one or more H atoms attached to the C atoms in Ar, $R_2$, $R_3$, $R_4$, $R_5$, $R_6$, $R_7$, $R_8$, $R_9$, $R_{10}$, $R_{11}$ and/or $R_{12}$ may be substituted with deuterium;
and/or one or more H atoms attached to the heteroatoms in Ar, $R_2$, $R_3$, $R_4$, $R_5$, $R_6$, $R_7$, $R_8$, $R_9$, $R_{10}$, $R_{11}$ and/or $R_{12}$ may be substituted with deuterium;
and/or the compound represented by general formula I contains at least one deuterium atom.
Moreover, the compound represented by general formula I contains at least one deuterium atom.

In an embodiment of the present invention, A is $CR_{10}$, in particular CH.

In an embodiment of the present invention, Q is O.

In an embodiment of the present invention, x is 0.

In an embodiment of the present invention, z is 1.

In an embodiment of the present invention, y is 1.

In an embodiment of the present invention, $B_1$, $B_2$, $B_3$, $B_4$ and $B_5$ are all C, i.e., in general formula I,

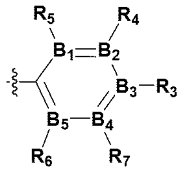

is

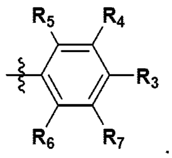

In another embodiment of the present invention, at least one of $B_1$, $B_2$, $B_3$, $B_4$ and $B_5$ is N.

In particular, $B_2$ is C, and at least one of $B_1$, $B_3$, $B_4$ and $B_5$ is N.

More particularly, $B_2$ is C, and $B_1$ is N.

More particularly, $B_2$ is C, and $B_3$ is N.

More particularly, $B_2$ is C, and $B_4$ is N.

More particularly, $B_2$ is C, and $B_5$ is N.

More particularly, $B_2$ is C, $B_3$ and $B_4$ are both N, or $B_3$ and $B_5$ are both N.

In particular, Ar is selected from thiazolyl, selenothiazolyl, imidazolyl, pyrazolyl and pyridinyl.

In an embodiment of the present invention, the compound represented by general formula I has the following structure:

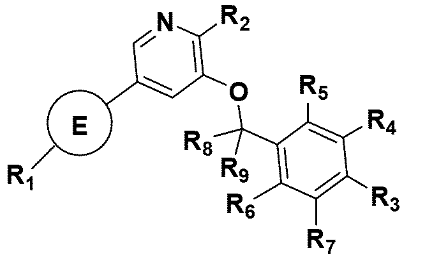

(II)

wherein ring E is selected from

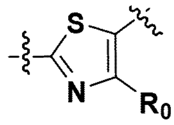,
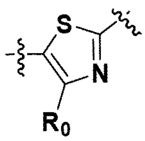,
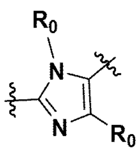,
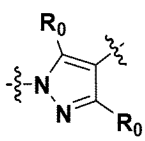,

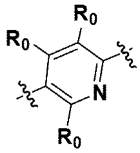 and 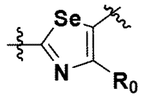;

in ring E, each $R_0$ is independently selected from -H, -D, $C_{1-10}$ straight-chain/branched alkyl, -N($C_{0-10}$ alkyl)($C_{0-10}$ alkyl), -O$C_{0-10}$ alkyl, -CO($C_{0-10}$ alkyl) or $C_{3-10}$ cycloalkyl; wherein the H atoms attached to the C atoms or heteroatoms may be substituted with deuterium; and

$R_1$ is selected from -H, -D, -O-containing heterocycloalkyl, -N-containing heterocycloalkyl, $C_{1-10}$ straight-chain/branched alkyl, $C_{3-10}$ cycloalkyl, -O$C_{0-10}$ alkyl, -N($C_{0-10}$ alkyl)($C_{0-10}$ alkyl), -$SO_2$($C_{0-10}$ alkyl), -CO($C_{0-10}$ alkyl), -O-phenyl, -S($C_{0-10}$ alkyl), -N-containing heteroaryl, -O-containing heteroaryl or -S-containing heteroaryl;

$R_{2-9}$ have the above corresponding definition in the present invention;

Further, the H atoms attached to the C atoms in $R_0$ and $R_1$ may be substituted with deuterium;

and/or the H atoms attached to the heteroatoms in $R_0$ and $R_1$ may be substituted with deuterium;

and/or at least one of $R_{0-9}$ contains a deuterium atom;

and/or the compound represented by general formula II contains at least one deuterium atom.

In an embodiment of the present invention, in general formula II, $R_1$ contains at least one deuterium atom; more particularly, for example, $R_1$ contains at least one deuterium atom, and $R_{2-9}$ contain no deuterium atom.

In another embodiment of the present invention, in general formula II, $R_2$ contains at least one deuterium atom.

In another embodiment of the present invention, in general formula II, $R_3$ contains at least one deuterium atom.

In another embodiment of the present invention, in general formula II, $R_4$ contains at least one deuterium atom.

In another embodiment of the present invention, in general formula II, $R_5$ contains at least one deuterium atom.

In another embodiment of the present invention, in general formula II, $R_6$ contains at least one deuterium atom.

In another embodiment of the present invention, in general formula II, $R_8$ and/or $R_9$ contain at least one deuterium atom.

In particular, each $R_0$ is independently selected from $C_{1-5}$ straight-chain/branched alkyl or -N($C_{0-10}$ alkyl)($C_{0-10}$ alkyl), wherein the H atoms attached to the C atoms may be substituted with deuterium.

More particularly, each $R_0$ is independently selected from -H, -D, -$CH_3$, -$CH_2CH_3$ or -$NH_2$.

In particular, $R_1$ is selected from -O-containing heterocycloalkyl or -N-containing heterocycloalkyl, -$SO_2$($C_{0-3}$ alkyl), -O-phenyl, -S($C_{0-4}$ alkyl), $C_{3-6}$ cycloalkyl or $C_{3-5}$ straight-chain/branched alkyl, wherein the H atoms attached to the C atoms or heteroatoms may be substituted with deuterium.

More particularly, $R_1$ is selected from

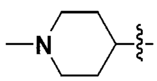, 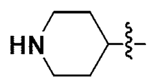, 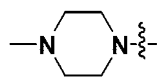,

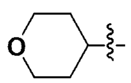, 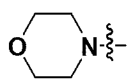, -$CH_3$, 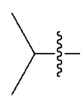, 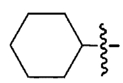, 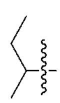, 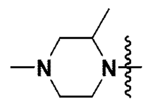, 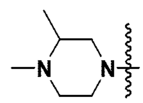, 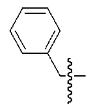,

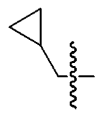, 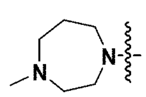, 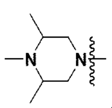, 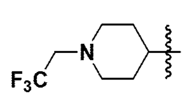, 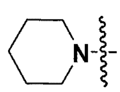, 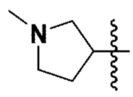, 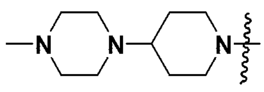,

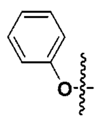, 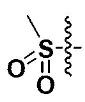, 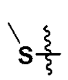, 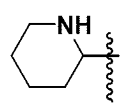, 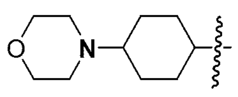, 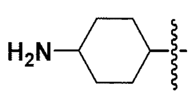 and 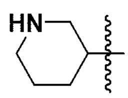,

wherein the H atoms attached to the C or N atoms may be substituted with deuterium.

More particularly, $R_1$ is selected from

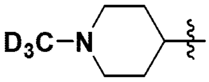, 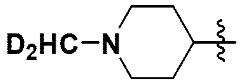,

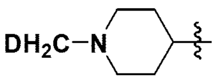, 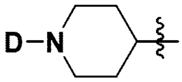, 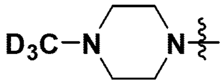, 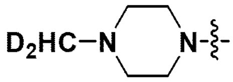, 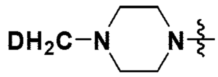,

$-CH_2D$, $-CHD_2$, $-CD_3$, 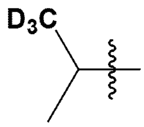, 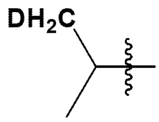, 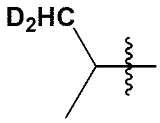, 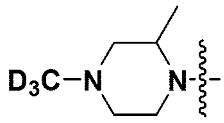,

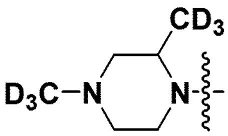, 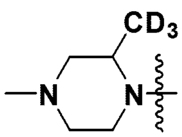, 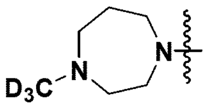, 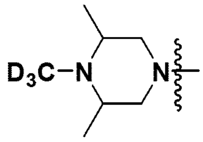, 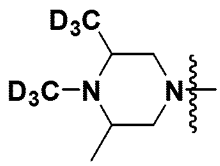,

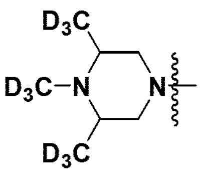, 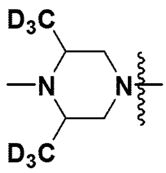, 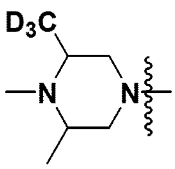, 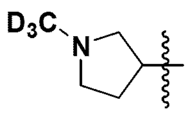, 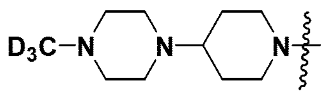,

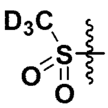, 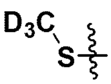, 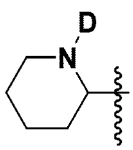, 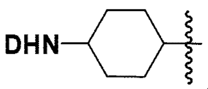, 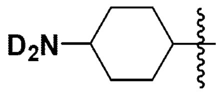 and 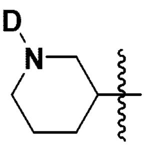.

In particular, when $R_0$ is adjacent to $R_1$, $R_0$ and $R_1$, together with the carbon atom between them, form $C_{3-8}$ cycloalkyl or -O- and -S-containing $C_{3-8}$ heterocycloalkyl, -N-containing heteroaryl, -O-containing heteroaryl or -S-containing heteroaryl and phenyl.

In particular, $R_2$ is selected from -H, -D, halogen, $-NO_2$, -CN, $C_{1-5}$ straight-chain/branched alkyl, $C_{3-10}$ cycloalkyl, $-N(C_{0-10}$ alkyl)($C_{0-10}$ alkyl), $-CF_3$, $-OCF_3$, $-OCHF_2$, $-OCH_2F$, or $-OC_{0-10}$ alkyl, wherein the H atoms attached to the C or N atoms may be substituted with deuterium.

More particularly, $R_2$ is selected from $-NO_2$, $-N(C_{0-10}$ alkyl)($C_{0-10}$ alkyl), $-OC_{0-10}$ alkyl and $-OCF_3$, wherein the H atoms attached to the C or N atoms may be substituted with deuterium.

Further, $R_2$ is selected from $-NH_2$, -NHD, $-ND_2$ or $-NO_2$.

In particular, $R_3$ is selected from -H, -D, halogen, $-OC_{0-10}$ alkyl, $-CO(C_{0-10}$ alkyl), $C_{1-10}$ straight-chain/branched alkyl, $-N(C_{0-10}$ alkyl)($C_{0-10}$ alkyl) or $C_{3-10}$ cycloalkyl, wherein the H atoms attached to the C atoms may be substituted with deuterium.

More particularly, $R_3$ is selected from -H, -D, halogen, $-OC_{0-10}$ alkyl and $C_{1-10}$ straight-chain/branched alkyl, wherein the H atoms attached to the C atoms may be substituted with deuterium.

Further, $R_3$ is selected from -H, -D, -F, $-OCH_3$, $-OCH_2D$, $-OCHD_2$ and $-OCD_3$.

In particular, $R_4$ is selected from -H, -D, halogen, $-OC_{0-10}$ alkyl, $-CO(C_{0-10}$ alkyl), -CN, $C_{3-10}$ cycloalkyl, $-C{\equiv}C-R_{10}$, $C_{1-10}$ straight-chain/branched alkyl, $-N(C_{0-10}$ alkyl)($C_{0-10}$ alkyl), -O-containing heterocycloalkyl or -N-containing heterocycloalkyl, wherein the H atoms attached to the C or N atoms may be substituted with deuterium.

More particularly, $R_4$ is selected from -H, -D, halogen, $-OC_{0-10}$ alkyl, -CN, $C_{3-10}$ cycloalkyl or $-C{\equiv}C-R_{10}$, wherein the H atoms attached to the C atoms may be substituted with deuterium. In an embodiment of the present invention, the H atoms attached to the C atoms in $R_{10}$ may be substituted with deuterium.

In an embodiment of the present invention, $R_4$ is selected from -H, -D, -F, -Cl, $-OCH_3$, $-OCH_2D$, $-OCHD_2$, $-OCD_3$, -CN,

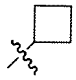

or $-C{\equiv}C-R_{10}$.

In particular, $R_5$, $R_6$ and $R_7$ are independently selected from -H, -D, halogen, -CN, $-OC_{0-10}$ alkyl, $-CO(C_{0-10}$ alkyl), $C_{1-10}$ straight-chain/branched alkyl, $-N(C_{0-10}$ alkyl)($C_{0-10}$ alkyl), $C_{3-10}$ cycloalkyl, $-C{\equiv}C-R_{10}$, -O-containing heterocycloalkyl or -N-containing heterocycloalkyl and O- or N-containing $C_{1-5}$ straight-chain/branched alkyl, or $R_6$ and $R_7$, together with the carbon atoms between $R_6$ and $R_7$, form $C_{3-8}$ cycloalkyl or -O- and -S-containing $C_{3-8}$ heterocycloalkyl, wherein the H atoms attached to the C atoms or heteroatoms may be substituted with deuterium.

More particularly, $R_5$, $R_6$ and $R_7$ are independently selected from -H, -D, halogen, -CN, $C_{1-3}$ straight-chain/-branched alkyl, $-OC_{0-3}$ alkyl, $-CO(C_{0-3}$ alkyl) and N-containing $C_{1-3}$ straight-chain/branched alkyl, or $R_6$ and $R_7$, together with the carbon atoms between $R_6$ and $R_7$, form $C_{3-8}$ cycloalkyl or -O-containing $C_{3-8}$ heterocycloalkyl, wherein the H atoms attached to the C or N atoms may be substituted with deuterium.

Further, $R_5$, $R_6$ and $R_7$ are independently selected from -H, -D, -F, -Cl, $-CH_3$, $-CH_2NH_2$, $-CH_2NH(CH_3)$, $-CH_2N(CH_3)_2$, -CN, $-OCH_3$ and $-COCH_3$, or $R_6$ and $R_7$, together with the carbon atoms between $R_6$ and $R_7$, form an -O-containing 5-membered cycloalkyl group, wherein the H atoms attached to the C or N atoms may be substituted with deuterium.

In an embodiment of the present invention, $R_5$ is selected from -H, -D, -F, -Cl, $-CH_3$, $-CH_2D$, $-CHD_2$, $-CD_3$, $-OCH_3$, $-COCH_3$, $-CH_2NH_2$, $-CH_2N(CH_3)_2$, -CN, $-OCH_2D$, $-OCHD_2$, $-OCD_3$, $-COCD_3$, $-CH_2N(CD_3)_2$ and $-CH_2N(CH_3)(CD_3)$.

In an embodiment of the present invention, $R_6$ is selected from -H, -D, -F, -Cl, $-CH_3$, $-CH_2D$, $-CHD_2$, $-CD_3$, $-OCH_3$, $-COCH_3$, $-CH_2NH_2$, $-CH_2N(CH_3)_2$, -CN, $-OCH_2D$, $-OCHD_2$, $-OCD_3$, $-COCD_3$, $-CH_2N(CD_3)_2$ and $-CH_2N(CH_3)(CD_3)$.

In an embodiment of the present invention, $R_7$ is selected from -H, -D, -F, -Cl, $-CH_3$, $-CH_2D$, $-CHD_2$, $-CD_3$, $-OCH_3$, $-COCH_3$, $-CH_2NH_2$, $-CH_2N(CH_3)_2$, -CN, $-OCH_2D$, $-OCHD_2$, $-OCD_3$, $-COCD_3$, $-CH_2N(CD_3)_2$ and $-CH_2N(CH_3)(CD_3)$.

In an embodiment of the present invention, $R_{10}$ is

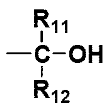

.

In an embodiment of the present invention, the H atoms attached to the C atoms in $R_{10}$ may be substituted with deuterium.

In particular, $R_{11}$ and $R_{12}$ are independently selected from -H, -D, $-CF_3$, $-CHF_2H$, $-CH_2F$, $C_{1-10}$ straight-chain/branched alkyl, $-CH{=}C(C_{0-10}$ alkyl)($C_{0-10}$ alkyl), $C_{3-10}$ cycloalkyl or an aromatic six-membered ring group, or $R_{11}$ and $R_{12}$, together with the carbon atom between $R_{11}$ and $R_{12}$, form $C_{3-8}$ cycloalkyl, $C_{4-7}$ fused cycloalkyl, $C_{5-9}$ spirocycloalkyl, $C_{4-9}$ bridged cycloalkyl, $C_{3-7}$ cyclic lactam, $C_{3-7}$ cyclic lactone and $C_{3-7}$ cyclic ketone, wherein the H atoms attached to the C atoms may be substituted with the following groups: $-SO_2$, $-SO_2N(C_{0-10}$ alkyl)($C_{0-10}$ alkyl), $-N(C_{0-10}$ alkyl)$SO_2(C_{0-10}$ alkyl), $-CON(C_{0-10}$ alkyl)($C_{0-10}$ alkyl), $-N(C_{0-10}$ alkyl)$CO(C_{0-10}$ alkyl), $-N(C_{0-10}$ alkyl)$COO(C_{0-10}$ alkyl), $-OCON(C_{0-10}$ alkyl)($C_{0-10}$ alkyl), halogen, -CN, $-OCH_2F$, $-OCHF_2$, $-OCF_3$, $C_{1-10}$ straight-chain/branched alkyl, $-N(C_{0-10}$ alkyl)($C_{0-10}$ alkyl), $-OC_{0-10}$ alkyl, $-CO(C_{0-10}$ alkyl), $C_{3-10}$ cycloalkyl, -O-containing heterocycloalkyl, -N-containing heterocycloalkyl, -N-containing heteroaryl, -O-containing heteroaryl or -S-containing heteroaryl, wherein the alkyl moiety may be optionally substituted with one or more of the following groups: $-SO_2$, $-SO_2N(C_{0-10}$ alkyl)($C_{0-10}$ alkyl), $-N(C_{0-10}$ alkyl)$SO_2(C_{0-10}$ alkyl), $-CON(C_{0-10}$ alkyl)($C_{0-10}$ alkyl), $-N(C_{0-10}$ alkyl)$CO(C_{0-10}$ alkyl), $-N(C_{0-10}$ alkyl)$COO(C_{0-10}$ alkyl), $-OCON(C_{0-10}$ alkyl)($C_{0-10}$ alkyl), halogen, -CN, $-OCH_2F$, $-OCHF_2$, $-OCF_3$, $-N(C_{0-10}$ alkyl)($C_{0-10}$ alkyl), $-OC_{0-10}$ alkyl, $-CO(C_{0-10}$ alkyl), -N-containing heteroaryl, -O-containing heteroaryl or -S-containing heteroaryl; wherein the H atoms attached to the C atoms or heteroatoms may be substituted with deuterium.

More particularly, $R_{11}$ and $R_{12}$ are independently selected from -H, -D, $-CF_3$, $-CHF_2$, $-CH_2F$, $C_{1-5}$ straight-chain/branched alkyl, $-CH{=}CH(C_{0-10}$ alkyl), $C_{3-10}$ cycloalkyl or an aromatic six-membered ring group, or $R_{11}$ and $R_{12}$, together with the carbon atom between $R_{11}$ and $R_{12}$, form $C_{3-6}$ cycloalkyl, $C_{4-6}$ fused cycloalkyl, $C_{5-8}$ spirocycloalkyl, $C_{4-8}$ bridged cycloalkyl, $C_{3-7}$ cyclic lactam, $C_{3-7}$ cyclic lactone and $C_{3-7}$ cyclic ketone, wherein the alkyl moiety may be substituted with the following groups: $-SO_2$, $-SO_2N(C_{0-10}$ alkyl)($C_{0-10}$ alkyl), $-N(C_{0-10}$ alkyl)$SO_2(C_{0-10}$ alkyl), $-CON(C_{0-10}$ alkyl)($C_{0-10}$ alkyl), $-N(C_{0-10}$ alkyl)$CO(C_{0-10}$ alkyl), $-N(C_{0-10}$ alkyl)$COO(C_{0-10}$ alkyl), $-OCON(C_{0-10}$ alkyl)($C_{0-10}$ alkyl), halogen, -CN, $-OCH_2F$, $-OCHF_2$, $-OCF_3$, $C_{1-10}$ straight-chain/branched alkyl, $-N(C_{0-10}$ alkyl)($C_{0-10}$ alkyl), $-OC_{0-10}$ alkyl, $-CO(C_{0-10}$ alkyl), $C_{3-10}$ cycloalkyl, -O-containing heterocycloalkyl, -N-containing heterocycloalkyl, -N-containing heteroaryl, -O-containing heteroaryl or -S-containing heteroaryl, wherein the alkyl moiety may be optionally substituted with one or more of the following groups: $-SO_2$, $-SO_2N(C_{0-10}$ alkyl)($C_{0-10}$ alkyl), $-N(C_{0-10}$ alkyl)$SO_2(C_{0-10}$ alkyl), $-CON(C_{0-10}$ alkyl)($C_{0-10}$ alkyl), $-N(C_{0-10}$ alkyl)$CO(C_{0-10}$ alkyl), $-N(C_{0-10}$ alkyl)$COO(C_{0-10}$ alkyl), $-OCON(C_{0-10}$ alkyl)($C_{0-10}$ alkyl), halogen, -CN, $-OCH_2F$, $-OCHF_2$, $-OCF_3$, $-N(C_{0-10}$ alkyl)($C_{0-10}$ alkyl), $-OC_{0-10}$ alkyl, $-CO(C_{0-10}$ alkyl), -N-containing heteroaryl, -O-containing heteroaryl or -S-containing heteroaryl; wherein the H atoms attached to the C atoms or heteroatoms may be

substituted with deuterium.

Further, $R_{11}$ and $R_{12}$ are independently selected from -H, -D, $-CF_3$, $-CHF_2$, $-CH_2F$, $-CH_3$,

$-CH_2CH_3$, $-CH{=}CH_2$, 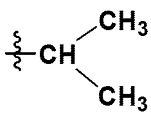, 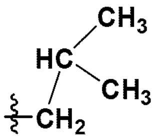, 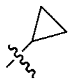, 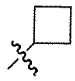, 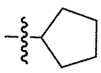, 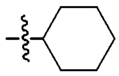

and

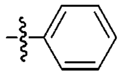,

, or $R_{11}$ and $R_{12}$, together with the carbon atom between $R_{11}$ and $R_{12}$, form

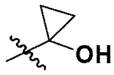,

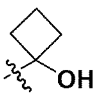, 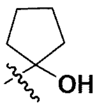, 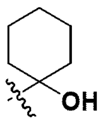, 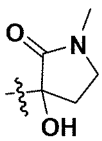, 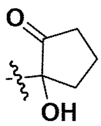, 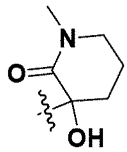, 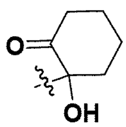, 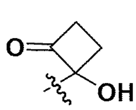,

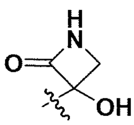, 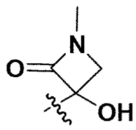, 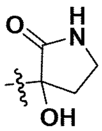, 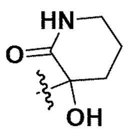, 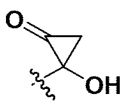, 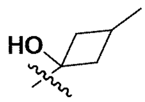, 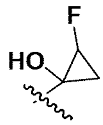,

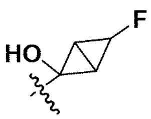 and 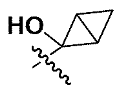,

wherein the H atoms attached to the C or N atoms may be substituted with deuterium.

Still more particularly, $R_{11}$ and $R_{12}$ are independently selected from -H, -D, $-CF_3$, $-CHF_2$, $-CDF_2$, $-CH_2F$, $-CD_2F$, $CH_3$, $-CH_2D$, $-CHD_2$, $-CD_3$, $-CH_2CH_3$, $-CH_2CD_3$,

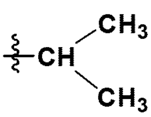,

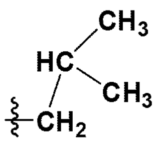, 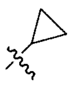, 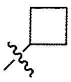, 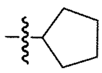, 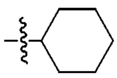 and 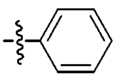.

In particular, $R_8$ and $R_9$ are independently selected from -H, -D and $C_{1-10}$ straight-chain/branched alkyl, wherein the H atoms attached to the C atoms may be substituted with deuterium.

More particularly, $R_8$ and $R_9$ are independently selected from -H, -D and $C_{1-3}$ straight-chain/branched alkyl, wherein the H atoms attached to the C atoms may be substituted with deuterium.

Further, $R_8$ and $R_9$ are independently selected from -H, -D, $-CH_3$, $-CH_2D$, $-CHD_2$ and $-CD_3$.

In particular, the compound represented by general formula I has the following structure:

(1)

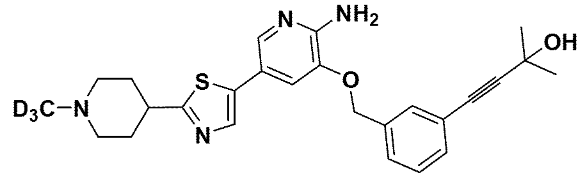

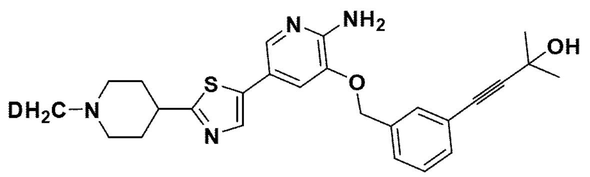

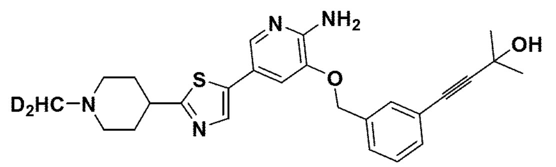

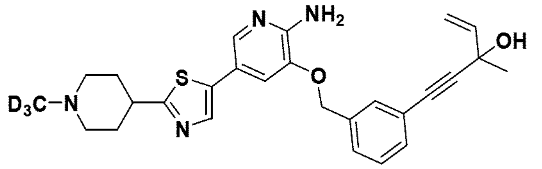

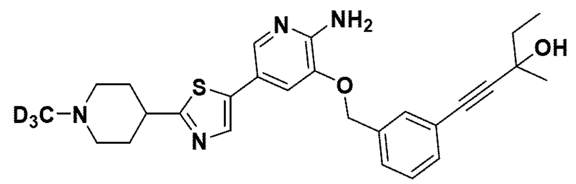

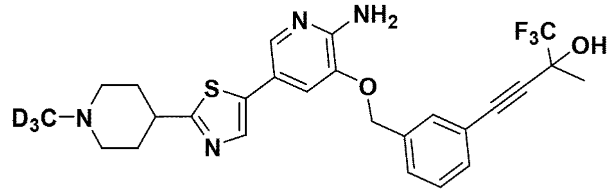

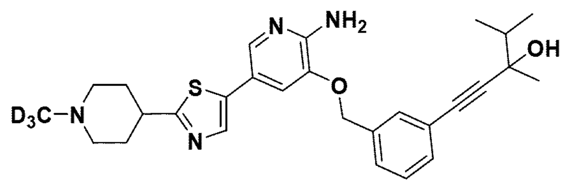

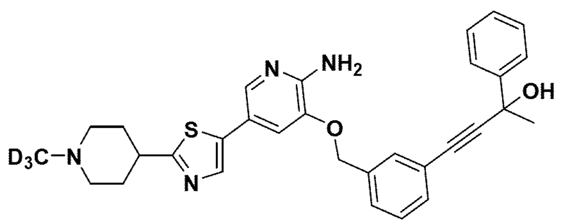

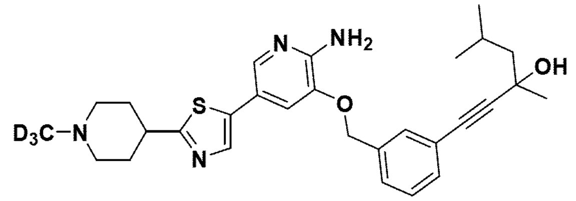

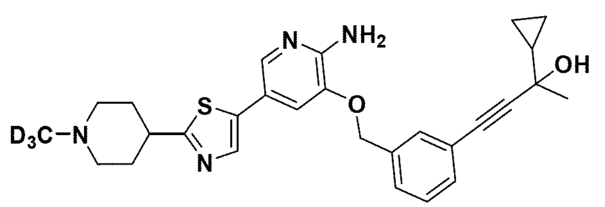

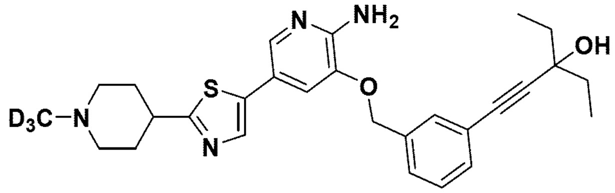

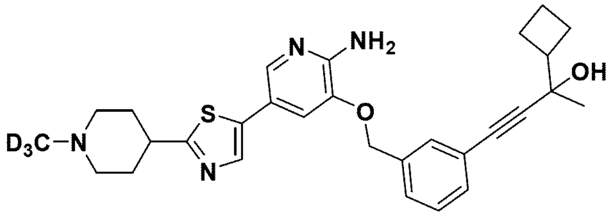

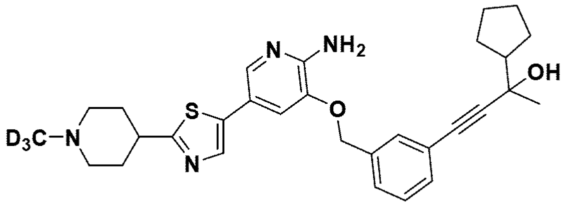

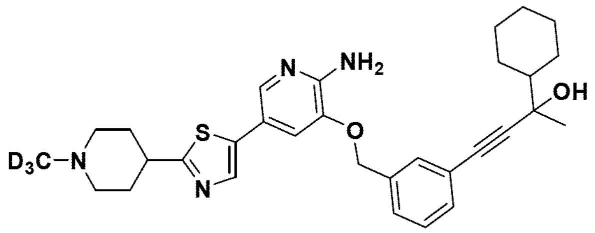

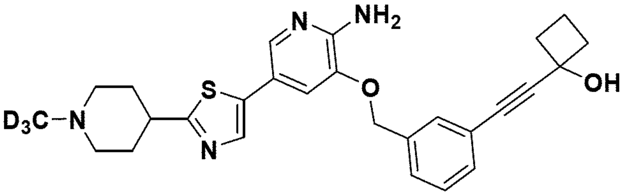

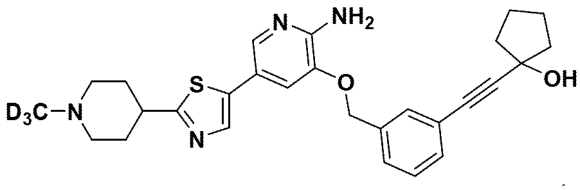

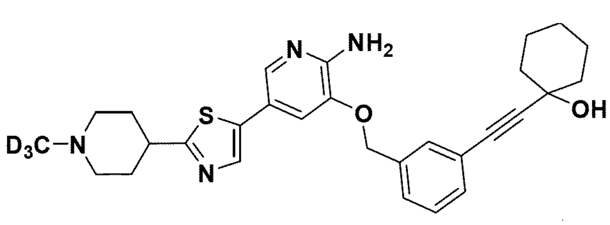

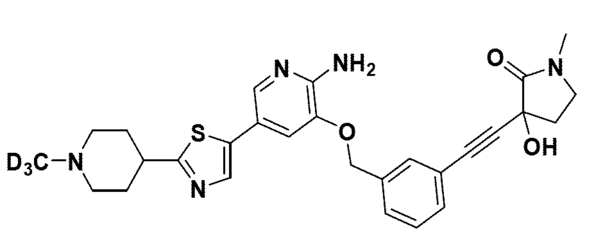

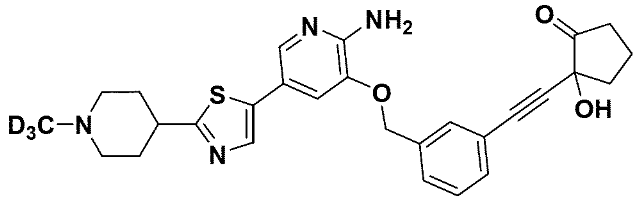

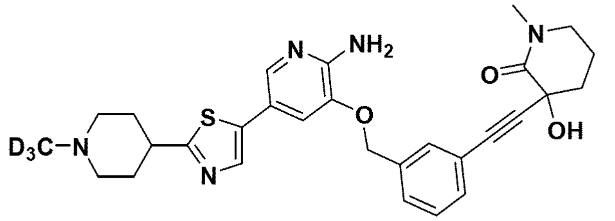

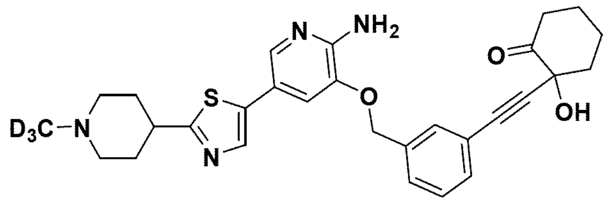

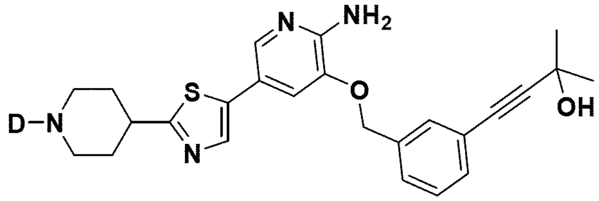

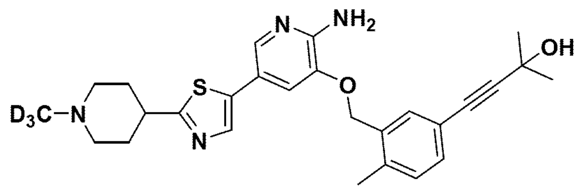

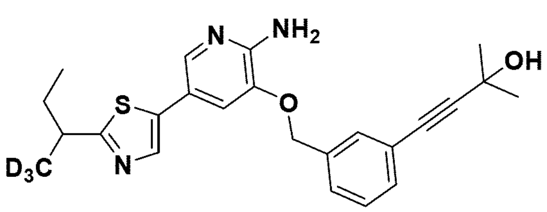

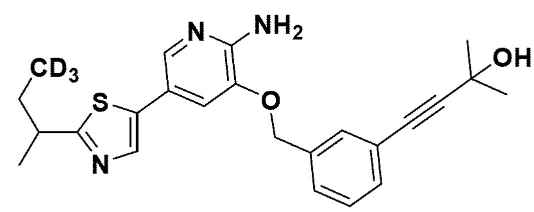

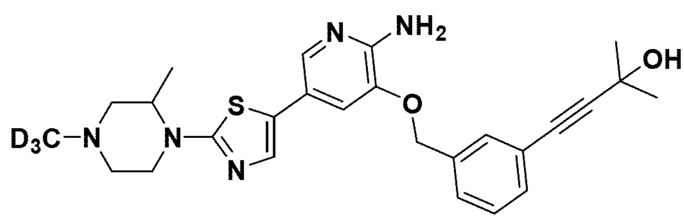

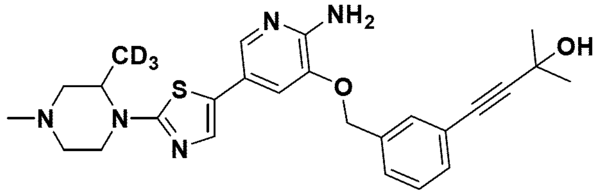

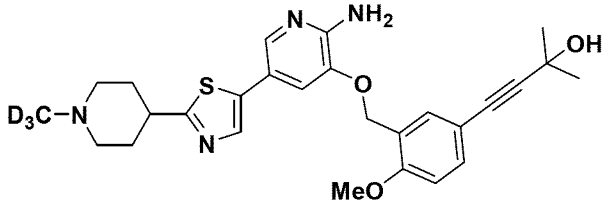

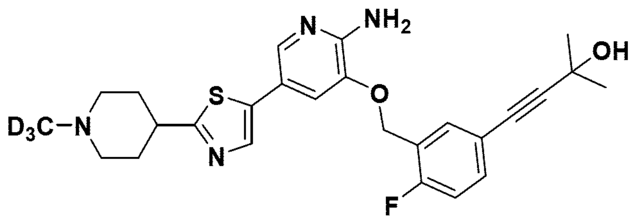

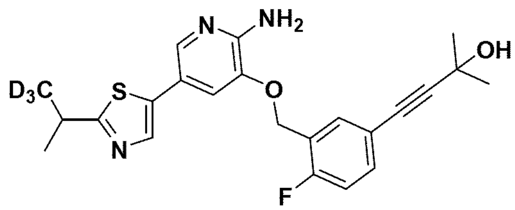

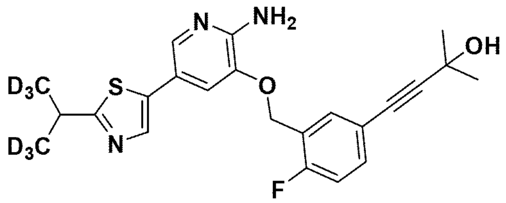

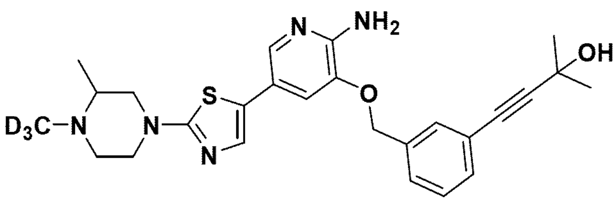

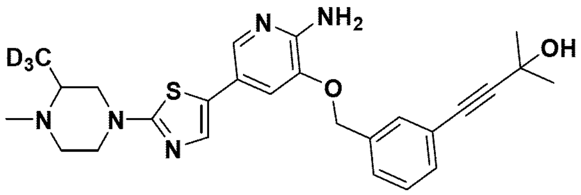

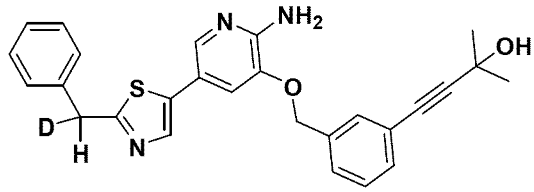

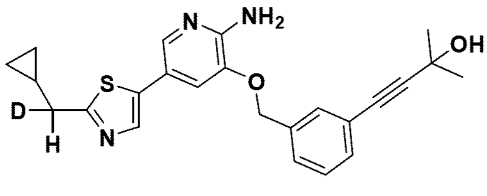

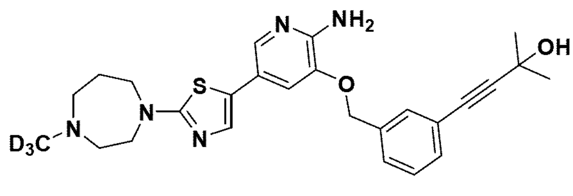

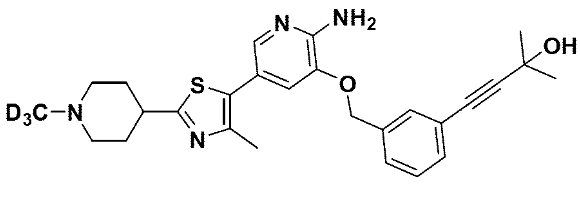

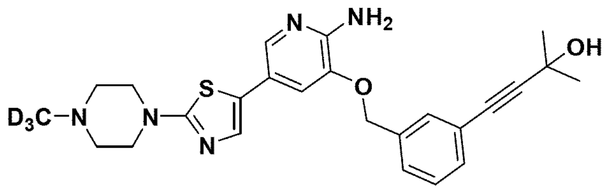

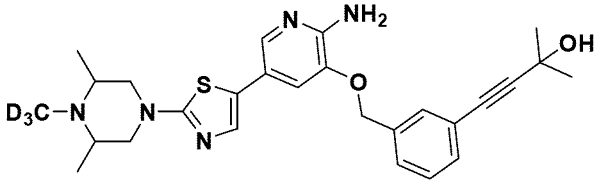

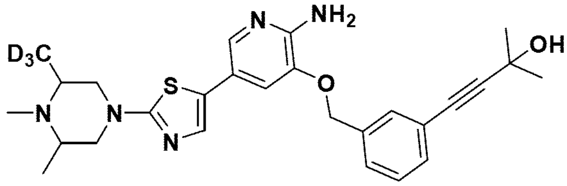

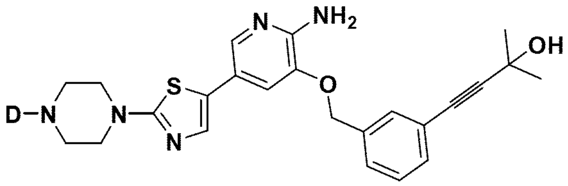

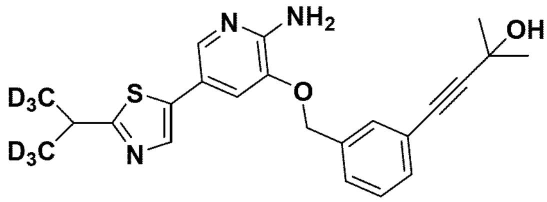

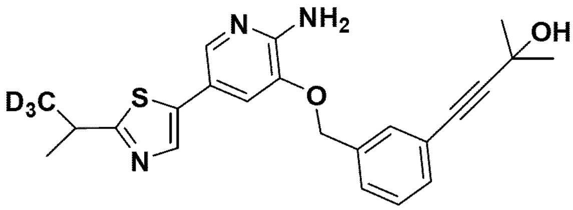

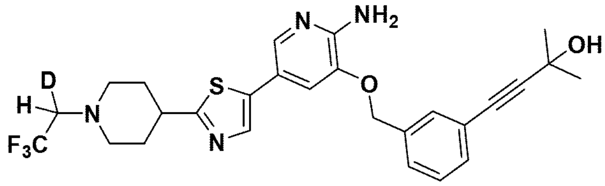

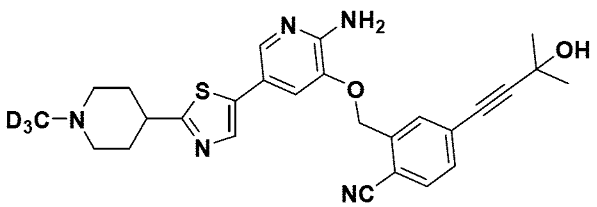

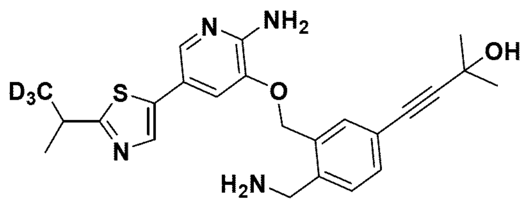

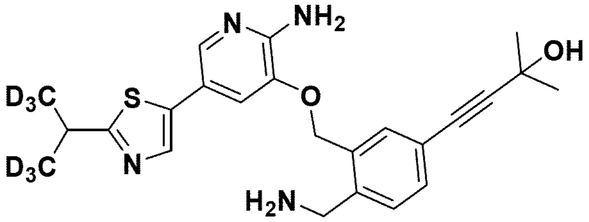

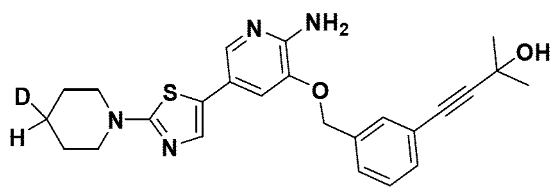

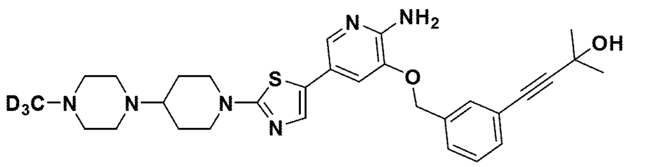

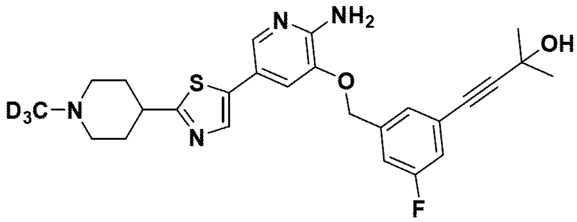

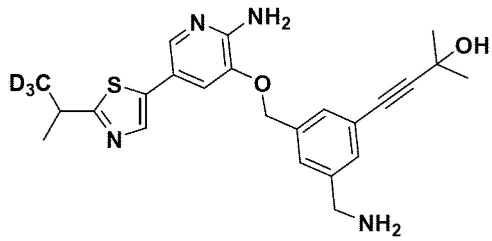

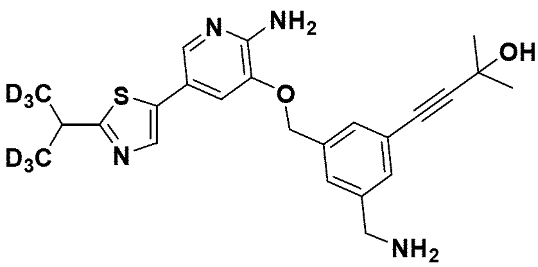

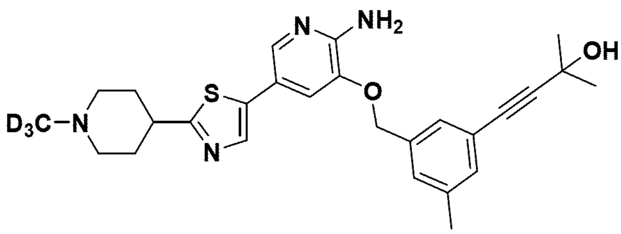

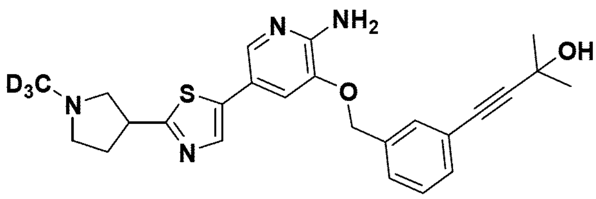

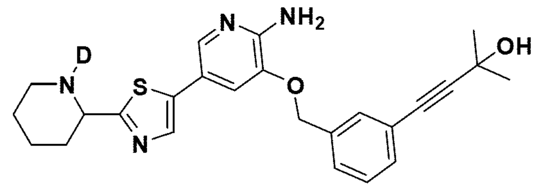

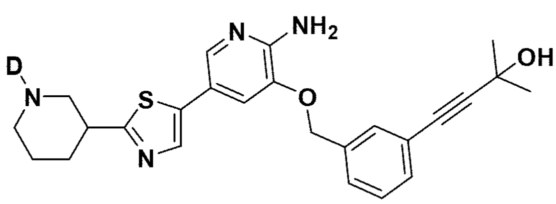

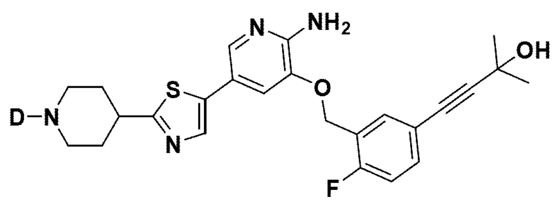

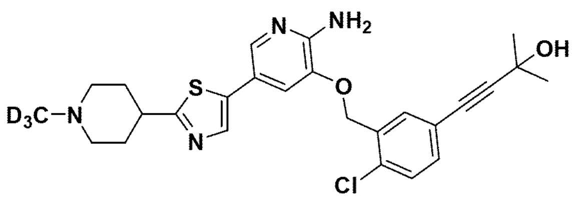

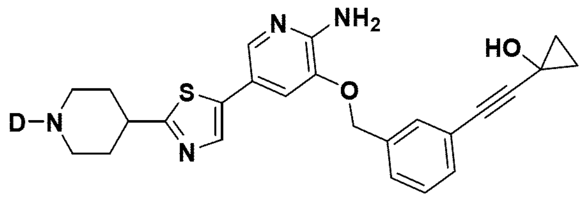

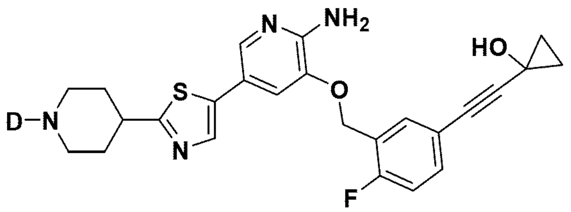

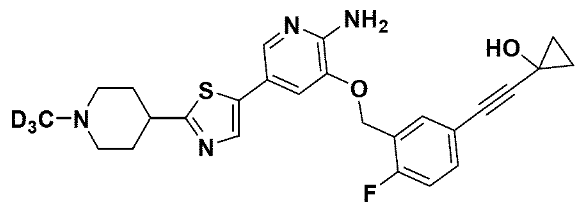

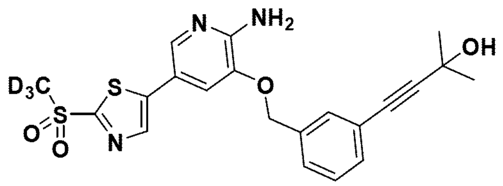

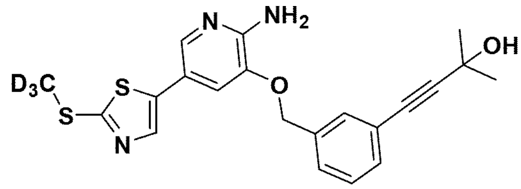

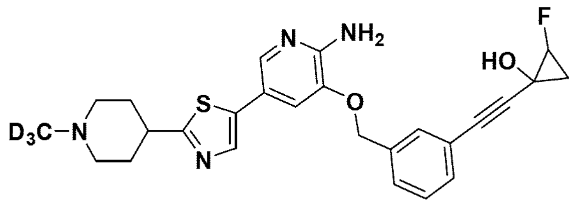

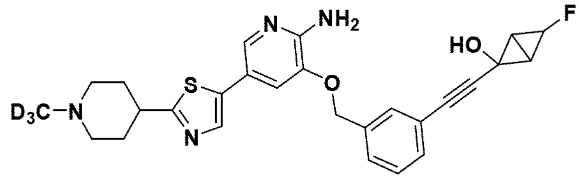

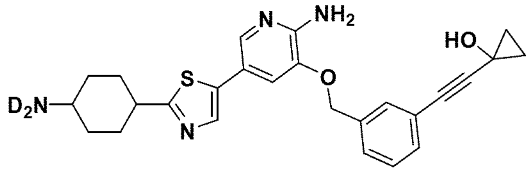

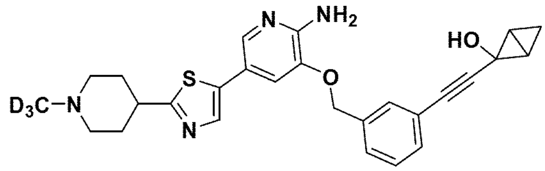

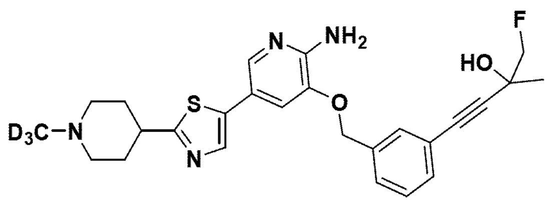

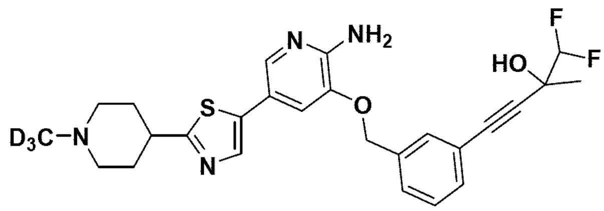

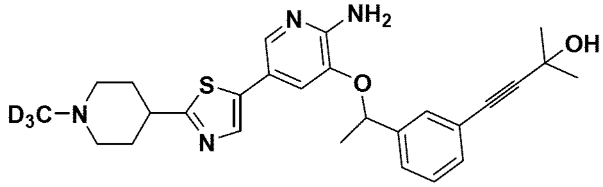

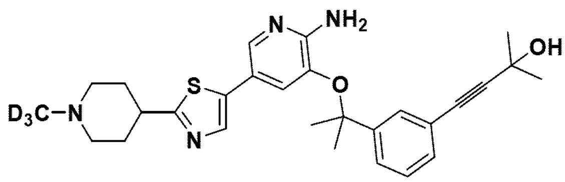

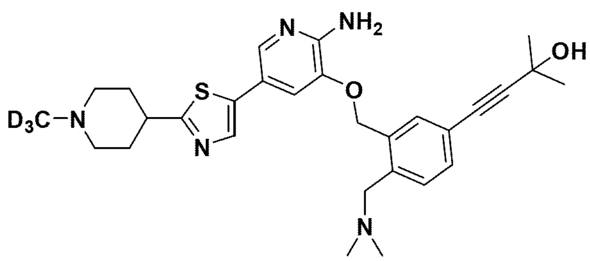

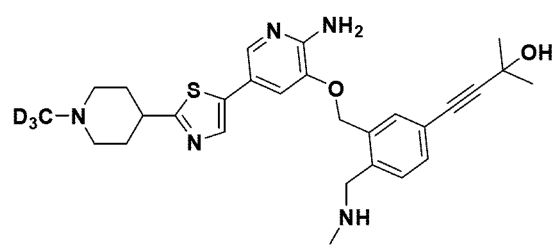

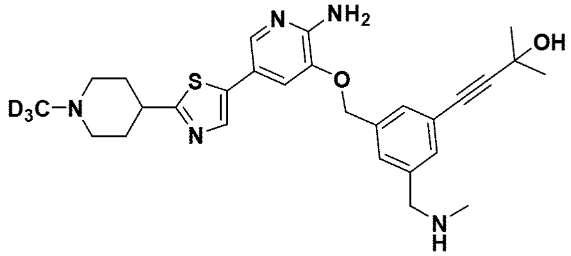

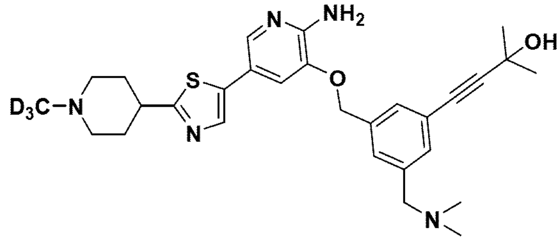

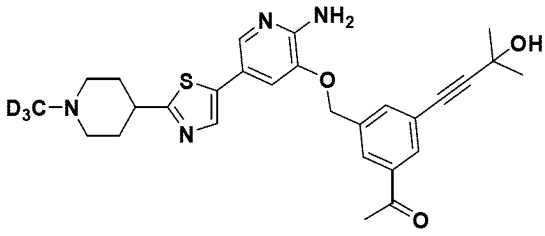
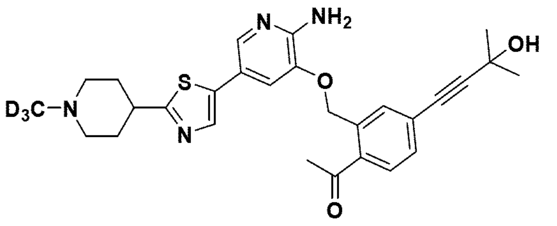

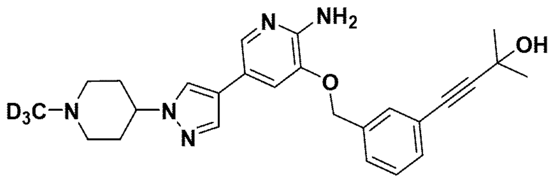

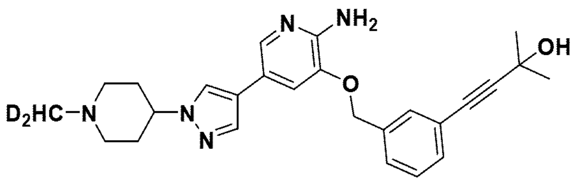

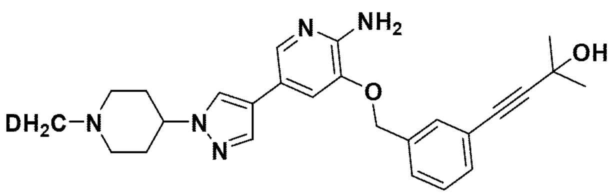

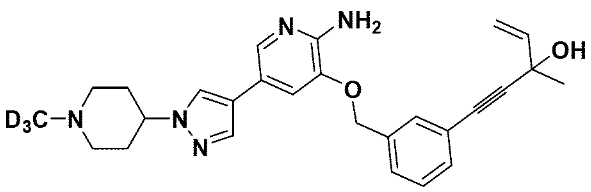
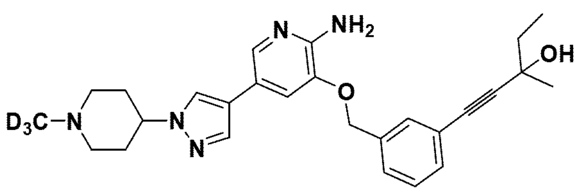

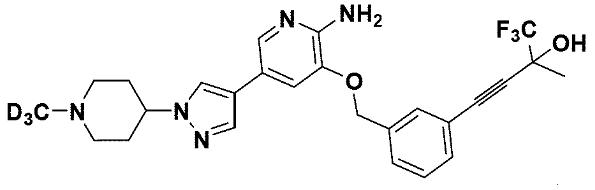

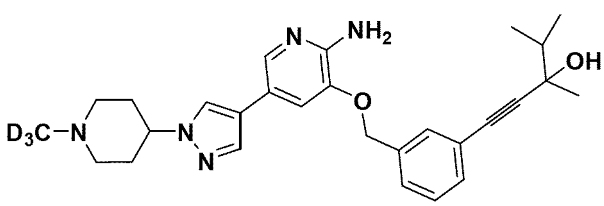

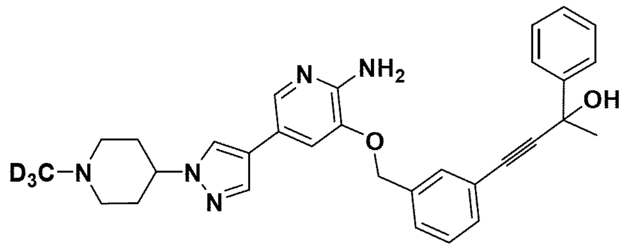

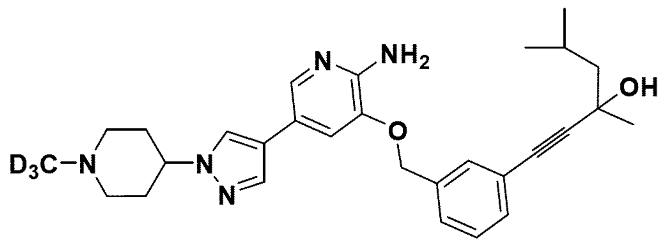

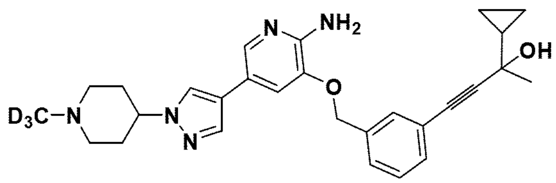

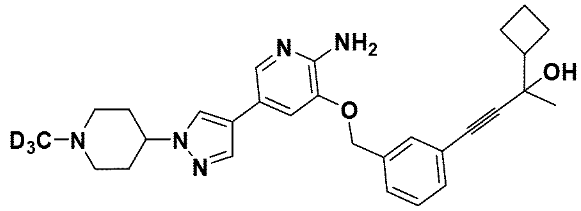

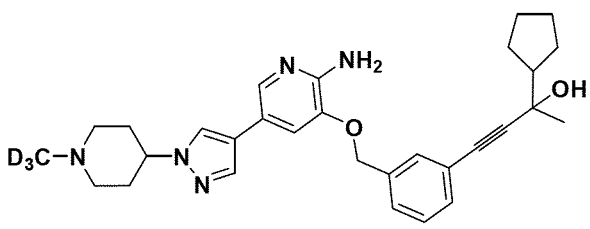

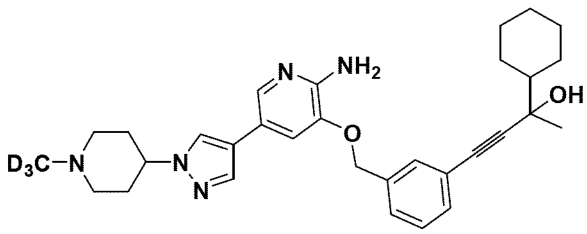

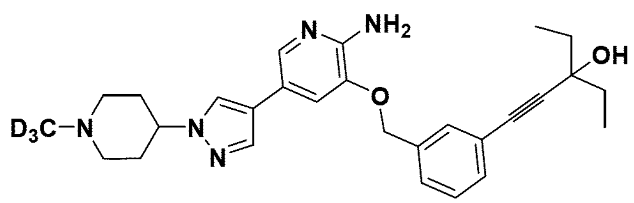

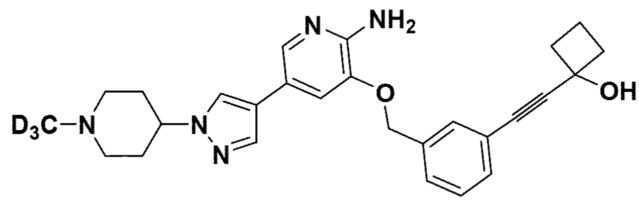

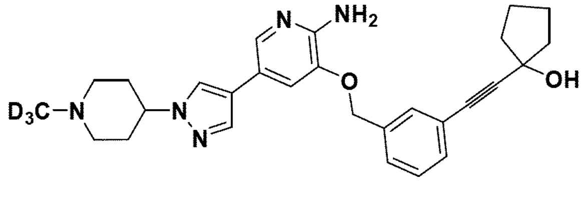

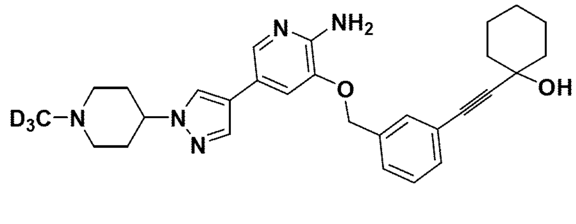

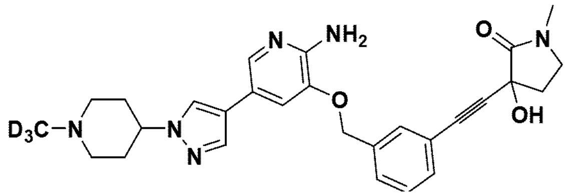

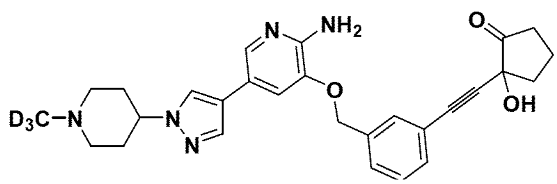

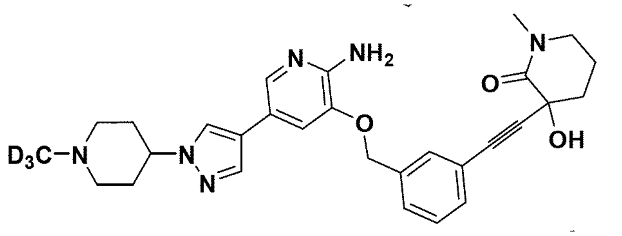

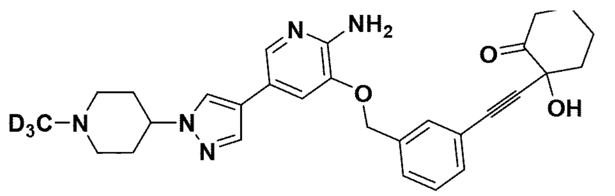

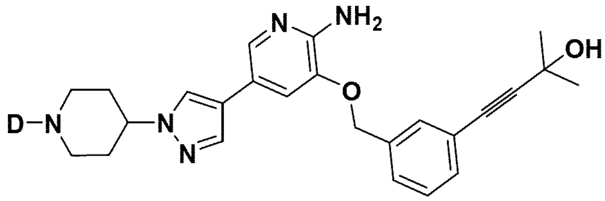

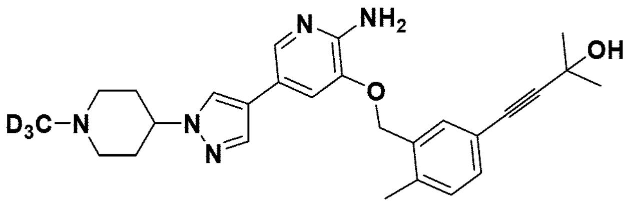

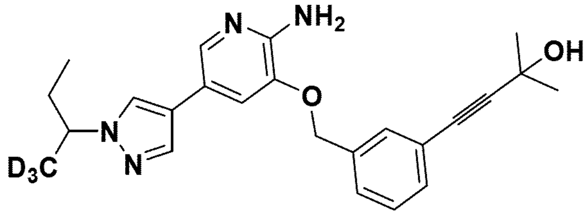

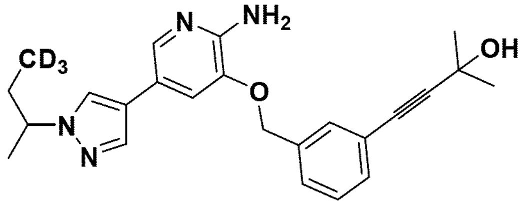

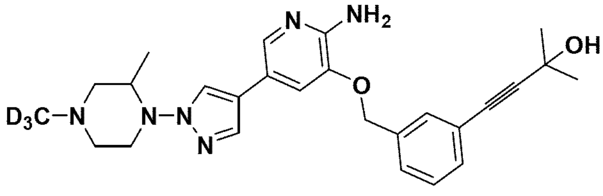

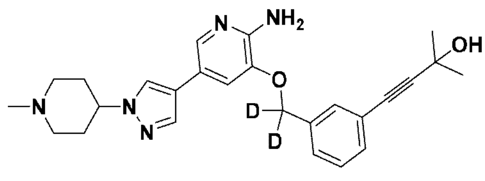

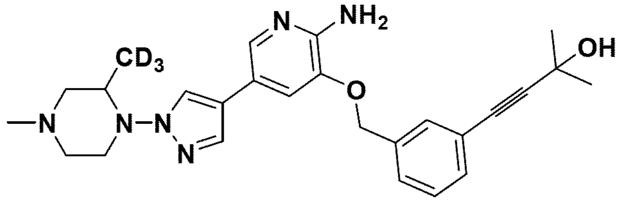

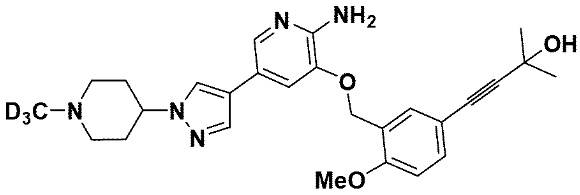

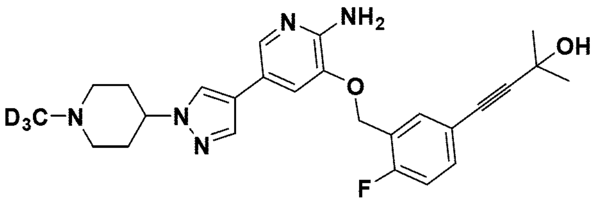

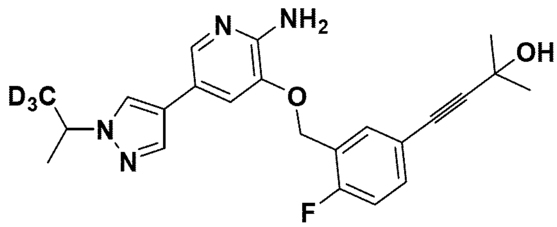

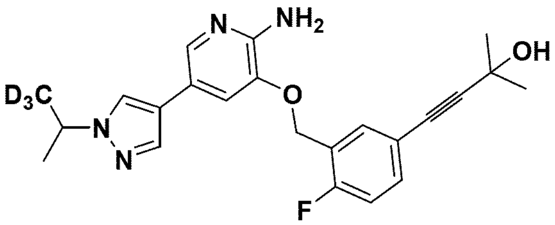

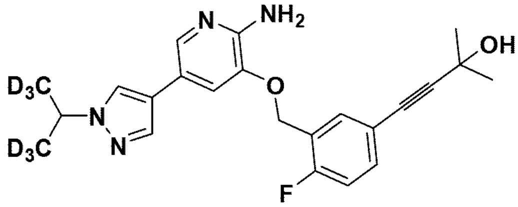

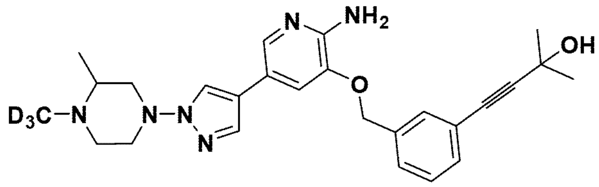

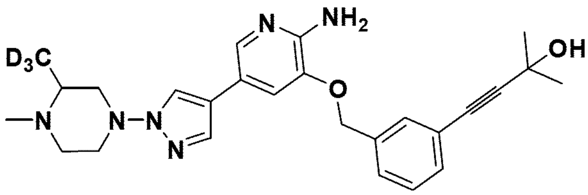

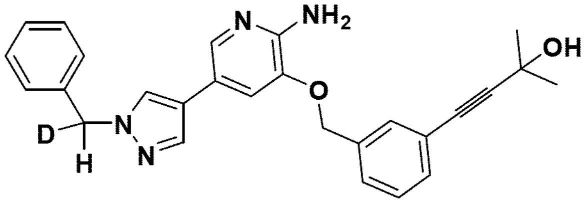

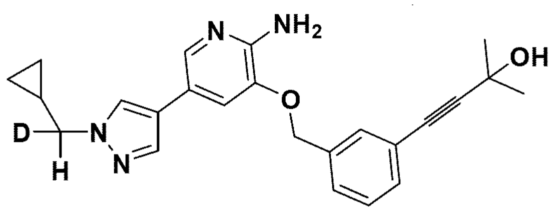

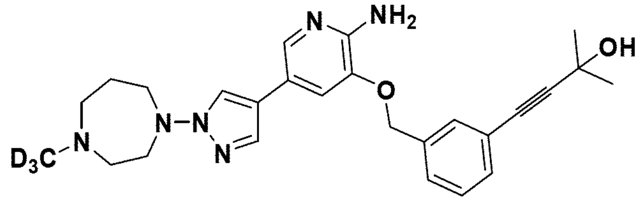

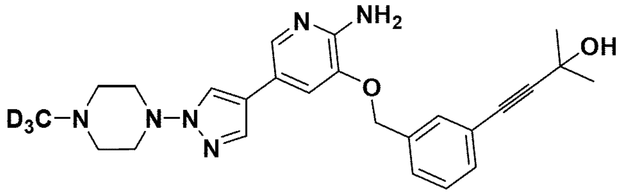

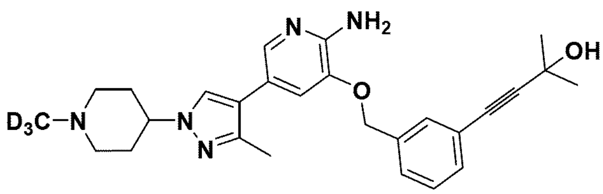

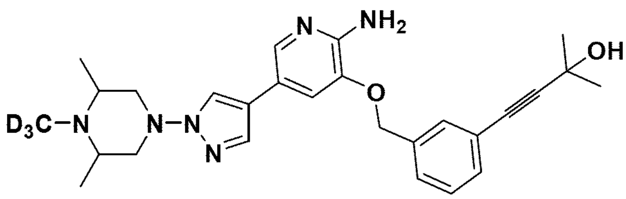

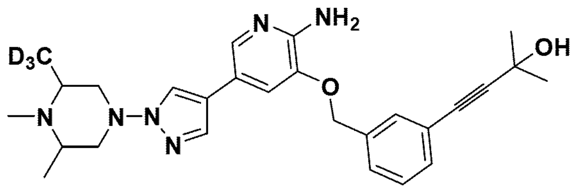

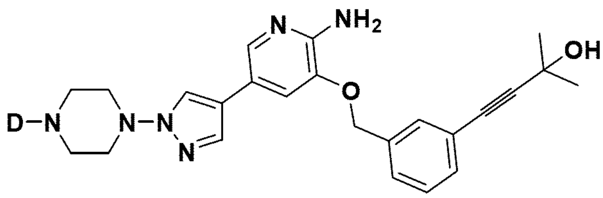

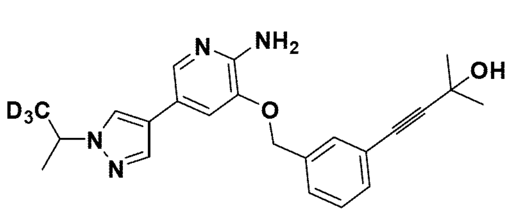

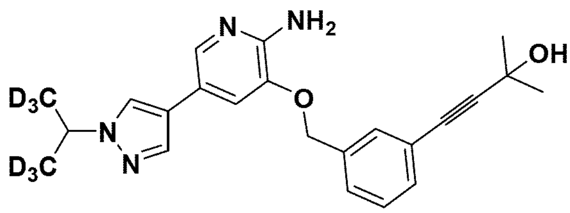

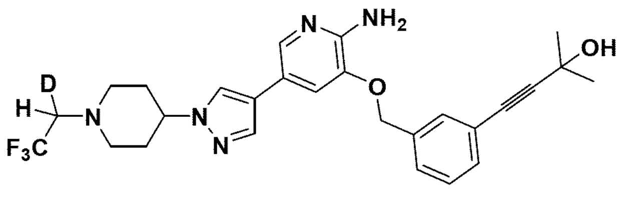

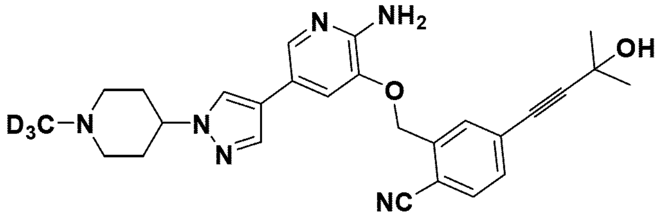

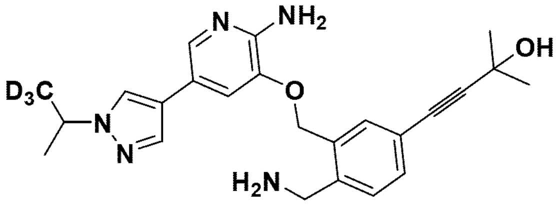

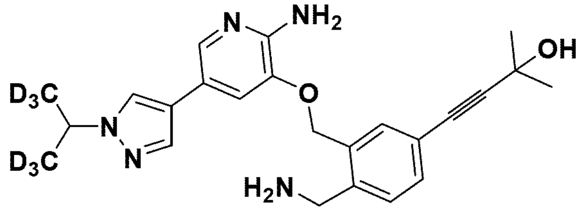

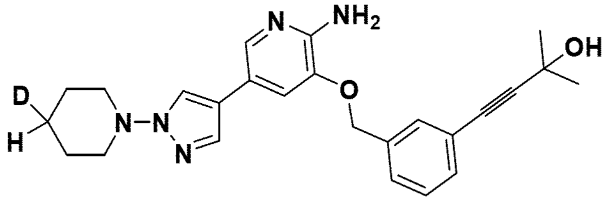

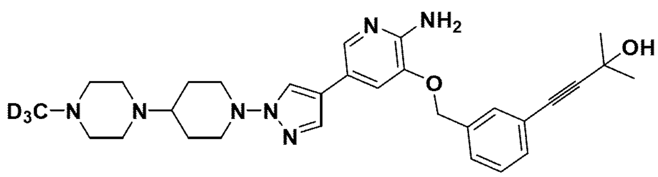

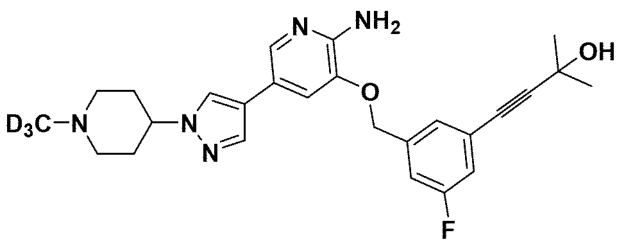

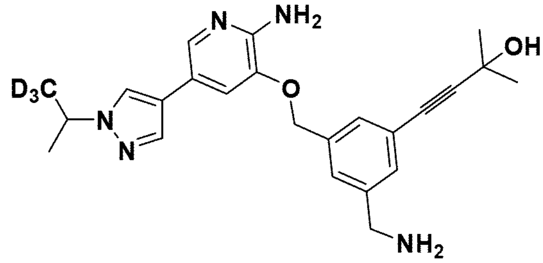

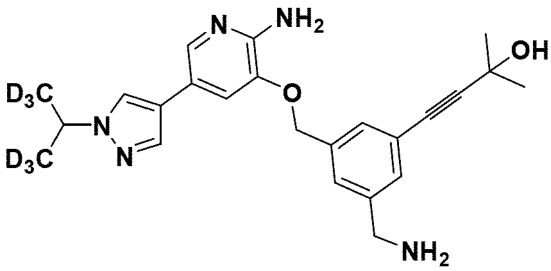

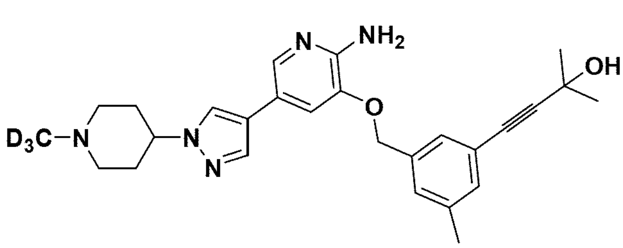

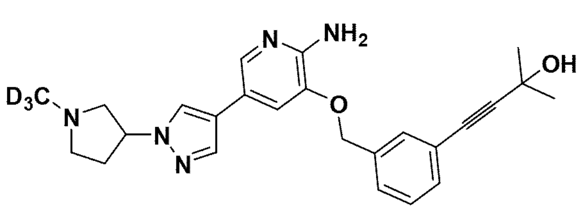

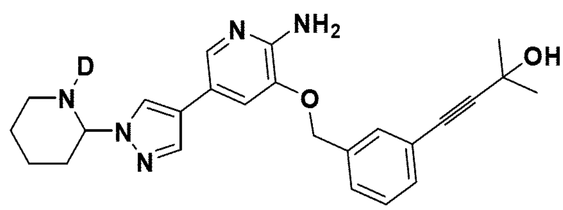

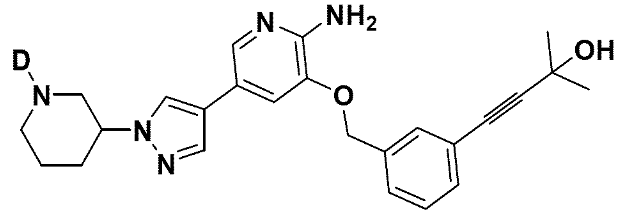

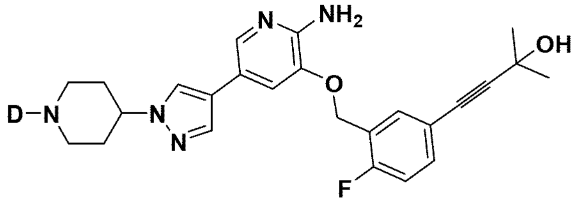

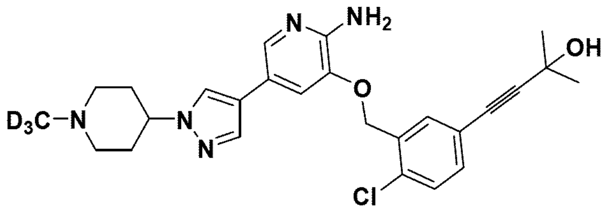

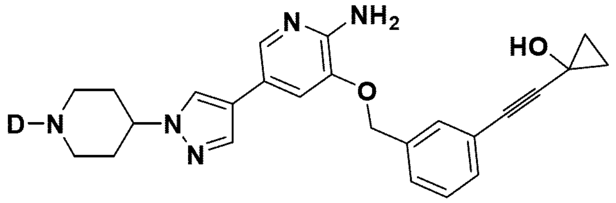

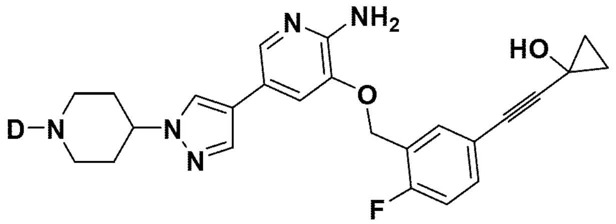

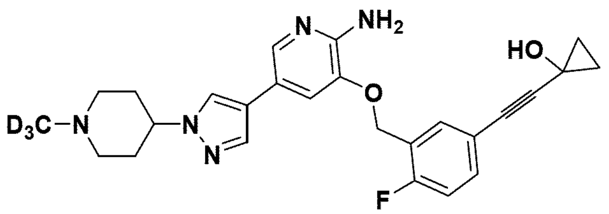

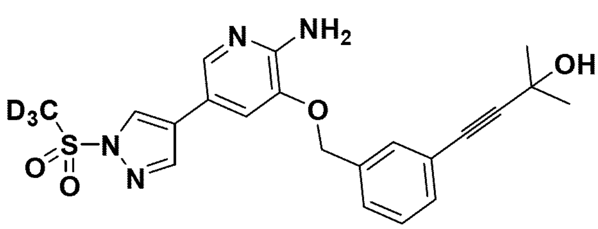

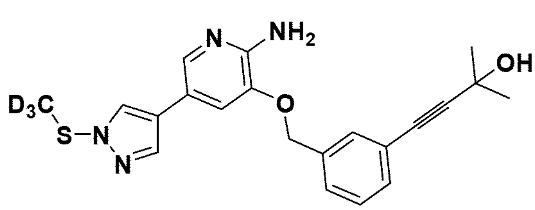

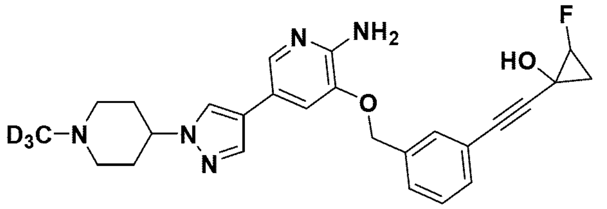

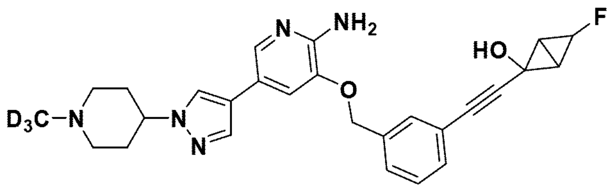

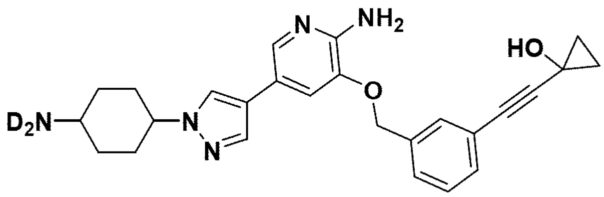

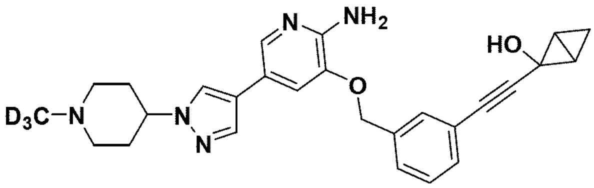

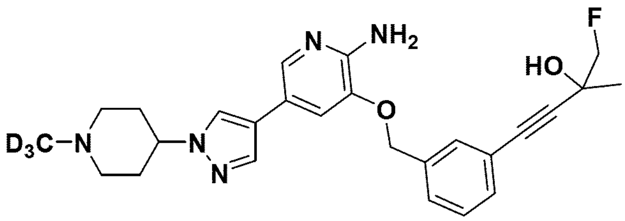

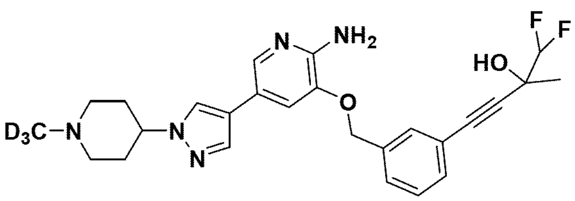

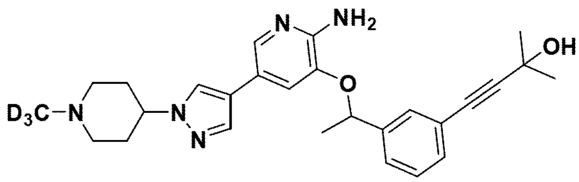

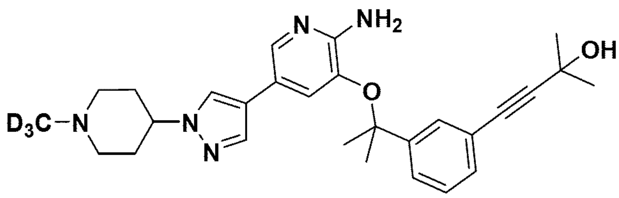

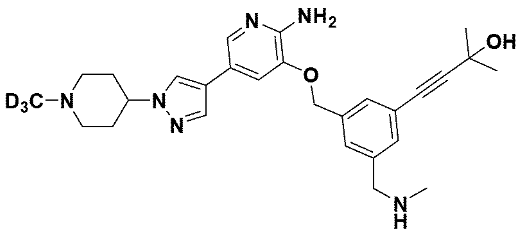

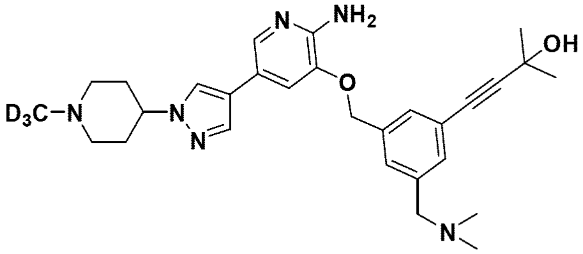

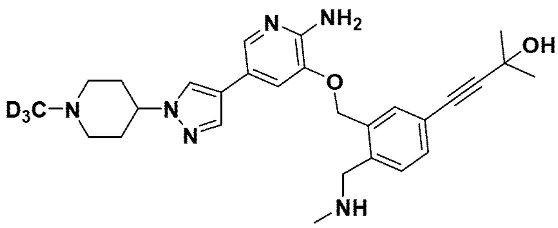

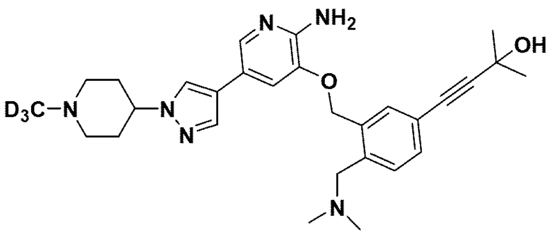

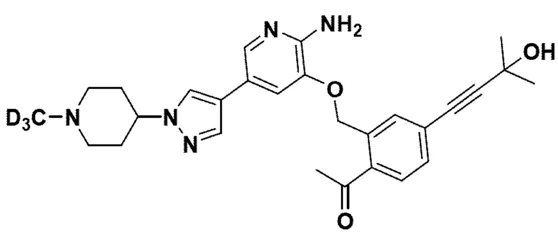

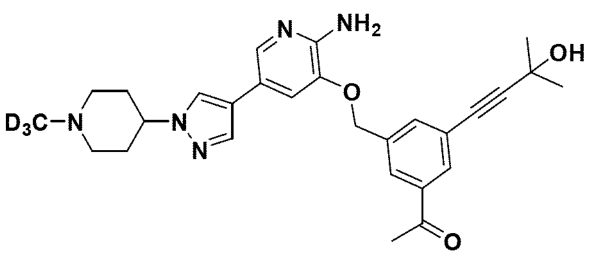

(3)

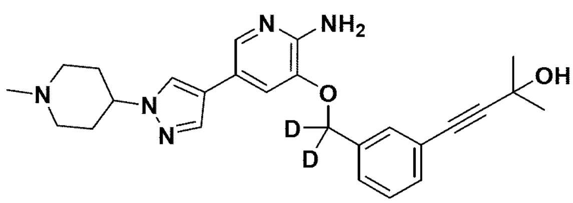

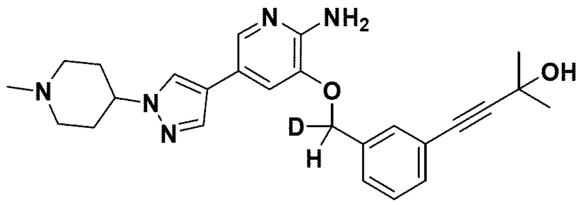

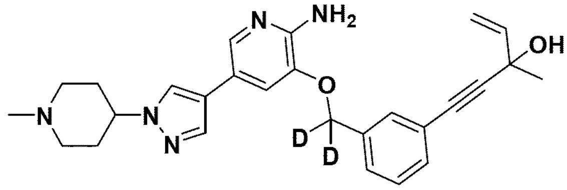

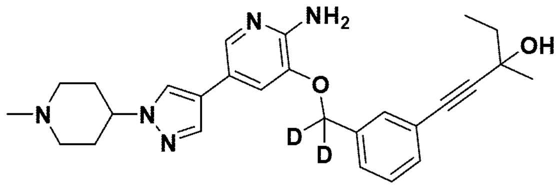

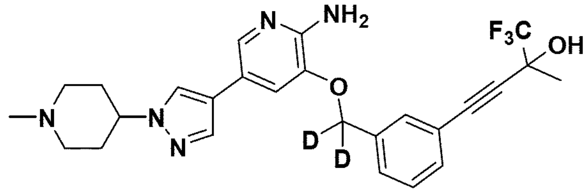

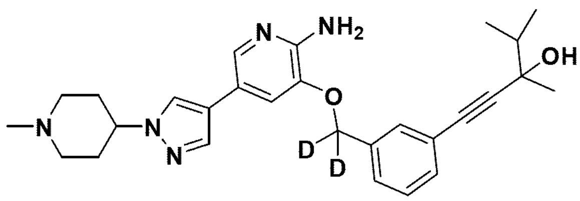

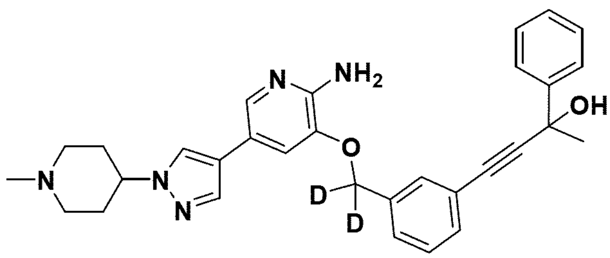

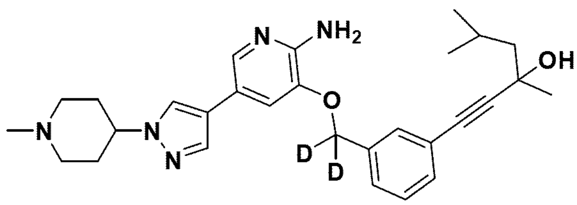

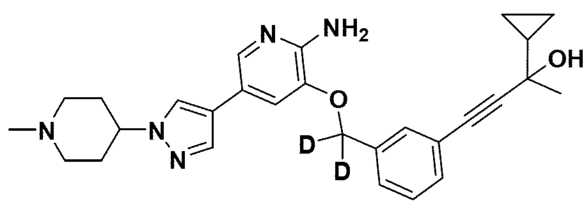

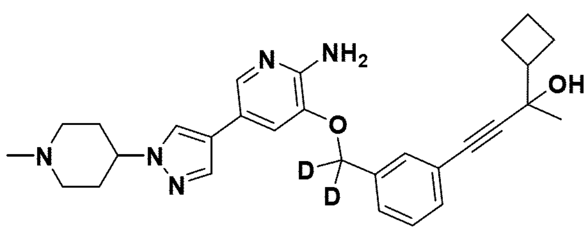

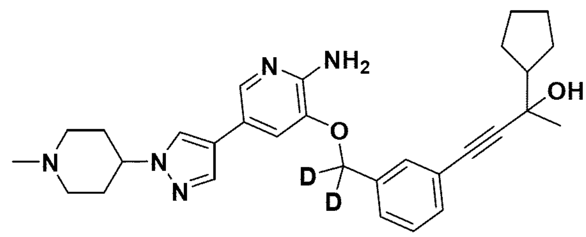

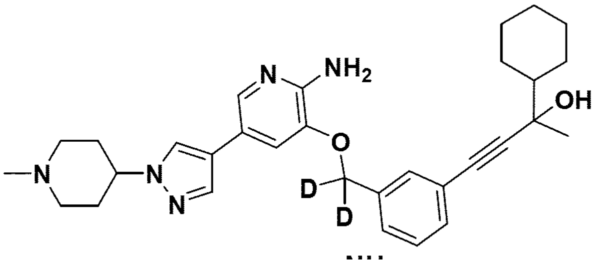

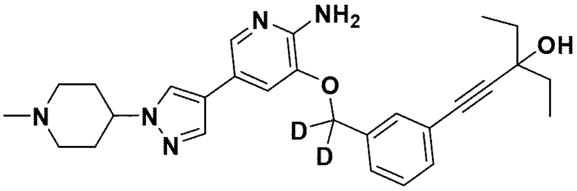

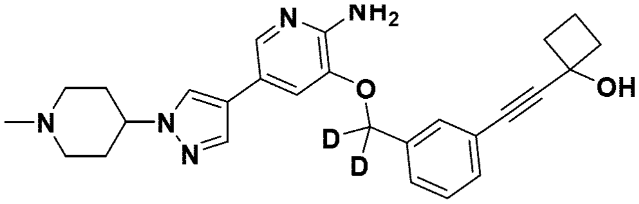

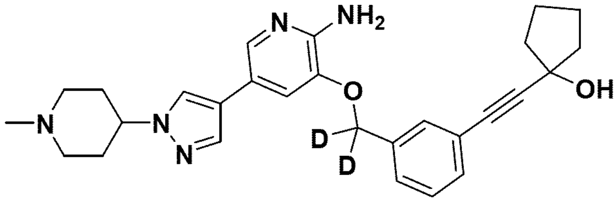

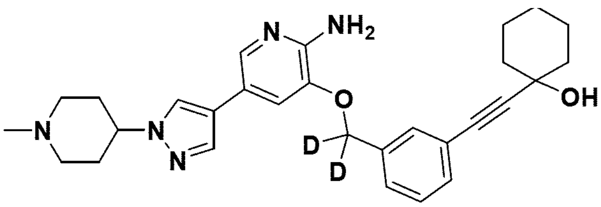

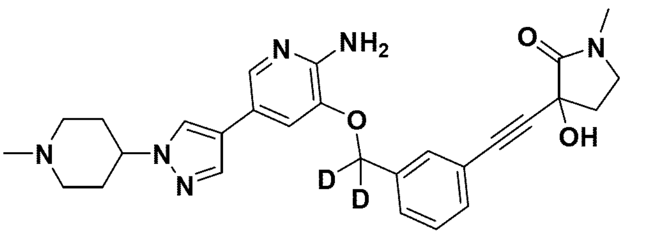

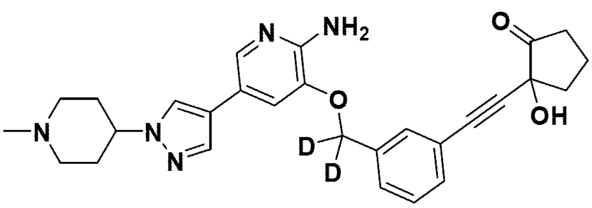

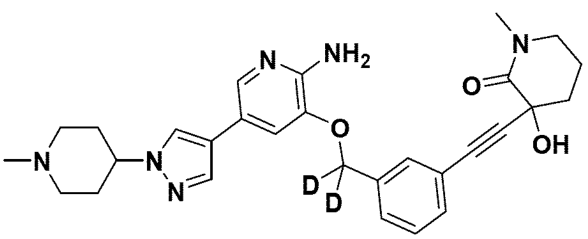

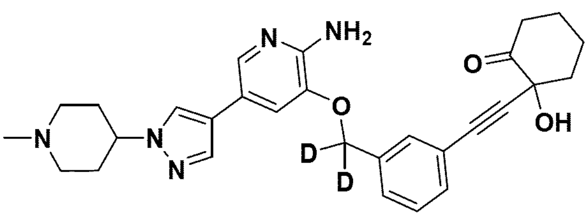

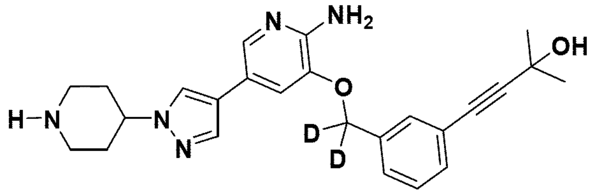

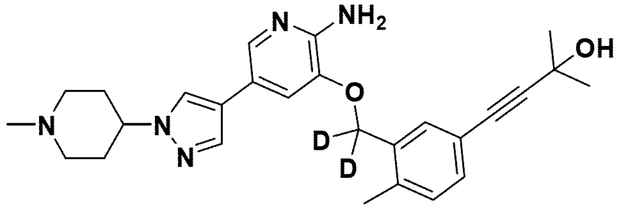

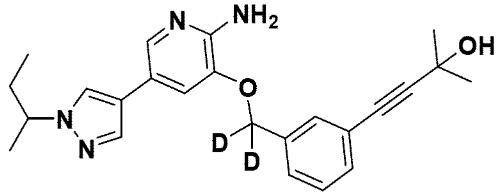

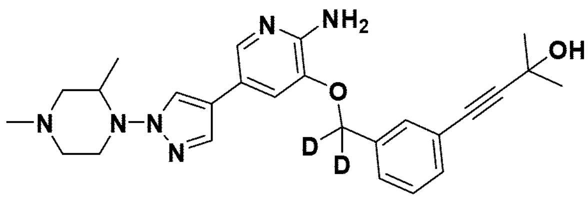

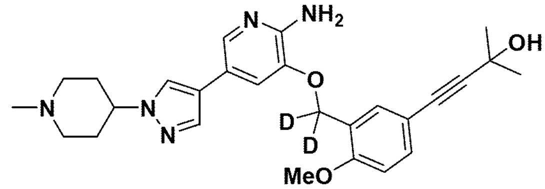

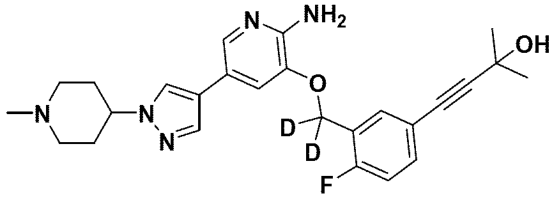

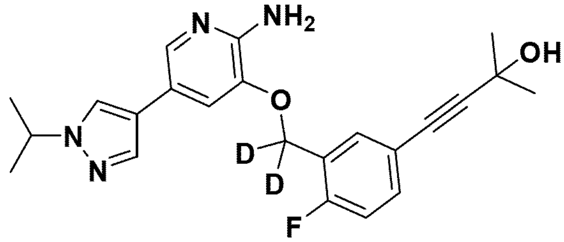

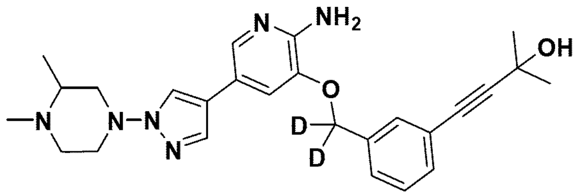

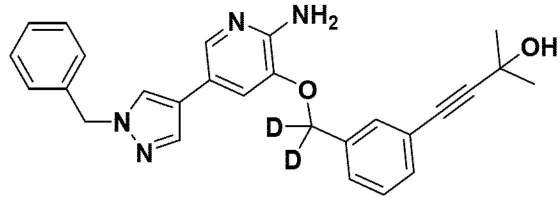

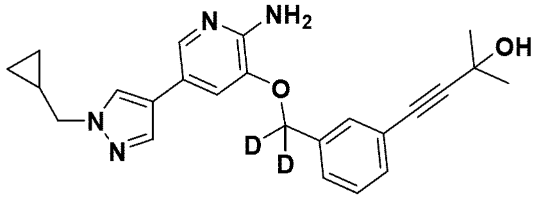

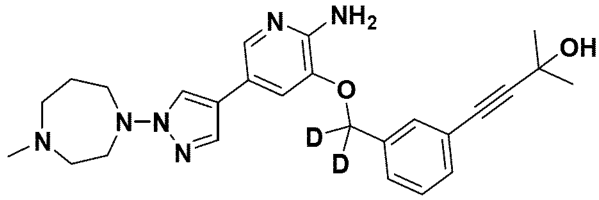

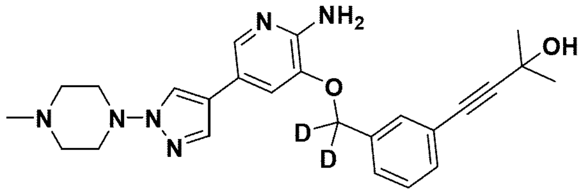

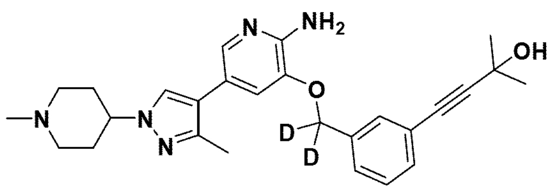

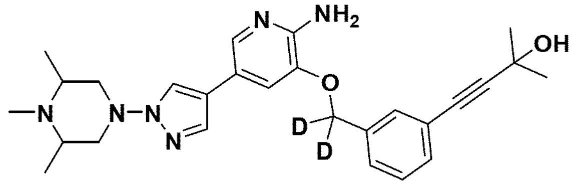

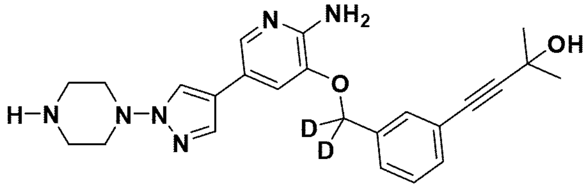

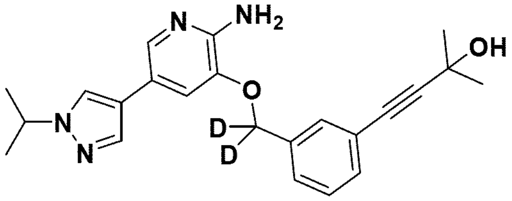

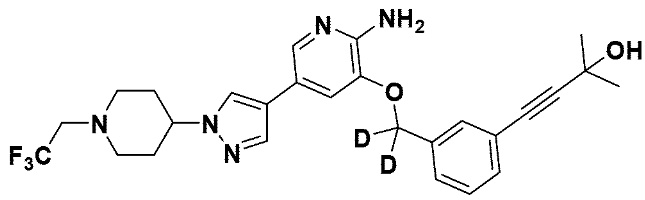

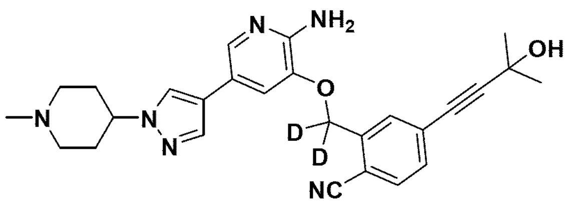

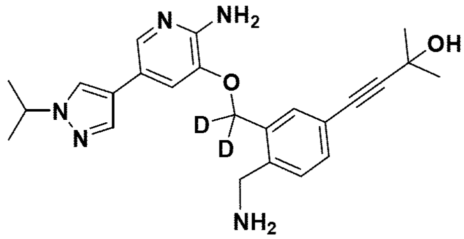

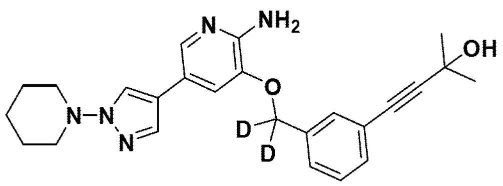

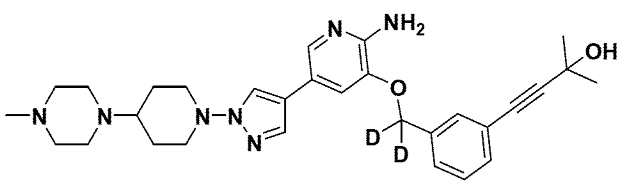

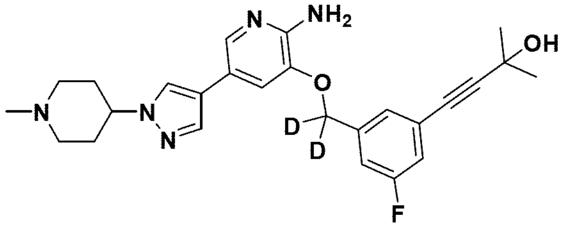

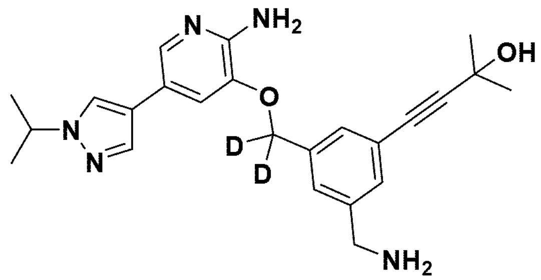

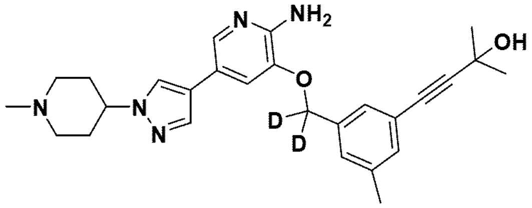

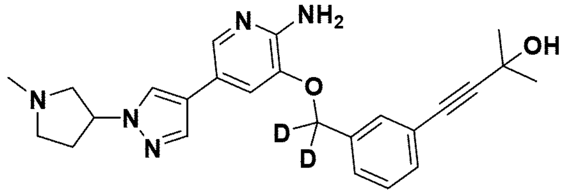

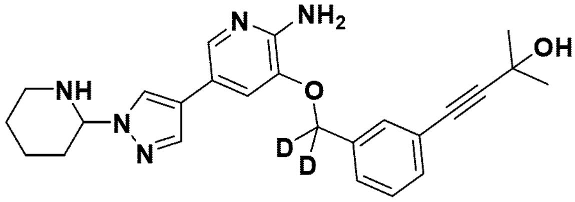

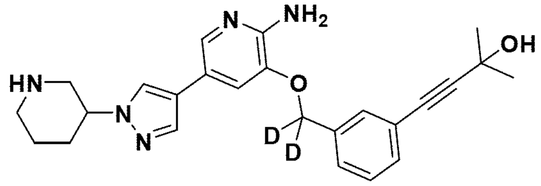

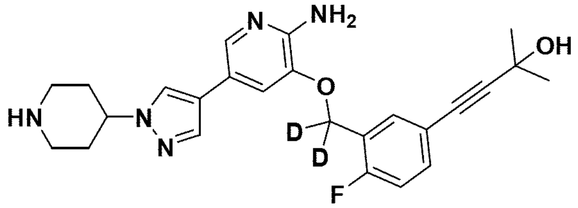
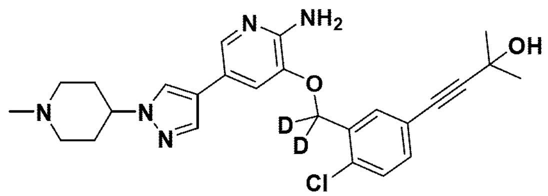

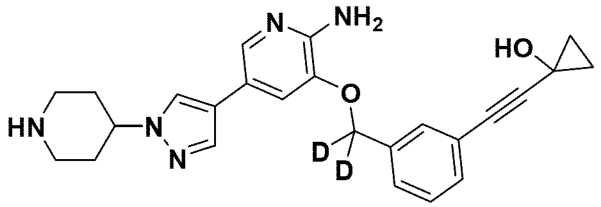
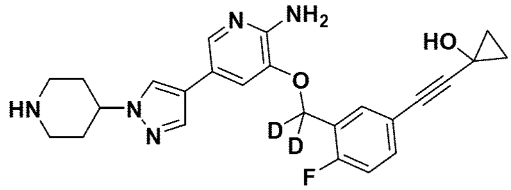

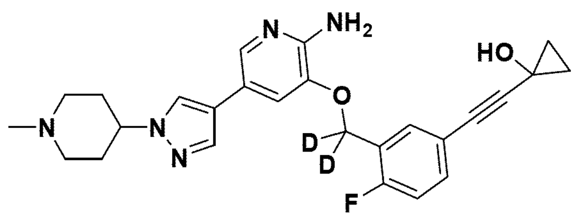
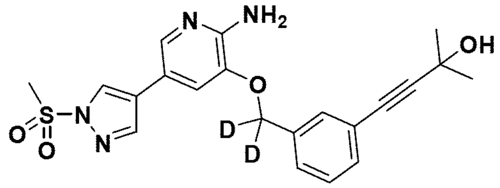

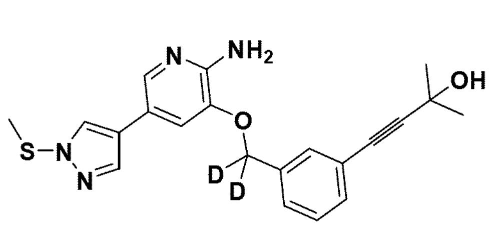
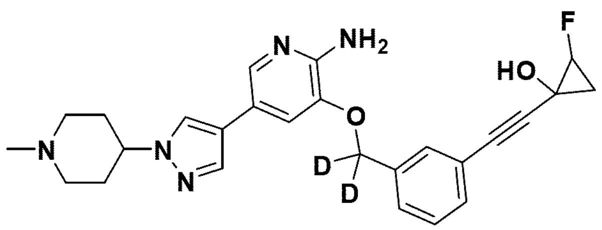

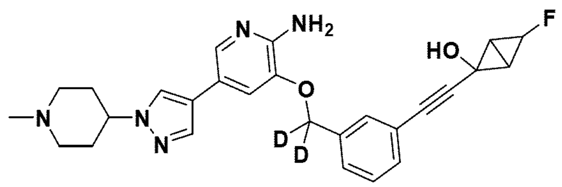
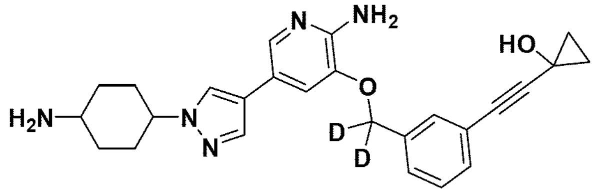

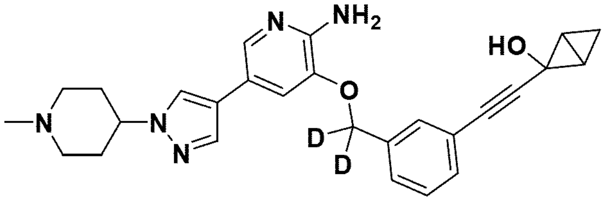
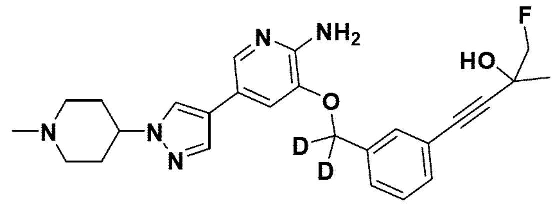

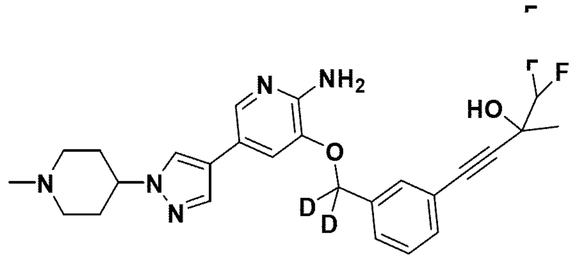
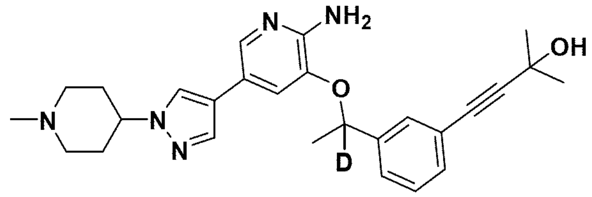

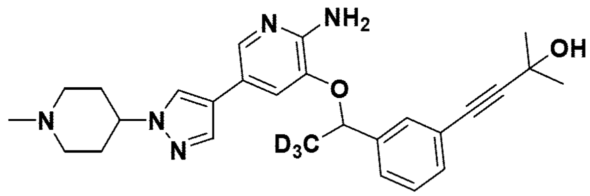

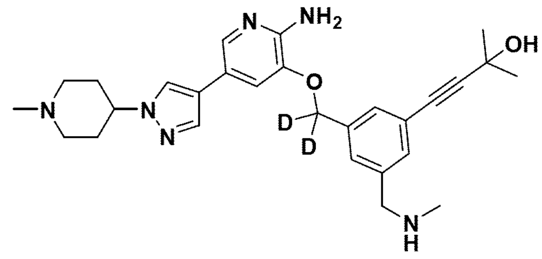

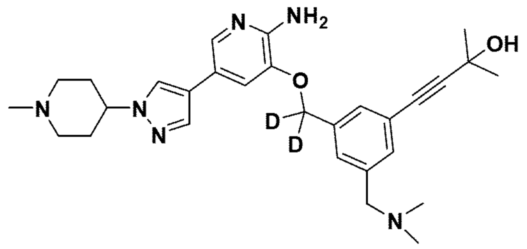

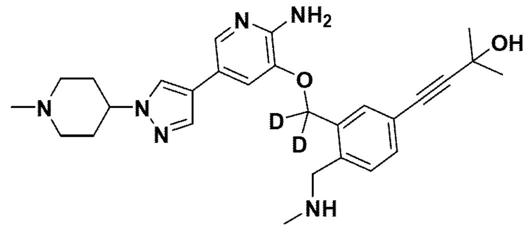

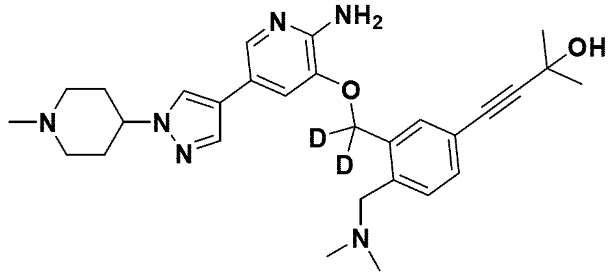

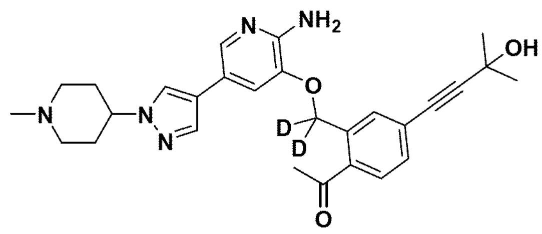

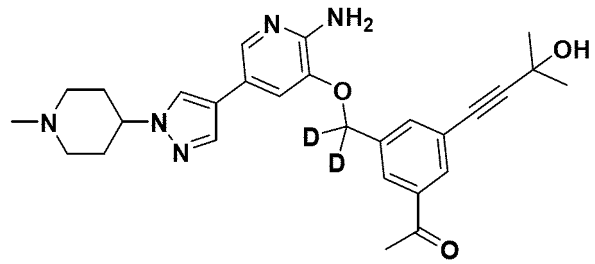

(4)

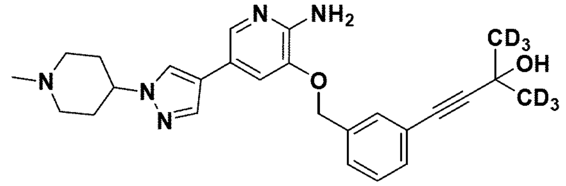

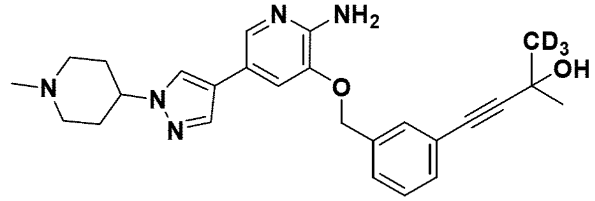

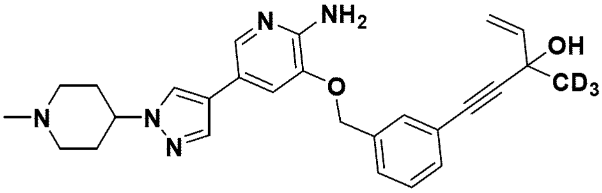

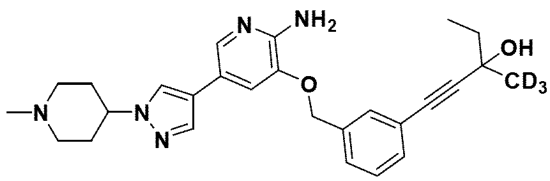

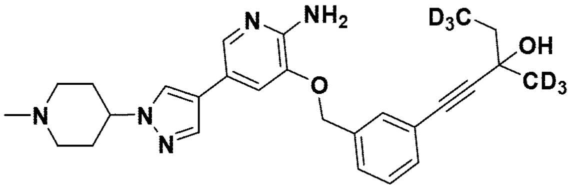

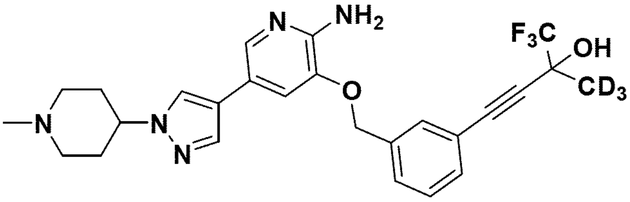

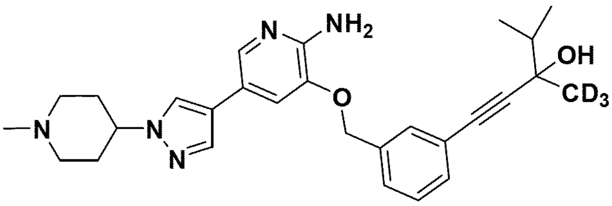

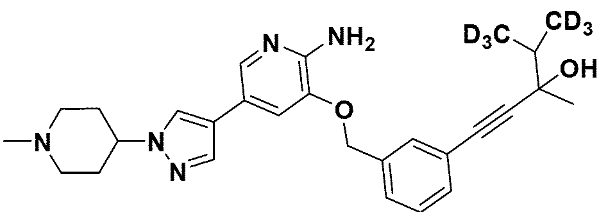

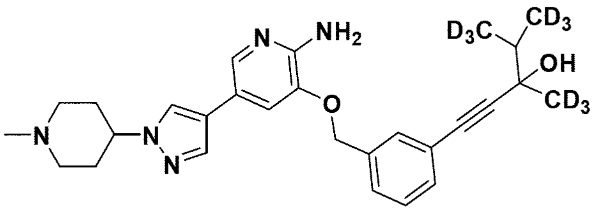

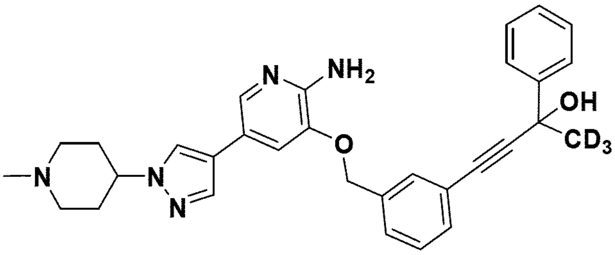

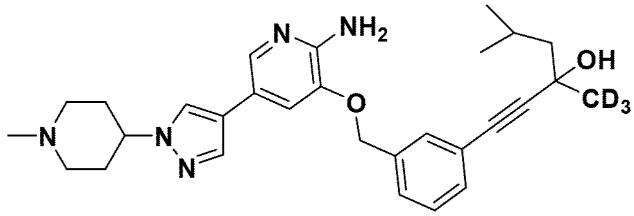

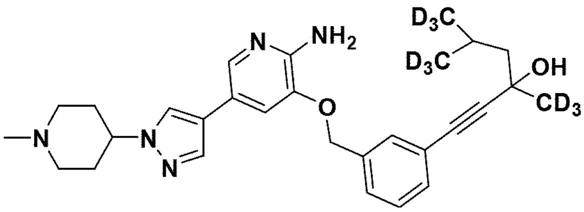

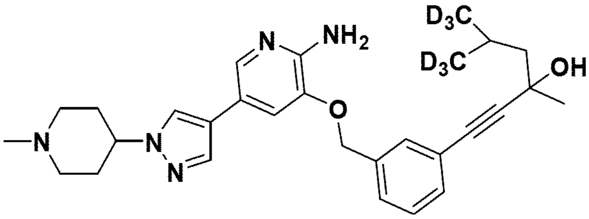

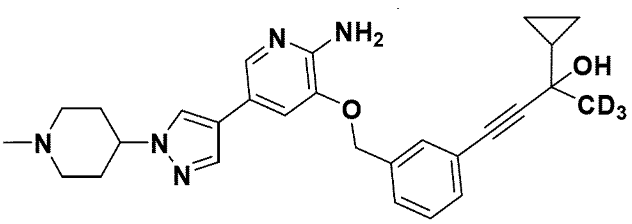

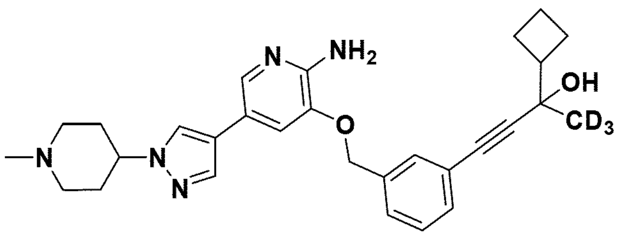

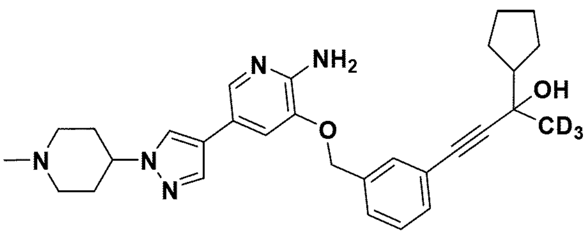

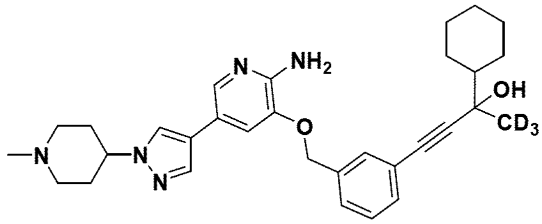

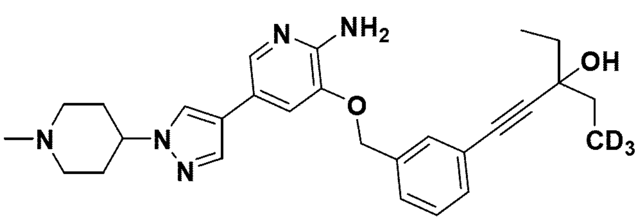

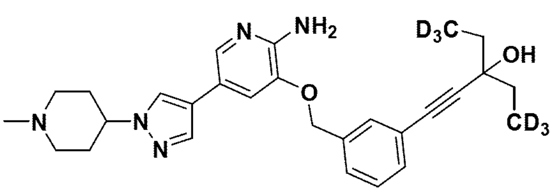

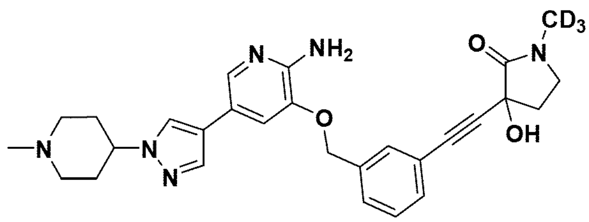

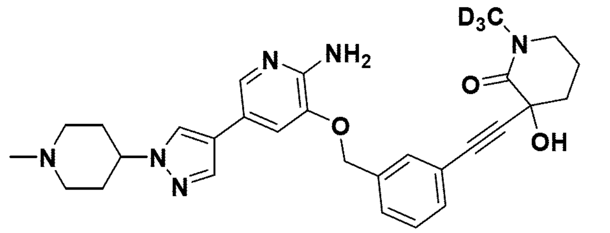

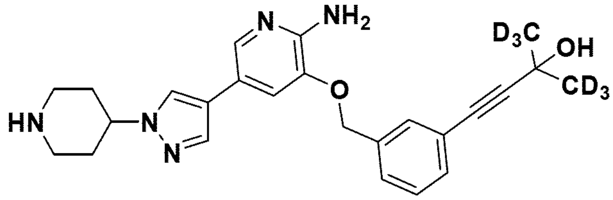

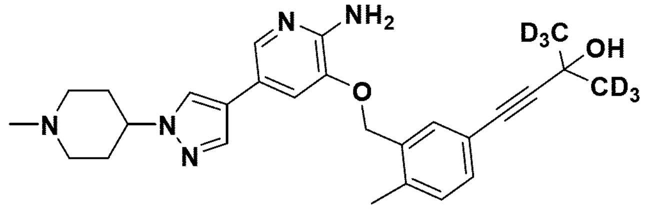

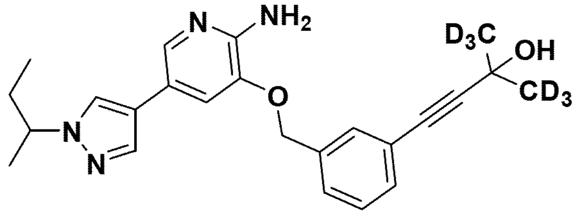

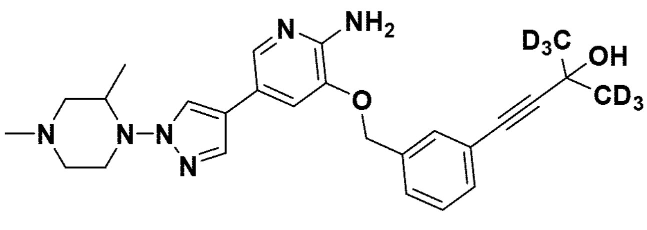

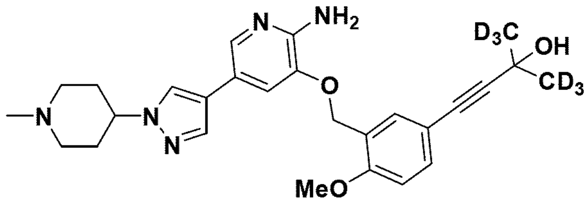

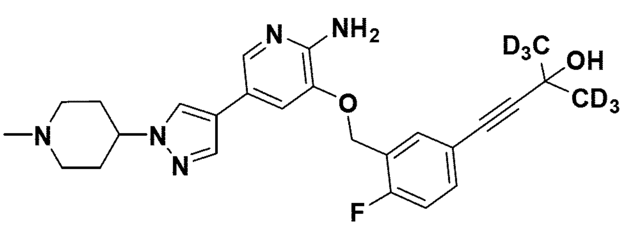

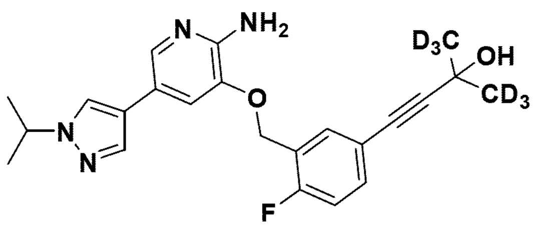

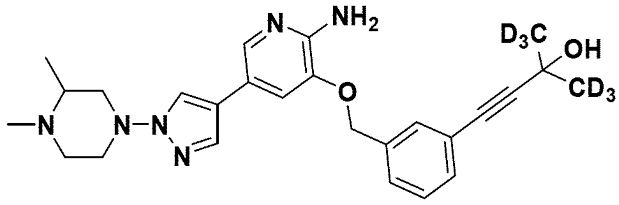

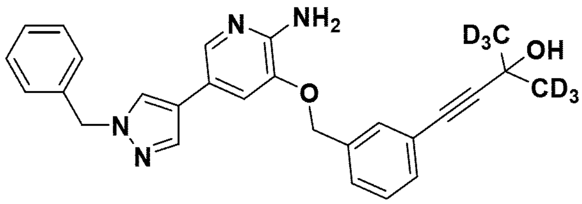

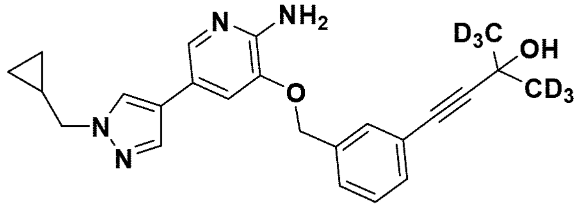

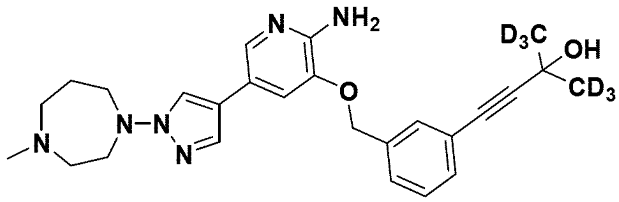

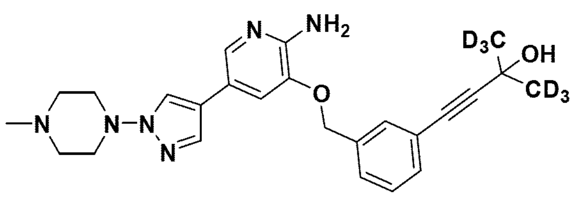

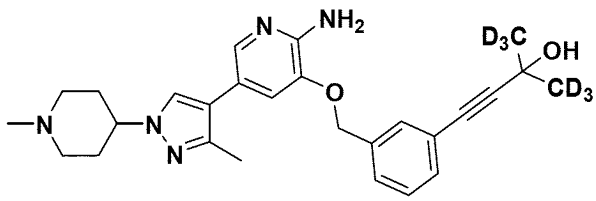

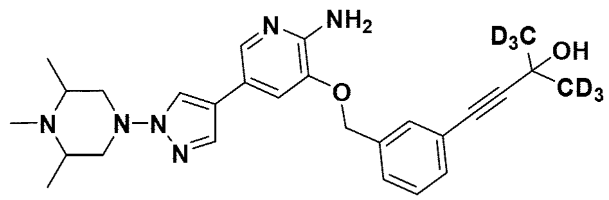

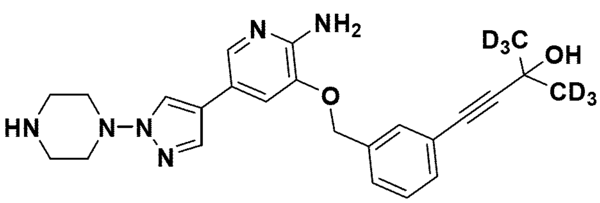

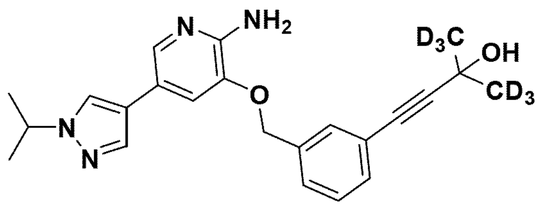

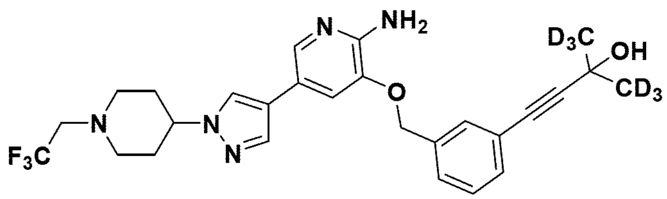

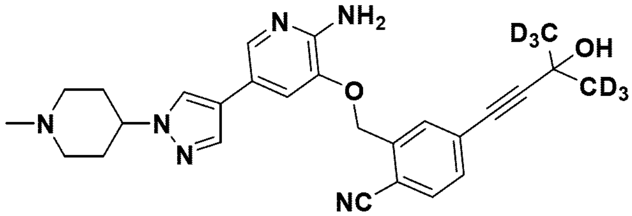

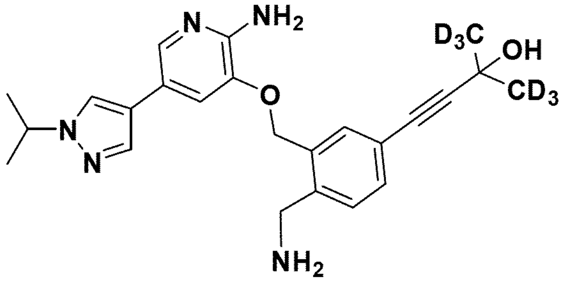

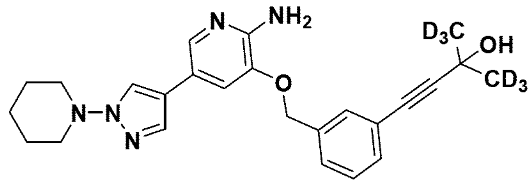

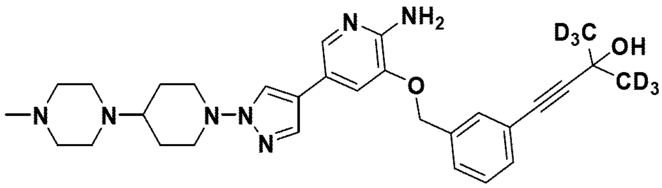

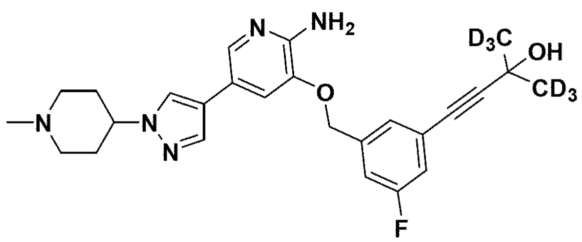

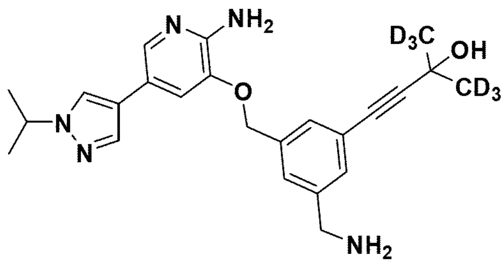

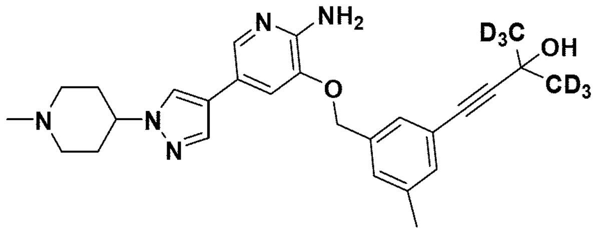

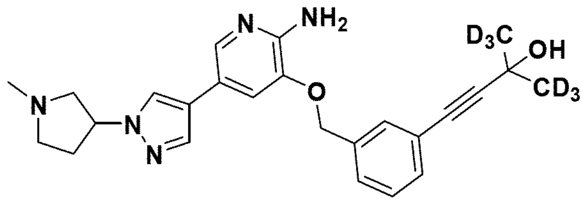

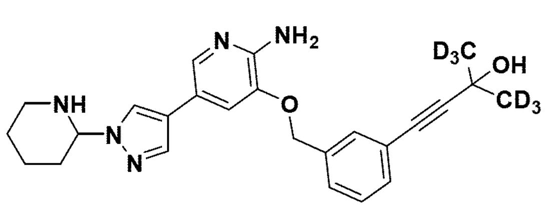

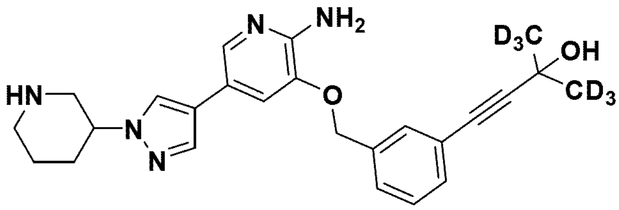

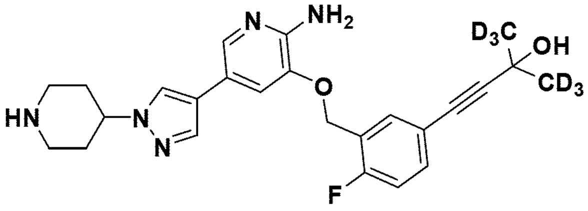

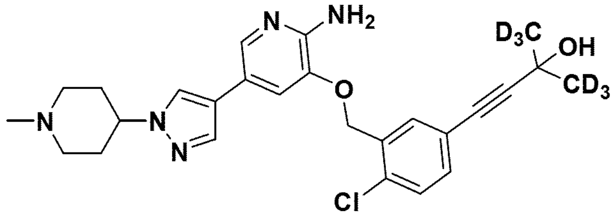

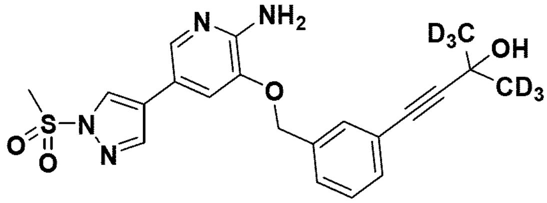

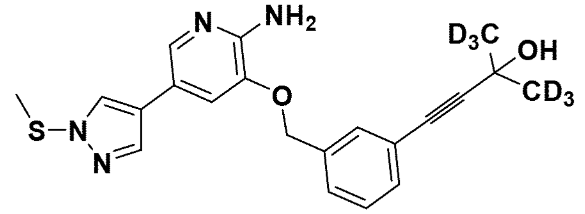

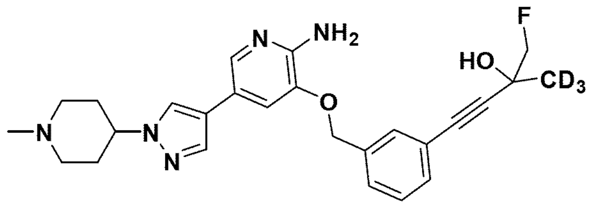

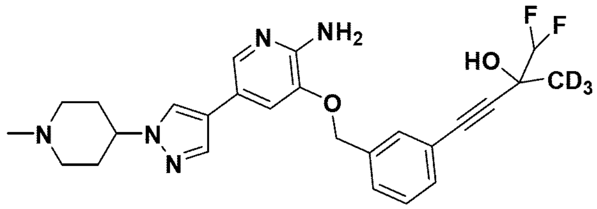

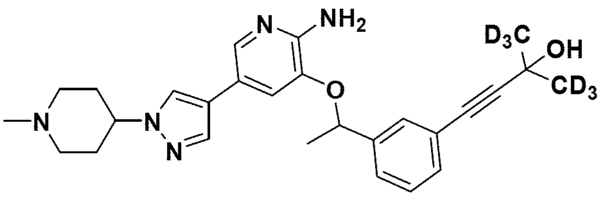

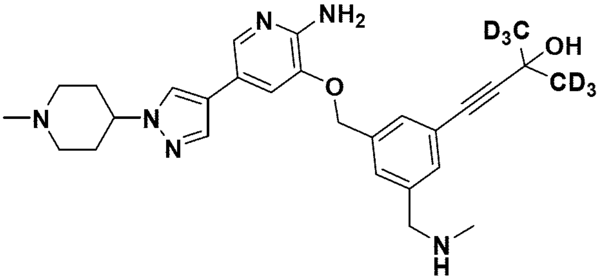

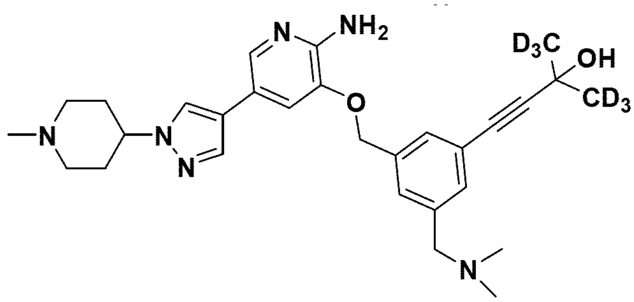

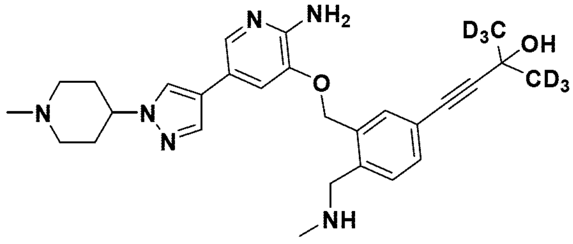

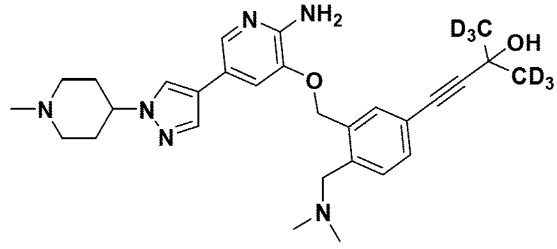

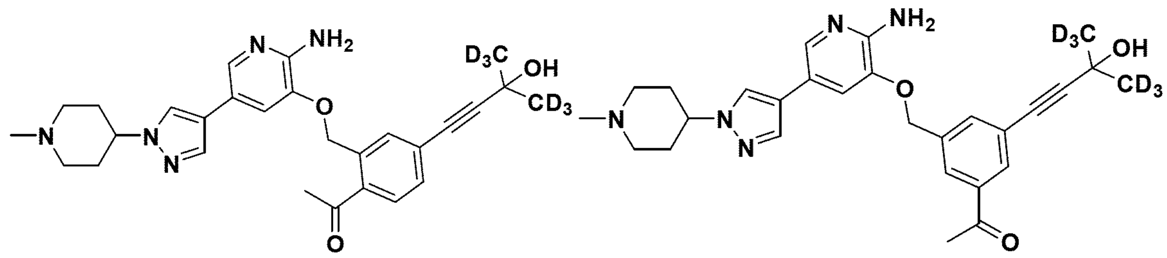

(5)

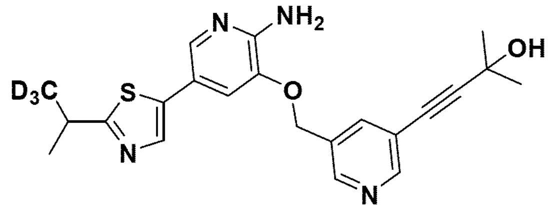

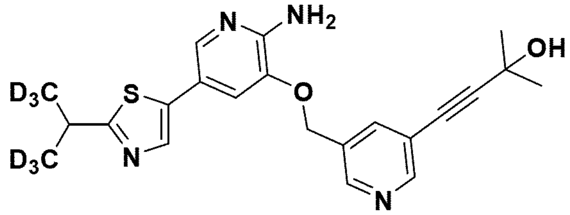

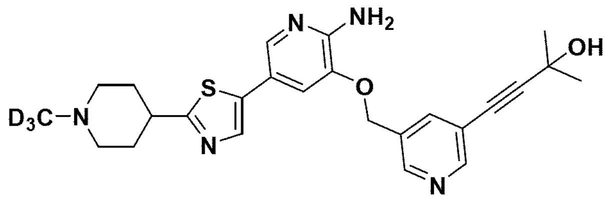

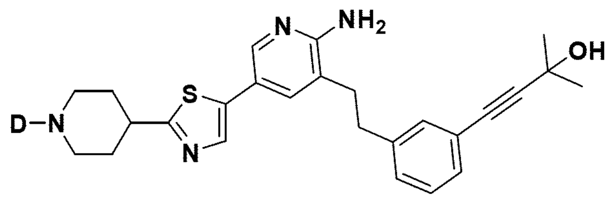

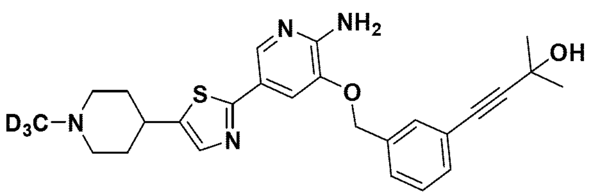

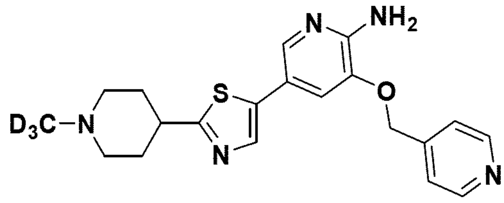

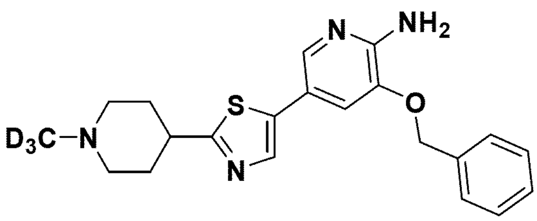

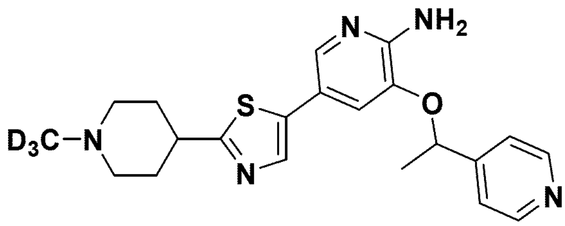

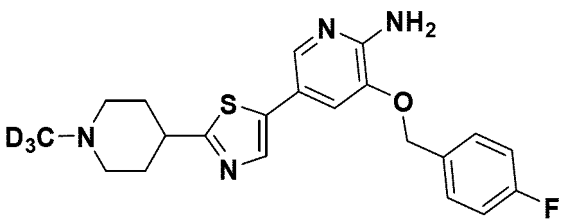

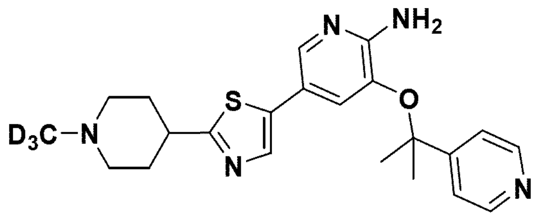

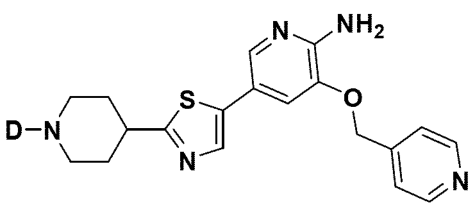

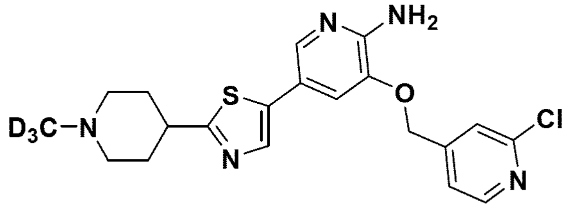

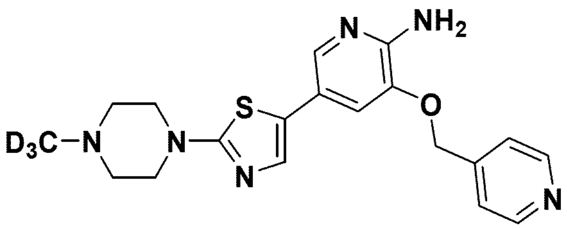

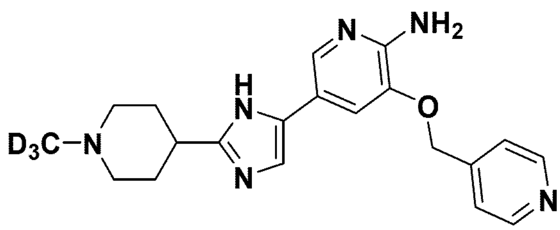

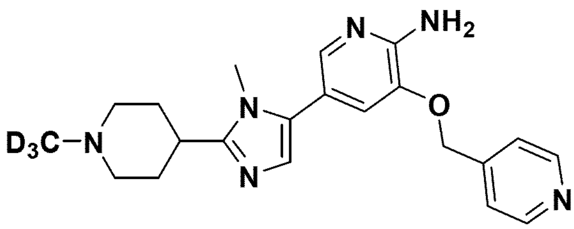

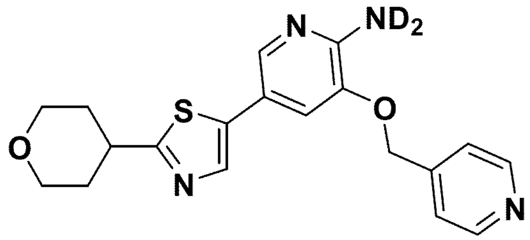

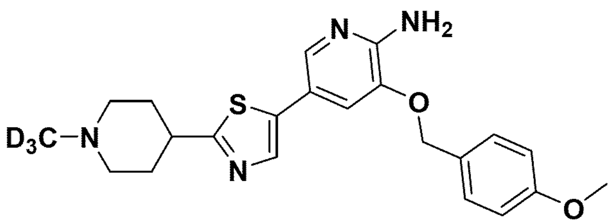

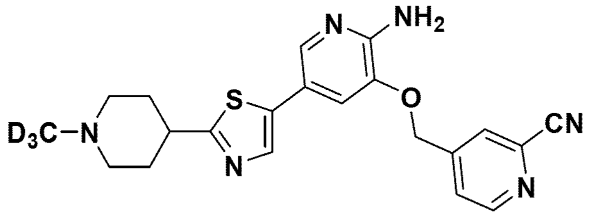

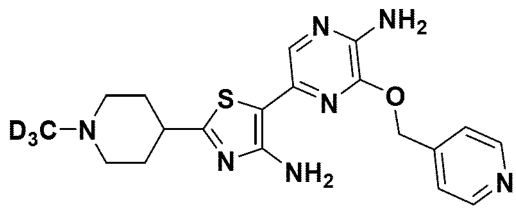

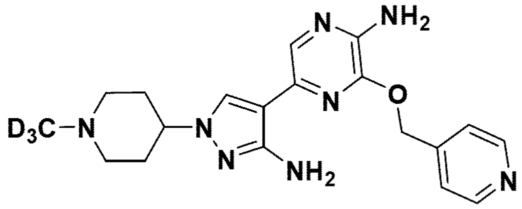

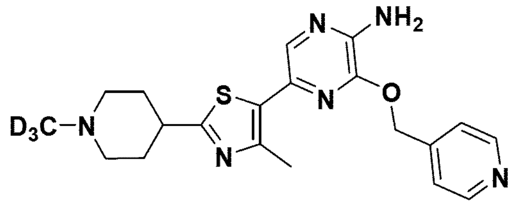

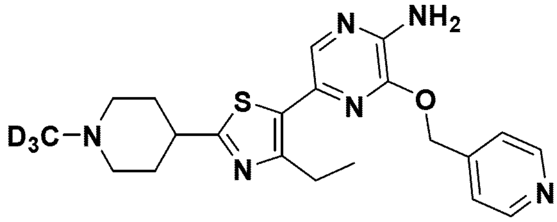

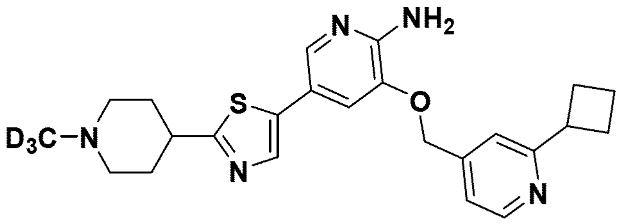

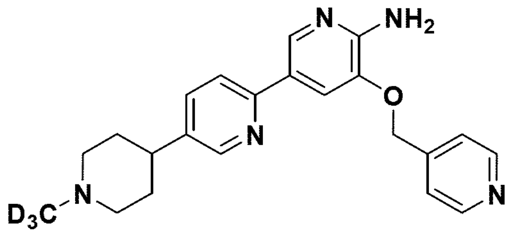

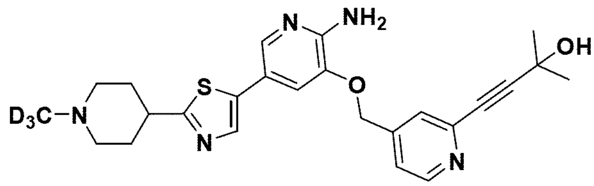

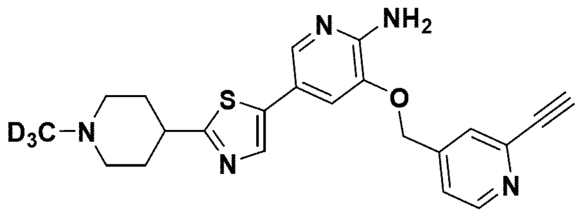

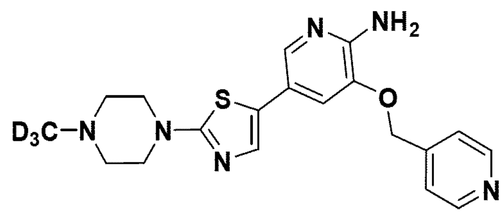

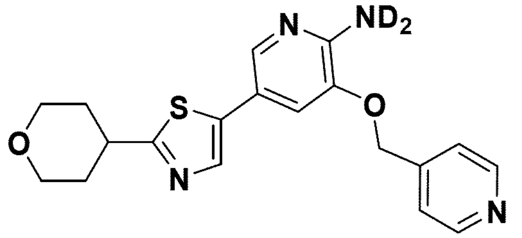

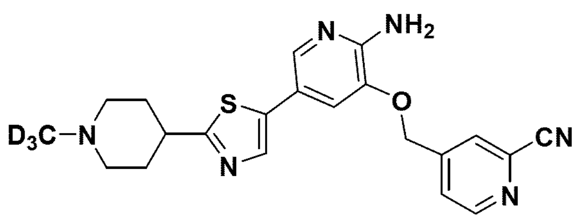

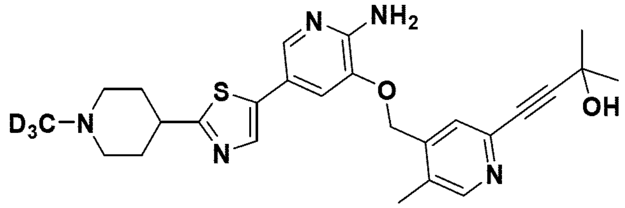

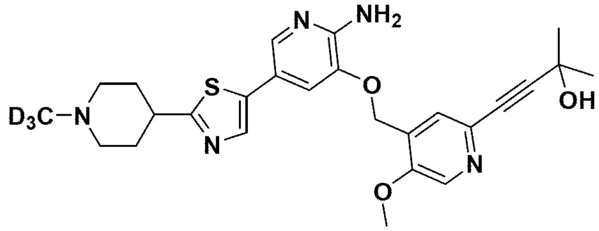

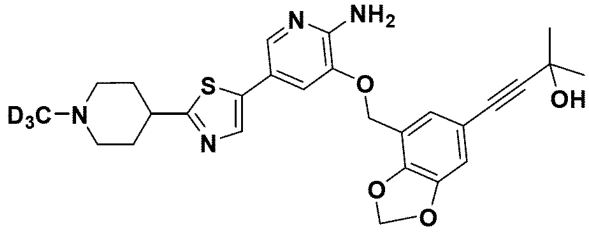

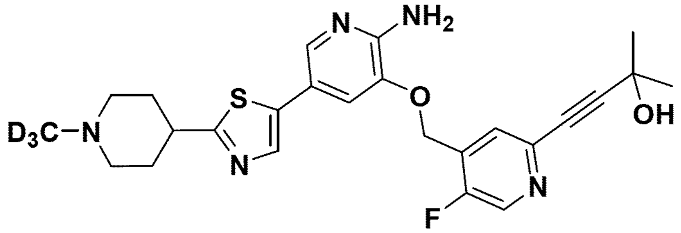

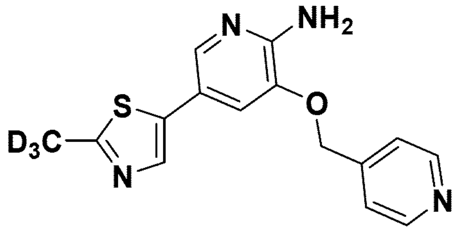

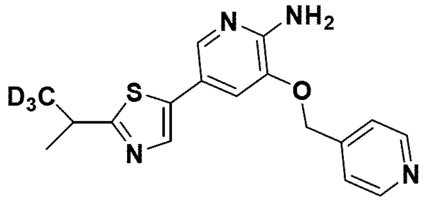

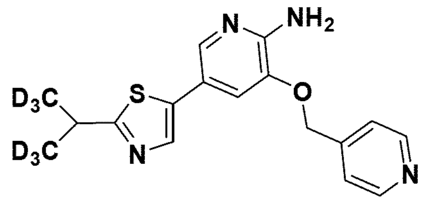

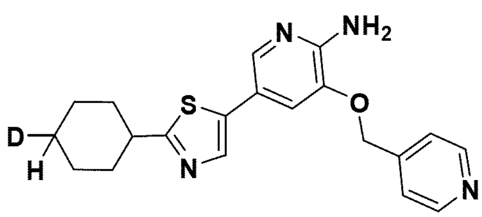

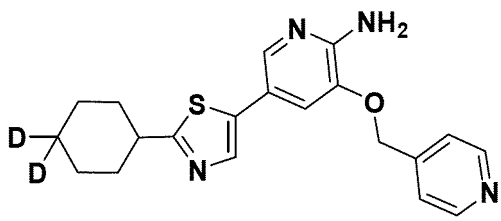

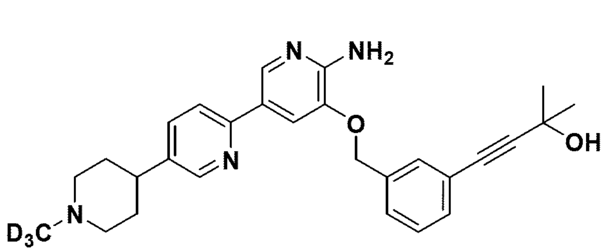

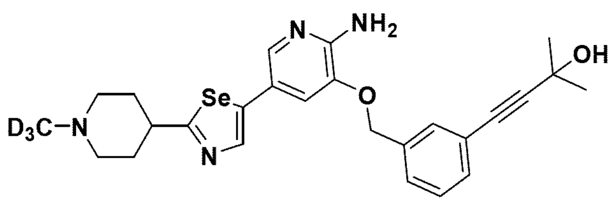

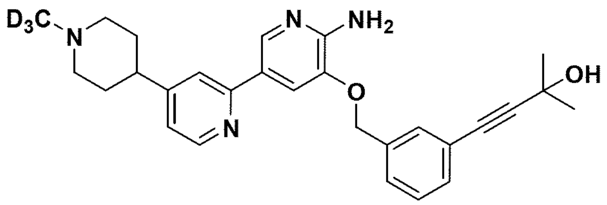

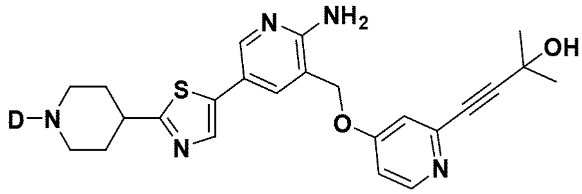

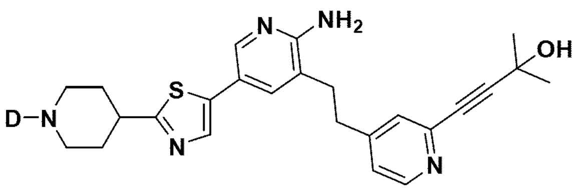

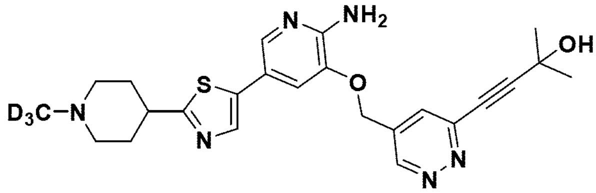

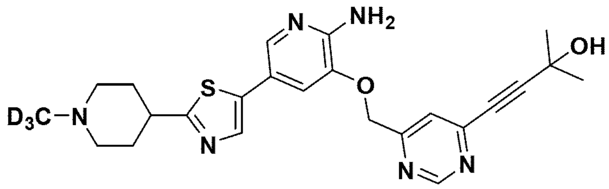
.

In particular, in the above compounds, any atom not designated as deuterium is present in its natural isotopic abundance.

In particular, in the above compounds, the positions designated as "deuterium" have at least, for example, 95% deuterium incorporation.

Provided in the present invention is a method for preparing the compound represented by general formula I, comprising the following steps:

(1) condensing

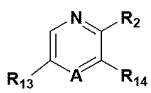

with

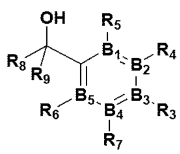

to generate

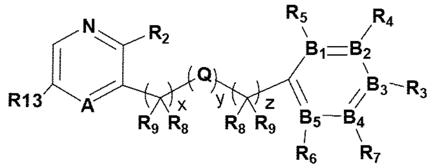

,

wherein $R_{13}$ is selected from halogen or

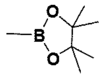

,

and $R_{14}$ is selected from -OH or -F; and

(2) condensing

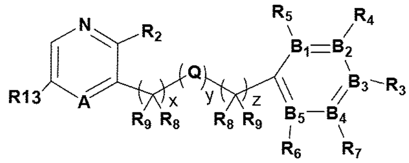

with **Ar-$R_{15}$** to generate

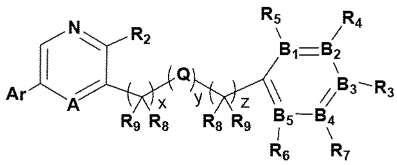

,

wherein $R_{15}$ is selected from -Br or -$SnBu_3$.

In particular, in step (1) above, $R_{13}$ is -Br.
In particular, in step (2) above, $R_{13}$ is -Br or

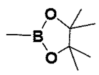

.

When $R_{13}$ is -Br, $R_{15}$ is -$SnBu_3$, and when $R_{13}$ is

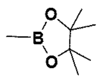

,

$R_{15}$ is -Br.

Further provided in the present invention are pharmaceutically acceptable salts, stereoisomers, esters, prodrugs and solvates of the compound represented by general formula I.

In particular, the pharmaceutically acceptable salts include acid addition salts and base addition salts.

In particular, the acid addition salts include, but are not limited to, salts derived from inorganic acids such as hydrochloric, nitric, phosphoric, sulfuric, hydrobromic, hydroiodic and phosphonic acids, and salts derived from organic acids such as aliphatic mono- and di-carboxylic acids, phenyl-substituted alkanoic acids, hydroxyalkanoic acids, alkanedioic acids, aromatic acids and aliphatic and aromatic sulfonic acids. Thus, these salts include, but are not limited to, sulfates, pyrosulfates, bisulfates, sulfites, bisulfites, nitrates, phosphates, monohydrogen phosphates, dihydrogen phosphates, metaphosphates, pyrophosphates, hydrochlorides, hydrobromides, iodates, acetates, propionates, octanoates, isobutyrates, oxalates, malonates, succinates, suberates, sebacates, fumarates, maleates, mandelates, benzoates, chlorobenzoates, methylbenzoates, dinitrobenzoates, phthalates, benzenesulfonates, tosylates, phenylacetates,

citrates, lactates, maleates, tartrates and methanesulfonates, as well as salts of amino acids, e.g., arginate, gluconate, galacturonate, etc. Acid addition salts may be prepared by contacting a free base form with a sufficient amount of the desired acid to form a salt in a conventional manner. The free base form may be regenerated by contacting the salt form with a base and isolated in a conventional manner.

In particular, the base addition salts are formed with metals or amines, such as hydroxides of alkali metals and alkaline earth metals, or with organic amines. Examples of the metals used as cations include, but are not limited to, sodium, potassium, magnesium and calcium. Examples of suitable amines include, but are not limited to, N,N'-dibenzylethylene diamine, chloroprocaine, choline, diethanolamine, ethylenediamine (ethane-1,2-diamine), N-methylglucamine and procaine. Base addition salts may be prepared by contacting a free acid form with a sufficient amount of the desired base to form a salt in a conventional manner. The free acid form may be regenerated by contacting the salt form with an acid and isolated in a conventional manner.

In an embodiment of the present invention, the pharmaceutically acceptable salts are hydrochloride salts.

In particular, the stereoisomers include enantiomers, diastereomers and geometric isomers. Some of the compounds of the present invention have cycloalkyl groups which may be substituted at more than one carbon atom, in which case all geometric forms thereof, including *cis* and *trans,* and mixtures thereof, are within the scope of the present invention.

In particular, the solvates refer to a physical association of a compound of the present invention with one or more solvent molecules. Such a physical association includes various degrees of ionic and covalent bonding, including hydrogen bonding. In some cases, the solvates may be isolated, for example when one or more solvent molecules are incorporated in the crystal lattice of the crystalline solid. "Solvates" encompass both solution-phase and isolatable solvates. Representative solvates include ethanolates, methanolates, etc. "Hydrates" are solvates in which one or more solvent molecules are $H_2O$.

In particular, the prodrugs refer to the forms of the compound represented by formula I that are suitable for administration to a patient without undue toxicity, irritation, allergic response, and the like, and that are effective for their application purpose, including acetal, ester and zwitterionic forms. The prodrugs are converted *in vivo,* for example by hydrolysis in blood, to give the parent compound represented by the above formula.

Further provided in the present invention is a crystalline form of the compound represented by general formula I, and pharmaceutically acceptable salts, stereoisomers, esters, prodrugs and solvates thereof.

In particular, provided in the present invention is a crystalline form of 4-(3-(((2-amino-5-(1-(1-trideuteriomethylpiperidin-4-yl)-1H-pyrazol-4-yl)pyridin-3-yl)oxy)meth yl)phenyl)-2-methylbut-3-yn-2-ol (which has the following structure).

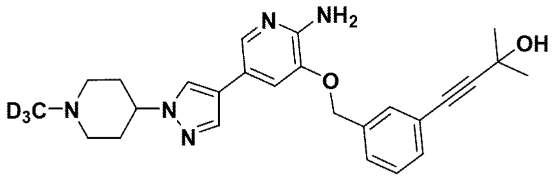

In particular, the crystalline form is crystalline form A, which has an XRPD pattern with characteristic peaks (main characteristic diffraction peaks) at at least three (or all) of the positions with 2θ values of 13.1° ± 0.2°, 16.3° ± 0.2°, 17.5° ± 0.2° and 23.8° ± 0.2°.

In particular, the XRPD pattern of the crystalline form A has further characteristic peaks (minor characteristic diffraction peaks) at at least three (at least four, at least five, at least six, at least seven or all) of the positions with 2θ values of 8.1° ± 0.2°, 12.2° ± 0.2°, 15.3° ± 0.2°, 18.0° ± 0.2°, 19.3° ± 0.2°, 19.5° ± 0.2°, 21.3° ± 0.2° and 21.6° ± 0.2°.

In particular, the crystalline form A has an XRPD pattern substantially as shown in FIG. 1.

In particular, the crystalline form A has a DSC pattern with an endothermic peak at around 168.8°C.

In particular, the crystalline form A has a DSC pattern substantially as shown in FIG. 2.

In particular, the crystalline form A has a weight loss of about 1.1% upon heating from room temperature to 170°C.

In particular, the crystalline form A has a TGA pattern substantially as shown in FIG. 2.

In particular, the crystalline form A is anhydrous.

In particular, the crystalline form is crystalline form B, which has an XRPD pattern with characteristic peaks (main characteristic diffraction peaks) at at least three (or all) of the positions with 2θ values of 5.7° ± 0.2°, 11.3° ± 0.2°, 22.7° ± 0.2° and 23.5° ± 0.2°.

In particular, the XRPD pattern of the crystalline form B has further characteristic peaks (minor characteristic diffraction peaks) at at least three (at least four, at least five or all) of the positions with 2θ values of 7.1° ± 0.2°, 8.8° ± 0.2°, 14.1° ± 0.2°, 17.0° ± 0.2°, 18.0° ± 0.2° and 18.8° ± 0.2°.

In particular, the crystalline form B has an XRPD pattern substantially as shown in FIG. 6.

In particular, the crystalline form B has a DSC pattern with endothermic peaks at at least one of around 59.5°C, 95.6°C, 150.8°C and 160.9°C.

In particular, the crystalline form B has a DSC pattern substantially as shown in FIG. 7.

In particular, the crystalline form B has a weight loss of about 13.2% upon heating from room temperature to 70°C and a weight loss of about 8.5% upon continued heating to 170°C.

In particular, the crystalline form B has a TGA pattern substantially as shown in FIG. 7.

In particular, the crystalline form B is an EtOAc solvate.

In particular, further provided in the present invention is a crystalline form of 4-(3-(((2-amino-5-(1-(1-trideuteriomethylpiperidin-4-yl)-1H-pyrazol-4-yl)pyridin-3-yl)oxy)meth yl)phenyl)-2-methylbut-3-yn-2-ol hydrochloride.

In particular, the crystalline form is crystalline form A, which has an XRPD pattern with characteristic peaks (main characteristic diffraction peaks) at at least three (at least four or all) of the positions with 2θ values of 13.0° ± 0.2°, 16.3° ± 0.2°, 17.5° ± 0.2°, 19.4° ± 0.2° and 23.8° ± 0.2°.

In particular, the XRPD pattern of the crystalline form A has further characteristic peaks (minor characteristic diffraction peaks) at at least three (at least four or all) of the positions with 2θ values of 8.1° ± 0.2°, 12.1° ± 0.2°, 15.3° ± 0.2°, 18.0° ± 0.2° and 21.4° ± 0.2°.

In particular, the crystalline form A has an XRPD pattern substantially as shown in FIG. 11.

In particular, the crystalline form A has a DSC pattern with endothermic peaks at around 81.9°C and around 156.0°C.

In particular, the crystalline form A has a DSC pattern substantially as shown in FIG. 12.

In particular, the crystalline form A has a weight loss of about 8.6% upon heating from room temperature to 150°C.

In particular, the crystalline form A has a TGA pattern substantially as shown in FIG. 12.

Further provided in the present invention is a method for preparing the above crystalline form.

In particular, the method is selected from one or more of anti-solvent addition, anti-anti-solvent addition, gas-solid diffusion, room temperature suspension stirring, 5°C suspension stirring, slow volatilization, slow cooling, gas-liquid diffusion and polymer induction.

In particular, the anti-solvent addition process includes dissolving the desired product in a good solvent, and then adding an anti-solvent to the obtained solution (then, for example, volatilizing at room temperature to give a solid, or stirring at -20°C to give a solid).

In particular, the anti-anti-solvent addition process includes dissolving the desired product in a good solvent, and then adding the obtained solution to an anti-solvent (then, for example, volatilizing at room temperature to give a solid, or stirring at -20°C to give a solid).

In particular, the gas-solid diffusion, room temperature suspension stirring, 5°C suspension stirring, slow volatilization, slow cooling and gas-liquid diffusion processes all include: dissolving the desired product in a solvent and then drying to give a solid.

In particular, the polymer induction process includes dissolving the desired product in a solvent, adding a polymer, and volatilizing at room temperature to give a solid.

In particular, the crystalline form A of 4-(3-(((2-amino-5-(1-(1-trideuteriomethylpiperidin-4-yl)-1H-pyraaol-4-yl) pyridin-3-yl)oxy)meth yl)phenyl)-2-methylbut-3-yn-2-ol is prepared by a method selected from one or more of anti-solvent addition, anti-anti-solvent addition, gas-solid diffusion, room temperature suspension stirring, 5°C suspension stirring, slow volatilization, slow cooling, gas-liquid diffusion and polymer induction.

In particular, regarding the preparation of the crystalline form A, for the anti-solvent addition process, the good solvent may be selected from MeOH, acetone, DMSO, EtOAc, EtOH, DCM, CHCl3, THF, IPA, ACN and 1,4-dioxane, and the anti-solvent may be selected from MTBE, toluene, n-heptane and water.

In an embodiment of the present invention, in the anti-solvent addition process, the good solvent may be selected from MeOH, acetone and DMSO, and the anti-solvent is MTBE.

In another embodiment of the present invention, in the anti-solvent addition process, the good solvent may be selected from EtOH and DCM, and the anti-solvent is toluene.

In another embodiment of the present invention, in the anti-solvent addition process, the good solvent may be selected from CHCl3, THF and IPA, and the anti-solvent is n-heptane.

In another embodiment of the present invention, in the anti-solvent addition process, the good solvent may be selected from acetone, ACN and 1,4-dioxane, and the anti-solvent is water.

In particular, regarding the preparation of the crystalline form A, for the anti-anti-solvent addition process, the good solvent may be selected from MeOH, MIBK, acetone, anisole, EtOH, THF, EtOAc and DCM, and the anti-solvent may be selected from toluene, n-heptane, water and MTBE.

In an embodiment of the present invention, in the anti-anti-solvent addition process, the good solvent may be MIBK, and the anti-solvent may be toluene.

In another embodiment of the present invention, in the anti-anti-solvent addition process, the good solvent may be selected from acetone and anisole, and the anti-solvent may be n-heptane.

In another embodiment of the present invention, in the anti-anti-solvent addition process, the good solvent may be selected from EtOH and THF, and the anti-solvent may be water.

In another embodiment of the present invention, in the anti-anti-solvent addition process, the good solvent may be selected from EtOAc and DCM, and the anti-solvent may be MTBE.

In particular, regarding the preparation of the crystalline form A, for the gas-solid diffusion process, the solvent may be selected from water, DCM, EtOH, MeOH, ACN, THF, CHCl3, acetone, DMSO, EtOAc, 1,4-dioxane and IPA.

In particular, regarding the preparation of the crystalline form A, for the room temperature suspension stirring process, the solvent may be selected from MTBE, IPAc, n-heptane, toluene, water, EtOH/toluene (e.g., in a ratio of 1:3, v/v), DMSO/MTBE (e.g., in a ratio of 1:4, v/v), acetone/water (e.g., in a ratio of 1:4, v/v), IPA/n-heptane (e.g., in a ratio of 1:4, v/v), EtOAc/n-heptane (e.g., in a ratio of 1:4, v/v), anisole/toluene (e.g., in a ratio of 1:4, v/v), DMAc/water (e.g., in a ratio of 1:4, v/v) and THF/water (e.g., in a ratio of 1:4, v/v).

In particular, regarding the preparation of the crystalline form A, for the 5°C suspension stirring process, the solvent may be selected from MTBE, toluene, water, IPA/n-heptane (e.g., in a ratio of 1:2, v/v), MEK/n-heptane (e.g., in a ratio of 1:2, v/v), EtOAc/toluene (e.g., in a ratio of 1:2, v/v), CPME/toluene (e.g., in a ratio of 1:2, v/v), NMP/water (e.g., in a ratio of 1:4, v/v), THF/water (e.g., in a ratio of 1:4, v/v), ACN/water (e.g., in a ratio of 1:2, v/v), IPAc/DCM (e.g., in a ratio of 1:1, v/v), MeOH/toluene (e.g., in a ratio of 1:4, v/v), DCM/MTBE (e.g., in a ratio of 1:4, v/v) and THF/n-heptane (e.g., in a ratio of 1:4, v/v).

In particular, regarding the preparation of the crystalline form A, for the slow volatilization process, the solvent may be selected from EtOH, acetone, IPAc, THF, CPME, anisole, ACN/water (e.g., in a ratio of 9:1, v/v), MeOH/DCM (e.g., in a ratio of 1:1, v/v), acetone/EtOAc (e.g., in a ratio of 2:1, v/v) and THF/water (e.g., in a ratio of 4:1, v/v).

In particular, regarding the preparation of the crystalline form A, for the slow cooling process, the solvent may be selected from CPME, toluene, ACN/toluene (e.g., in a ratio of 1:2, v/v), acetone/n-heptane (e.g., in a ratio of 1:1, v/v), THF/toluene (e.g., in a ratio of 1:2, v/v), MeOH/water (e.g., in a ratio of 1:1, v/v) and $CHCl_3$/MTBE (e.g., in a ratio of 1:1, v/v).

In particular, regarding the preparation of the crystalline form A, for the gas-liquid diffusion process, the good solvent may be selected from EtOH, THF and DMSO, and the anti-solvent may be selected from n-heptane, MTBE, toluene, cyclohexane and water.

In an embodiment of the present invention, in the gas-liquid diffusion process, the good solvent is EtOH, and the anti-solvent may be selected from n-heptane, MTBE and toluene.

In another embodiment of the present invention, in the gas-liquid diffusion process, the good solvent is THF, and the anti-solvent may be selected from n-heptane, cyclohexane and MTBE.

In another embodiment of the present invention, in the gas-liquid diffusion process, the good solvent is DMSO, and the anti-solvent may be selected from toluene, MTBE and water.

In particular, regarding to the preparation of the crystalline form A, for the polymer induction process, the solvent may be selected from MEK, ACN/toluene (e.g., in a ratio of 4:1, v/v), THF/water (e.g., in a ratio of 9:1, v/v), EtOAc, acetone/2-MeTHF (e.g., in a ratio of 1:1, v/v) and MeOH/DCM (e.g., in a ratio of 1:1, v/v), and the polymer may be selected from one or more of polyvinylpyrrolidone, polyvinyl alcohol, polyvinyl chloride, polyvinyl acetate, hypromellose, methylcellulose, polycaprolactone, polyethylene glycol, polymethyl methacrylate, sodium alginate and hydroxyethyl cellulose.

In an embodiment of the present invention, in the polymer induction process, the polymer is a mixture of polyvinylpyrrolidone, polyvinyl alcohol, polyvinyl chloride, polyvinyl acetate, hypromellose and methylcellulose (e.g., in a mass ratio of 1:1:1:1:1:1), and the solvent may be selected from MEK, ACN/toluene (e.g., in a ratio of 4:1, v/v) and THF/water (e.g., in a ratio of 9:1, v/v).

In another embodiment of the present invention, in the polymer induction process, the polymer is a mixture of polycaprolactone, polyethylene glycol, polymethyl methacrylate, sodium alginate and hydroxyethyl cellulose (e.g., in a mass ratio of 1:1:1:1:1), and the solvent may be selected from EtOAc and acetone/2-MeTHF (e.g., in a ratio of 1:1, v/v).

In particular, the crystalline form B of 4-(3-(((2-amino-5-(1-(1-trideuteriomethylpiperidin-4-yl)-1H-pyrazol-4-yl) pyridin-3-yl)oxy)meth yl)phenyl)-2-methylbut-3-yn-2-ol is prepared by an anti-solvent addition process.

In an embodiment of the present invention, regarding the preparation of the crystalline form B, the good solvent is EtOAc, and the anti-solvent is toluene.

Further provided in the present invention is a pharmaceutical composition comprising the compound represented by general formula I, or pharmaceutically acceptable salts, stereoisomers, esters, prodrugs and solvates thereof, or the above crystalline form, and pharmaceutically acceptable excipients.

In particular, the excipients are selected from one or more of carriers, diluents, binders, lubricants, wetting agents, etc. In particular, the pharmaceutical composition comprises a therapeutically effective amount of the compound represented by general formula I. In certain embodiments, these pharmaceutical compositions may be used for treating diseases or symptoms mediated by HPK1 kinase.

In particular, the pharmaceutical composition may be in the form of tablets (e.g., sugar-coated tablets, film-coated tablets, sublingual tablets, orally disintegrating tablets, buccal tablets, etc.), pills, powders, granules, capsules (e.g., soft capsules, microcapsules), lozenges, syrups, emulsions, suspensions, controlled release preparations (e.g., immediate

release preparations, sustained release preparations, sustained release microcapsules), aerosols, films (e.g., oral disintegrating films, oral mucosa-adherent films), injections (e.g., subcutaneous injections, intravenous injections, intramuscular injections, intraperitoneal injections), intravenous drips, transdermal absorption preparations, ointments, lotions, adhesive preparations, suppositories (e.g., rectal suppositories, vaginal suppositories), pellets, nasal preparations, pulmonary preparations (inhalants), eye drops, etc.

In particular, various dosage forms of the pharmaceutical composition may be prepared by conventional production methods in the pharmaceutical field, such as mixing the active ingredient with one or more excipients and then formulating them into the desired dosage form.

In particular, the pharmaceutical composition may contain 0.1-99.5% (e.g., 0.1%, 1%, 5%, 10%, 20%, 30%, 40%, 50%, 60%, 70%, 80%, 90%, 95%, 99%, 99.5%) by weight of the active ingredient.

Further provided in the present invention is the use of the compound represented by general formula I, and pharmaceutically acceptable salts, stereoisomers, esters, prodrugs and solvates thereof, or the above crystalline form, or the above pharmaceutical composition, in preparation of drugs for preventing and/or treating tumors.

Further provided in the present invention is the use of the compound represented by general formula I, and pharmaceutically acceptable salts, stereoisomers, esters, prodrugs and solvates thereof, and the above crystalline form, in combination with PD-1, PD-L1, CTLA-4, TIM-3, TGF-β and its receptors, LAG3 antagonists or TLR4, TLR7, TLR8, TLR9 and STING agonists, in tumor immunotherapy.

Further provided in the present invention is the use of the compound represented by general formula I, and pharmaceutically acceptable salts, stereoisomers, esters, prodrugs and solvates thereof, and the above crystalline form, in combination with CAR-T immunotherapy, in tumor immunotherapy.

In particular, CAR-T immunotherapy refers to chimeric antigen receptor T cell immunotherapy, the basic principle of which is to use the patient's immune cells to clear cancer cells, which belongs to cell therapy.

In particular, the tumors are malignant tumors, including, but not limited to, lymphoma, blastoma, medulloblastoma, retinoblastoma, sarcoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia or lymphoid malignancies, squamous cell carcinoma, epithelial squamous cell carcinoma, lung cancer, small cell lung cancer, non-small cell lung cancer, lung adenocarcinoma, lung squamous cell carcinoma, peritoneal cancer, hepatocellular cancer, gastric cancer, intestinal cancer, pancreatic cancer, glioblastoma, cervical cancer, ovarian cancer, liver cancer, bladder cancer, hepatoma, breast cancer, metastatic breast cancer, colon cancer, rectal cancer, colorectal cancer, uterine cancer, salivary gland cancer, kidney cancer, prostate cancer, vulval cancer, thyroid cancer, hepatic carcinoma, anal cancer, penile cancer, merkel cell cancer, esophageal cancer, biliary tract cancer, head and neck cancer and hematologic malignancies.

Further provided in the present invention is a method for preventing and/or treating tumors, comprising the step of administering to a subject in need an effective amount of the compound represented by general formula I, and pharmaceutically acceptable salts, stereoisomers, esters, prodrugs and solvates thereof, or the above crystalline form, or the pharmaceutical composition according to the present invention.

In particular, the tumors have the above corresponding definition in the present invention.

Further provided in the present invention is a method for preventing and/or treating diseases caused by pathogen infection or related to pathogen infection, comprising the step of administering to a subject in need an effective amount of the compound represented by general formula I according to the present invention, and pharmaceutically acceptable salts, stereoisomers, esters, prodrugs and solvates thereof, or the above crystalline form, or the pharmaceutical composition according to the present invention.

In particular, the pathogens and diseases have the above corresponding definitions in the present invention.

## BRIEF DESCRIPTION OF THE DRAWINGS

FIG. 1 shows an XRPD pattern of Compound A2 free base crystalline form A.
FIG. 2 shows a TGA/DSC pattern of Compound A2 free base crystalline form A.
FIG. 3 shows a $^1$H NMR pattern of Compound A2 free base crystalline form A.
FIG. 4 shows an HPLC pattern of Compound A2 free base crystalline form A.
FIG. 5 shows an XRPD pattern of the starting sample of Compound A2 free base crystalline form A and after 1 day of closed placement at 60°C.
FIG. 6 shows an XRPD pattern of Compound A2 free base crystalline form B.
FIG. 7 shows a TGA/DSC pattern of Compound A2 free base crystalline form B.
FIG. 8 shows a $^1$H NMR pattern of Compound A2 free base crystalline form B.
FIG. 9 shows an XRPD pattern of Compound A2 free base crystalline form B before and after being placed at room

temperature.

FIG. 10 shows an XRPD pattern of Compound A2 free base crystalline form B before and after nitrogen purging.

FIG. 11 shows an XRPD pattern of Compound A2 hydrochloride.

FIG. 12 shows a TGA/DSC pattern of Compound A2 hydrochloride.

FIG. 13 shows an XRPD overlay of Compound A2 free base crystalline form A and Compound A2 hydrochloride.

FIG. 14 shows the concentration of iL-2 in human PBMCs.

FIG. 15 shows keratitis lesions in mice post HSV virus inoculation.

FIG. 16 shows the clinical scores of mice post HSV virus inoculation.

FIG. 17 shows the corneal neovascularization scoring of mice post HSV virus inoculation.

FIG. 18 shows the body weight changes of hamsters.

FIG. 19 shows the lung weight changes of hamsters.

FIG. 20 shows the Kaplan-Meier survival of H1N1-infected mice (n = 6), with the dotted line at 35% indicating the humanitarian endpoint. Any mice with a weight loss below this criteria (-35%) will be euthanized.

FIG. 21 shows the virus specific T cell proliferation rate in mice infected with low-dose virus.

## DETAILED DESCRIPTION

Unless otherwise defined, all scientific and technical terms used herein have the same meaning as those commonly understood by those skilled in the art to which the present invention belongs.

For the term "$C_{0-10}$ alkyl" in the present invention, $C_0$ alkyl refers to H, and thus, $C_{0-10}$ alkyl includes H, $C_1$ alkyl, $C_2$ alkyl, $C_3$ alkyl, $C_4$ alkyl, $C_5$ alkyl, $C_6$ alkyl, $C_7$ alkyl, $C_8$ alkyl, $C_9$ alkyl and $C_{10}$ alkyl.

The term "$C_{1-10}$ straight-chain/branched alkyl" in the present invention includes methyl, ethyl, $C_3$ straight-chain/branched alkyl, $C_4$ straight-chain/branched alkyl, $C_5$ straight-chain/branched alkyl, $C_6$ straight-chain/branched alkyl, $C_7$ straight-chain/branched alkyl, $C_8$ straight-chain/branched alkyl, $C_9$ straight-chain/branched alkyl and $C_{10}$ straight-chain/branched alkyl.

The term "$C_{3-10}$ branched alkyl" in the present invention includes isopropyl, isobutyl, tert-butyl and isoamyl.

The term "$C_{3-10}$ cycloalkyl" in the present invention includes $C_3$ cycloalkyl, $C_4$ cycloalkyl, $C_5$ cycloalkyl, $C_6$ cycloalkyl, $C_7$ cycloalkyl, $C_8$ cycloalkyl, $C_9$ cycloalkyl and $C_{10}$ cycloalkyl.

The term "$C_{3-8}$ cycloalkyl" in the present invention includes $C_3$ cycloalkyl, $C_4$ cycloalkyl, Cs cycloalkyl, $C_6$ cycloalkyl, $C_7$ cycloalkyl and $C_8$ cycloalkyl.

The term "$C_{4-8}$ cycloalkyl" in the present invention includes $C_4$ cycloalkyl, $C_5$ cycloalkyl, $C_6$ cycloalkyl, $C_7$ cycloalkyl and $C_8$ cycloalkyl.

The term "$C_{4-6}$ cycloalkyl" in the present invention includes $C_4$ cycloalkyl, Cs cycloalkyl and $C_6$ cycloalkyl.

The term "halogen" in the present invention includes fluorine, chlorine, bromine and iodine.

The term "heterocycloalkyl" in the present invention refers to a non-aromatic saturated monocyclic or polycyclic ring system containing 3 to 10 ring atoms, preferably 5 to 10 ring atoms, in which one or more of the ring atoms are not carbon atoms but, for example, nitrogen, oxygen or sulfur atoms. A preferred heterocycloalkyl group contains 5 to 6 ring atoms. The prefix, aza-, oxa- or thia-, in front of the heterocycloalkyl group refers to at least one nitrogen, oxygen or sulfur atom as a ring atom.

The term "heteroaryl" in the present invention refers to an aromatic monocyclic or polycyclic ring system containing 5 to 14 ring atoms, preferably 5 to 10 ring atoms, in which one or more of the ring atoms are not carbon atoms but, for example, nitrogen, oxygen or sulfur atoms. A preferred heteroaryl group contains 5 to 6 ring atoms. Representative heteroaryl groups include pyrazinyl, furanyl, thienyl, pyridinyl, pyrimidinyl, isoxazolyl, isothiazolyl, oxazolyl, thiazolyl, pyrazolyl, furazanyl, pyrrolyl, pyrazolyl, triazolyl, 1,2,4-thiadiazolyl, pyrazinyl, pyridazinyl, quinoxalinyl, 2,3-diazanaphthalenyl, imidazo[1,2-a]pyridine, imidazo[2,1-b]thiazolyl, benzofurazanyl, indolyl, azaindolyl, benzimidazolyl, benzothienyl, quinolinyl, imidazolyl, thienopyridinyl, quinazolinyl, thienopyrimidinyl, pyrrolopyridinyl, imidazopyridinyl, isoquinolinyl, benzoazaindolyl, 1,2,4-triazinyl, benzothiazolyl, etc.

In the present invention, the "D" refers to deuterium; "substituted with deuterium" refers to the replacement of one or more hydrogen atoms with a corresponding number of deuterium atoms.

It will be recognized that there may be some variations in the natural isotopic abundance in the synthesized compounds, depending on the source of the chemical materials used in synthesis. Thus, the compound of the present invention will inherently have a small amount of deuterated isotopologues. Despite this variation, the concentration of stable hydrogen and carbon isotopes with natural abundance is low and insignificant as compared to the degree of stable isotopic substitution of the compound of the present invention. See, e.g., Wada, E et al. Seikagaku, 1994, 66: 15; Gannes, LZ et al. Comp Biochem Physiol Mol Integr Physiol, 1998, 119: 725.

In the compound of the present invention, any atom not designated as deuterium is present in its natural isotopic abundance. Unless otherwise indicated, when a position is designated specifically as "H" or "hydrogen", the position is understood to have hydrogen at its natural abundance isotopic composition. Likewise, unless otherwise indicated, when a

position is specifically designated as "D" or "deuterium", the position is understood to have deuterium at an abundance that is at least 3000 times greater than the natural abundance of deuterium, which is 0.015% (i.e., at least 45% deuterium incorporation).

As used herein, the term "isotope enrichment factor" refers to the ratio of the isotopic abundance of a particular isotope to its natural abundance.

In other embodiments, the compound of the present invention has an isotope enrichment factor of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation) or at least 6633.3 (99.5% deuterium incorporation) for each designated deuterium atom.

The term "isotopologue" refers to a substance whose chemical structure differs from a particular compound of the present invention only in its isotopic composition.

The term "compound", when referring to the compound of the present invention, refers to a collection of molecules having the same chemical structure, except that there may be isotopic variation among the constituent atoms of the molecule. It will thus be clear to those skilled in the art that a compound represented by a particular chemical structure containing a designated deuterium atom will also contain a small quantity of isotopologues having hydrogen atoms at one or more of the designated deuterium positions of the structure. The relative amount of such isotopologues in the compound of the present invention will depend upon a variety of factors, including the isotopic purity of the deuterated reagents used to make the compound and the efficiency of deuterium incorporation in the various synthetic steps used to prepare the compound. However, the overall relative amount of such isotopologues will be less than 49.9% of the compound, as described above. In other embodiments, the overall relative amount of such isotopologues will be less than 47.5%, less than 40%, less than 32.5%, less than 25%, less than 17.5%, less than 10%, less than 5%, less than 3%, less than 1% or less than 0.5%, of the compound.

Some abbreviations used in the present invention are explained as follows:

XRPD: X-ray Powder Diffraction
DSC: Differential Scanning Calorimetry
TGA: Thermogravimetric Analysis
$^1$H HMR: Liquid Proton Nuclear Magnetic Resonance

In the present invention, the term "crystalline form" is confirmed by X-ray powder diffraction pattern characterization. It will be understood by those skilled in the art that the physicochemical properties discussed herein may be characterized where experimental error depends on instrument conditions, sample preparation, sample purity, etc. Especially, it is well known to those skilled in the art that the X-ray diffraction pattern will generally vary with the instrument conditions. It is important to note that the relative intensity of the X-ray powder diffraction pattern may also vary with experimental conditions, such that the order of the peak intensity cannot be taken as the sole or determining factor. Indeed, the relative intensity of the diffraction peaks in the XRPD pattern is related to the preferred orientation of the crystal, and the peak intensity shown herein is for illustrative purposes and not for absolute comparison. In addition, the experimental error of peak angles is usually 5% or less, and the error of these angles should also be taken into account, usually allowing for an error of $\pm$ 0.2°. In addition, due to experimental factors such as sample thickness, an overall shift in peak angles may occur. A certain degree of shift is usually allowed. As a result, it will be understood by those skilled in the art that the X-ray powder diffraction pattern of one crystalline form in the present invention need not be the same as the X-ray powder diffraction pattern in the Examples herein. The "XRPD pattern is the same" as described herein does not mean identical, wherein the same peak positions may differ by $\pm$ 0.2°, and some variability in peak intensity is allowed. Any crystalline form having the same or similar pattern as the characteristic peaks in these patterns is within the scope of the present invention. Those skilled in the art would be able to compare the patterns set forth herein with the pattern of an unknown crystalline form to confirm whether the two patterns reflect the same or different crystalline forms.

In some embodiments, the crystalline form A of the present invention is pure, single, and substantially free of any other crystalline forms admixed. In the present invention, "substantially free", when referring to a new crystalline form, means that this crystalline form includes less than 20% by weight of other crystalline forms, especially less than 10% by weight of other crystalline forms, even less than 5% by weight of other crystalline forms, and even less than 1% by weight of other crystalline forms.

It is noted that the numerical values and numerical ranges mentioned in the present invention should not be construed narrowly as numerical values or numerical ranges per se. It should be understood by those skilled in the art that they may vary depending on the particular technical environments and fluctuate around specific numerical values without departing from the spirits and principles of the present invention. In the present invention, such predictable fluctuation range for those skilled in the art is often represented by the term "about". When the term "about" is used in front of a

numerical value of the present invention and refers to the numerical value, it means any value within ± 10%, preferably within ± 5%, more preferably within ± 2%, and preferably within ± 1% of that value. For example, "about 10" should be understood to mean 9 to 11, preferably 9.5 to 10.5, more preferably 9.8 to 10.2, and more preferably 9.9 to 10.1.

In the present invention, the term "room temperature" means that the temperature of an article is close to or the same as the temperature of a space (e.g., the place of a fume hood in which the article is located). Typically, room temperature is about 20°C to about 30°C, or about 22°C to 27°C, or about 25°C.

Anti-solvent addition (also known as anti-solvent crystallization, precipitation crystallization, salting-out or forced crystallization) process is generally performed by adding one or more anti-solvents to a solution in which the desired product is dissolved in a good solvent, so that the product is in a slightly soluble state in the solution, thereby bringing the solution to a supersaturated state and then precipitating crystals. Anti-anti-solvent addition is generally performed by adding a solution in which the desired product is dissolved in a good solvent to one or more anti-solvents, so that the product is in a slightly soluble state in the solution, thereby bringing the solution to a supersaturated state and then precipitating crystals.

The anti-solvents have a poor ability to dissolve the desired product than the good solvent, e.g., by more than 10%, 20%, 30%, 40%, 50%, 60%, 70% or 80%, and thus, the anti-solvents in the system are relative. The good solvent and the anti-solvents may be polar solvents or non-polar solvents, such as may be selected from one or more of dimethylformamide (DMF), dimethyl sulfoxide (DMSO), water, alcohol solvents, ether solvents, ketone solvents, ester solvents, alkane solvents, aromatic hydrocarbon solvents and nitrile solvents. Among these, the alcohol solvents include, but are not limited to, methanol, ethanol, propanol, isopropanol or 1,3-propanediol, 1,2-propanediol or chlorobutanol, or combinations thereof; the ether solvents include, but are not limited to, tetrahydrofuran, methyl tert-butyl ether or 1,4-dioxane, or combinations thereof; the ketone solvents include, but are not limited to, acetone, methyl ethyl ketone or 4-methyl-2-pentanone, or combinations thereof; the ester solvents include, but are not limited to, ethyl acetate, isopropyl acetate, n-butyl acetate or t-butyl acetate, or combinations thereof; the alkane solvents include, but are not limited to, dichloromethane, chloroform, n-hexane, cyclohexane or pentane or n-heptane, or combinations thereof; the aromatic hydrocarbon solvents include, but are not limited to, benzene, toluene, or combinations thereof; the nitrile solvents include, but are not limited to, acetonitrile and malononitrile.

The anti-solvent addition and the anti-anti-solvent addition may be operated by batch, semi-batch or continuous crystallization. The addition of the anti-solvents to the solution (anti-solvent crystallization) or the addition of the product solution to the anti-solvent (anti-anti-solvent crystallization) may be done dropwise at a constant rate, or slowly at the beginning and then gradually increasing the rate.

In the present invention, the term "hPBMs" (human-Peripheral blood mononuclear cell) refers to cells that have a single nucleus in human peripheral blood, including lymphocytes and monocytes.

In the present invention, the term "iL-2" refers to interleukin-2, a cytokine of the chemokine family, which is derived from multiple cells (mainly produced by activated T cells) and has pleiotropic effects (mainly promoting lymphocyte growth, proliferation and differentiation). iL-2 plays an important role in the body's immune response and anti-virus infection, including stimulating the proliferation of T cells activated by specific antigens or mitogens; activating T cells to promote cytokine production; stimulating NK cell proliferation to enhance NK killing activity and produce cytokines, thereby inducing LAK cell production; promoting B cell proliferation and secreting antibodies; and activating macrophages.

In the present invention, the term "LPS" (Lipopolysaccharide) is a constituent of the outer wall of the cell wall of Gram-negative bacteria, consisting of lipids and polysaccharides (glycolipids). The structure of LPS is composed of antigen-core polysaccharide-lipid A. LPS is an endotoxin that exhibits various biological activities when it acts on cells of other organisms such as humans or animals. The physiological role of LPS is manifested by the presence of Toll-like receptor (TLR) 4 (TLR4) on the cell membrane surface of host cells.

TNF-$\alpha$ (tumor necrosis factor) in the present invention refers to a tumor necrosis factor produced by activated macrophages, NK cells and T lymphocytes. The TNF produced by macrophages is named as TNF-$\alpha$. TNF-$\alpha$ can kill and inhibit tumor cells, regulate the body's immune function, promote the killing of tumor cells by T cells and other killer cells, improve the phagocytic capacity of neutrophils, increase the production of superoxide anion, enhance the ADCC function, stimulate cell degranulation, and secrete myeloperoxidase. TNF-$\alpha$ can also inhibit viral replication, suppress viral protein synthesis, production and infectivity of viral particles, kill virus-infected cells, and promote the differentiation of myeloid leukemia cells into macrophages, such as promoting the differentiation of myeloid leukemia cell ML-1, monocytic leukemia cell U937 and promyelocytic leukemia cell HL60. TNF-$\alpha$ can promote the expression of MHC class I antigens on T cells, enhance the proliferation ability of IL-2-dependent thymocytes and T cells, promote the differentiation of lymphokine such as IL-2, CSF and IFN-$\gamma$, and enhance the proliferation of B cells stimulated by mitogen or foreign antigen and Ig secretion.

The disclosures of the various publications, patents and published patent specifications cited herein are incorporated herein by reference in their entirety.

The following clearly and completely describes the technical solutions of the present invention with reference to Examples of the present invention. Apparently, the described examples are merely some rather than all of the examples of the present invention. All other examples obtained by a person of ordinary skill in the art based on the examples of the

present invention without creative efforts shall fall within the scope of the present invention.

Example 1. Synthesis of Compound A2

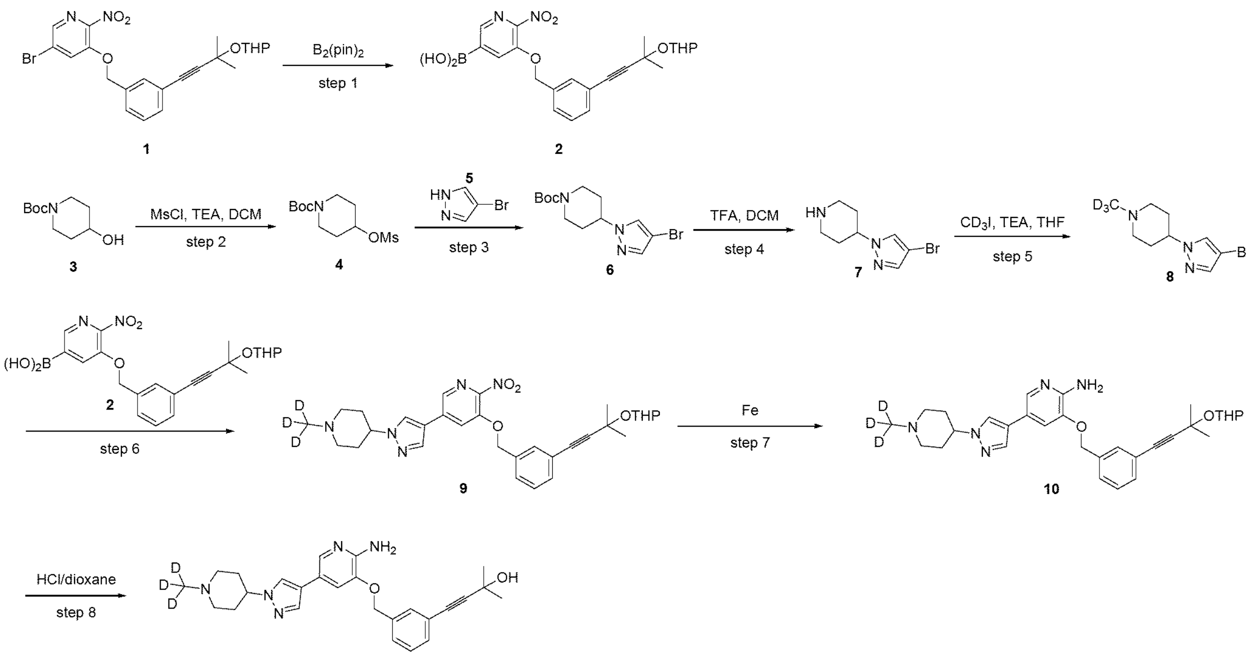


1 2 3 4 5 6 7 8 9 10

The experimental steps were as follows:

**Step 1:**

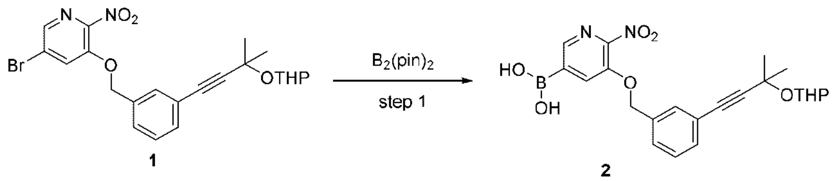


1 2

**1** (11.3 g, 23.7 mmol), bis(pinacolato)diboron (9.06 g, 35.6 mmol), bis(diphenylphosphino)ferrocene dichloropalladium (1.74 g, 2.37 mmol), potassium acetate (6.99 g, 71.3 mmol) and dimethyl sulfoxide (150 mL) were added to a 500 mL single-neck flask and reacted at 95°C for 16 hours in the presence of protective nitrogen. The reaction was quenched with water (300 mL). The obtained mixture was extracted with ethyl acetate (150 mL x 3), washed with saturated brine (150 mL x 2) and spin-dried to give the desired product as a black solid (13.0 g, crude product). LC-MS: 463 $[M+Na]^+$.

**Step 2:**

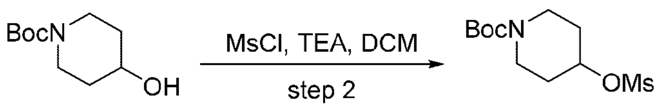


3 4

**3** (15 g, 74.6 mmol), triethylamine (22.7 g, 223.8 mmol) and dichloromethane (150 mL) were added to a 500 mL three-neck flask, followed by methanesulfonyl chloride (12.1 g, 111.9 mmol) in an ice bath, and reacted for 1 hour in an ice

bath. The reaction was quenched with water (300 mL). The obtained mixture was extracted with dichloromethane (100 mL x 3), washed with saturated brine, dried over anhydrous sodium sulfate, spin-dried and purified by column chromatography (petroleum ether:ethyl acetate = 40:1) to give the desired product as a yellow solid (20.7 g, crude product). LC-MS: 280 [M+H]+.

**Step 3:**

5

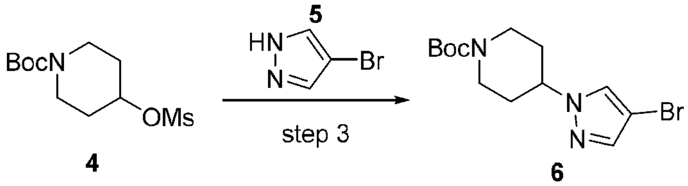


4 6

5 (7.27 g, 49.4 mmol) and DMF (200 mL) were added to a 500 mL single-neck flask, followed by 60% NaH (2.96 g, 74.1 mmol) in portions at 0°C, and reacted at room temperature for 1 hour. Then, 4 (20.7 g, 74.1 mmol) was added and reacted at 70°C for 16 h in the presence of protective nitrogen. The reaction was quenched with water (500 mL). The obtained mixture was extracted with ethyl acetate (200 mL x 3). The organic phase was washed with saturated brine (200 mL x 2), dried over anhydrous sodium sulfate, spin-dried and purified by column chromatography (petroleum ether:ethyl acetate = 30:1) to give the desired product as a white solid (9.8 g, yield: 61.2%). LC-MS: 330 [M+H]+.

**Step 4:**

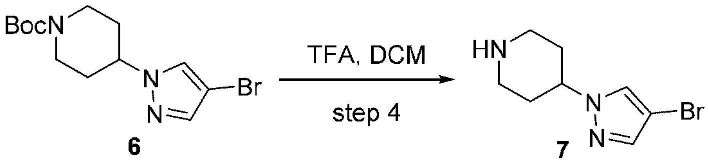


6 7

In a 500 mL single-neck flask, 6 (9.8 g, 35.8 mmol) was dissolved in DCM (80 mL), and TFA (16 mL) was added dropwise at 0°C. The system was reacted at room temperature for 16 h, concentrated at low temperature, and diluted with DCM (200 mL). The reaction was quenched with ice water. The obtained mixture was adjusted to pH = 10 with ammonia at 0°C and extracted with DCM (200 mL x 3). The organic phase was washed with saturated brine (200 mL x 2), dried over anhydrous sodium sulfate and spin-dried to give 6.59 g of the desired product as a white solid (yield: 96.6%). LC-MS: 230 $[M+H]^+$.

**Step 5:**

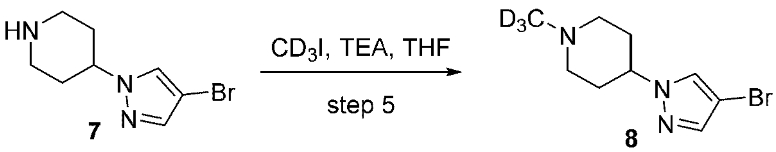


7 8

In a 250 mL three-neck flask, 7 (5.53 g, 24.0 mmol) and TEA (10 mL, 72.1 mmol) were dissolved in THF (80 mL) and reacted at room temperature for 1 hour. The reaction was cooled to 0°C in an ice bath, and $CD_3I$ (1.65 mL, 26.4 mmol) was added dropwise. The system was reacted at room temperature for 2 hours in the presence of protective nitrogen. The reaction was quenched with water. The obtained mixture was extracted with DCM (100 mL x 3). The organic phase was washed with saturated brine (100 mL x 2), dried over anhydrous sodium sulfate and spin-dried to give 3.1 g of the desired product as a yellow oil (yield: 52.2%). LC-MS: 248[M+H]+.

**Step 6:**

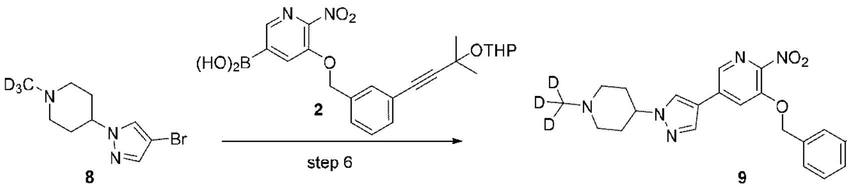


2 (7.13 g, 16.2 mmol), **8** (2.67 g, 10.8 mmol), xphosPdG II (850 mg, 1.08 mmol), xphos (515 mg, 1.08 mmol), potassium phosphate (4.58 g, 21.6 mmol) and DMF/$H_2O$ (150 mL/30 mL) were added to a 500 mL single-neck flask and reacted at 95°C for 2.5 hours in the presence of protective nitrogen. The reaction was quenched with water (300 mL). The obtained mixture was extracted with ethyl acetate (150 mL x 3), washed with saturated brine (150 mL x 2), dried over anhydrous sodium sulfate, spin-dried and purified by Flash to give the desired product as a black oil (3.3 g, crude product). LC-MS: 563[M+H]$^+$.

**Step 7:**

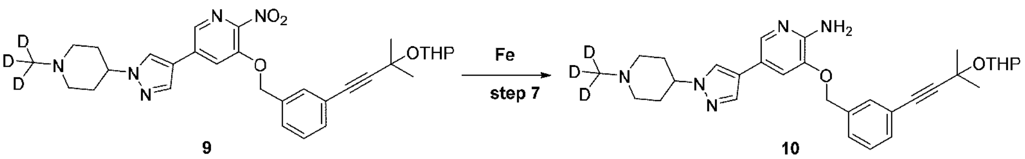


**9** (3.3 g, 7.14 mmol), Fe (2.0 g, 35.7 mmol), ammonium chloride (1.93 g, 35.7 mmol), ethanol (40 mL) and water (8 mL) were added to a 250 mL three-neck flask and reacted at 85°C for 2.5 hours. The obtained mixture was filtered by suction, and the filtrate was concentrated to give 3.5 g of the crude desired product as a black solid. LC-MS: 533[M+H]+.

**Step 8:**

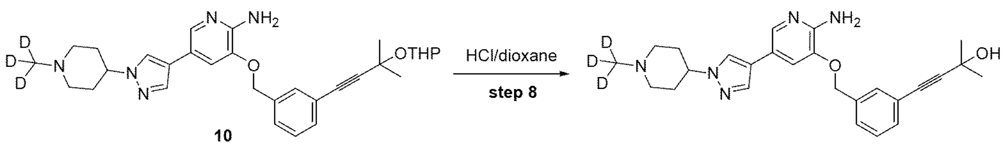


In a 250 mL three-neck flask, **10** (3.5 g, 6.55 mmol) was dissolved in tetrahydrofuran (40 mL), and HCl/Dioxane (8 mL) was added dropwise at 0°C. The system was reacted at room temperature for 1 hour, concentrated at low temperature, and diluted with DCM (100 mL). The reaction was quenched with ice water. The obtained mixture was adjusted to pH = 10 with ammonia at 0°C and extracted with DCM (100 mL x 3). The organic phase was washed with saturated brine (100 mL x 2), dried over anhydrous sodium sulfate, spin-dried and purified by Prep-HPLC to give 450 mg of the desired product as a white solid (yield: 15.3%). LC-MS: 449 [M+H]+, 1H NMR (400 MHz, MeOD) δ 8.38 (s, 1H), 8.01 (s, 1H), 7.81 (s, 1H), 7.73 (d, J = 1.5 Hz, 1H), 7.54 (s, 1H), 7.48 (td, J = 4.7, 1.7 Hz, 1H), 7.38 (s, 2H), 5.21 (s, 2H), 4.51 (t, J = 6.9 Hz, 1H), 3.59 (d, J = 12.6 Hz, 2H), 3.19 (s, 2H), 2.37 - 2.32 (m, 4H), 1.56 (s, 6H).

Example 2. Synthesis of Compound B2

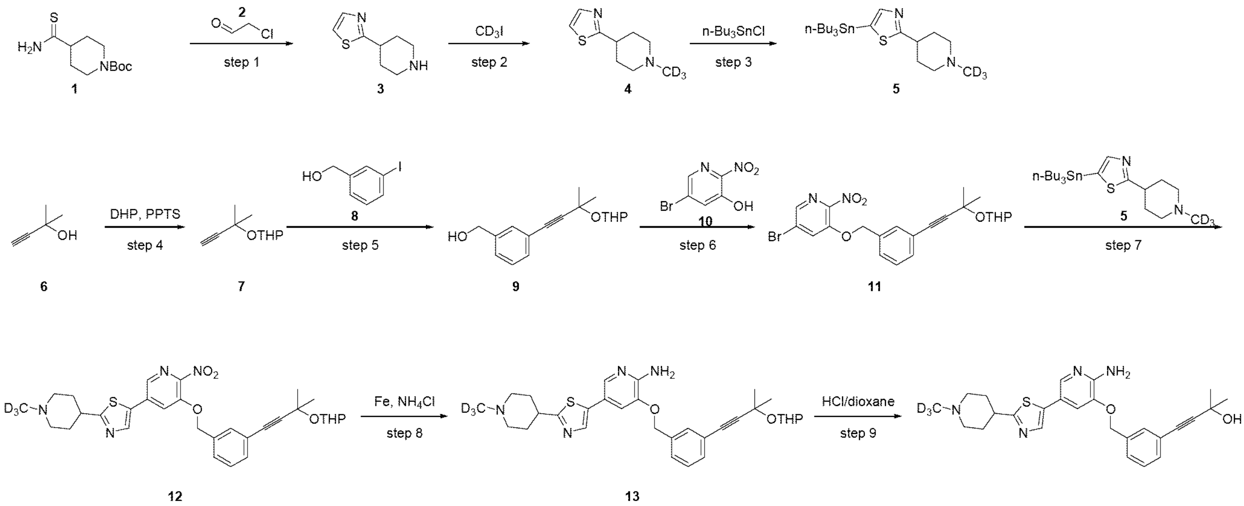


The experimental steps were as follows:

**Step 1:**

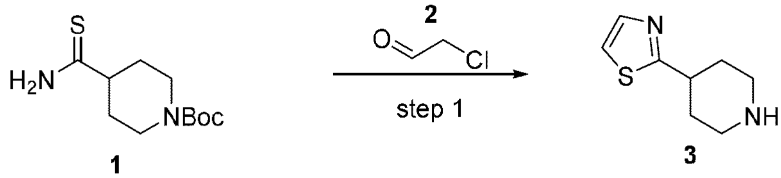


**1** (25.0 g, 102 mmol), aqueous chloroacetaldehyde solution (12.0 g, 154 mmol) and acetone (500 mL) were added to a 2000 mL single-neck flask and reacted at 50°C for 16 hours. The obtained mixture was spin-dried and purified by column chromatography (MeOH in DCM, from 0% to 10%, v/v) to give the desired product as a yellow oil (9.20 g, yield: 53.4%). LC-MS: 169 $[M+H]^+$.

**Step 2:**

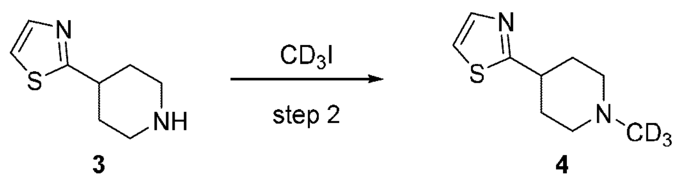


**3** (4.00 g, 23.8 mmol), triethylamine (6.01 g, 59.5 mmol) and tetrahydrofuran (200 mL) were added to a 1000 mL single-neck flask. Deuterated iodomethane (3.62 g, 25.0 mmol) was added at room temperature and stirred at room temperature for 2 hours. The reaction was quenched with water (250 mL). The obtained mixture was spin-dried, extracted with ethyl acetate (150 mL x 3), dried over anhydrous sodium sulfate, spin-dried and purified by column chromatography (MeOH in DCM, from 0% to 10%, v/v) to give the desired product as a yellow solid (2.70 g, yield: 61.3%). LC-MS: 186 [M+H] +.

**Step 3:**

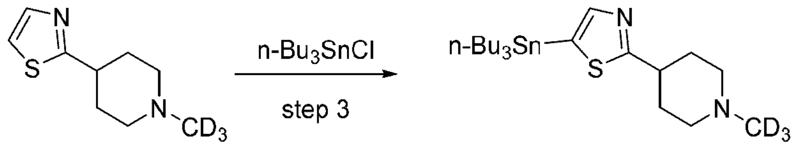


4 5

**4** (2.70 g, 14.6 mmol) and tetrahydrofuran (100 mL) were added to a 500 mL three-neck flask, followed by dropwise addition of n-butyllithium (2.4 M in tetrahydrofuran, 7.30 mL, 17.5 mmol) at -78°C in the presence of protective nitrogen, and stirring was continued for 1 h while maintaining the temperature. Then, tri-n-butyltin chloride (7.14 g, 21.9 mmol) was added dropwise, and the reaction was continued at -78°C for 1 h. Upon completion of the reaction, the reaction was quenched with saturated aqueous ammonium chloride solution (100 mL). The obtained mixture was extracted with ethyl acetate (120 mL x 3). The organic layer was washed with saturated brine, dried over anhydrous sodium sulfate, filtered by suction and concentrated under reduced pressure to give the desired product as a yellow oil, which was directly used in the next step. (7.05 g, crude product). LC-MS: 476 [M+H]+.

**Step 4:**

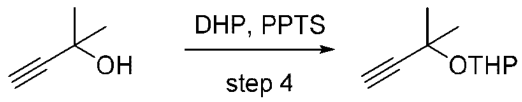


**6** **7**

**6** (59.4 g, 707 mmol), tetrahydrofuran (1.5 L), DHP (68.4 g, 813 mmol) and PPTS (3.55 g, 14.1 mmol) were added to a 3 L single-neck flask and reacted at room temperature for 16 hours. The reaction was quenched with saturated sodium bicarbonate (1000 mL * 3). The obtained mixture was washed with saturated brine (500 mL), dried over anhydrous sodium sulfate and spin-dried to give the desired product as a colorless oil (120 g, crude product), which was directly used in the next step. LC-MS: 169 [M+H]+.

**Step 5:**

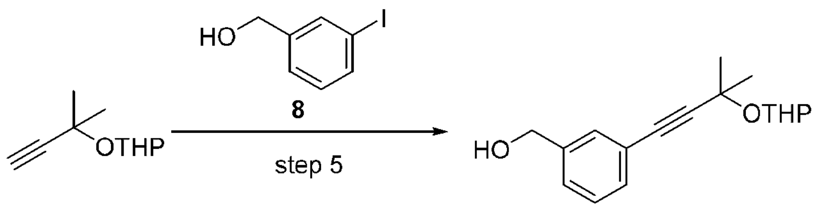


**7** **9**

**7** (35.8 g, 213.15 mmol), **8** (47.5 g, 203.00 mmol), bis(triphenylphosphine)palladium(II) chloride (1.43 g, 2.03 mmol), cuprous iodide (1.93 g, 10.16 mmol), triethylamine (40.01 g, 406.00 mmol) and anhydrous dichloromethane (1 L) were added to a 3 L single-neck flask and reacted at room temperature for 16 hours in the presence of protective nitrogen. The reaction mixture was washed with saturated ammonium chloride (1000 M1 * 3) and saturated brine (500 mL), dried over anhydrous sodium sulfate and spin-dried to give the desired compound as a yellow oil (59.4 g, crude product), which was directly used in the next step. LC-MS: 275 [M+H]+.

**Step 6:**

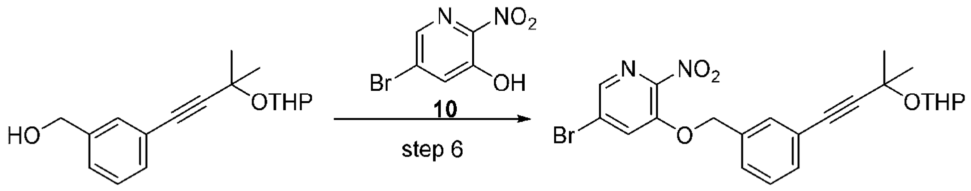


9 11

**9** (50.3 g, 184 mmol), tetrahydrofuran (500 mL), **10** (40.1 g, 184 mmol) and triphenylphosphine (72.3 g, 276 mmol) were added to a 2000 mL three-neck flask. Diethyl azodicarboxylate (55.8 g, 276 mmol) was then added with stirring at room temperature in the presence of protective nitrogen and reacted at room temperature for 16 hours. The obtained mixture was spin-dried and purified by column chromatography (petroleum ether:ethyl acetate = 10:1) to give the desired product as a yellow solid (70.2 g, yield: 80.6%). LC-MS: 476 $[M+H]^+$.

**Step 7:**

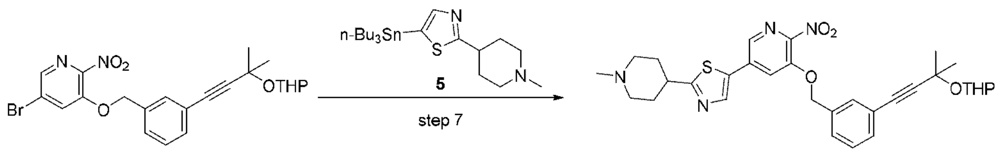


11 12

**11** (5.05 g, 10.7 mmol), 5 (9.15 g, 19.3 mmol), bis(triphenylphosphine)palladium(II) chloride (376 mg, 0.535 mmol), cuprous iodide (305 mg, 1.61 mmol) and 1,4-dioxane (200 mL) were added to a 1000 mL single-neck flask and stirred at 90°C for 5 hours in the presence of protective nitrogen. The obtained mixture was diluted with ethyl acetate (400 mL), washed with saturated aqueous ammonium chloride solution (300 mL x 3), spin-dried and purified by column chromatography (MeOH in DCM, from 0% to 5%, v/v) to give the desired product as a yellow solid (2.50 g, yield: 40.5%). LC-MS: 580 $[M+H]^+$.

**Step 8:**

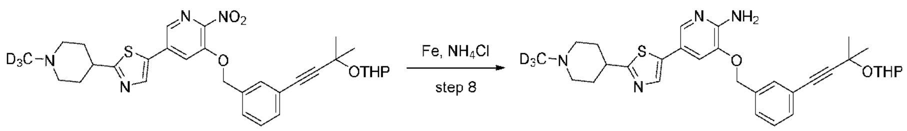


12 13

12 (2.50 g, 4.32 mmol), reduced iron powder (1.21 g, 21.6 mmol), ammonium chloride (1.14 g, 21.6 mmol), ethanol (100 mL) and water (20 mL) were added to a 500 ml single-neck flask and reacted at 80°C for 2 hours. The obtained mixture was diluted with dichloromethane (100 mL), filtered by suction, spin-dried and purified by column chromatography (MeOH in DCM, from 0% to 12%, v/v) to give the desired product as a yellow solid (1.40 mg, 59.1%). LC-MS: 550 [M+H]+.

**Step 9:**

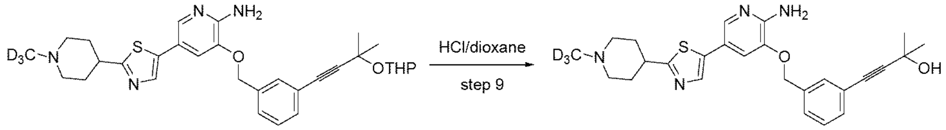


13

13 (1.02 g, 1.86 mmol) and tetrahydrofuran (25 mL) were added to a 250 ml single-neck flask and stirred in an ice bath. Then, 4M/L hydrochloric acid gas in 1,4-dioxane (5 mL) was added dropwise and reacted at room temperature for 20 minutes. The obtained mixture was spin-dried, dissolved in dichloromethane, adjusted pH to 9 with ammonia, spin-dried with dichloromethane (30 mL x 3) and purified by high-pressure liquid chromatography to give the desired product as a light yellow solid (350 mg, 40.5%). LC-MS: 466 [M+H]+. $^1$H NMR (400 MHz, DMSO) δ 7.88 (s, 1H), 7.77 (d, *J* = 1.8 Hz, 1H), 7.54 (d, *J* = 5.4 Hz, 2H), 7.42 - 7.37 (m, 1H), 7.34 (dd, *J* = 5.4, 1.6 Hz, 2H), 6.10 (s, 2H), 5.50 (s, 1H), 5.22 (s, 2H), 2.96 - 2.78 (m, 3H), 2.02 (dd, *J* = 16.9, 6.7 Hz, 4H), 1.72 (qd, *J* = 12.5, 3.6 Hz, 2H), 1.47 (s, 6H).

Example 3. Synthesis of Compound C

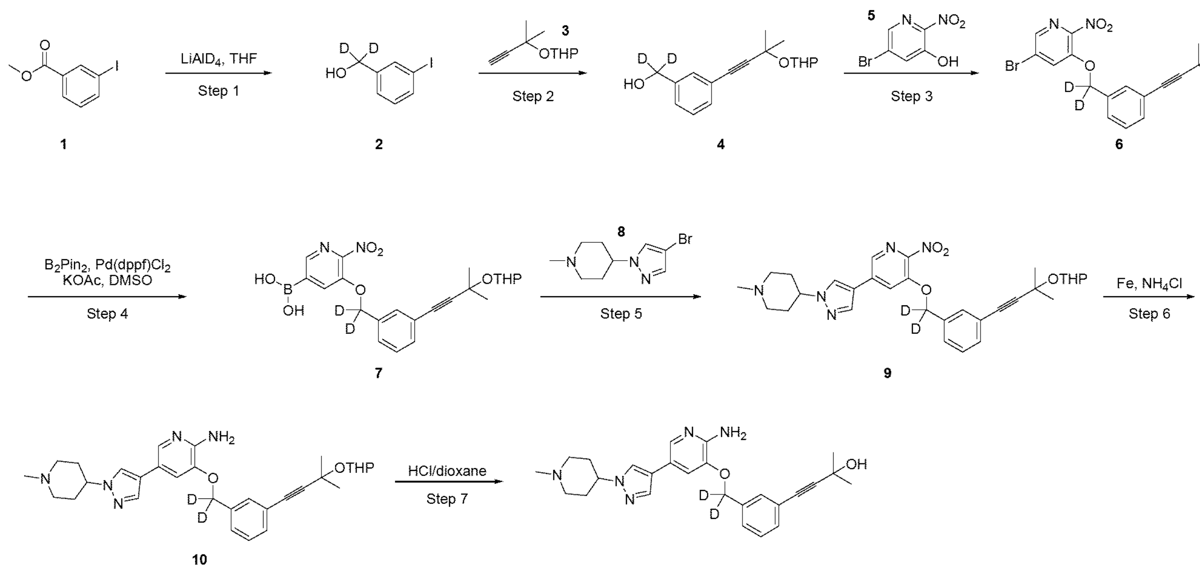


The experimental steps were as follows:

**Step 1:**

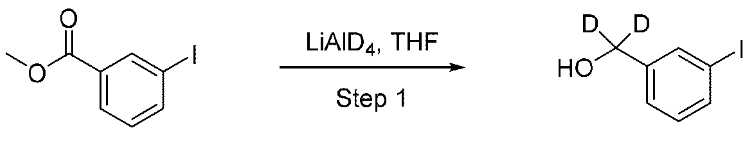


1 2

1 (7.86 g, 30.0 mmol) and tetrahydrofuran (500 mL) were added to a 2000 mL single-neck flask, followed by lithium aluminum deuteride (3.15 g, 75.0 mmol) in portions at 0°C, and reacted at 0°C for 1 hour. The reaction was quenched with acetic acid (50 mL). The obtained mixture was extracted with ethyl acetate (500 mL x 3), washed with saturated brine (500 mL x 3) and spin-dried to give the desired product as a yellow solid (3.50 g, crude product). LC-MS: 259 [M+Na]$^+$.

**Step 2:**

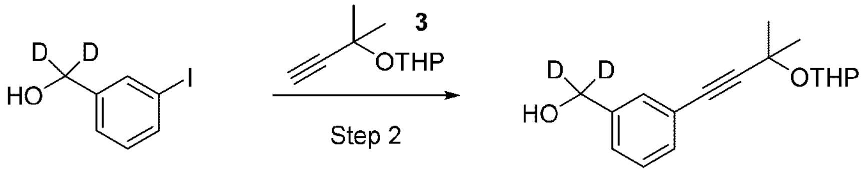


2 4

**3** (3.50 g, 14.8 mmol), bis(triphenylphosphine)palladium(II) chloride (519 mg, 0.740 mmol), cuprous iodide (281 mg, 1.48 mmol), triethylamine (4.48 g, 44.4 mmol) and dichloromethane (60 mL) were added to a 250 mL three-neck round bottom flask, followed by 3 (2.50 g, 14.8 mmol) at room temperature in the presence of protective nitrogen, and reacted at room temperature for 16 hours. The obtained mixture was diluted with dichloromethane (400 mL), washed with saturated aqueous ammonium chloride solution (300 mL x 3), spin-dried and purified by column chromatography (petroleum ether:ethyl acetate = 4:1) to give the desired product as a yellow solid (1.03 g, yield: 25.2%). LC-MS: 299 $[M+Na]^+$.

**Step 3:**

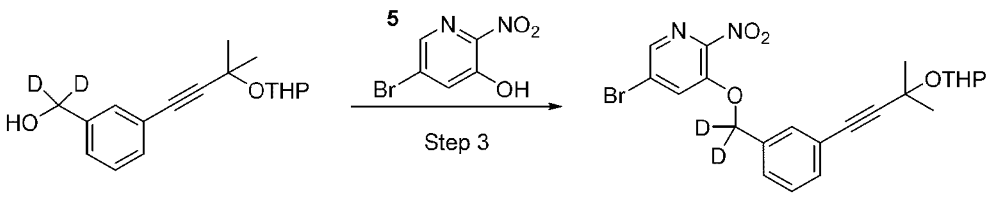


4 6

**4** (1.03 g, 3.73 mmol), tetrahydrofuran (30 mL), **5** (854 mg, 3.92 mmol) and triphenylphosphine (1.47 g, 5.60 mmol) were added to a 250 mL three-neck flask. Diethylpropyl azodicarboxylate (1.13 g, 5.60 mmol) was added dropwise at 0°C in the presence of protective nitrogen. The system was reacted at room temperature for 16 hours in the presence of protective nitrogen. The obtained mixture was spin-dried and purified by column chromatography (petroleum ether:ethyl acetate = 10:1) to give the desired product as a yellow solid (1.30 g, yield: 73.2%). LC-MS: 499 [M+Na]+.

**Step 4:**

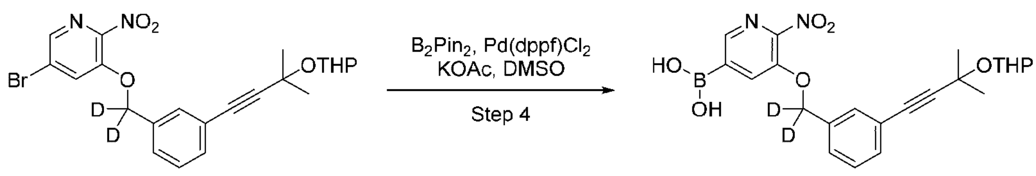


6 7

**6** (300 mg, 0.630 mmol), bis(pinacolato)diboron (240 mg, 0.945 mmol), Pd(dppf)$Cl_2$ (23.5 mg, 0.0315 mmol), potassium acetate (154 mg, 1.58 mmol) and dimethyl sulfoxide (10 mL) were added to a 100 mL single-neck flask and reacted at 90°C for 16 hours under a nitrogen atmosphere. The reaction was quenched with water (80 mL). The obtained mixture was extracted with ethyl acetate (50 mL x 3), washed with saturated brine (50 mL x 2), dried over anhydrous sodium sulfate and spin-dried to give the desired product as a brown oil (400 mg, crude product). LC-MS: 443 [M+H]+.

**Step 5:**

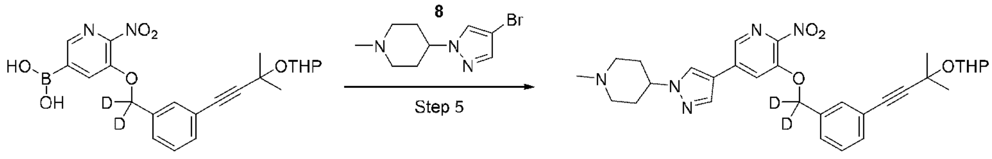


7 9

**7** (400 mg, 0.905 mmol), **8** (200 mg, 0.905 mmol), XPhosPdG2 (35.6 mg, 0.0453 mmol), XPhos (43.2 mg, 0.0905 mmol), potassium phosphate (384 mg, 1.81 mmol), DMF (10 mL) and water (2 mL) were added to a 100 mL single-neck flask and reacted at 90°C for 2 hours in the presence of protective nitrogen. The reaction was quenched with water (80 mL). The obtained mixture was extracted with ethyl acetate (50 mL x 3), washed with saturated brine (50 mL x 2), dried over anhydrous sodium sulfate, spin-dried and purified by column chromatography (dichloromethane:methanol = 18:1) to give the desired product as a yellow solid (250 mg, yield: 49.2%). LC-MS: 562 $[M+H]^+$.

**Step 6:**

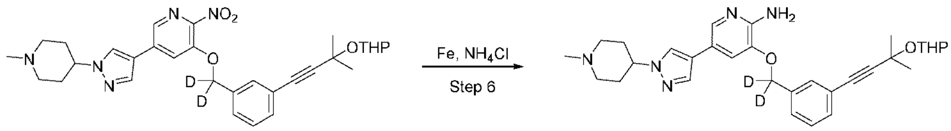


9 10

**9** (250 mg, 0.446 mmol), reduced iron powder (125 mg, 2.23 mmol), ammonium chloride (118 mg, 2.23 mmol), ethanol (10 mL) and water (2 mL) were added to a 100 ml single-neck flask and reacted at 80°C for 2 hours. The obtained mixture was diluted with dichloromethane (50 mL), filtered by suction, spin-dried and purified by column chromatography (dichloromethane:methanol = 10:1) to give the desired product as a yellow solid (220 mg, 93.0%). LC-MS: 532 [M+H]+.

**Step 7:**

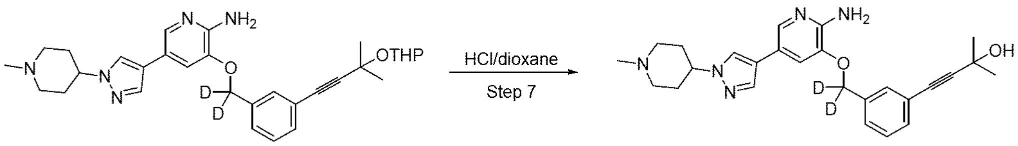


10

**10** (220 mg, 0.414 mmol) and tetrahydrofuran (10 mL) were added to a 250 ml single-neck flask and stirred in an ice bath. Then, 4M/L hydrochloric acid in 1,4-dioxane (3 mL) was added dropwise and reacted at room temperature for 20 minutes. The obtained mixture was spin-dried, dissolved in dichloromethane, adjusted pH to 9 with ammonia, spin-dried with dichloromethane (30 mL x 3) and purified by high-pressure liquid chromatography to give the desired product as a light yellow solid (85.0 mg, 45.9%). LC-MS: 448 [M+H]+. $^1$H NMR (400 MHz, DMSO) δ 8.10 (s, 1H), 7.79 (d, *J* = 1.8 Hz, 1H), 7.75 (s, 1H), 7.58 - 7.49 (m, 2H), 7.44 - 7.30 (m, 3H), 5.66 (s, 2H), 5.49 (s, 1H), 4.14 - 4.00 (m, 1H), 2.86 (d, *J* = 11.5 Hz, 2H), 2.21 (s, 3H), 1.99 (d, *J* = 3.1 Hz, 6H), 1.46 (s, 6H).

Example 4. Synthesis of Compound D

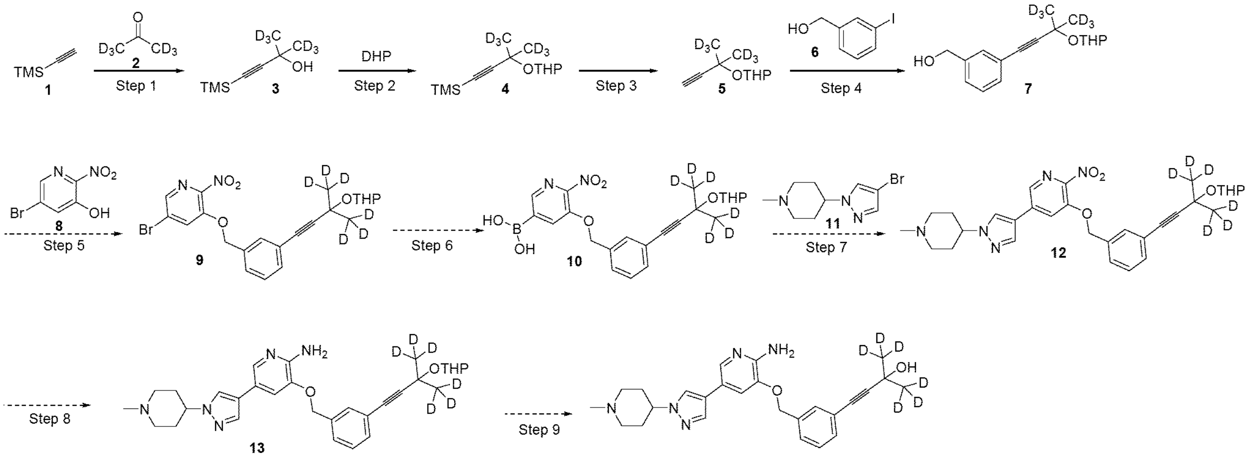


The experimental steps were as follows:

**Step 1:**

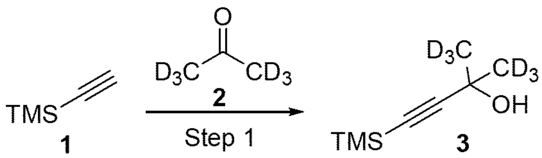


**1** (3.0 g, 30.61 mmol) and tetrahydrofuran (30 mL) were added to a 100 mL three-neck flask, followed by dropwise addition of n-butyllithium (2.4 M in tetrahydrofuran, 12.75 mL, 30.61 mmol) at -78°C in the presence of protective nitrogen, and stirring was continued for 1 h while maintaining the temperature. Then, **2** (2.0 g, 30.61 mmol) was added dropwise. The reaction was continued at -78°C for 1 h and warmed to room temperature for another 2 h. Upon completion of the reaction, the reaction was quenched with saturated aqueous ammonium chloride solution (30 mL). The obtained mixture was extracted with ethyl acetate (100 mL x 3). The organic layer was washed with saturated brine, dried over anhydrous sodium sulfate, filtered by suction and concentrated under reduced pressure to give the desired product as a yellow oil, which was directly used in the next step. (6.1 g, crude product). LC-MS: 163 $[M+H]^+$.

**Step 2:**

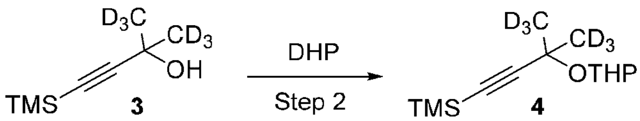


**3** (6.1 g, 37.65 mmol), tetrahydrofuran (50 mL), DHP (4.76 g, 56.63 mmol) and PPTS (158.8 mg, 0.63 mmol) were added to a 250 mL single-neck flask and reacted at room temperature for 16 hours. The reaction was quenched with saturated sodium bicarbonate (100 mL x 3). The obtained mixture was washed with saturated brine (100 mL), dried over anhydrous sodium sulfate and spin-dried to give the desired product as a colorless oil (8.5 g, crude product), which was directly used in the next step. LC-MS: 247 $[M+H]^+$.

**Step 3:**

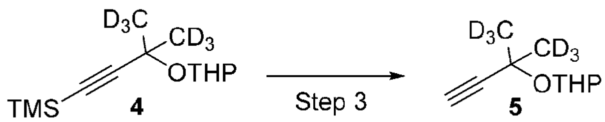


**4** (8.5 g, 34.55 mmol), methanol and dichloromethane (10 mL/10 mL) and potassium carbonate (7.15 g, 51.83 mmol) were added to a 250 mL single-neck flask and reacted at room temperature for 3 hours. The obtained mixture was filtered by suction. The filtrate was concentrated and spin-dried to give the desired product as a colorless oil (6.3 g, crude product), which was directly used in the next step. LC-MS: 175 $[M+H]^+$.

**Step 4:**

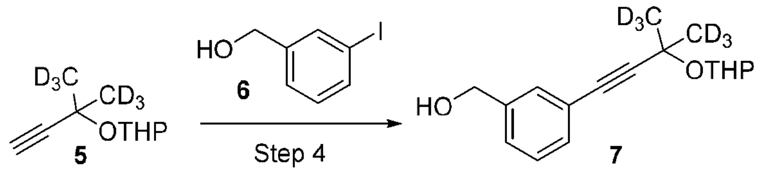


**5** (6.3 g, 36.21 mmol), **6** (8.05 g, 34.40 mmol), bis(triphenylphosphine)palladium(II) chloride (238 mg, 0.34 mmol), cuprous iodide (322 mg, 1.69 mmol), triethylamine (6.95 g, 68.8 mmol) and anhydrous dichloromethane (80 mL) were added to a 250 mL single-neck flask and reacted at room temperature for 16 hours in the presence of protective nitrogen. The reaction mixture was washed with saturated ammonium chloride (200 mL x 3) and saturated brine (100 mL), dried over anhydrous sodium sulfate, spin-dried and purified by column chromatography (petroleum ether:ethyl acetate = 10:1) to give the desired compound as a yellow oil (3.2 g). LC-MS: 281 $[M+H]^+$.

**Step 5:**

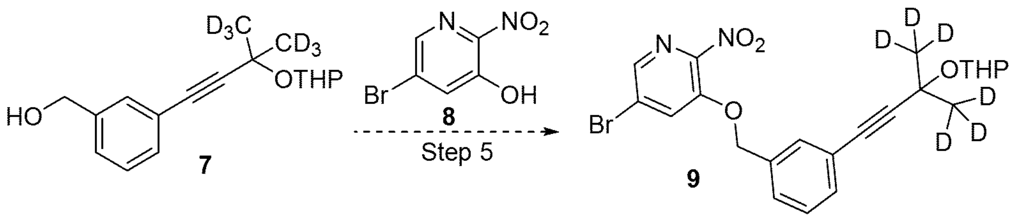


7 (1.5 g, 5.36 mmol), **8** (1.752 g, 8.04 mmol), triphenylphosphine (2.106 g, 8.04 mmol) and anhydrous tetrahydrofuran (20 mL) were added to a 100 mL three-neck flask. Diisopropyl azodicarboxylate (1.624 g, 8.04 mmol) was then added with stirring at room temperature in the presence of protective nitrogen and reacted for 16 hours at room temperature. The obtained mixture was spin-dried and purified by column chromatography (petroleum ether:ethyl acetate = 4:1) to give the desired product as a yellow solid (450 mg, yield: 17.50%). LC-MS: 481 $[M+H]^+$.

**Step 6:**

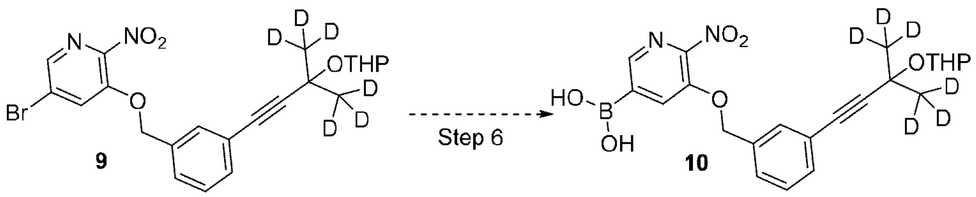


**9** (450 mg, 0.94 mmol), bis(pinacolato)diboron (359 mg, 1.41 mmol), bis(diphenylphosphino)ferrocene dichloropalladium (35 mg, 0.047 mmol), potassium acetate (276 mg, 2.82 mmol) and dimethyl sulfoxide (10 mL) were added to a 100 mL single-neck flask and reacted at 90°C for 16 hours in the presence of protective nitrogen. The reaction was quenched with water (30 mL). The obtained mixture was extracted with ethyl acetate (50 mL x 3), washed with saturated brine (100 mL x 2) and spin-dried to give the desired product as a black solid (500 mg, crude product). LC-MS: 447[M+H]+.

**Step 7:**

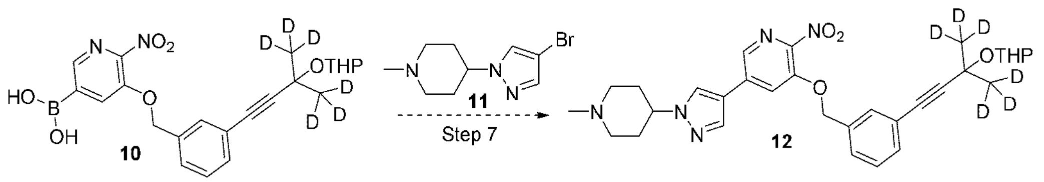


**10** (363 mg, 0.81 mmol), **11** (178 g, 0.73 mmol), chloro(2-dicyclohexylphosphino-2',4',6'-triisopropyl-1,1'-biphenyl)[2-(2'-amino-1,1'-biphenyl)]p alladium(II) (59 mg, 0.081 mmol), 2-dicyclohexylphospho-2,4,6-triisopropylbiphenyl (39 mg, 0.082 mmol) and potassium phosphate (343 mg, 1.62 mmol), DMF (10 mL) and $H_2O$ (2 mL) were added to a 100 mL single-neck flask and stirred at 95°C for 1.5 h in the presence of protective nitrogen. The reaction was quenched with water (30 mL). The obtained mixture was extracted with ethyl acetate (100 mL x 3). The organic phases were combined, washed with saturated brine (100 mL x 3), dried over anhydrous sodium sulfate, spin-dried and purified by TCL (dichloromethane:methanol = 15:1) to give the desired product as a brown liquid (266 mg, yield: 57.9%). LC-MS: 566 [M+H]+.

**Step 8:**

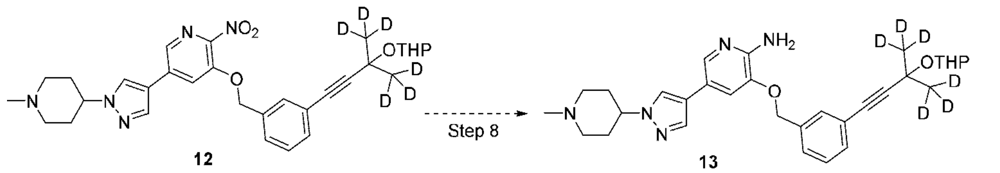


**12** (266 mg, 0.47 mmol), iron powder (201 mg, 3.77 mmol), ammonium chloride (203 mg, 3.77 mmol), ethanol (10 mL) and water (2 mL) were added to a 100 ml single-neck flask and reacted at 80°C for 1 h. The obtained mixture was cooled to room temperature and filtered by suction. The filtrate was concentrated and purified by TLC (methanol:dichloromethane = 1:10, v/v) to give the desired product as a yellow solid (95 mg, 37.6%). LC-MS: 536 [M+H]+.

Step 9:

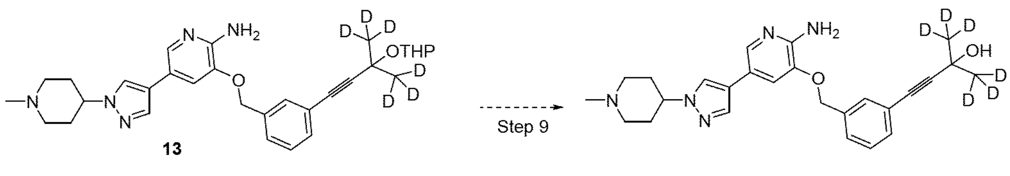


13 (95 mg, 0.17 mmol) and tetrahydrofuran (5 mL) were added to a 50 ml single-neck flask and stirred in an ice bath. Then, 4M/L hydrochloric acid in 1,4-dioxane (0.01 mL, 0.34 mmol) was added dropwise and reacted at room temperature for 1 h. The obtained mixture was spin-dried at 0°C and purified by high-pressure liquid chromatography to give the desired product as a black solid (15 mg, 18.7%). LC-MS: 452 [M+H]+. $^1H$ NMR (400 MHz, DMSO) δ 8.20 (s, 1H), 8.10 (s, 1H), 7.80 (d, *J*= 1.7 Hz, 1H), 7.76 (s, 1H), 7.52 (d, *J*= 1.4 Hz, 2H), 7.40 (s, 1H), 7.35 - 7.32 (m, 2H), 5.67 (s, 2H), 5.18 (s, 2H), 4.09 (dd, *J*= 10.4, 5.1 Hz, 1H), 2.89 (d, *J*= 11.1 Hz, 2H), 2.24 (s, 3H), 2.10 (d, *J*= 11.3 Hz, 2H), 2.00 (d, *J* = 3.3 Hz, 4H).

Example 5: Compound A2 free base crystalline form A

Compound A2 prepared in Example 1 was passed through a silica gel column to give 1.6 g of a crude product (about 80% purity). The crude product was purified by preparative high-performance liquid chromatography, concentrated to 100 mL, adjusted to pH = 9 with sodium bicarbonate solution, and extracted three times with dichloromethane (100 mL). The organic phase was washed once with saturated sodium chloride solution (80 mL), dried over sodium sulfate and spin-dried to give 570 mg of a light yellow solid (97% purity). The solid was slurried with 50 mL of petroleum ether/ethyl acetate (3:1) and filtered to give 460 mg of an off-white solid (> 99% purity).

Repeat preparation:

(1) 230.9 mg of the above free base sample was weighed into a 20 mL glass bottle, and 1mL of IPAc was added to give a clear solution;
(2) After stirring (1000 rpm) at room temperature for about 5 minutes, a large amount of solid was precipitated, and 1mL of IPAc was further added;
(3) After stirring in suspension at room temperature for about 1 day, the mixture was centrifuged (10000 rpm, 2 min) to give a solid; and
(4) The solid was vacuum dried at room temperature for 3 hours, and a total of 195.4 mg of sample was collected (yield: 84.6%).

The XRPD pattern of the above sample is shown in FIG. 1, and the XRPD test parameters and the data table of results are shown in Tables 1 and 2, respectively, indicating that the sample is crystalline, named as free crystalline form A, and can be prepared repeatedly. The entire list of peaks or corresponding d-values in Table 2, or a subset thereof, and an XRPD pattern substantially similar to FIG. 1 can be sufficient to characterize the crystalline form.
The TGA/DSC pattern of Compound A2 free base crystalline form A is shown in FIG. 2, and TGA and DSC test parameters are shown in Table 3. In FIG. 2, TGA results showed a weight loss of 1.3% upon heating from room temperature to 170°C; DSC results showed a sharp endothermic peak at 168.8°C (starting temperature).
The $^1$H NMR pattern of Compound A2 free base crystalline form A is shown in FIG. 3. The $^1$H NMR results showed that the molar ratio of residual solvent IPAc to free base crystalline form A was 0.01:1 (corresponding to a TGA weight loss of 0.2%).
The HPLC pattern of Compound A2 free base crystalline form A is shown in FIG. 4, and the results are shown in Table 4.
The Compound A2 free base crystalline form A is presumed to be anhydrous.

Table 1. XRPD test parameters.

| Instrument model | X'Pert$^3$ |
|---|---|
| X-ray | Cu, Kα; Kα1(Å): 1.540598<br>Kα2(Å): 1.544426<br>Intensity ratio Kα2/Kα1: 0.50 |
| X-ray tube setting | 45 kV, 40 mA |
| Divergence slit | 1/8° |
| Scan mode | continuous |
| Scan range (°2TH) | 3° to 40° |
| Scan step (°2TH) | 0.0263 |
| Scan time per step (s) | 46.665 |
| Scan time (s) | 5 min 04 s |

Table 2. XRPD data table of Compound A2 free base crystalline form A

| Diffraction angle (°2θ) | d-value (Å) | Relative intensity (%) |
|---|---|---|
| 8.056970 | 10.97382 | 15.13 |

(continued)

| Diffraction angle (°2θ) | d-value (Å) | Relative intensity (%) |
|---|---|---|
| 9.692430 | 9.12550 | 5.99 |
| 12.171430 | 7.27188 | 16.70 |
| 13.057630 | 6.78028 | 32.80 |
| 15.305380 | 5.78921 | 16.44 |
| 16.324030 | 5.43018 | 33.09 |
| 17.530630 | 5.05905 | 32.94 |
| 18.025570 | 4.92125 | 16.88 |
| 19.336490 | 4.59047 | 16.94 |
| 19.450710 | 4.56377 | 17.83 |
| 20.519450 | 4.32842 | 2.47 |
| 21.306330 | 4.17031 | 18.82 |
| 21.588990 | 4.11634 | 16.08 |
| 23.778260 | 3.74208 | 100.00 |
| 27.036990 | 3.29800 | 4.69 |
| 27.711080 | 3.21928 | 3.95 |
| 28.487430 | 3.13329 | 2.77 |
| 31.912120 | 2.80443 | 3.52 |

Table 3. TGA and DSC test parameters

| Parameter | TGA | DSC |
|---|---|---|
| Method | Linear temperature increase | Linear temperature increase |
| Sample plate | Aluminum plate, open | Aluminum plate, gland |
| Temperature range | Room temperature-350°C | 25-350°C |
| Heating rate | 10°C/min | 10°C/min |
| Protective gas | Nitrogen | Nitrogen |

Table 4. HPLC results of Compound A2 free base crystalline form A

| Peak | RRT | Area (%) |
|---|---|---|
| 1 | 0.95 | 0.42 |
| **2** | **1.00** | **99.26** |
| 3 | 1.05 | 0.22 |
| 4 | 1.06 | 0.10 |

Solid state stability of the free base crystalline form A

The XRPD pattern of the starting sample of the above free base crystalline form A and after 1 day of closed placement at 60°C is shown in FIG. 5, and the HPLC results are shown in Table 5.

Table 5. HPLC results of Compound A2 free base crystalline form A

| Peak | RRT | Area (%) | |
|---|---|---|---|
| | | Starting sample | 60°C / 1 day |
| 1 | 0.95 | 0.42 | 0.41 |
| 2 | 1.00 | 99.26 | 99.33 |
| 3 | 1.05 | 0.22 | 0.17 |
| 4 | 1.06 | 0.10 | 0.09 |

Example 6: Compound A2 free base crystalline form B

(1) 9.9 of the free base sample (as described in Example 5) was weighed into a 20 mL glass bottle, and 2 mL of EtOAc was added to dissolve the sample. The obtained mixture was filtered with a 0.45 μm PTFE filter to give a clear API solution;
(2) Toluene as an anti-solvent was added dropwise to the API solution while stirring magnetically (~ 1000 rpm), totaling 10 mL of toluene;
(3) The obtained clear solution was stirred at room temperature for ~ 2 hours, transferred to 5°C and stirred for about 15 hours, still remaining a clear solution;
(4) The obtained clear solution was transferred to -20°C and stirred for about 6 hours, still remaining a clear solution; and
(5) The clear solution was transferred to room temperature and volatilized for 8 days to give a solid.

The XRPD pattern of the above sample is shown in FIG. 6, the XRPD test parameters are shown in Table 1 above, and the data table of results is shown in Table 6, indicating that the sample is crystalline, named as free crystalline form B. The entire list of peaks or corresponding d-values in Table 6, or a subset thereof, and an XRPD pattern substantially similar to FIG. 5 can be sufficient to characterize the crystalline form.

Table 6. XRPD data table of Compound A2 free base crystalline form B

| Diffraction angle (°2θ) | d-value (Å) | Relative intensity (%) |
|---|---|---|
| 5.683550 | 15.54998 | 87.55 |
| 7.085257 | 12.47650 | 19.72 |
| 8.848269 | 9.99413 | 46.82 |
| 11.330050 | 7.80994 | 100.00 |
| 13.025680 | 6.79684 | 3.84 |
| 14.152280 | 6.25821 | 29.61 |
| 14.979470 | 5.91443 | 6.30 |
| 17.004010 | 5.21453 | 36.50 |
| 17.683430 | 5.01568 | 10.18 |
| 18.091520 | 4.90345 | 30.51 |
| 18.752700 | 4.73203 | 18.19 |
| 21.597990 | 4.11465 | 8.09 |
| 22.297300 | 3.98716 | 3.65 |
| 22.731990 | 3.91189 | 96.70 |
| 23.470920 | 3.79038 | 94.14 |
| 24.190420 | 3.67924 | 4.18 |
| 26.220830 | 3.39877 | 7.82 |

The TGA/DSC pattern of Compound A2 free base crystalline form B is shown in FIG. 7, and TGA and DSC test parameters are shown in Table 3 above. TGA results showed that the sample had a weight loss of 13.2% from room temperature to 70°C and a weight loss of 8.5% upon continued heating to 170°C. DSC results showed that the sample had four endothermic peaks at 59.5°C, 95.6°C, 150.8°C and 160.9°C (peak temperature).

The $^1H$ NMR pattern of Compound A2 free base crystalline form B is shown in FIG. 8. The $^1H$ NMR results showed that the molar ratio of residual solvent EtOAc to free base was 0.8:1 (corresponding to a TGA weight loss of 18.1%).

The XRPD pattern of Compound A2 free base crystalline form B before and after being placed at room temperature is shown in FIG. 9. The results showed that the free base crystalline form B exhibited a diffraction peak of the free base crystalline form A after 5 days of closed placement at room temperature, indicating a tendency to convert to the free base crystalline form A after being placed at room temperature.

The XRPD pattern of Compound A2 free base crystalline form B before and after nitrogen purging is shown in FIG. 10. The results showed that the free base crystalline form B (containing a diffraction peak of crystalline form A) underwent a crystal transformation after nitrogen purging at 30°C for 20 minutes. It is presumed that the removal of EtOAc from the free base crystalline form B after nitrogen purging causes the crystal transformation.

Combining the characterization results, the free base crystalline form B is presumed to be an EtOAc solvate.

Example 7: Preparation of Compound A2 hydrochloride

100 mg (0.223 mmol) of Compound A2 was weighed, dissolved in 5 mL of anhydrous dichloromethane, and stirred at room temperature for 5 minutes. Then, 1.11 mL of hydrogen chloride ether solution (1N) was added dropwise and stirred at room temperature for another 30 minutes upon completion of the dropwise addition. TLC showed a complete disappearance of the starting material. The obtained mixture was purged with nitrogen for 10 minutes and concentrated under reduced pressure at 10°C to give an off-white solid (> 99% purity). The solid was placed in a lyophilizer for 12 hours to give 108 mg of A2 hydrochloride (off-white, > 99% purity).

The XRPD pattern of Compound A2 hydrochloride is shown in FIG. 11, the XRPD test parameters are shown in Table 1 of Example 5, and the data table of XRPD results is shown in Table 7, indicating that the compound is crystalline, named as hydrochloride crystalline form A. The entire list of peaks or corresponding d-values in Table 7, or a subset thereof, and an XRPD pattern substantially similar to FIG. 11 can be sufficient to characterize the crystalline form.

The TGA/DSC pattern of Compound A2 hydrochloride is shown in FIG. 12, and TGA and DSC test parameters are shown in Table 3 of Example 5. TGA results showed a weight loss of 8.6% upon heating from room temperature to 150°C; DSC results showed two endothermic peaks at 81.9°C and 156.0°C (peak temperature).

Table 7. XRPD data table of Compound A2 hydrochloride

| Diffraction angle (°2θ) | d-value (Å) | Relative intensity (%) |
|---|---|---|
| 8.138280 | 10.86436 | 10.23 |
| 12.12130 | 7.29535 | 13.51 |
| 13.048080 | 6.78522 | 28.41 |
| 15.250920 | 5.80976 | 13.92 |
| 16.320850 | 5.43123 | 26.84 |
| 17.509650 | 5.06507 | 43.49 |
| 17.990580 | 4.93074 | 20.44 |
| 19.378850 | 4.58053 | 27.22 |
| 21.438430 | 4.14491 | 17.23 |
| 23.765500 | 3.74406 | 100.00 |
| 27.305560 | 3.26617 | 2.56 |

The XRPD overlay of Compound A2 free base crystalline form A and Compound A2 hydrochloride is shown in FIG. 13. The XRPD results of the two are essentially the same, but the crystallinity of the hydrochloride salt is relatively low.

Example 8

The procedure for identifying microsomal metabolites is as follows:

The compounds (test compounds and their numbers are shown below, among which compounds A1 and B1 are prepared by referring to the relevant method disclosed in CN110396087A) were weighed and dissolved into dimethyl sulfoxide to prepare a 20 mM solution. The compound stock solution was diluted with 50% acetonitrile in water (v/v) to a concentration of 1.0 mM as a working solution. Liver microsomes (20 mg/mL) were diluted to 1.27 mg/mL with 50 mM dipotassium hydrogen phosphate buffer as a liver microsome working solution. Reduced coenzyme was weighed and dissolved in 3307 μL of phosphate buffer (50 mM) to 5.0 mM as a reduced coenzyme working solution. For T = 60 minutes samples, 4 μL of test working solution (1.0 mM) was added, followed by 316 μL of liver microsome working solution (1.27 mg/mL), and finally, 80 μL of reduced coenzyme working solution was added to start the reaction. For T = 0 minutes samples, 316 μL of liver microsome working solution (1.27 mg/mL) was added and then 80 μL of reduced coenzyme working solution was added to start the reaction, without test solution incubation. After incubation at 37°C for 60 minutes, 1200 μL of stop solution was added to stop the enzyme reaction. For T = 0 minute samples, 4 μL of test working solution (1.0 mM) was added. The sample plate was placed on a vortexer and vortexed at 600 rpm for 5 minutes. Then, the sample plate was centrifuged at 4000 rpm for 10 minutes. The supernatant was removed, mixed, and dried at room temperature with nitrogen. The residue dried with nitrogen was redissolved in 300 μL of 10% acetonitrile (0.1% FA) solvent and then centrifuged at 4000 rpm for 15 minutes. The supernatant was transferred to a detection plate for mass spectrometry. Mass spectrometry was performed using LC/Q-Exactive plus.

7-Ethoxycoumarin (10 μM) was selected as a positive control compound and operated in parallel with the compounds.

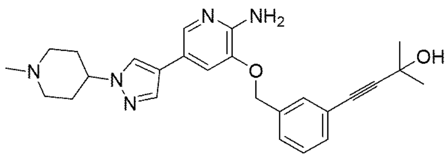

A1

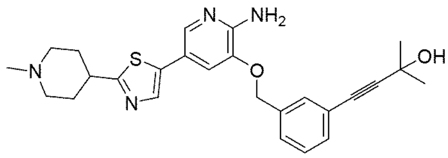

B1

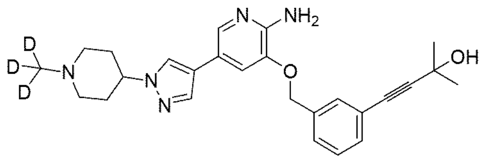

A2

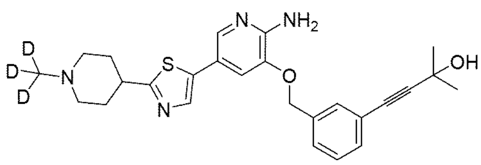

B2

The remaining percentage of the parent cores after 60 min is shown in the table below.

Table 8. Stability study results of the compounds

| Test species | A1 (%) | A2 (%) | B1 (%) | B2 (%) |
|---|---|---|---|---|
| Mouse | 1.13 | 55.0 | 1.24 | 42.8 |
| Rat | 2.44 | 52.1 | 11.21 | 43.9 |
| Beagle | 0.17 | 7.0 | 0.12 | 8.9 |
| Cynomolgus macaques | 0.36 | 29.6 | 9.11 | 23.4 |
| Human | 43.84 | 83.9 | 51.82 | 70.4 |

Example 9. Pharmacokinetic experiments

Rat pharmacokinetic experiment

Six male Sprague Dawley rats were administrated a single dose via intravenous injection (iv, n = 3) and oral administration (po, n = 3), respectively. Intravenous injection was performed by formulating the compounds into 0.25 mg/ml or 0.5 mg/mL solutions using 10% DMSO/30% PEG400/60% water and administering it at a volume of 2 mL/kg. Oral administration was performed by formulating the compounds into 0.6 mg/ml or 2.0 mg/mL homogeneous suspensions using 0.5% methylcellulose and administering it at a volume of 5 mL/kg. The specific administration doses are shown in the table below.

Table 9. Administration doses

| Compound | iv (mg/kg) | po (mg/kg) |
|---|---|---|
| A1 | 1 | 10 |
| A2 | 1 | 10 |
| C | 0.5 | 3 |
| D | 0.3 | 2 |

After intravenous injection and oral administration, blood samples were collected at 0.0833, 0.25, 0.5, 1, 2, 4, 8 and 24 hours, and the concentration of the compounds in the samples was determined using LC-MS/MS with a lower limit of quantitation of 1 ng/mL. The pharmacokinetic parameters of the compounds were calculated using the WinNolin non-compartmental model.

The structures and numbers of the compounds used in this example are shown below:

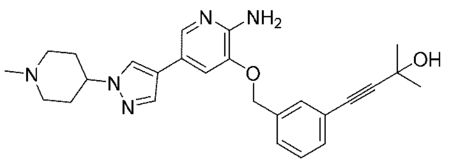

A1

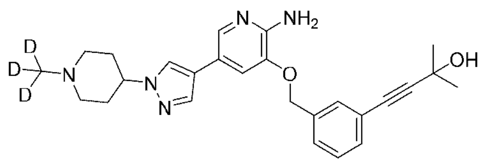

A2

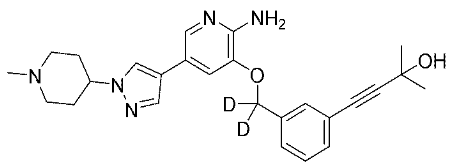

C

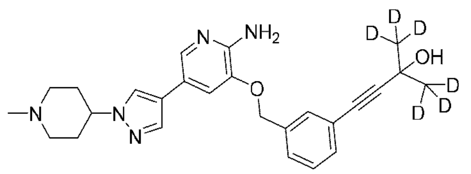

D

Results

After intravenous injection administration, the *in vivo* exposure ($AUC_{last}$) of A1, A2, C and D was 116 hr*ng/mL, 247 hr*ng/mL, 74.8 hr*ng/mL and 28.7 hr*ng/mL, respectively; the average total clearance (CL) was 129 mL/min/kg, 71 mL/min/kg, 101 mL/min/kg and 151 mL/min/kg, respectively.

After oral administration, the *in vivo* exposure ($AUC_{last}$) of Al, A2, C and D was 79.8 ng*hr/mL, 572 ng*hr/mL, 42.8 ng*hr/mL and 9.94 ng*hr/mL, respectively. Compared with the data for intravenous injection, the oral bioavailability of rats was 6.84%, 23.2%, 9.52% and 5.2%, respectively.

Cynomolgus macaques pharmacokinetic experiment

Three male Cynomolgus macaques were administrated a single dose via intravenous injection (iv, 1 mg/kg, n = 3), followed by oral administration (po, 10 mg/kg, n = 3) after washing out for one week. Intravenous injection was performed by formulating the compounds into 1.0 mg/mL solutions using water or 10% DMSO/30% PEG400/water and administering it at a volume of 1 mL/kg. Oral administration was performed by formulating the compounds into 2.0 mg/mL solutions or suspensions using water or 0.5% methylcellulose/1.7 meq 1N hydrochloric acid and administering it at a volume of 5 mL/kg.

After intravenous injection and oral administration, blood samples were collected at 0.0833, 0.25, 0.5, 1, 2, 4, 8, 12 and 24 hours, and the concentration of the compounds in the samples was determined using LC-MS/MS with a lower limit of quantitation of 1 ng/mL. The pharmacokinetic parameters of the compounds were calculated using the non-compartmental model.

Results

After intravenous injection administration, the *in vivo* exposure ($AUC_{last}$) of A1 and A2 was 881 hr*ng/mL and 949 hr*ng/mL, respectively; the average total clearance (CL) was 18.9 mL/min/kg and 16.7 mL/min/kg, respectively.

After oral administration, the *in vivo* exposure ($AUC_{last}$) of A1 and A2 was 388 ng*hr/mL and 1758 ng*hr/mL, respectively. Compared with the data for intravenous injection, the oral bioavailability of Cynomolgus macaques was 4.61% and 18.8%, respectively.

Example 10. Pharmacodynamic evaluation

The structures and numbers of the compounds used in this example are shown below:

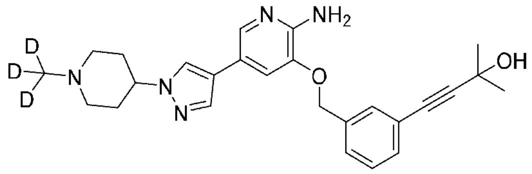

**A2**

**Pharmacodynamic evaluation for hPBMCs**

The hPBMCs used in this experiment were purchased from Shanghai AoNeng Biotechnology Co., Ltd. CD3 antibody was pre-coated onto a 96-well plate for future use. Cells were seeded into the 96-well plate at 2 x 106/ml, with 200 μL per well. CD28 antibody was added for activation, and Compound A2 was added at a final concentration of 1 μM. The supernatant was collected after 24 hours of treatment. IL-2 levels were determined by ELISA, and the effects of A2 and CD3/CD28 on the activation of hPBMCs were compared.

Results

The results are shown in FIG. 14. When 1 uM A2 was used to activate PBMC cells, the concentration ratio (stimulated by A2 - unstimulated) / (stimulated - unstimulated) = 5.89, indicating that IL-2 expressed by hPBMS stimulated by A2 was 5.89-fold higher than iL-2 expressed by hPBMCs stimulated by CD3/CD28 (ELISA).

Conclusion

A2 can effectively enhance human immunity.

**Pharmacodynamic evaluation for viral keratitis (HSV)**

Fifteen C57 mice (body weight: 22 g to 25 g), aged 8 weeks, were subjected to viral keratitis modeling in their right eyes. During the modeling process, a sterile 25G needle was used to make #/M/parallel scratches on the cornea of the right eye of all the animals. The scratch depth should be in the superficial zone and cannot penetrate the stroma layer. The scratches between each eyeball should be moderately uniform in depth and evenly distributed in density. Then, 4 μl of well-mixed HSV virus solution was dripped using a fiber syringe (titer: 2.0 * 106 PFU/ml) to complete the modeling. The day of modeling was set as D0. One day after modeling, the animals' right eyes were scored for viral keratitis, and 9 mice with uniform modeling were selected for the experiment. These 9 mice were randomly divided into 3 groups: control group, 1% A2 group and 0.2% A2 group, with 3 mice per group. According to the grouping, each animal's right eye surface was treated with the corresponding drugs, vehicle or 3024 eye drops with different concentrations, once every other day. On the day of administration, each eye was given 3 drops of 10 μl each time for 2 consecutive weeks. The degree of keratitis lesions was scored and analyzed on days 1, 3, 5, 7, 9, 11 and 14 after modeling.
(the HSV virus of the present invention is cultured and proliferated in African green monkey kidney cell lines, is active, and the titer of HSV virus suspension is good).

Results

One day after modeling, each mice's right eye showed typical corneal ulcer signs of viral keratitis, from which 9 mice with uniform modeling were selected for the experiment and randomly grouped. The results showed that the mice in the experimental control group developed severe corneal ulcers, and 2 eyes showed corneal perforation on the fifth day of the experiment until the end of the experiment, while the mice in the low concentration group (0.2% A2) and high concentration group (1% A2) showed no corneal perforation. See FIG. 15 for details.
FIG. 16 shows the clinical scores of mice within a few days post HSV virus inoculation. As can be seen from the

data in FIG. 16, the keratitis lesion scores after administration of A2 eye drops at concentrations of 0.2% and 1.0% for 2 weeks were both statistically different from that of the model control group ($P < 0.01$ and $P < 0.05$), demonstrating that the drug of the present invention has a good therapeutic effect on viral keratitis. At the highest peak of the disease (day 5), the clinical score of the control group was above 6, while the clinical scores of the low-concentration group (0.2% A2) and the high-concentration group (1% A2) were both less than 6, further indicating that both concentrations of A2 had good therapeutic effect on viral keratitis, which was manifested as A2 could effectively reduce corneal edema and the depth and turbidity of corneal ulcer during disease progression, and improve the depth of corneal inflammatory infiltration. There were no corneal perforation lesions at the peak of the disease and throughout the dosing period. FIGs. 14 and 15 illustrate that A2 eye drops at 0.2% and 1.0% can effectively inhibit viral keratitis lesions in mice.

FIG. 17 shows the neovascularization scoring of the control group, the low-concentration group (0.2% A2) and the high-concentration group (1% A2). It can be seen that the scoring of the control group was above 1, the scoring of the low-concentration group (0.2% A2) was above 0.5 and below 1, and the scoring of the high-concentration group (1% A2) was below 0.5, indicating that the effect of high concentration A2 suspension is slightly superior to that of low concentration A2 clear solution.

According to the results shown in FIGs. 15 to 17, the medication of the disclosure is effective. At the peak of the disease, the model control group treated with a saline eye drop showed perforation, while the therapy group did not. The drug of the disclosure can effectively prevent corneal perforation. The medication of the disclosure can effectively reduce corneal edema and the depth and turbidity of corneal ulcers during disease progression, and improve the depth of inflammatory infiltration. In addition, the effect of high-concentration A2 suspension of the disclosure is superior to that of low-concentration A2 clear solution.

Conclusion

A2 eye drops at 0.2% and 1.0% can effectively inhibit viral keratitis lesions in mice.

Disease model scoring criteria:

Grading (i.e., scoring) was performed based on the three criteria of corneal ulcer area, depth and shape, and the total score was recorded by adding.

(1) Corneal ulcer area: Score 1 = 1% to 25%; Score 2 = 26% to 50%; Score 3 = 51% to 75%; and Score 4 = 76% to 100%.
(2) Lesion depth: Score 1 = mild corneal opacity with clear iris texture; Score 2 = off-white superficial corneal opacity with visible iris texture; Score 3 = off-white deep corneal opacity with unclear iris texture; and Score 4 = off-white full-layer dense corneal opacity with unclear anterior chamber.
(3) Corneal ulcer shape: Score 1 = mild surface irregularity; Score 2 = rough corneal surface with mild swelling; Score 3 = severe edema with swelling descemet's membrane; and Score 4 = corneal perforation or severe swelling descemet's membrane.
(4) If there is hypopyon and/or hyphema, the score is increased by 1.

Evaluation criteria for corneal neovascularization:

Four corneal quadrants had a neovascularization scoring of 0 to 2, with a maximum score of 8. Criteria for corneal neovascularization (0 to 2): Score 0 indicates neovascularization into the cornea; Score 1 indicates centrality neovascularization, but not reaching the pupil center or not exceeding the corneal radius; and Score 2 indicates that the neovascularization reaches the pupil center or exceeds the corneal radius.

$$\text{Clinical score} = \text{lesion area score} + \text{lesion depth score.}$$

**Pharmacodynamic evaluation for Covid 19**

A total of 20 wild-type Syrian golden hamsters, aged 6-8 weeks, with half male and half female, were randomly divided into two groups: control group (n = 4), and therapy group consisting of 5 mg/kg and 20 mg/kg groups, with 8 mice per group. Starting from day 0 (D0), the hamsters in the therapy group were given A2 orally at a total daily dose of 5 mg/kg or 20 mg/kg, and the control group was given an equal amount of blank drug orally daily. Meanwhile, on day 0, all hamsters were anesthetized and then inoculated intranasally with 100 uL of $1.375 \times 10^6$ PFU/mL SARS-Cov-2 virus. Clinical manifestations and body weight were monitored daily. At the end of the study, tissues (e.g., lungs) were collected and weighed, and

virus RNA and infectious virus were then quantified.

Results

FIG. 18 shows the effect of A2 on the body weight of SARS-Cov-2 infected hamsters. As can be seen, the body weights of all three groups of hamsters decreased, however, the animals in the 20 mg/kg group gained weight at a much faster rate. To quantify lung inflammation, lung weights were measured and normalized to total body weight in the present invention. In this example, the increase in lung weight is used as a reliable and indirect measure to characterize the progression of lung inflammation, that is, the change in lung weight can indicate the development of lung inflammation. As shown in FIG. 19, the lung weight decreased in all A2 dose groups. These results indicate that A2 accelerates the clearance of the SARS-CoV-2 virus in hamsters, indicating that A2 can prevent diseases.

Conclusion

The drug of the present invention can prevent Covid 19.

**Pharmacodynamic evaluation for H1N1**

Six to eight-week-old female SPFC 57BL/6 mice (VitalRiver Laboratory Animal Technology Co., Ltd., SCXK (Beijing) 2016-0006, Beijing version) were infected with H1N1 (PR8 strain) and stimulated intranasally with 1 x $10^4$ TCID50 virus nose drops. Mice were randomly divided into a therapy group and a control group. The therapy group was given 3 mg/kg of A2 orally, and the control group was given an equal amount of blank drug orally. The weight loss of mice in all groups was monitored daily.

Results

The results are shown in FIG. 20. The survival rate of the A2 (3 mg/kg) group was significantly higher than that of the blank control group.

Conclusion

The drug of the present invention can treat infections caused by H1N1.

**Pharmacodynamic evaluation for MHV**

The effect of A2 on the prevention and treatment of MHV was investigated.

Low-dose virus group: 21 male SPF grade C57BL/6 mice, aged 4 weeks, were divided into three groups: control group, precaution group and therapy group. These mice were anesthetized and then injected intrahepatically (i.h.) with 100 $\mu$L of DMEM containing 4 x $10^5$ PFU MHV-A59 virus. The precaution group was given the first dose of A2 at 10 mg/kg 24 hours before the challenge, and the therapy group was given the first dose of A2 at 10 mg/kg 24 hours after the challenge, followed by daily continuous administration (both the precaution group and the therapy group were given a daily dose of 10 mg/kg). The infected mice were monitored daily. Blood samples were collected from the retro-orbital venous plexus on day 0 and day 4 after infection, and serum was extracted to detect alanine aminotransferase (ALT) / alanine aminotransferase (GPT) levels. On day 8 after infection, all groups of mice were sacrificed. Liver tissues were taken for virus titer determination and histological analysis, and spleen tissues were taken for virus specific T proliferation assay. The results are shown in FIG. 21.

High-dose virus group: 21 male SPF grade C57BL/6 mice, aged 4 weeks, were divided into three groups: control group, precaution group and therapy group. These mice were anesthetized and then injected intrahepatically (i.h.) with 100 $\mu$L of DMEM containing 2 x $10^6$ PFU MHV-A59 virus. The precaution group was given the first dose of A2 at 10 mg/kg 24 hours before the challenge, and the therapy group was given the first dose of A2 at 10 mg/kg 24 hours after the challenge, followed by daily continuous administration, both the precaution group and the therapy group being given a daily dose of 10 mg/kg. The weight change, clinical symptoms of disease and mortality of infected mice were monitored daily.

Results

The specific T cell proliferation rate of mice in the low-dose virus group is shown in FIG. 21. The average proliferation rates of spleen cells in the control group, the precaution group and the therapy group were 19.19%, 28.18%

and 52.95%, respectively. As can be seen from the above data, the virus specific Tcells in the therapy group in the low-dose virus group proliferated significantly faster.

During the observation of mice in the high-dose virus group, it was found that the body weight of the precaution group increased. Specifically, from day 1 to day 8 of the experiment, the average body weight of mice in the control group decreased from 12.39 g to 10.91 g, the average body weight of mice in the precaution group increased from 13.39 g to 16.34 g, and the average body weight of mice in the therapy group decreased from 12.48 g to 11.39 g. The survival rate of mice in the precaution group in the high-dose virus group was 42.86%, the survival rate of mice in the high-dose therapy group was 28.57%, and all the mice in the high-dose control group died. From the above experimental results of the high-dose virus group, the body weight of the therapy group and the control group was slightly decreased, and the survival rate of the precaution group was higher. When the observation period was prolonged, it was found that the survival curve of the precaution group was superior to that of the therapy group.

Conclusion

The drug of the present invention can better prevent and treat diseases caused by MHV.

The above are only the preferred examples of the present invention and are not intended to limit the present invention. Any modifications, equivalent replacements, and the like made within the spirit and principles of the present invention should fall within the scope of the present invention.

The foregoing examples and methods described in the present invention may vary based on the ability, experience and preferences of those skilled in the art.

The steps of a method are only listed in a certain order in the present invention and are not intended to constitute any limitation on the order of the method steps.

## Claims

1. Use of an HPK1 inhibitor in preparation of drugs for preventing and/or treating human diseases or symptoms caused by pathogen infection or related to pathogen infection.

2. The use according to claim 1, wherein the pathogens may be microorganisms, parasites or other agents;

   preferably, the microorganisms are selected from one or more of viruses, chlamydias, rickettsias, mycoplasmas, bacteria, spirochetes, fungi, etc.;
   more preferably, the pathogens are viruses, comprising, but not limiting to, adenoviridae, herpesviridae, HSV2, VZV, EBV, CMV, poxviridae, papovaviridae, parvoviridae, hepadnaviridae, polyomaviridae, reoviridae, picornaviridae, caliciviridae, togaviridae, arenaviridae, retroviridae, flaviviridae, orthomyxoviridae, paramyxoviridae, bunyaviridae, coronaviridae, astroviridae and bornaviridae.

3. The use according to claim 1, wherein the inhibitor is a compound represented by following general formula I, or pharmaceutically acceptable salts, stereoisomers, esters, prodrugs, solvates and deuterated compounds of the compound represented by formula I:

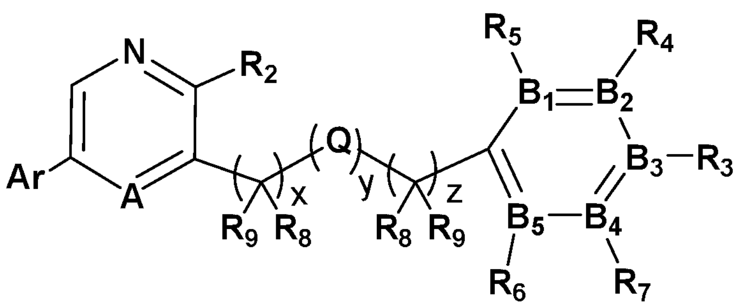

(I)

   wherein

   A is selected from $CR_{10}$ or N;

Q is selected from O or S;
x and z are independently selected from integers between 0 and 6;
y is 0 or 1;
Ar is selected from an aromatic five-membered heterocyclic group, an aromatic six-membered heterocyclic group or phenyl, wherein the aromatic five-membered heterocyclic group is selected from furyl, thienyl, pyrrolyl, pyrazolyl, imidazolyl, oxazolyl, thiazolyl or selenothiazolyl; the aromatic six-membered heterocyclic group is selected from pyridinyl, pyridazinyl, pyrimidinyl or pyrazinyl; optionally, the H atoms in the aromatic five-membered heterocyclic group, the aromatic six-membered heterocyclic group or the phenyl may be substituted with the following groups: -D, $-SO_2$, $-SO_2N(C_{0-10}$ alkyl)($C_{0-10}$ alkyl), $-N(C_{0-10}$ alkyl)$SO_2$($C_{0-10}$ alkyl), $-CON(C_{0-10}$ alkyl)($C_{0-10}$ alkyl), $-N(C_{0-10}$ alkyl)CO($C_{0-10}$ alkyl), $-N(C_{0-10}$ alkyl)COO($C_{0-10}$ alkyl), $-OCON(C_{0-10}$ alkyl)($C_{0-10}$ alkyl), halogen, -CN, $-OCH_2F$, $-OCHF_2$, $-OCF_3$, $C_{1-10}$ straight-chain/branched alkyl, $-N(C_{0-10}$ alkyl)($C_{0-10}$ alkyl), $-OC_{0-10}$ alkyl, $C_{3-10}$ cycloalkyl, -O-containing heterocycloalkyl, -N-containing heterocycloalkyl, -N-containing heteroaryl, -O-containing heteroaryl or -S-containing heteroaryl, wherein the alkyl moiety may be optionally substituted with one or more of the following groups: $-SO_2$, $-SO_2N(C_{0-10}$ alkyl)($C_{0-10}$ alkyl), $-N(C_{0-10}$ alkyl)$SO_2$ ($C_{0-10}$ alkyl), $-CON(C_{0-10}$ alkyl)($C_{0-10}$ alkyl), $-N(C_{0-10}$ alkyl)CO($C_{0-10}$ alkyl), $-N(C_{0-10}$ alkyl)COO($C_{0-10}$ alkyl), $-OCON(C_{0-10}$ alkyl)($C_{0-10}$ alkyl), halogen, -CN, $-OCH_2F$, $-OCHF_2$, $-OCF_3$, $-N(C_{0-10}$ alkyl)($C_{0-10}$ alkyl), $-OC_{0-10}$ alkyl, $-CO(C_{0-10}$ alkyl), $-COO(C_{0-10}$ alkyl), -N-containing heteroaryl, -O-containing heteroaryl or -S-containing heteroaryl;
$R_2$ is selected from -H, -D, halogen, $-NO_2$, -CN, $C_{1-10}$ straight-chain/branched alkyl, $C_{3-10}$ cycloalkyl, $-N(C_{0-10}$ alkyl)($C_{0-10}$ alkyl), $-CF_3$, $-OCF_3$, $-OCHF_2$, $-OCH_2F$ or $-OC_{0-10}$ alkyl;
$B_1$, $B_2$, $B_3$, $B_4$ and $B_5$ are independently selected from C or N;
$R_3$, $R_4$, $R_5$, $R_6$ and $R_7$ are independently selected from -H, -D, halogen, -CN, $-OC_{0-10}$ alkyl, $-CO(C_{0-10}$ alkyl), $-CON(C_{0-10}$ alkyl)($C_{0-10}$ alkyl), $C_{1-10}$ straight-chain/branched alkyl, O- or N-containing heteroalkyl, $-N(C_{0-10}$ alkyl) ($C_{0-10}$ alkyl), $C_{3-10}$ cycloalkyl, $-C\equiv C-R_{10}$, -O-containing heterocycloalkyl and -N-containing heterocycloalkyl, or $R_5$ and $R_4$, $R_4$ and $R_3$, $R_3$ and $R_7$, $R_7$ and $R_6$, together with the carbon atoms between them, form $C_{3-8}$ cycloalkyl or -O- and -S-containing $C_{3-8}$ heterocycloalkyl, -N-containing heteroaryl, -O-containing heteroaryl or -S-containing heteroaryl and phenyl;
$R_8$ and $R_9$ are independently selected from -H, -D, halogen and $C_{1-10}$ straight-chain/branched alkyl;
$R_{10}$ is selected from H, -D, $C_{1-5}$ straight-chain/branched alkyl, $C_{3-10}$ cycloalkyl and

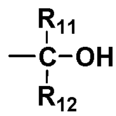 ;

and
$R_{11}$ and $R_{12}$ are independently selected from -H, -D, $-CF_3$, $-CHF_2H$, $-CH_2F$, $C_{1-10}$ straight-chain/branched alkyl, $-CH=C(C_{0-10}$ alkyl)($C_{0-10}$ alkyl), $-C\equiv C(C_{0-10}$ alkyl), $C_{3-10}$ cycloalkyl, an aromatic five-membered ring group or an aromatic six-membered ring group, or $R_{11}$ and $R_{12}$, together with the carbon atom between $R_{11}$ and $R_{12}$, form $C_{3-8}$ cycloalkyl or -O- and -S-containing $C_{3-8}$ heterocycloalkyl, $C_{4-9}$ fused cycloalkyl, $C_{5-10}$ spirocycloalkyl, $C_{4-9}$ bridged cycloalkyl, $C_{3-7}$ cyclic lactam, $C_{3-7}$ cyclic lactone and $C_{3-7}$ cyclic ketone;

**4.** The use according to claim 3, wherein one or more H atoms attached to the C atoms in Ar, $R_2$, $R_3$, $R_4$, $R_5$, $R_6$, $R_7$, $R_8$, $R_9$, $R_{10}$, $R_{11}$ and/or $R_{12}$ may be substituted with deuterium;

and/or one or more H atoms attached to the heteroatoms in Ar, $R_2$, $R_3$, $R_4$, $R_5$, $R_6$, $R_7$, $R_8$, $R_9$, $R_{10}$, $R_{11}$ and/or $R_{12}$ may be substituted with deuterium;
and/or the compound represented by general formula I contains at least one deuterium atom.

**5.** The use according to claim 3, wherein the compound has the following structure:

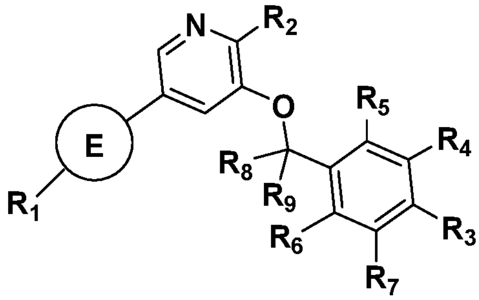

(II)

wherein ring E is selected from

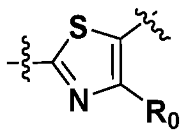, 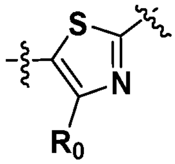, 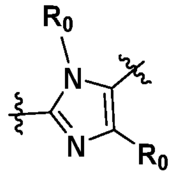,

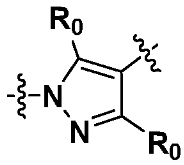, 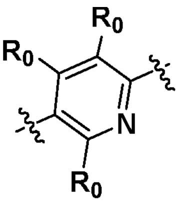 and 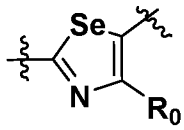;

in ring E, each $R_0$ is independently selected from **-H, -D,** $C_{1-10}$ straight-chain/branched alkyl, -N($C_{0-10}$ alkyl)($C_{0-10}$ alkyl), -O$C_{0-10}$ alkyl, -CO($C_{0-10}$ alkyl) or $C_{3-10}$ cycloalkyl; wherein the H atoms attached to the C atoms or heteroatoms may be substituted with deuterium; and

$R_1$ is selected from **-H, -D,** -O-containing heterocycloalkyl, -N-containing heterocycloalkyl, $C_{1-10}$ straight-chain/branched alkyl, $C_{3-10}$ cycloalkyl, -O$C_{0-10}$ alkyl, -N($C_{0-10}$ alkyl)($C_{0-10}$ alkyl), -$SO_2$($C_{0-10}$ alkyl), -CO($C_{0-10}$ alkyl), -O-phenyl, -S($C_{0-10}$ alkyl), -N-containing heteroaryl, -O-containing heteroaryl or -S-containing heteroaryl.

**6.** The use according to claim 5, wherein the H atoms attached to the C atoms in $R_0$ and $R_1$ may be substituted with deuterium;

and/or the H atoms attached to the heteroatoms in $R_0$ and $R_1$ may be substituted with deuterium;
and/or at least one of $R_{0-9}$ contains a deuterium atom;
and/or the compound represented by general formula II contains at least one deuterium atom.

**7.** The use according to claim 5, wherein at least one of $R_1$, $R_4$, $R_8$ and $R_9$ contains a deuterium atom.

**8.** The use according to claim 5, wherein $R_1$ is selected from

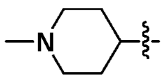, 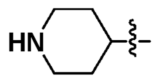,

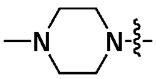, 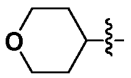, 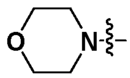, $-CH_3$, 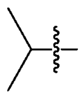, 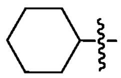, 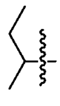, 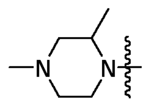, 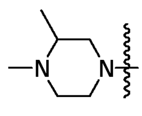,

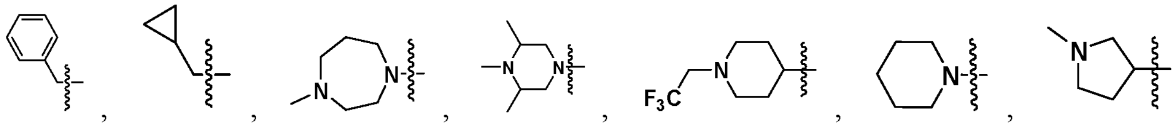

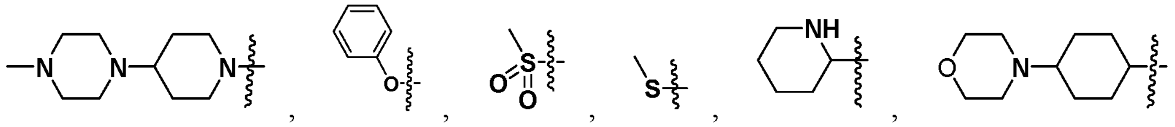

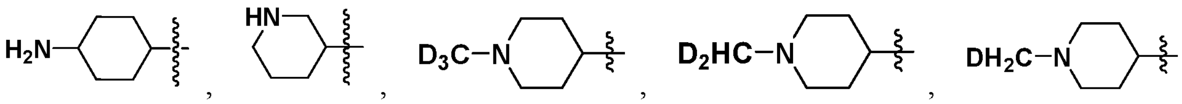

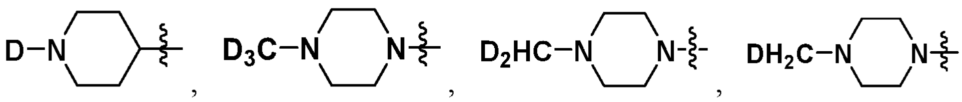 , $-CH_2D$, $-CHD_2$,

$-CD_3$, 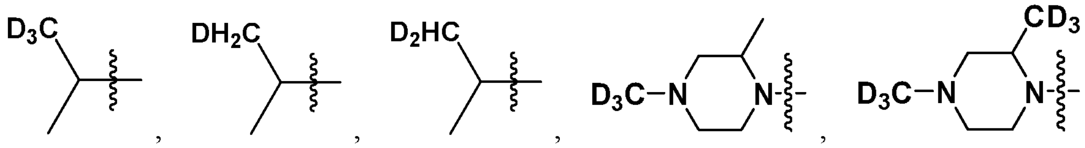

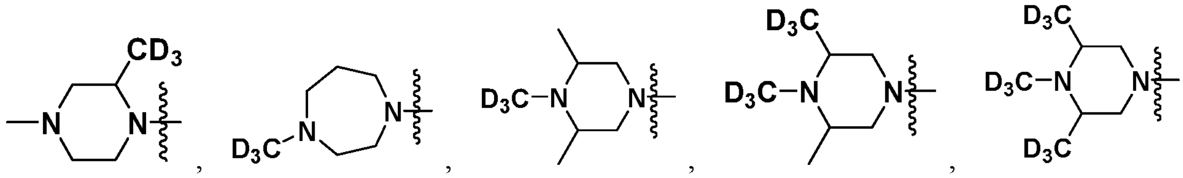

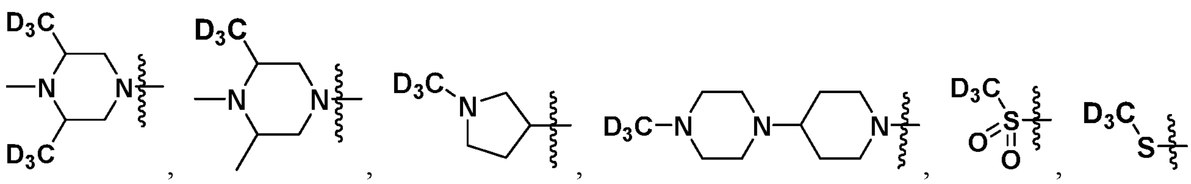

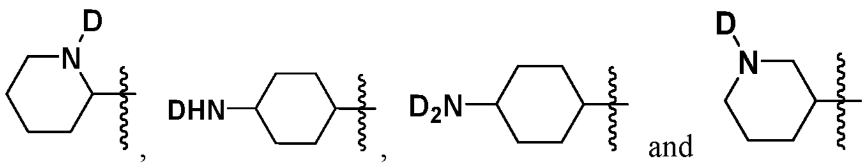 and .

**9.** The use according to claim 5, wherein $R_4$ is selected from -H, -D, -F, -Cl, $-OCH_3$, $-OCH_2D$, $-OCHD_2$, $-OCD_3$, -CN,

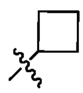

or $-C{\equiv}C-R_{10}$.

**10.** The use according to claim 9, wherein $R_{11}$ and $R_{12}$ are independently selected from -H, -D, $-CF_3$, $-CHF_2$, $-CH_2F$, $-CH_3$, $-CH_2CH_3$, $-CH=CH_2$,

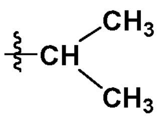, 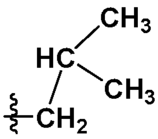, 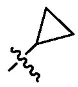, 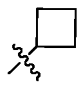,

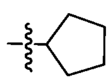, 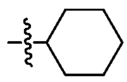 and 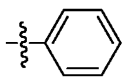,

or $R_{11}$ and $R_{12}$, together with the carbon atom between $R_{11}$ and $R_{12}$, form

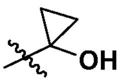, 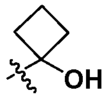, 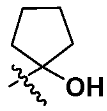, 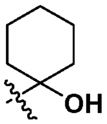, 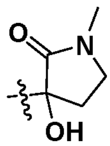, 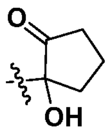, 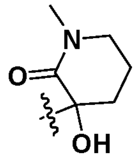,

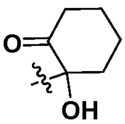, 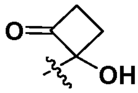, 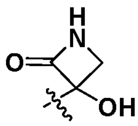, 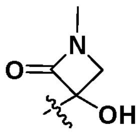, 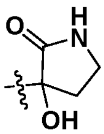, 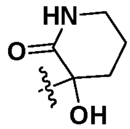, 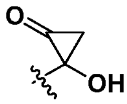,

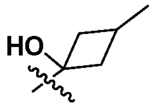, 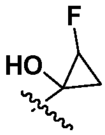, 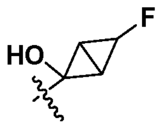 and 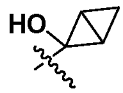,

wherein the H atoms attached to the C or N atoms may be substituted with deuterium;
preferably, $R_{11}$ and $R_{12}$ are independently selected from -H, -D, $-CF_3$, $-CHF_2$, $-CDF_2$, $-CH_2F$, $-CD_2F$, $-CH_3$, $-CH_2D$, $-CHD_2$, $-CD_3$, $-CH_2CH_3$, $-CH_2CD_3$,

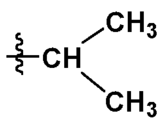, 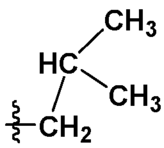, 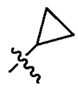,

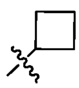, 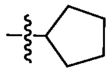, 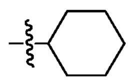 and 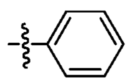.

**11.** The use according to claim 5, wherein each $R_0$ is independently selected from -H, -D, $-CH_3$, $-CH_2CH_3$ or $-NH_2$.

**12.** The use according to claim 5, wherein $R_2$ is selected from $-NO_2$, $-N(C_{0-10}$ alkyl)($C_{0-10}$ alkyl), $-OC_{0-10}$ alkyl and $-OCF_3$; wherein the H atoms attached to the C or N atoms may be substituted with deuterium;
preferably, $R_2$ is selected from $-NH_2$, -NHD, $-ND_2$ or $-NO_2$.

**13.** The use according to claim 5, wherein $R_3$ is selected from -H, -D, halogen, $-OC_{0-10}$ alkyl, $-CO(C_{0-10}$ alkyl) and $C_{1-10}$ straight-chain/branched alkyl, wherein the H atoms attached to the C atoms may be substituted with deuterium;
preferably, $R_3$ is selected from -H, -D, -F, $-OCH_3$, $-OCH_2D$, $-OCHD_2$ and $-OCD_3$.

**14.** The use according to claim 5, wherein $R_5$, $R_6$ and $R_7$ are independently selected from -H, -D, halogen, -CN, $C_{1-3}$ straight-chain/branched alkyl, $-OC_{0-3}$ alkyl, $-CO(C_{0-3}$ alkyl) and N-containing $C_{1-3}$ straight-chain/branched alkyl, or $R_6$

and $R_7$, together with the carbon atoms between $R_6$ and $R_7$, form $C_{3-8}$ cycloalkyl or -O-containing $C_{3-8}$ heterocycloalkyl, wherein the H atoms attached to the C or N atoms may be substituted with deuterium; preferably, $R_5$, $R_6$ and $R_7$ are independently selected from -H, -D, -F, -Cl, $-CH_3$, $-CH_2D$, $-CHD_2$, $-CD_3$, $-OCH_3$, $-COCH_3$, $-CH_2NH_2$, $-CH_2N(CH_3)_2$, -CN, $-OCH_2D$, $-OCHD_2$, $-OCD_3$, $-COCD_3$, $-CH_2N(CD_3)_2$ and $-CH_2N(CH_3)(CD_3)$.

**15.** The use according to claim 5, wherein $R_8$ and $R_9$ are independently selected from -H, -D and $C_{1-3}$ straight-chain/branched alkyl, wherein the H atoms attached to the C atoms may be substituted with deuterium; preferably, $R_8$ and $R_9$ are independently selected from -H, -D, $-CH_3$, $-CH_2D$, $-CHD_2$ and $-CD_3$.

**16.** The use according to claim 1, wherein the compound is selected from the following structures:

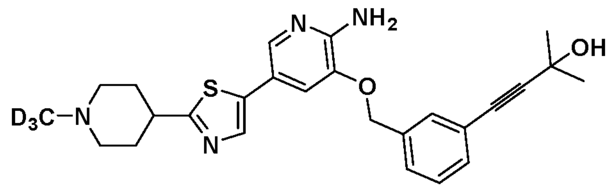

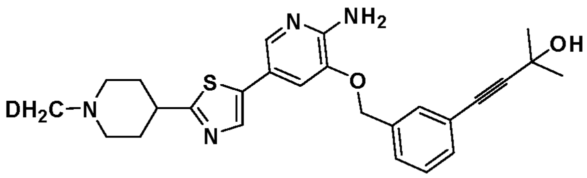

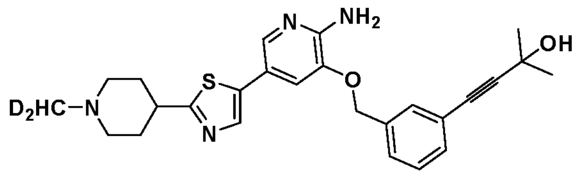

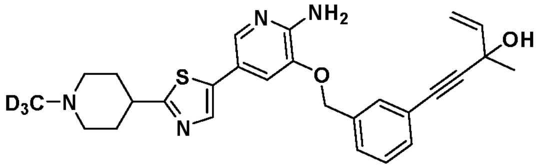

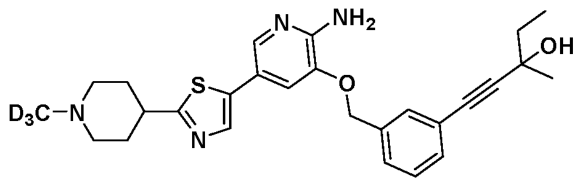

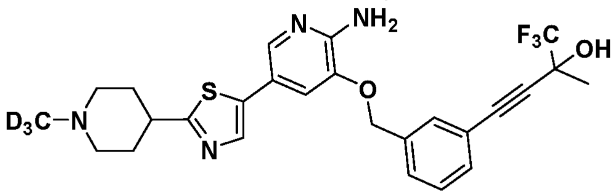

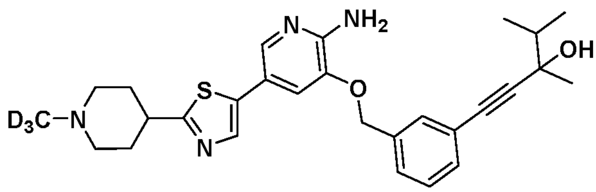

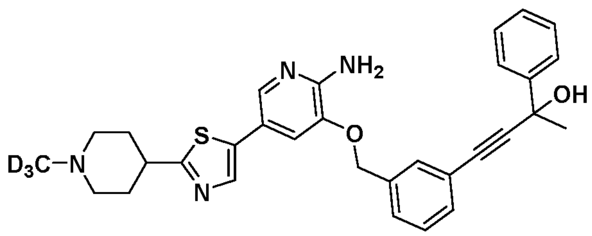

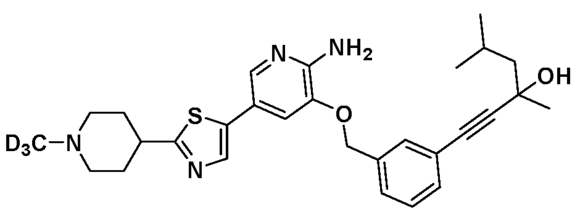

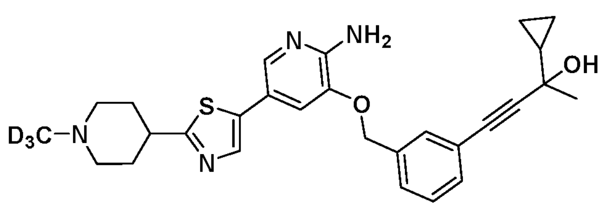

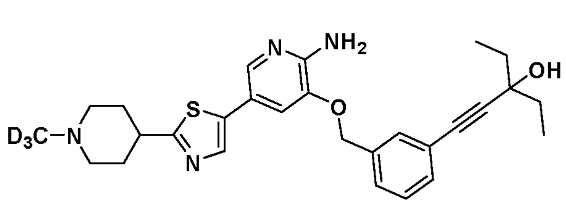

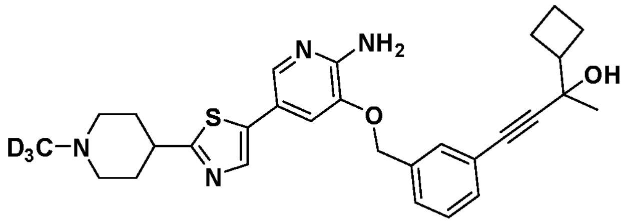

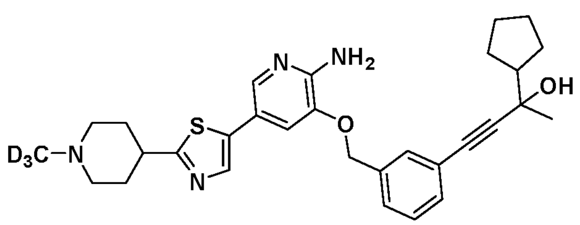

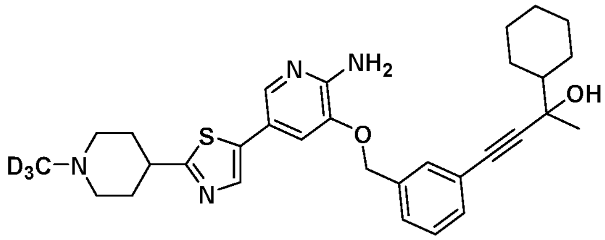

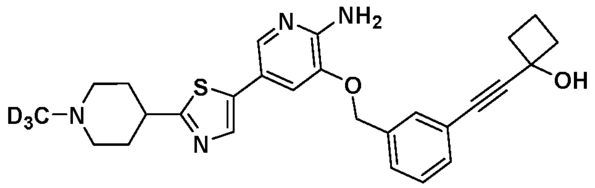

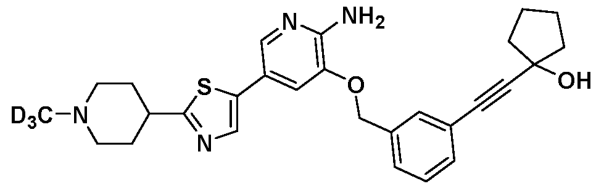

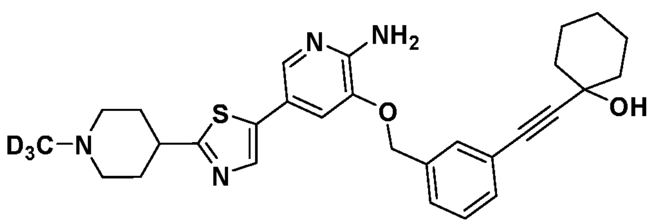

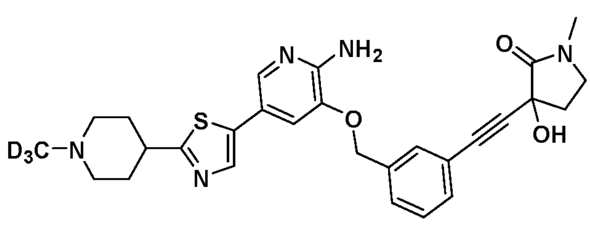

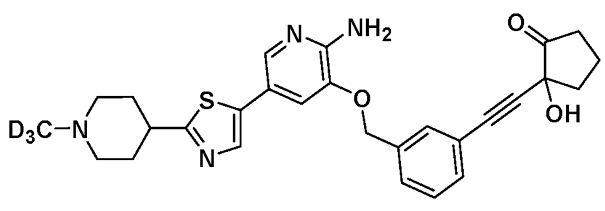

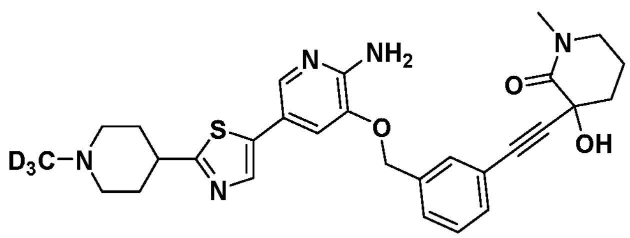

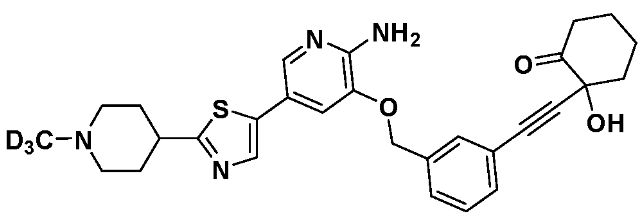

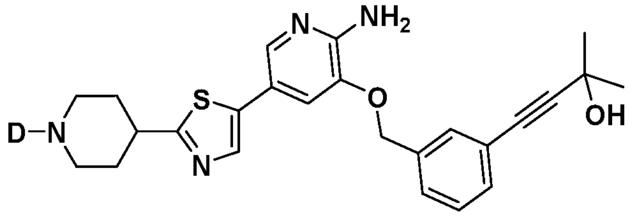

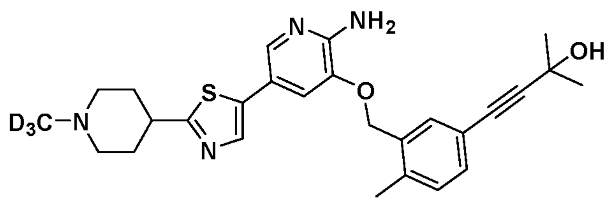

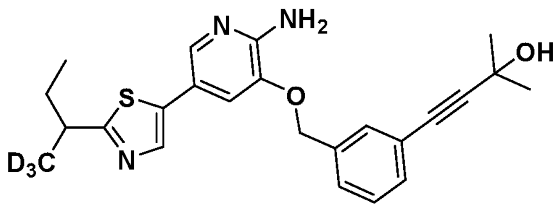

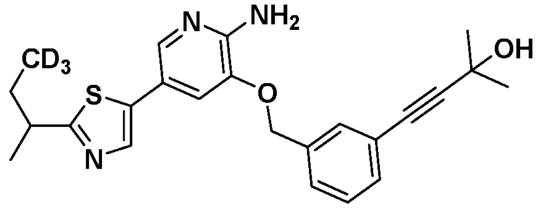

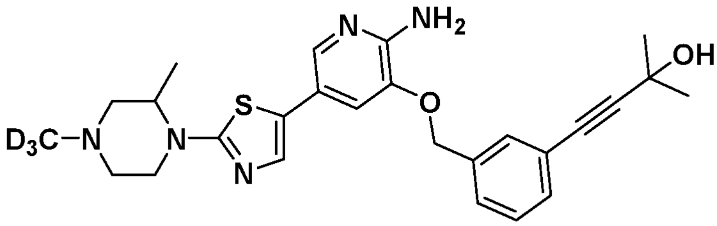

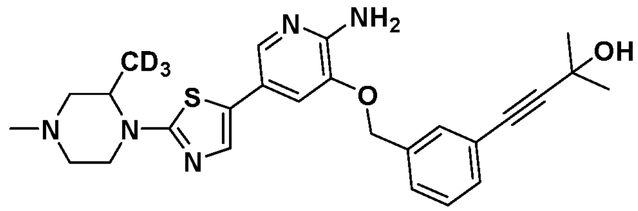

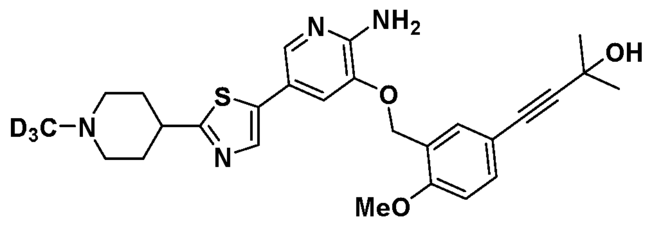

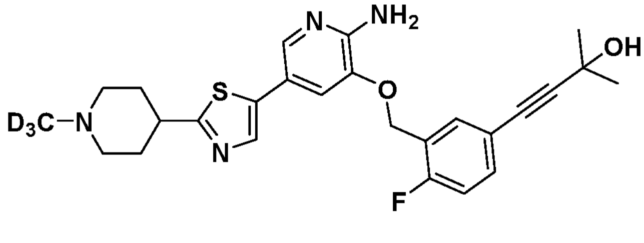

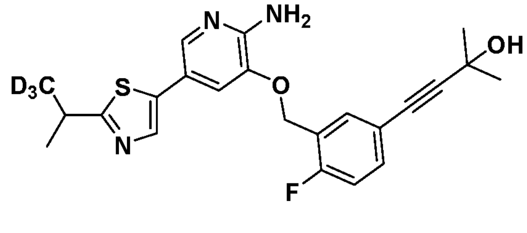

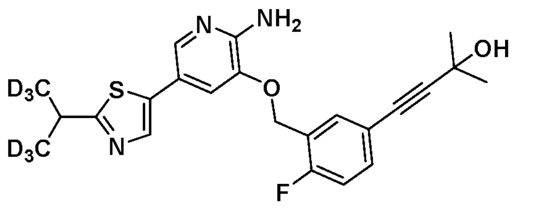

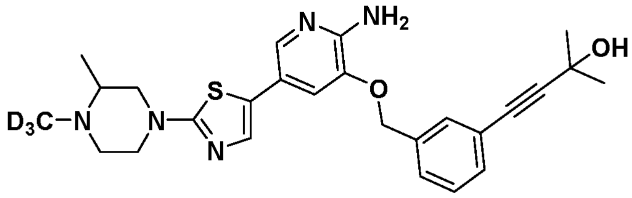

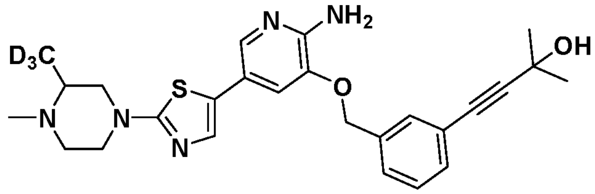

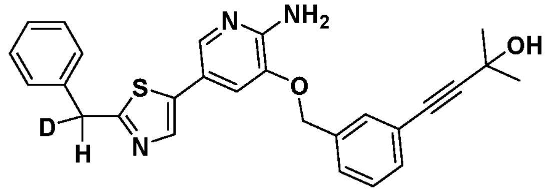

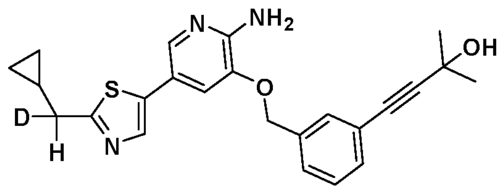

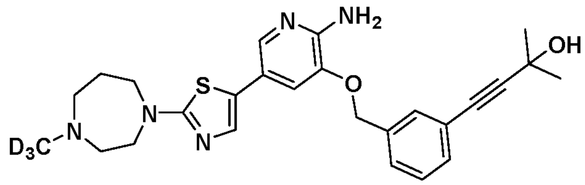

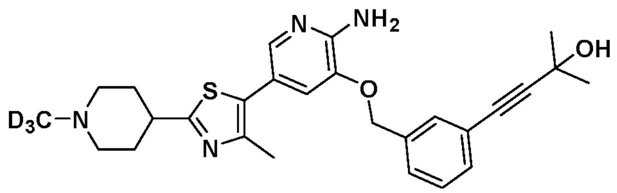

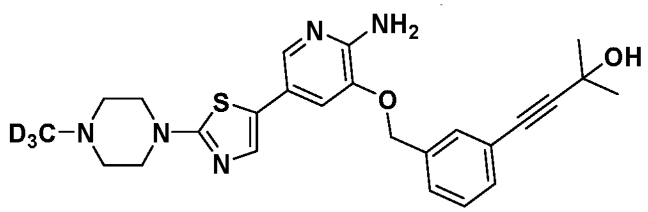

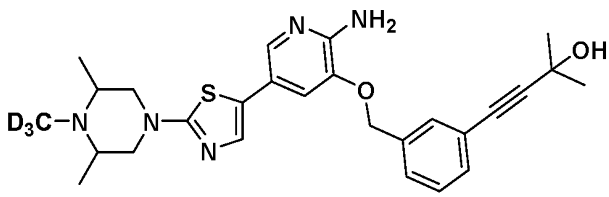

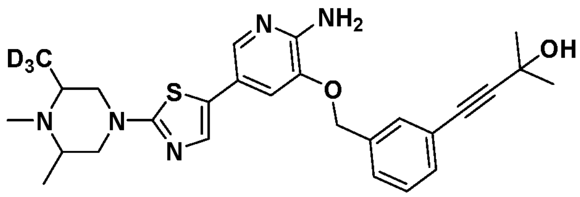

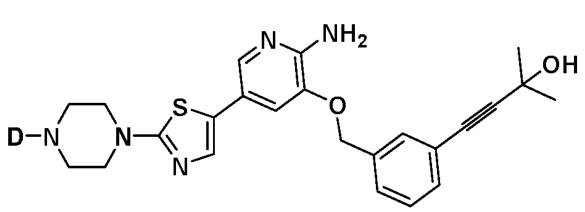

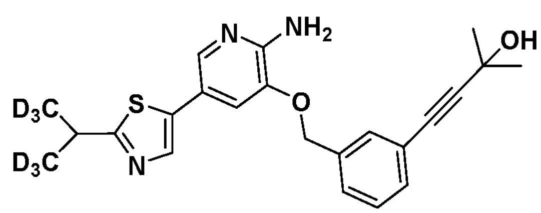

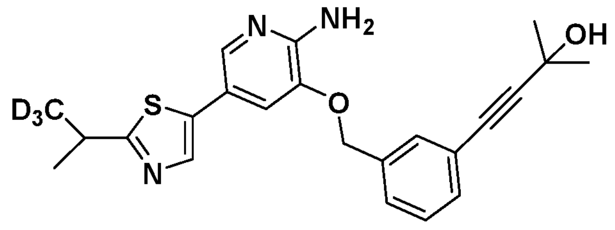

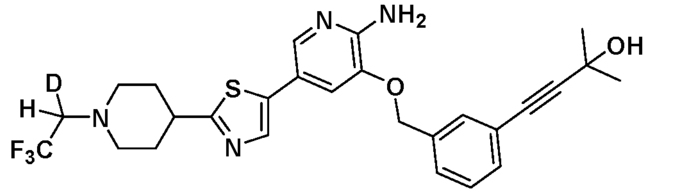

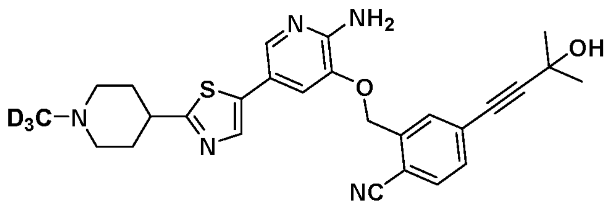

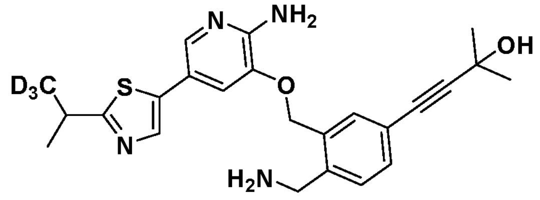

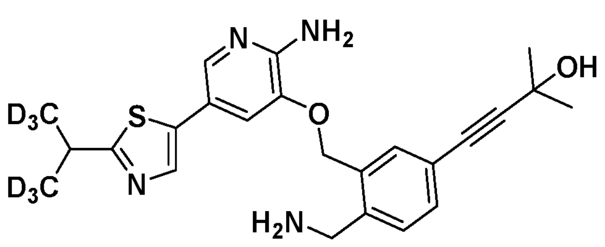

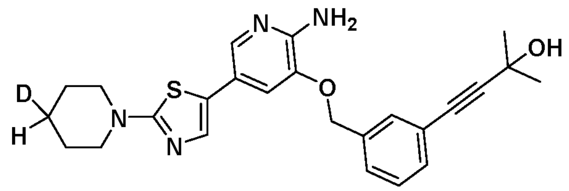

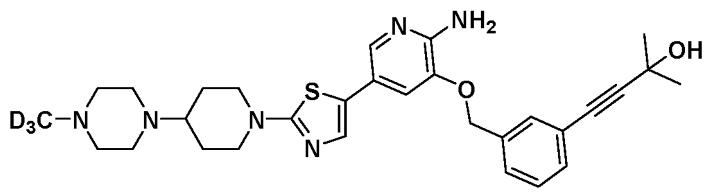

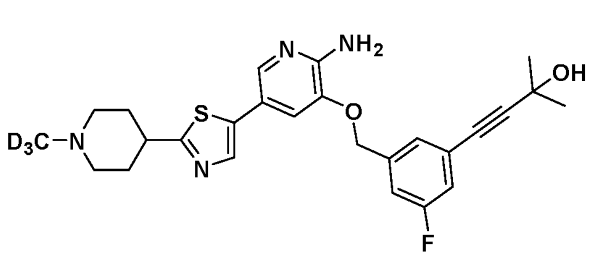

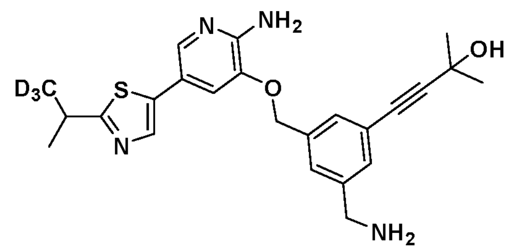

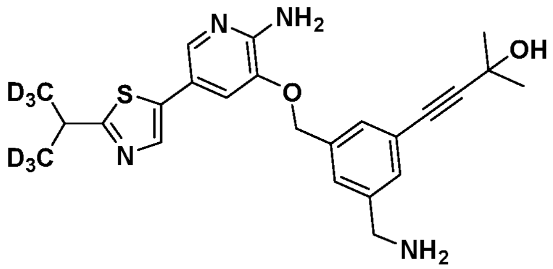

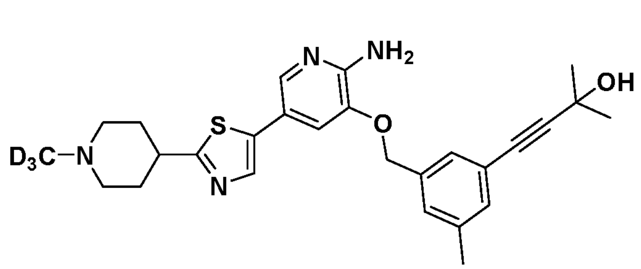

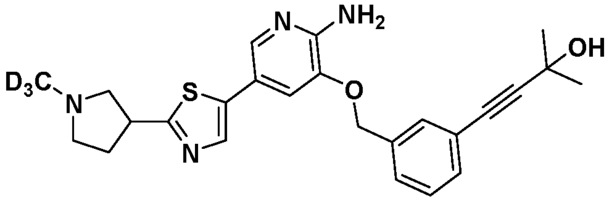

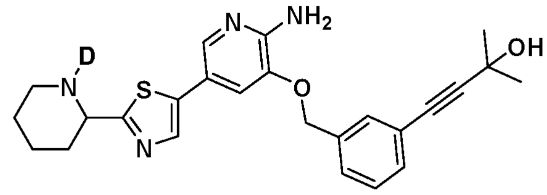

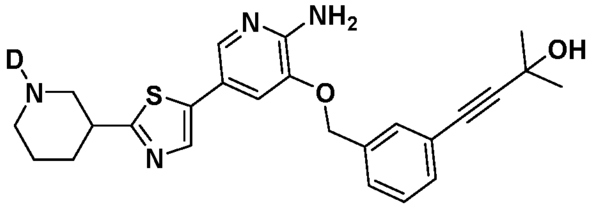

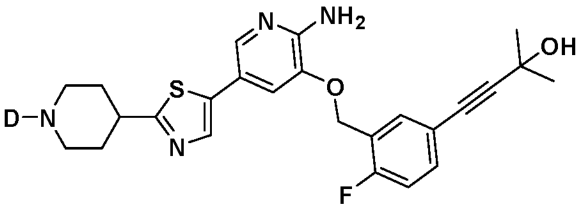

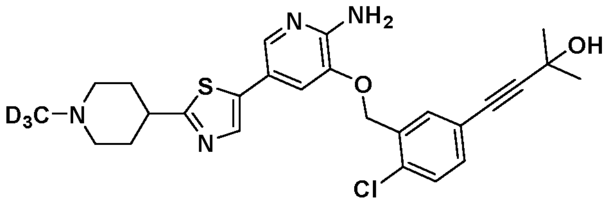

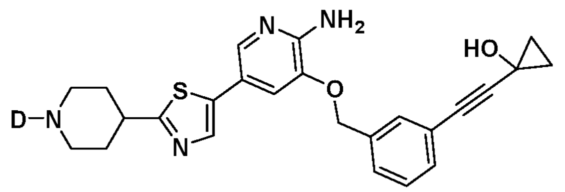

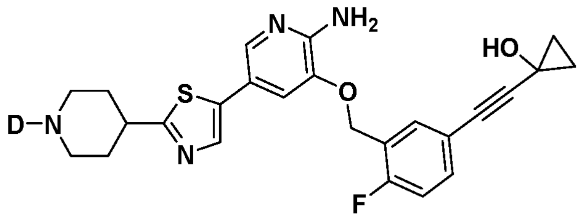

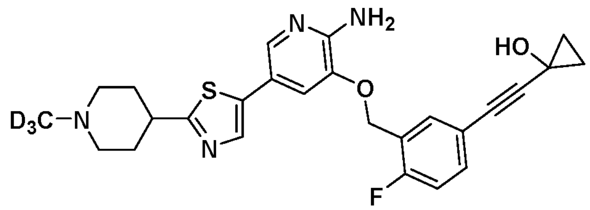

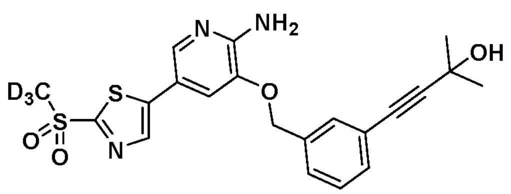

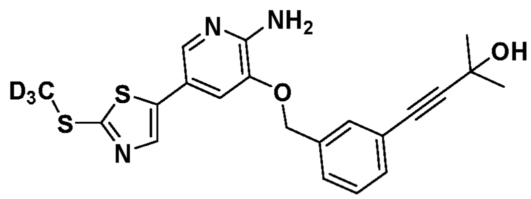

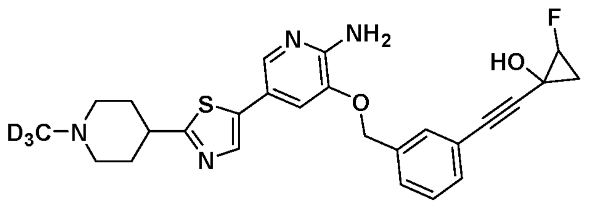

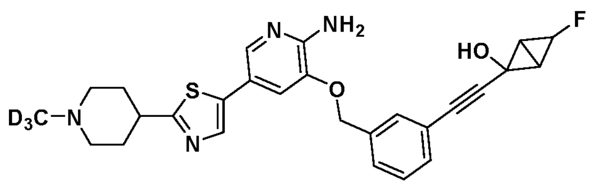

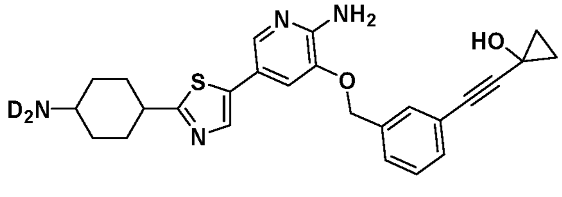

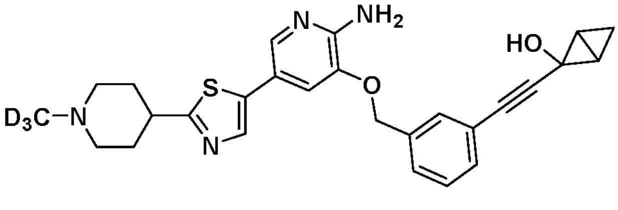

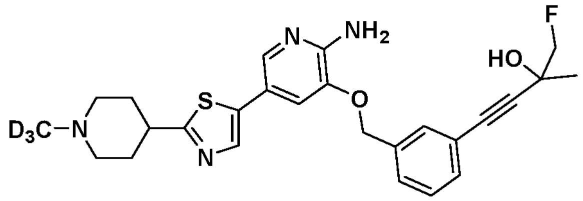

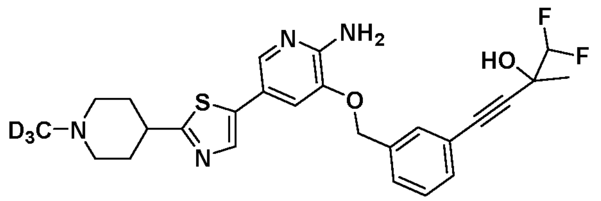

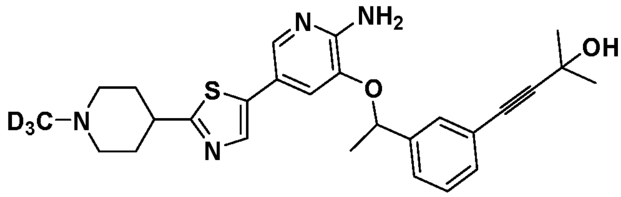

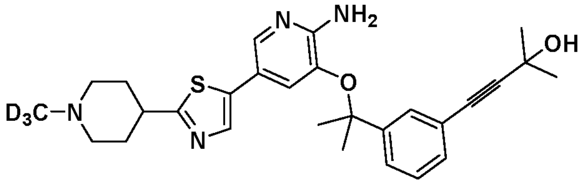

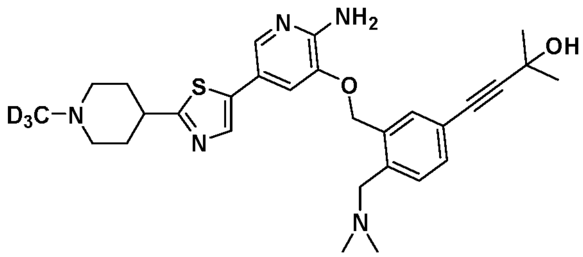

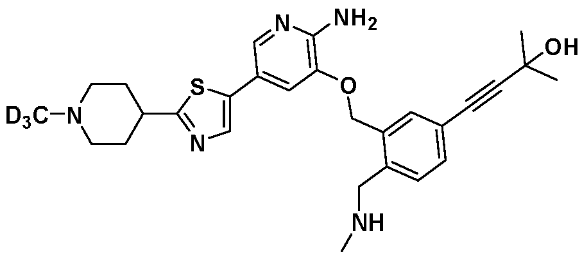

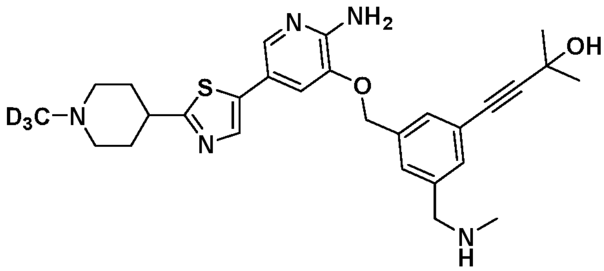

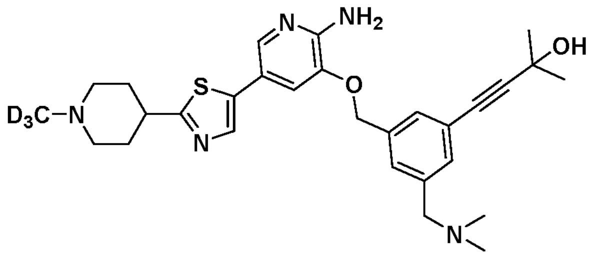

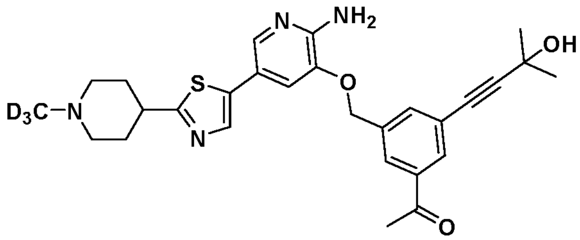

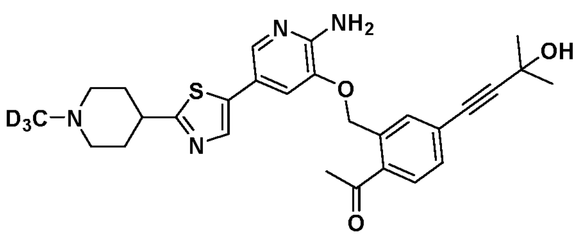

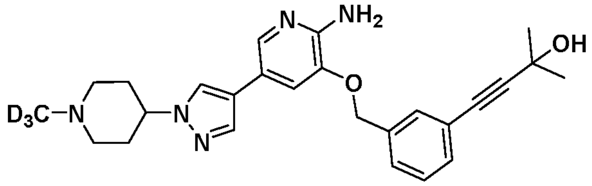

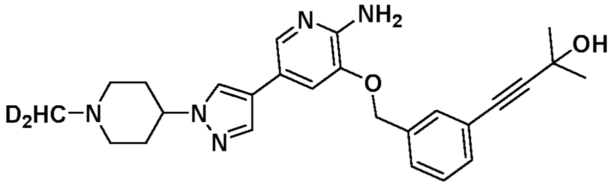

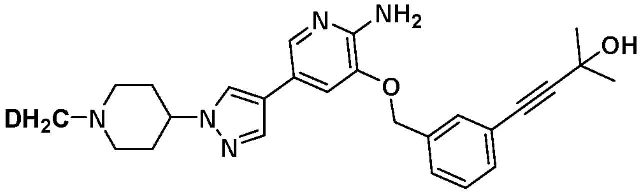

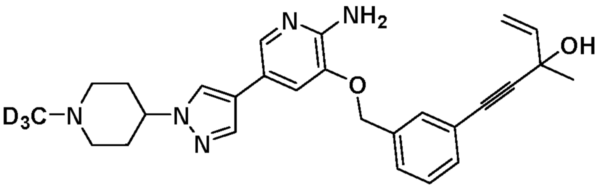

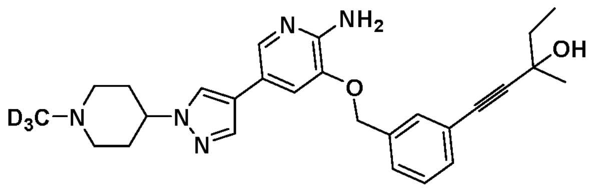

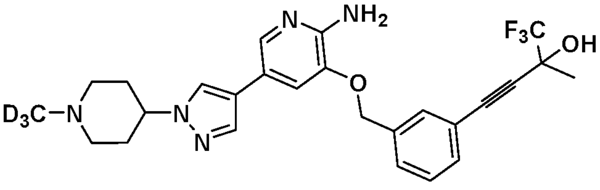

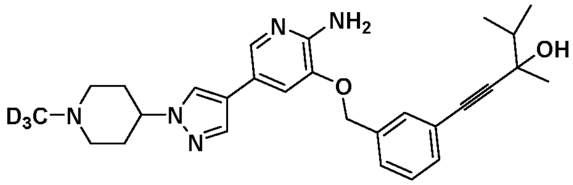

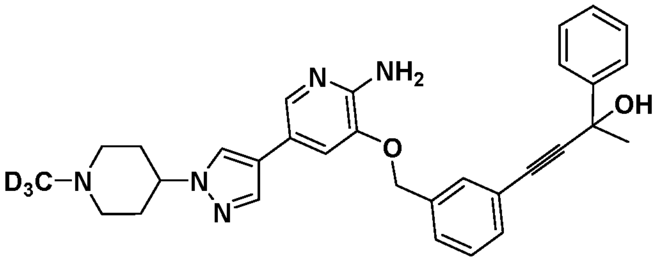

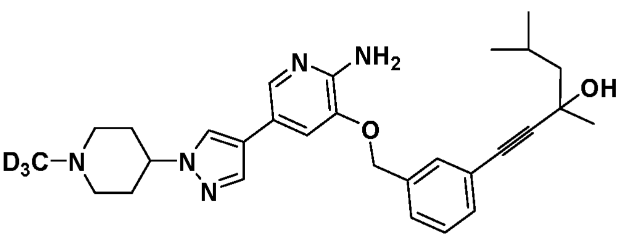

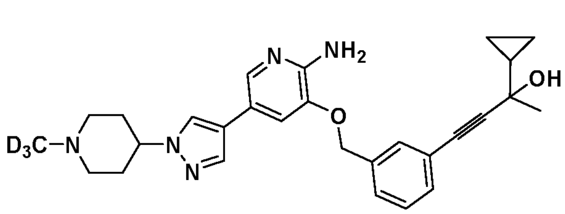

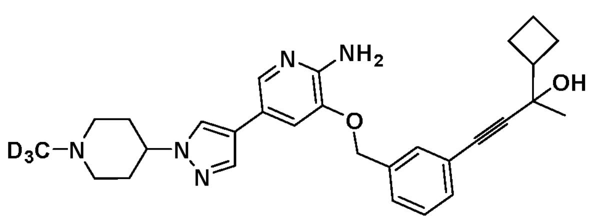
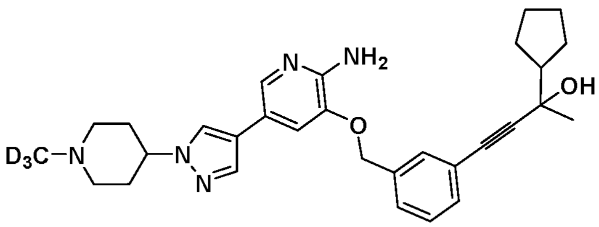

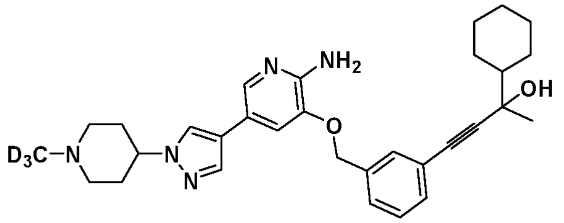
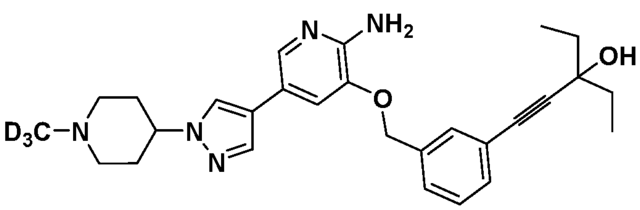

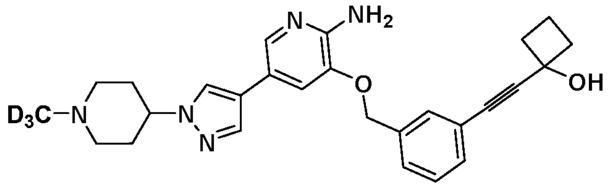
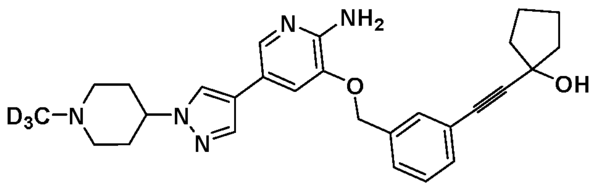

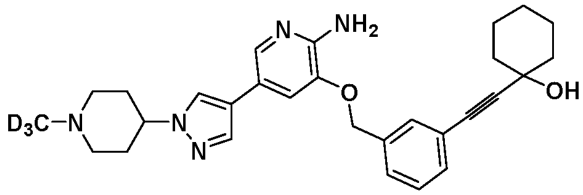
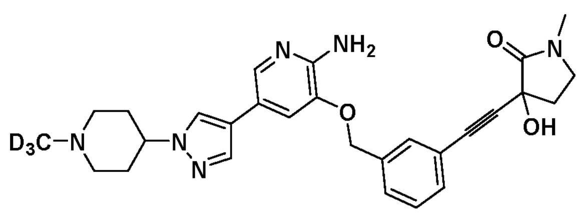

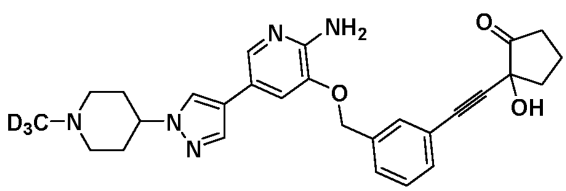
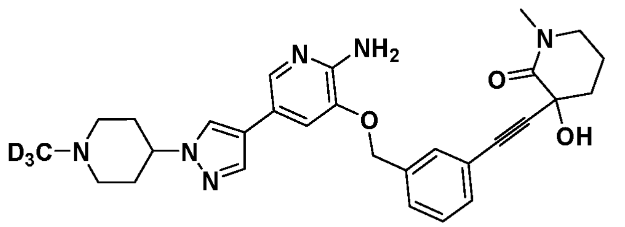

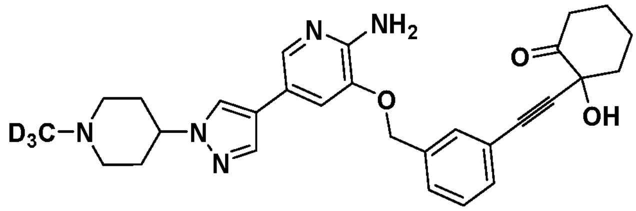

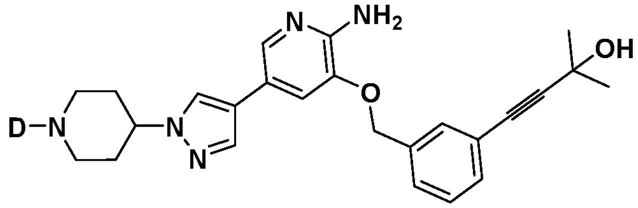

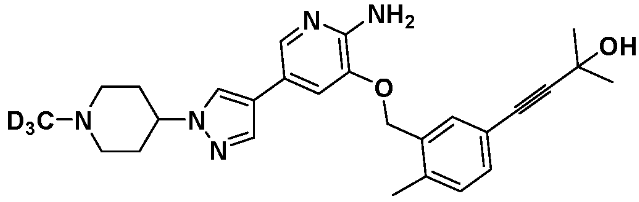

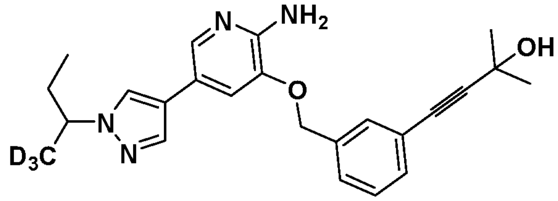

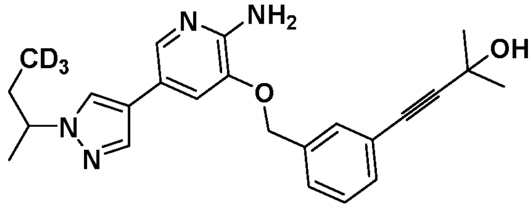

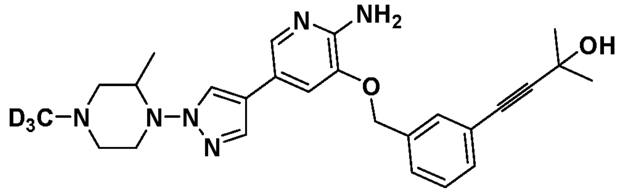

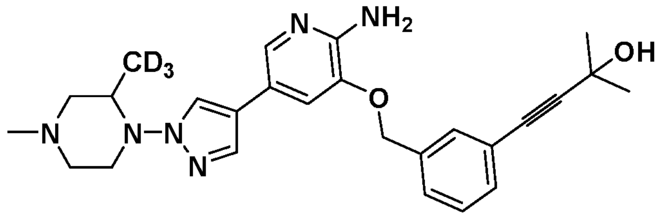

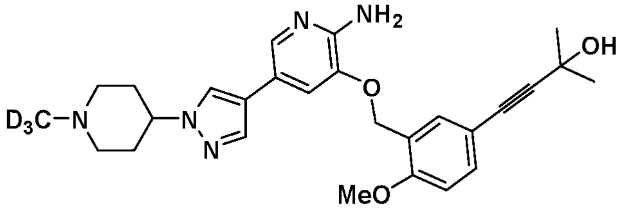

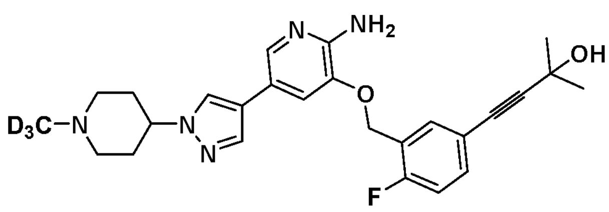

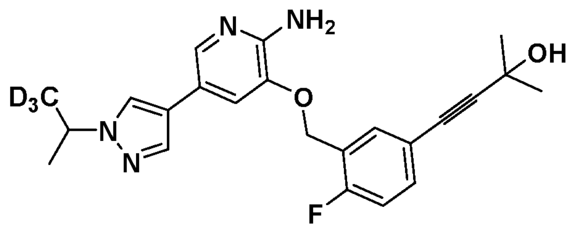

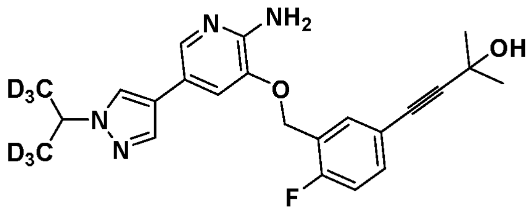

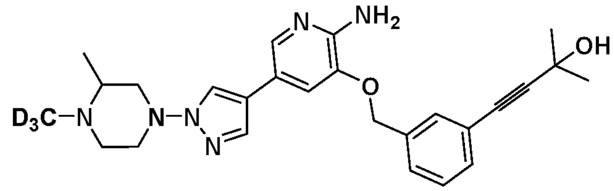

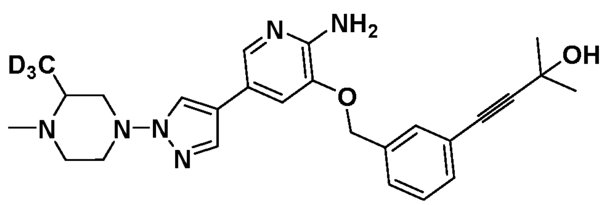

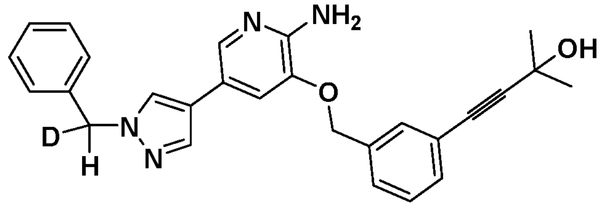

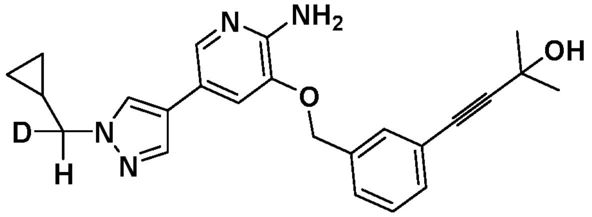

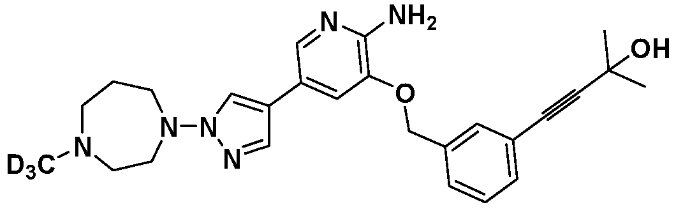

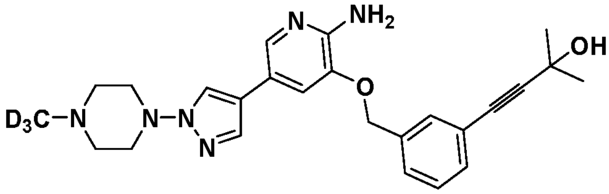

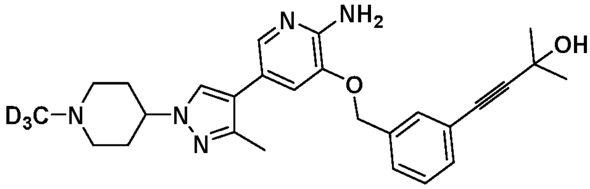

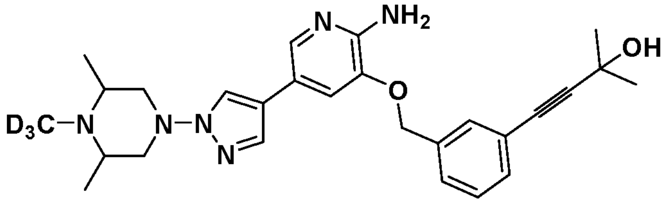

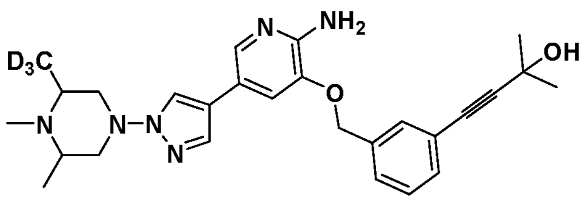

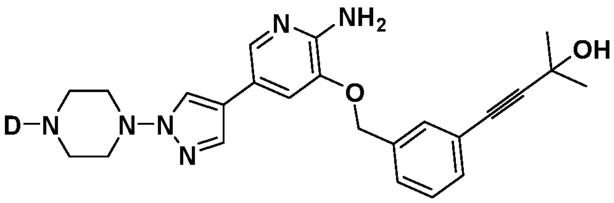

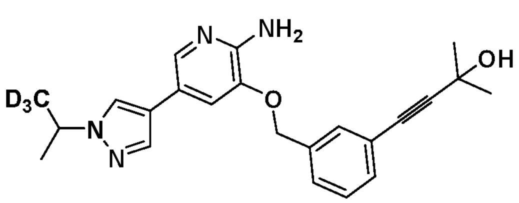

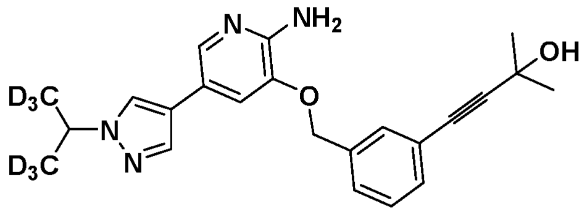

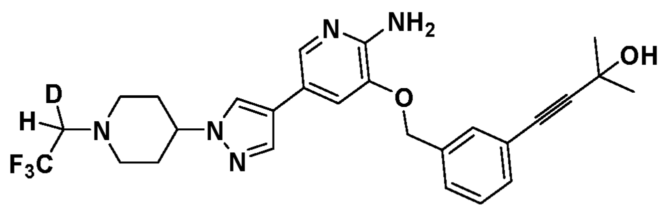

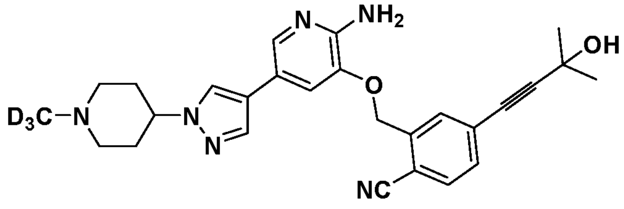

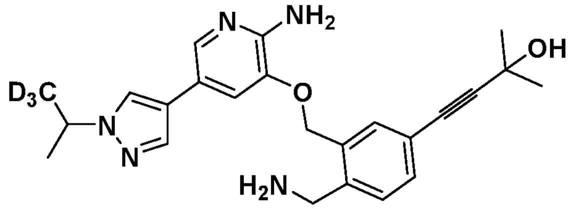

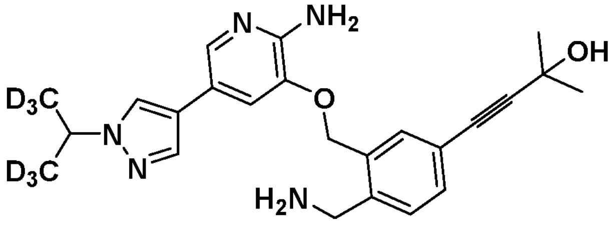

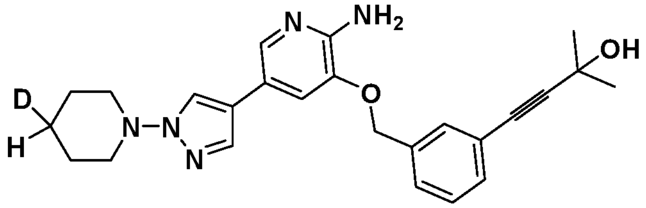

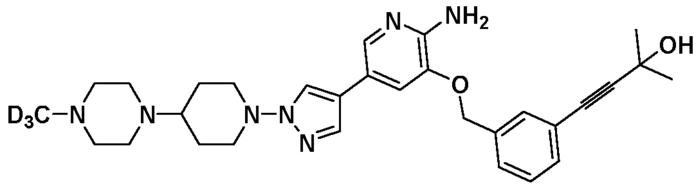

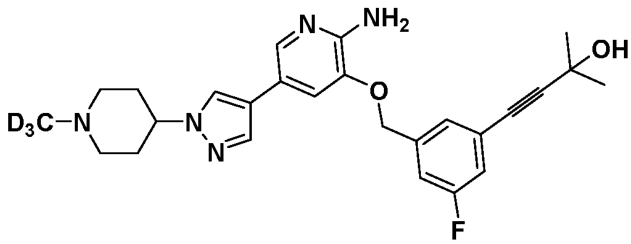

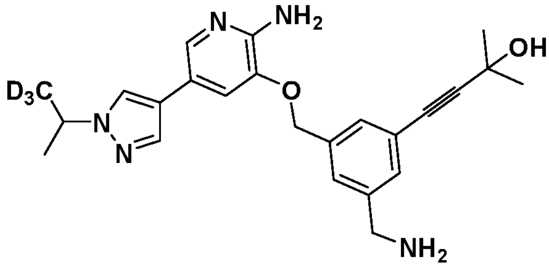

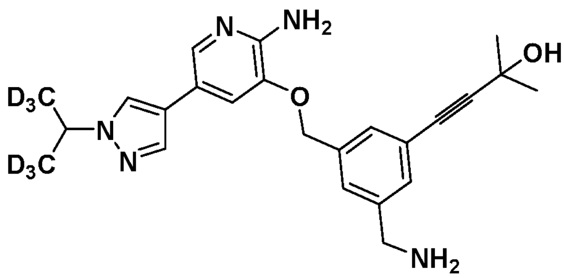

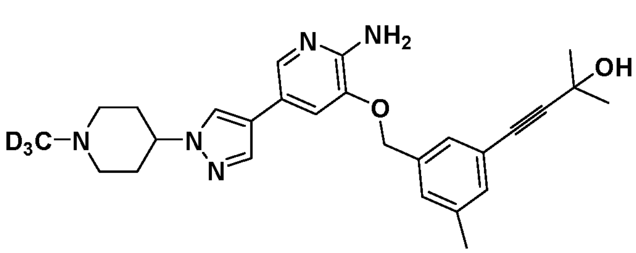

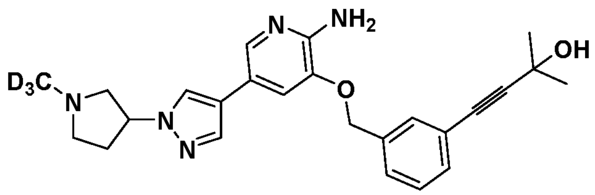

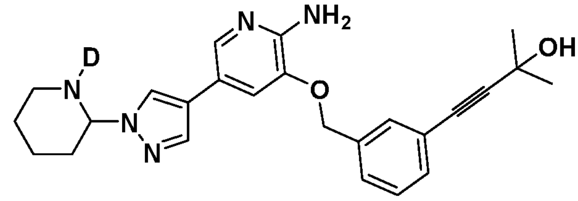

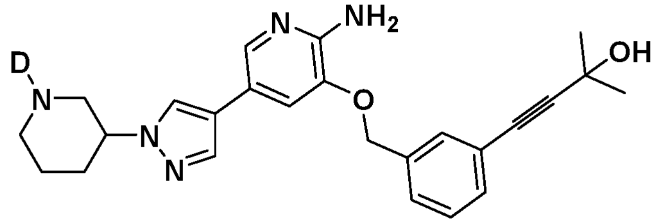

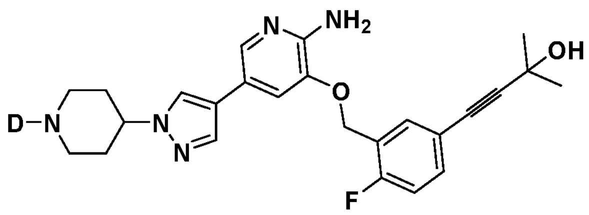

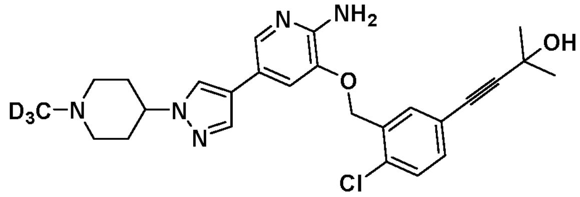

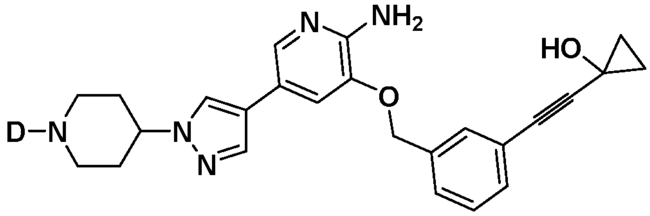

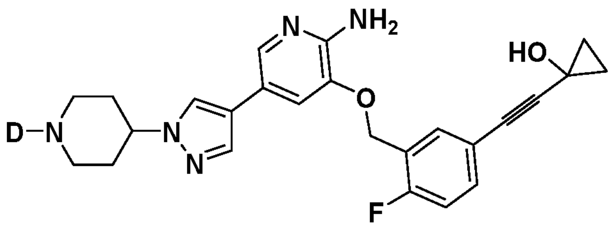

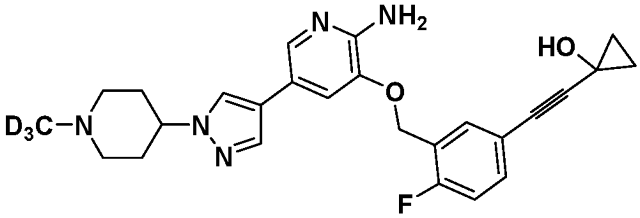

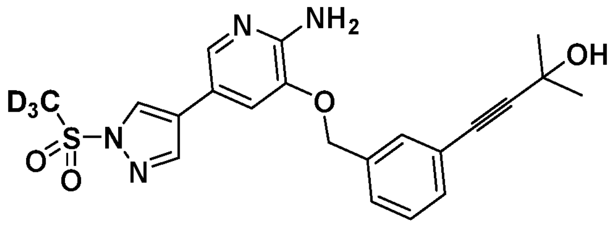

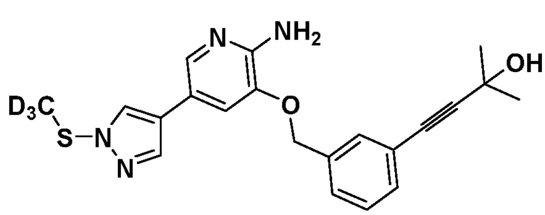

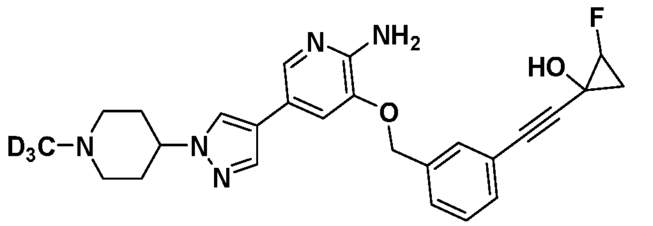

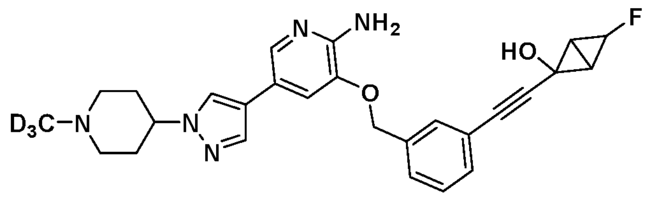

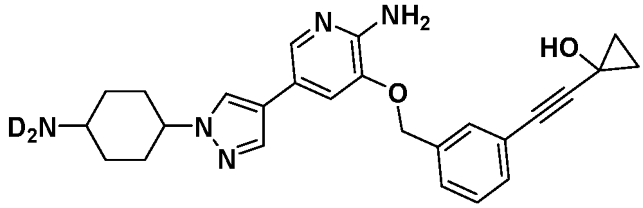

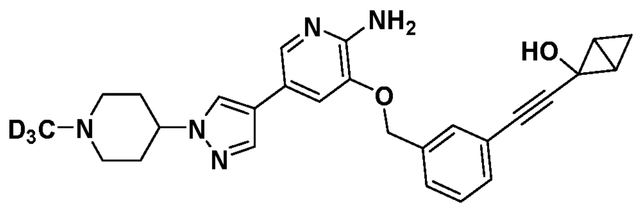

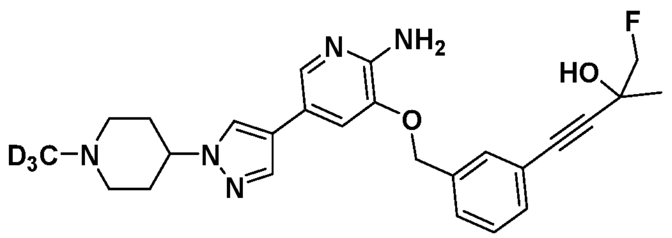

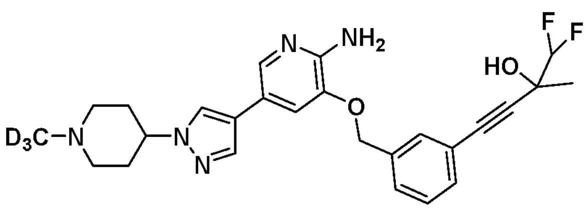

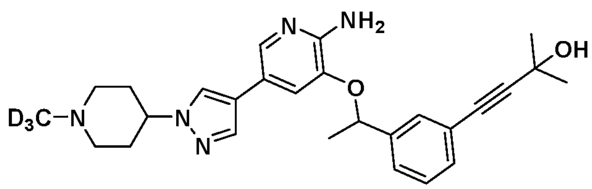

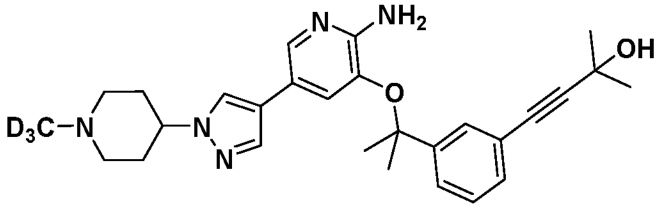

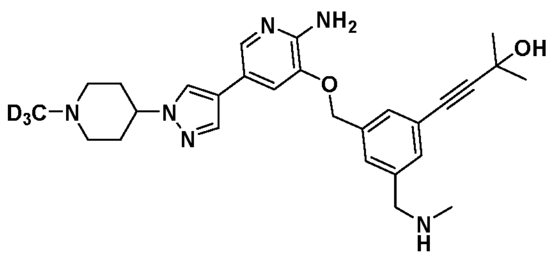

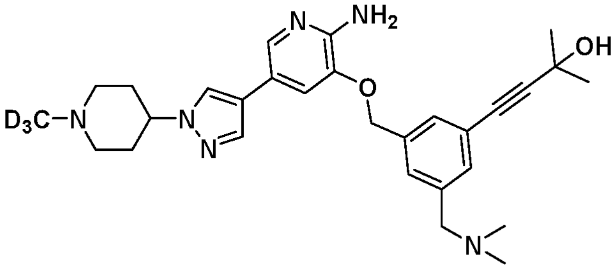

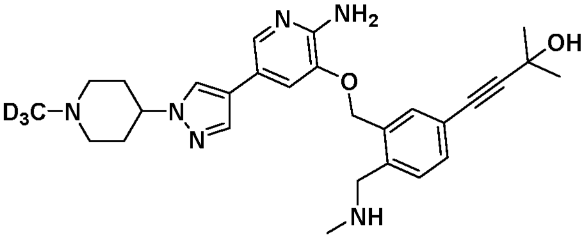

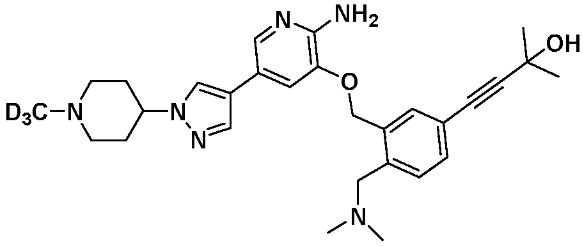

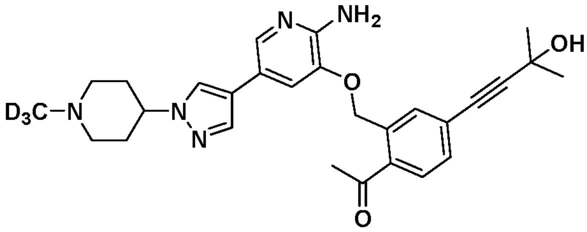

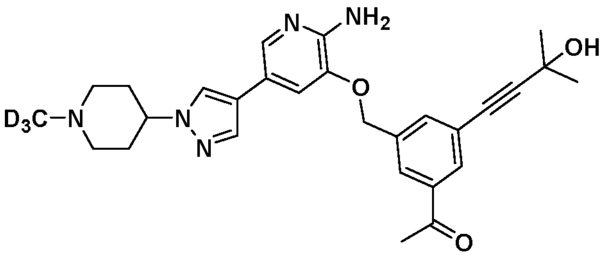

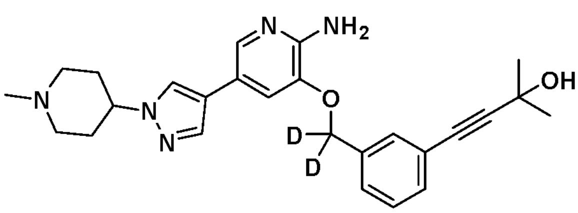

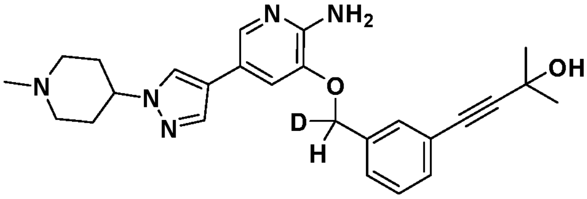

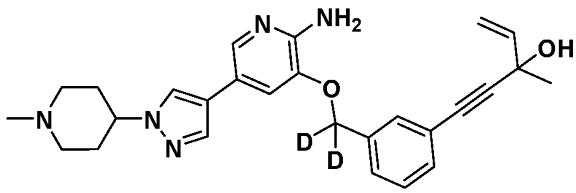

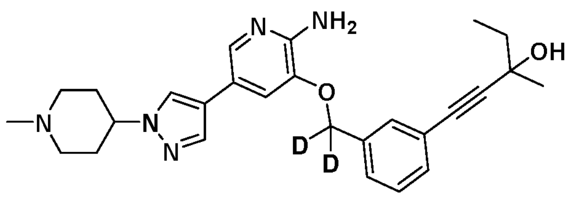

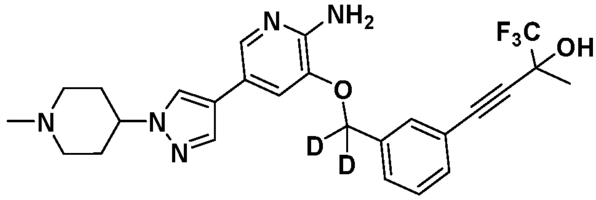

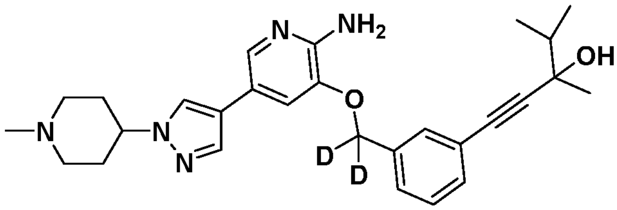

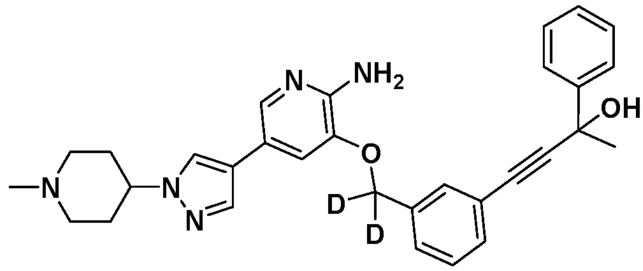

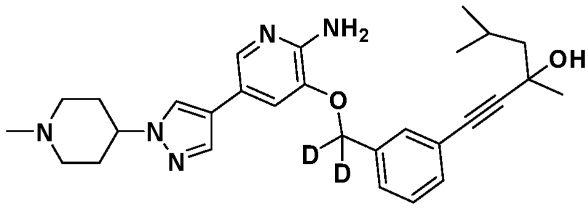

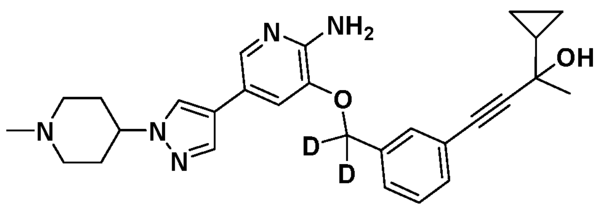

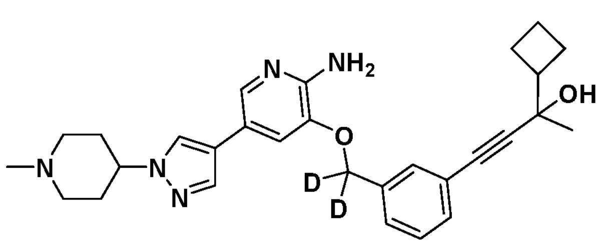

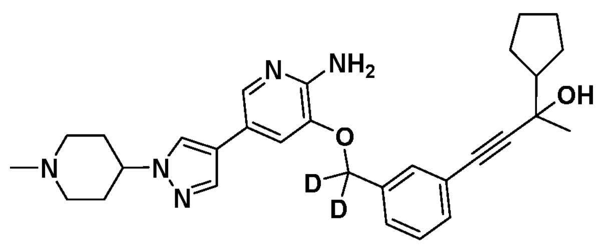

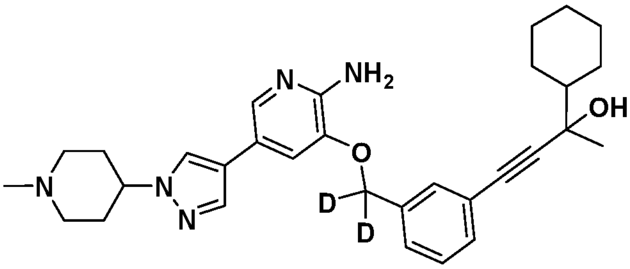

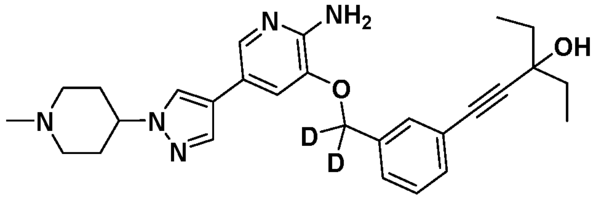

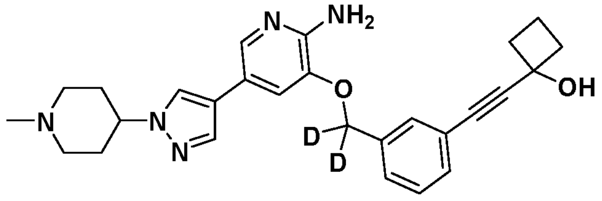

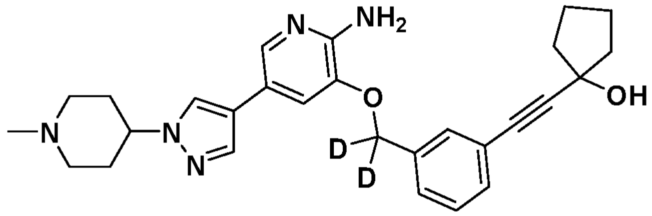

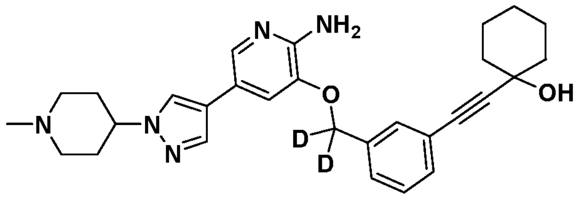

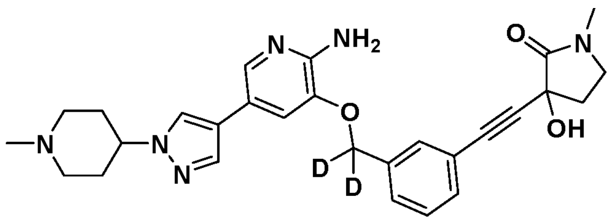

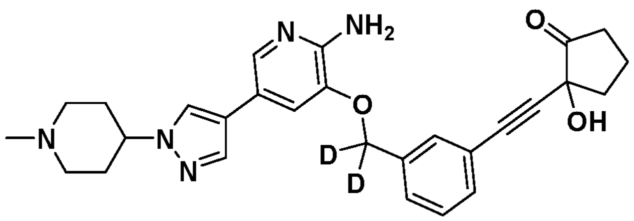

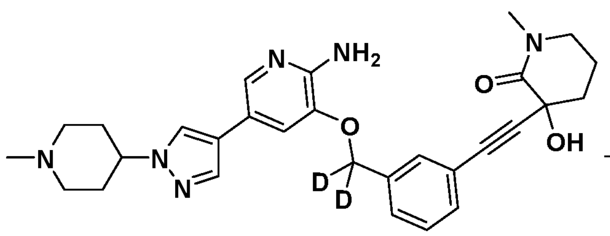

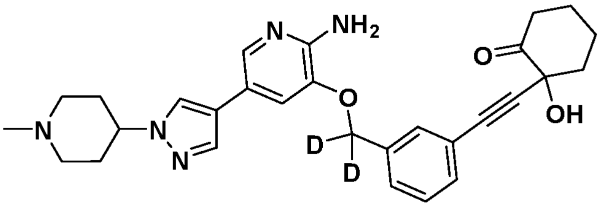

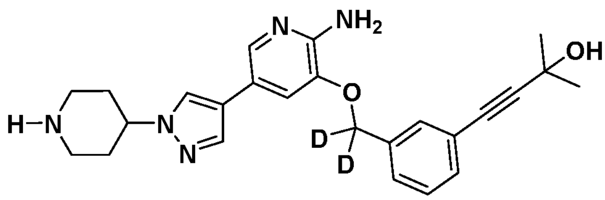

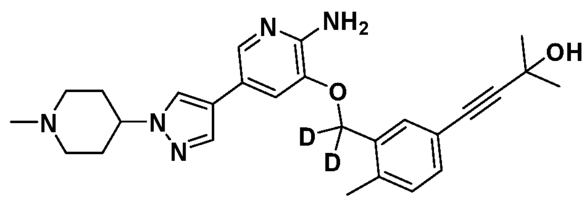

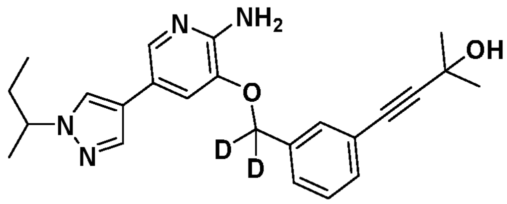

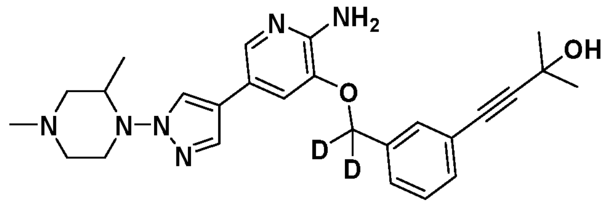

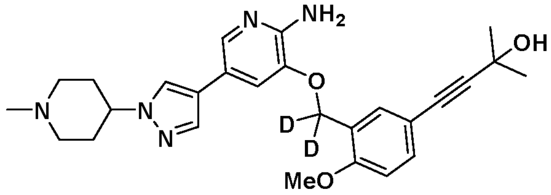

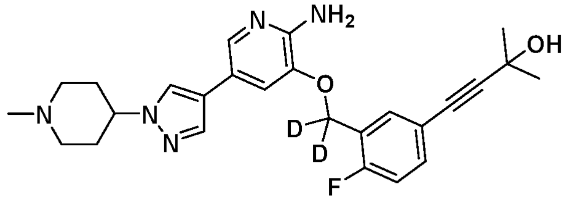

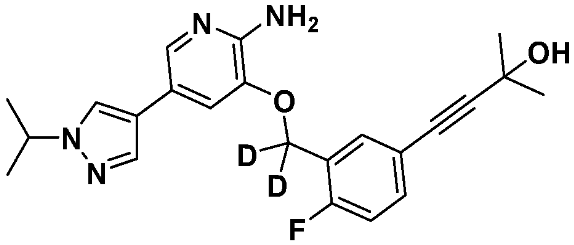

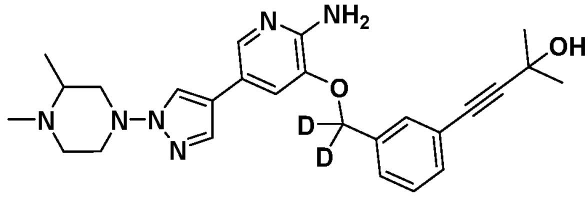

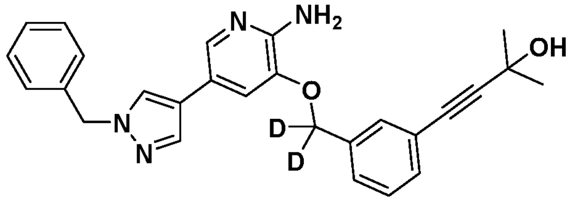

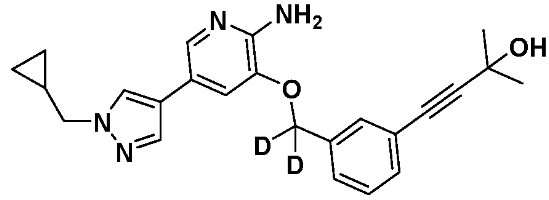

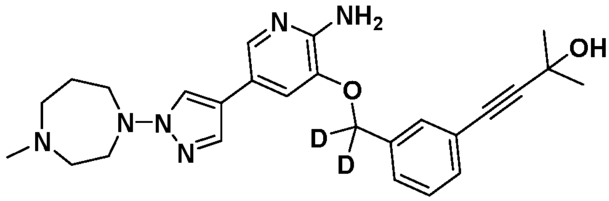

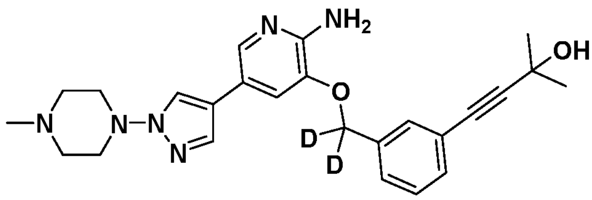

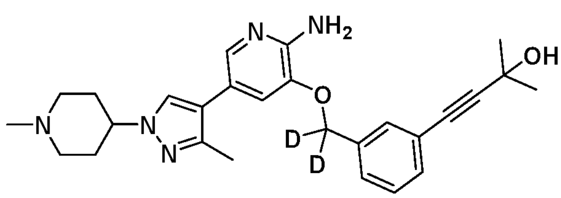

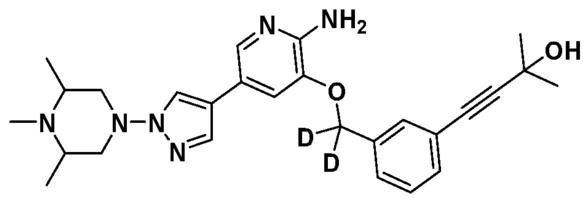

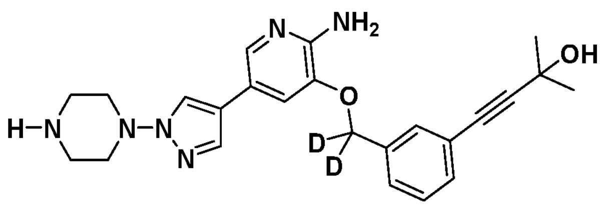

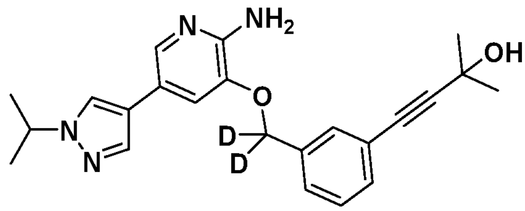

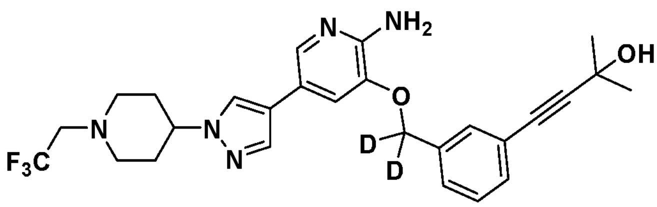

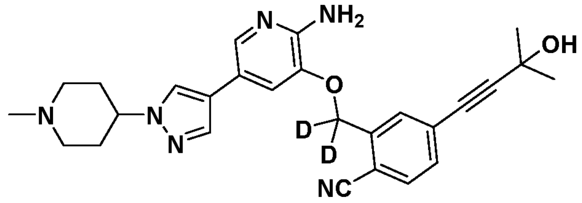

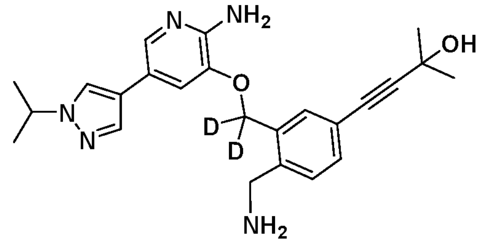

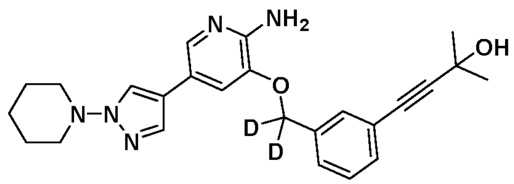

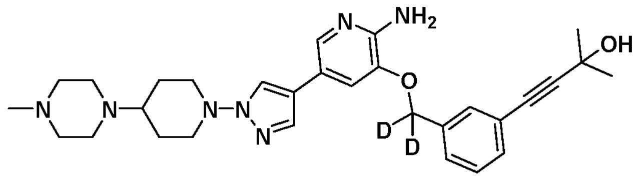

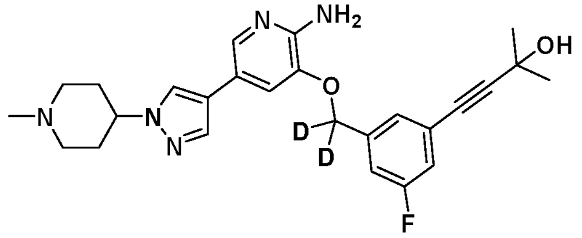

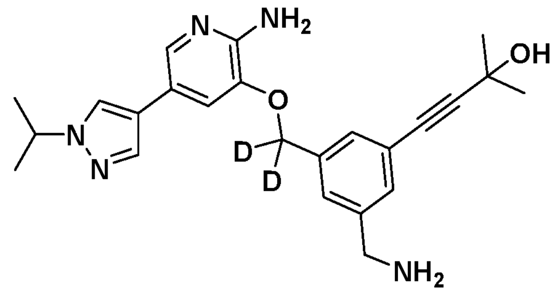

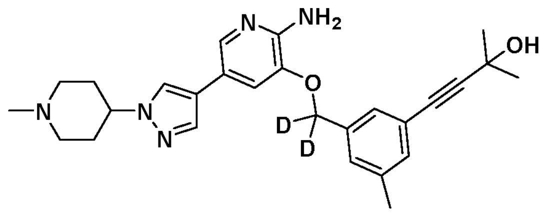

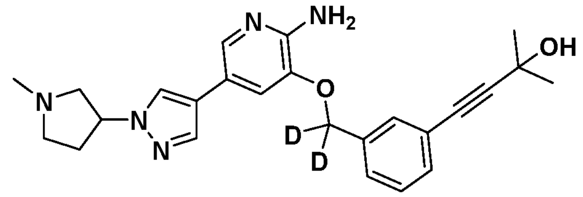

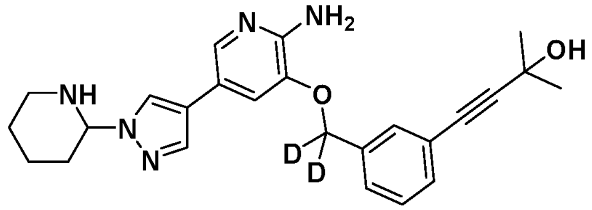

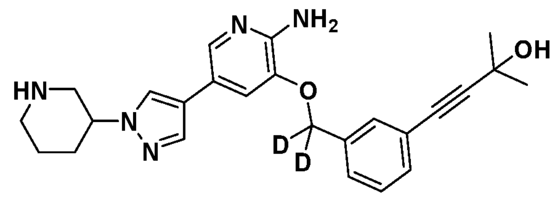

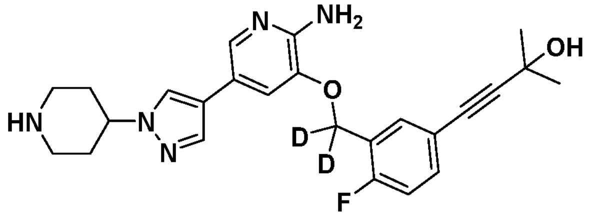

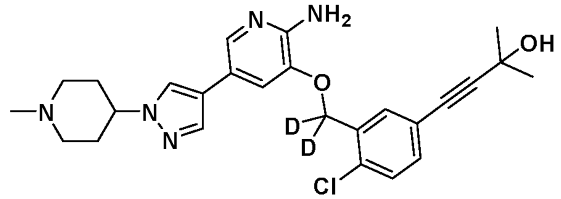

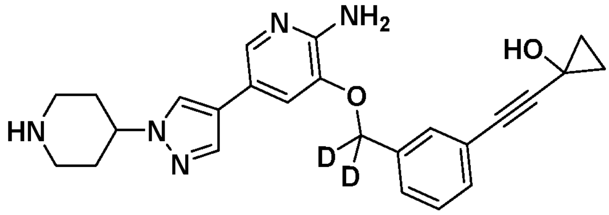

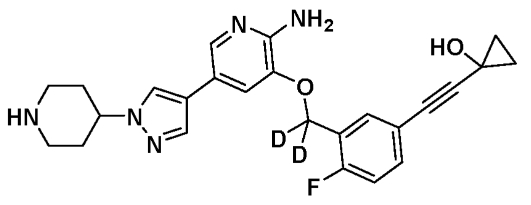

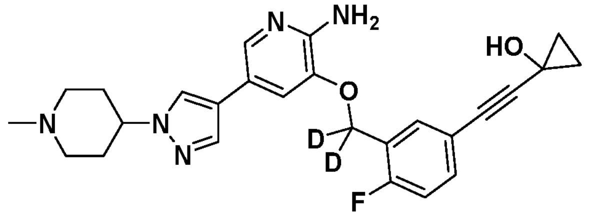

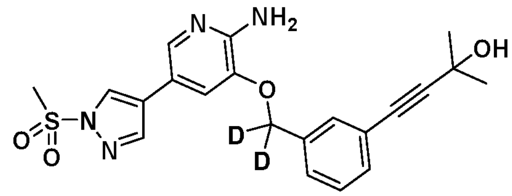

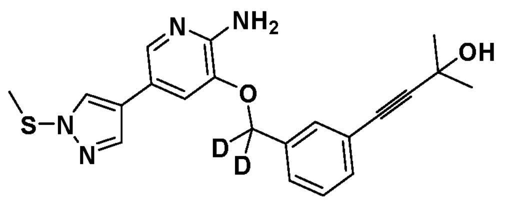

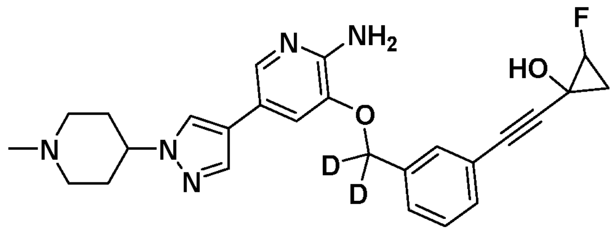

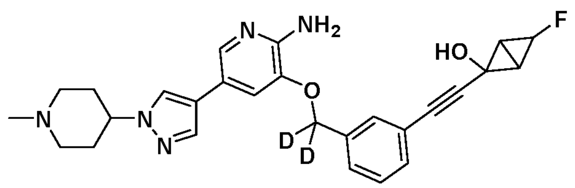

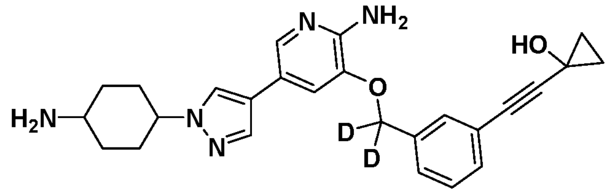

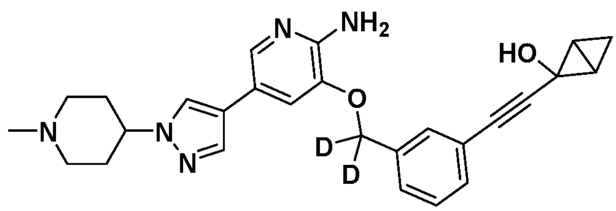

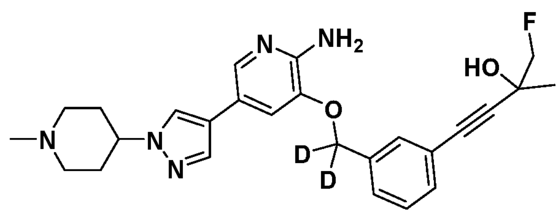

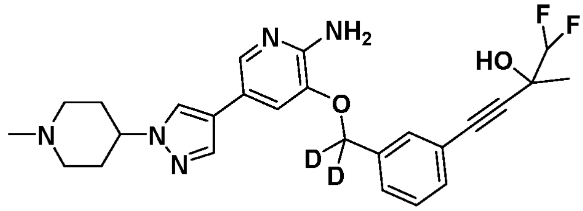

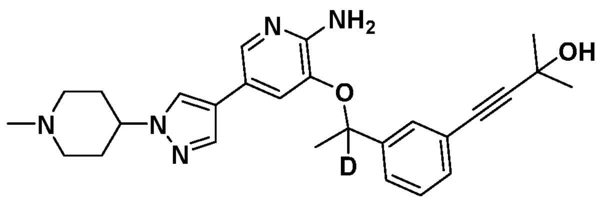

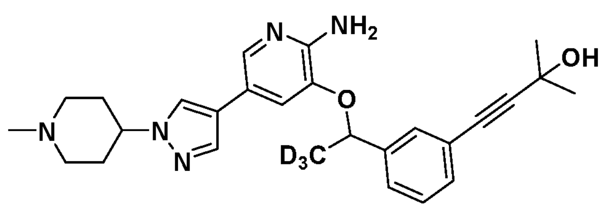

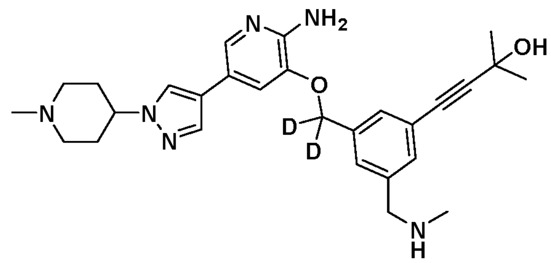

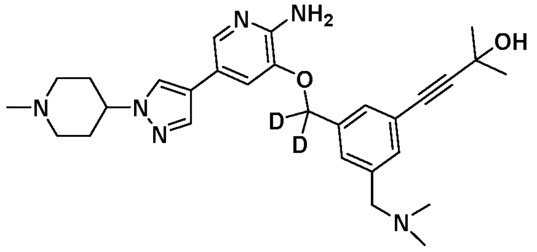

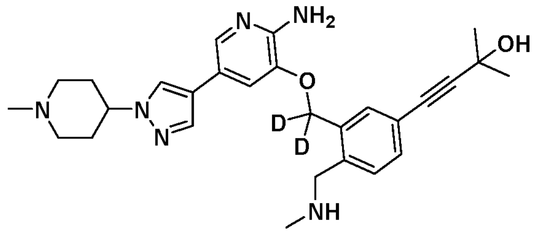

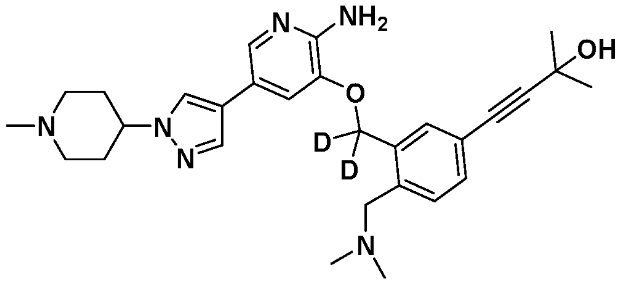

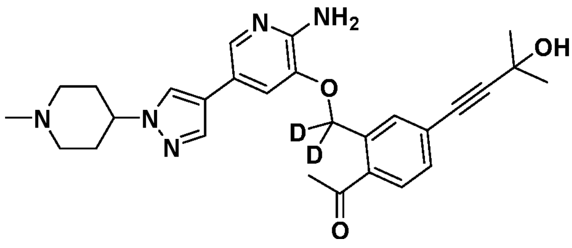

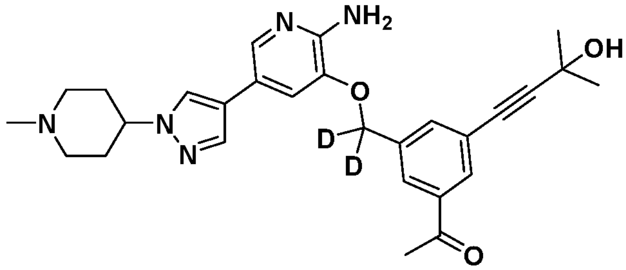

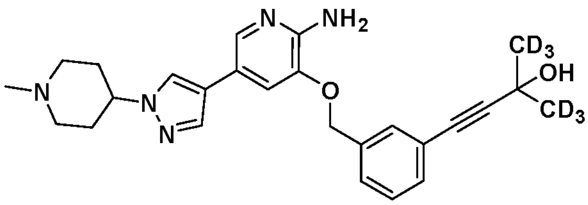

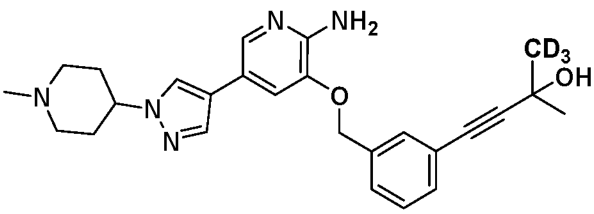

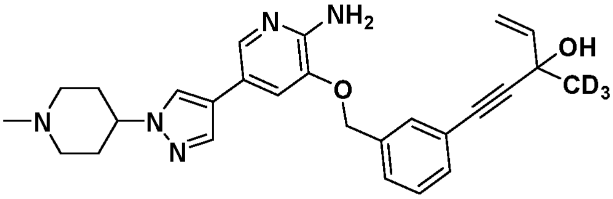

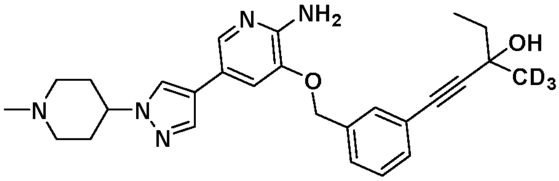

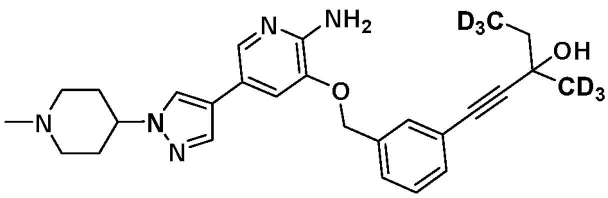

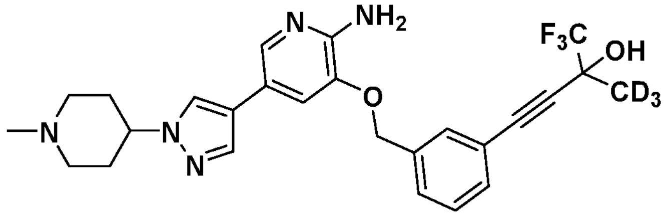

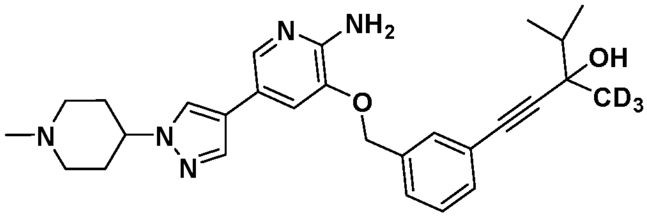

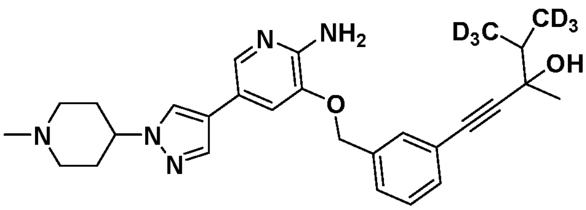

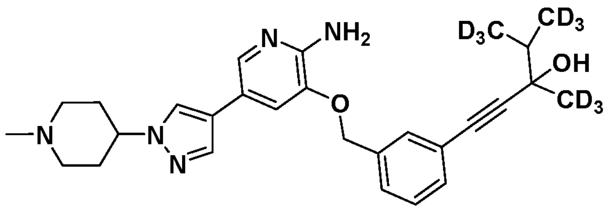

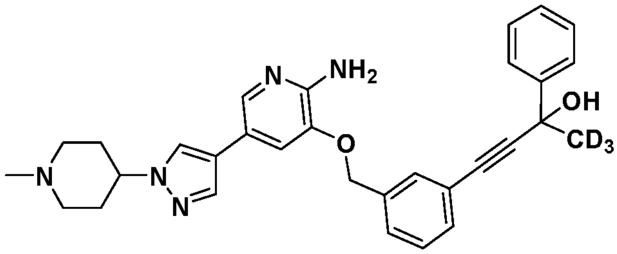

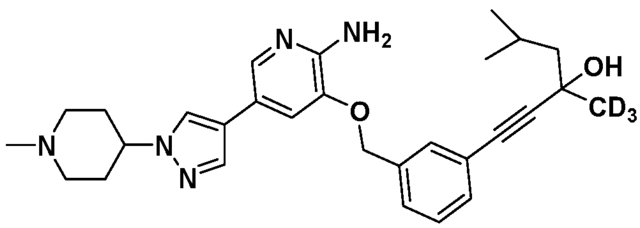

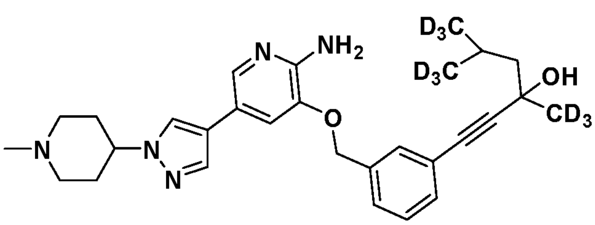

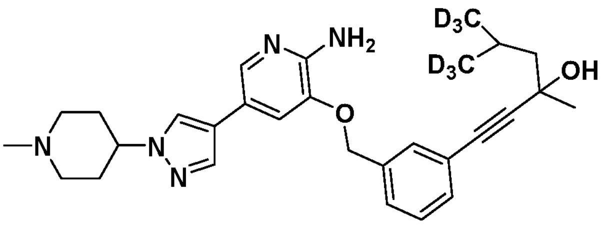

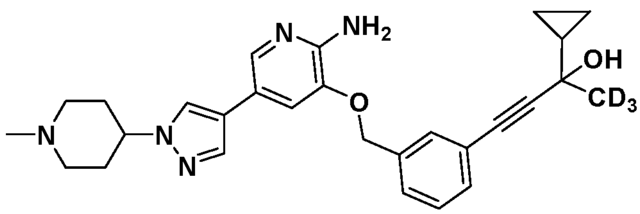

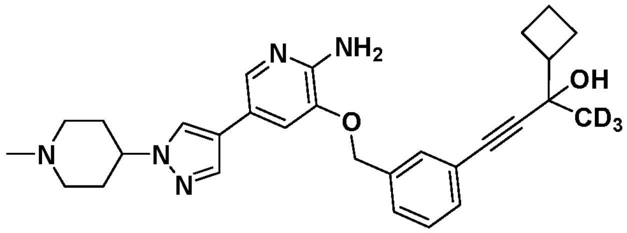

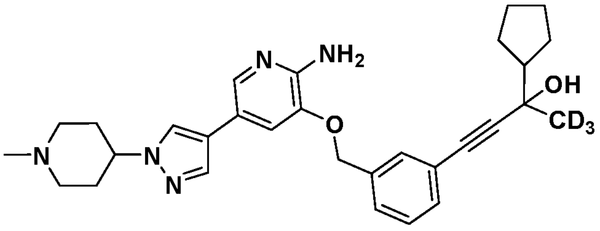

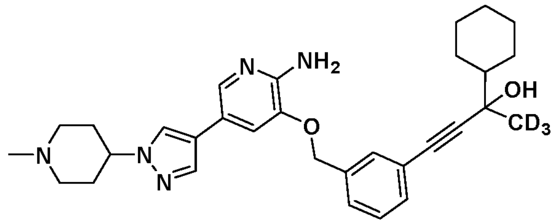

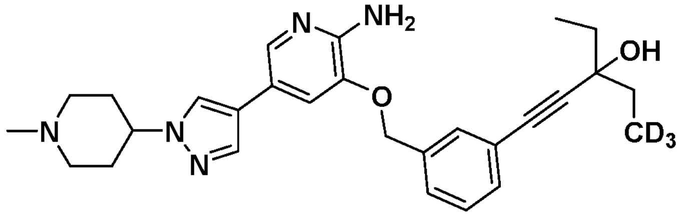

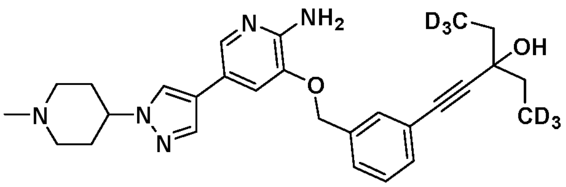

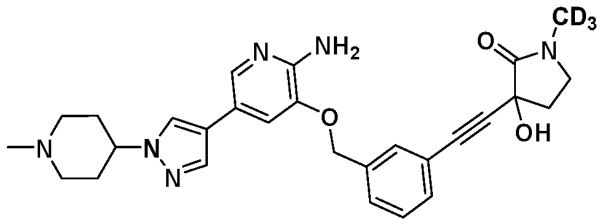

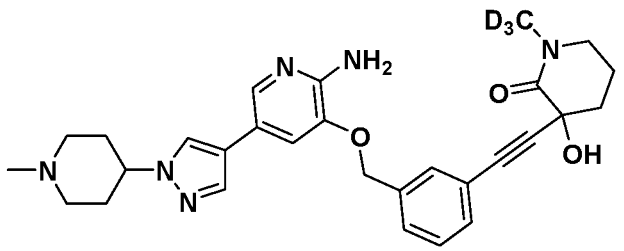

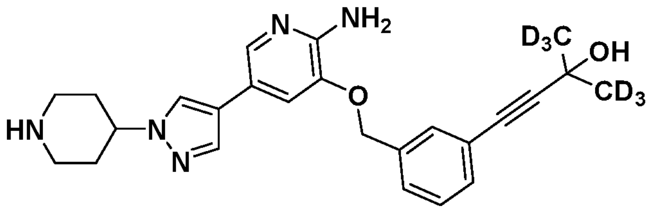

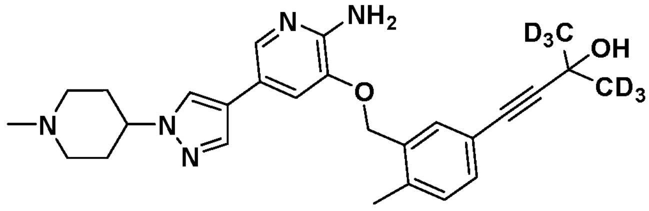

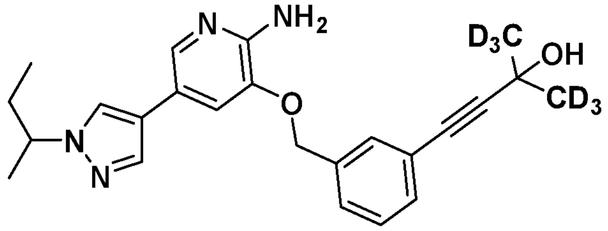

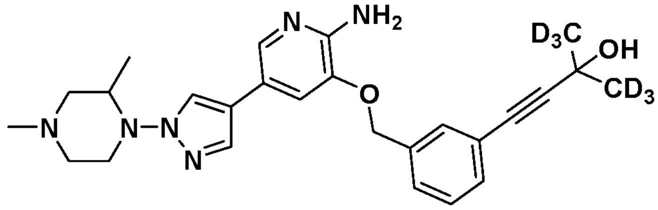

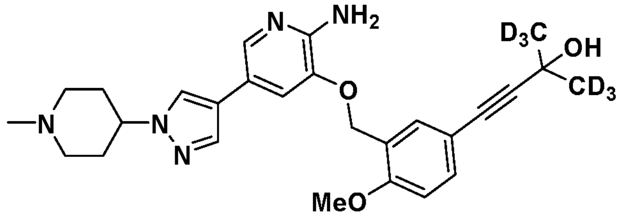

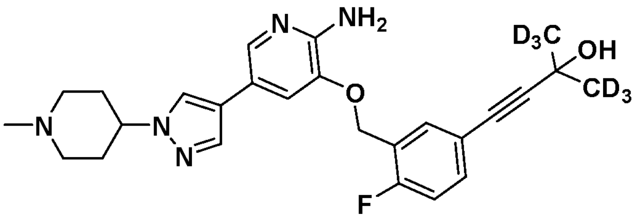

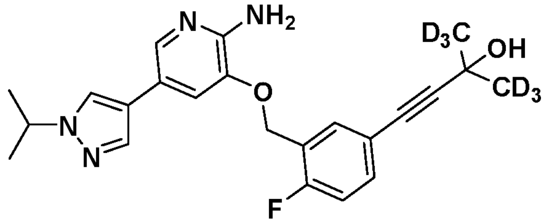

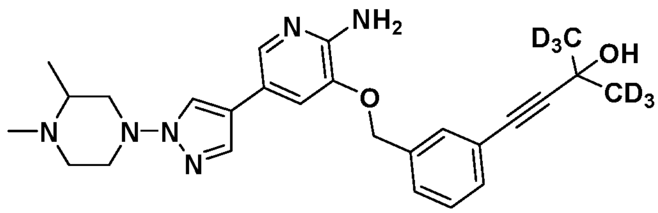

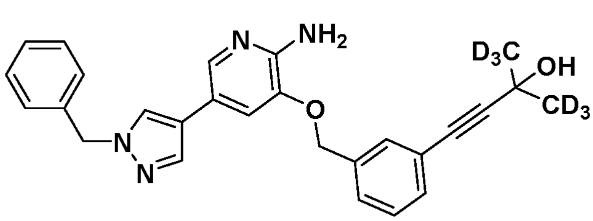

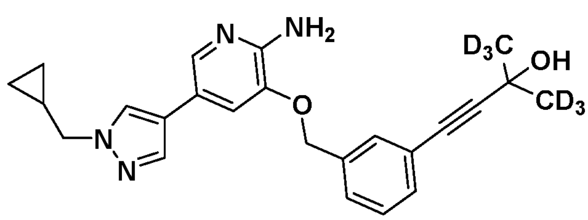

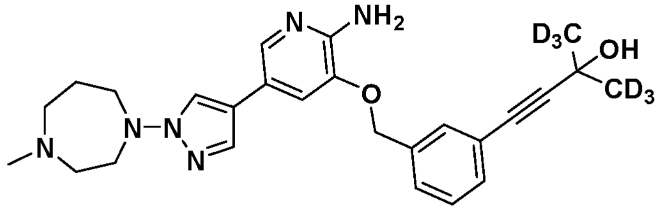

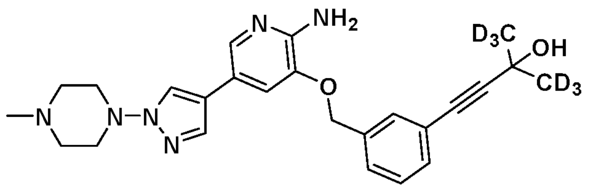

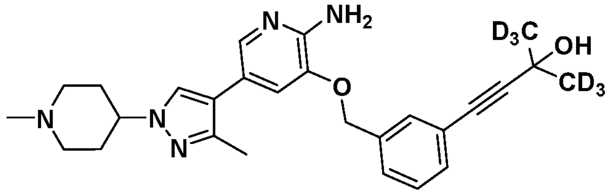

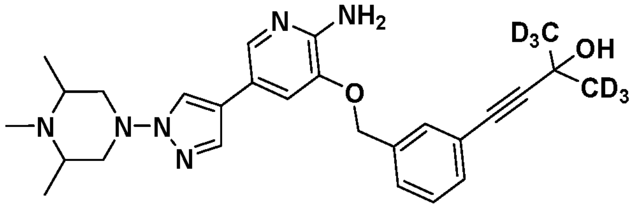

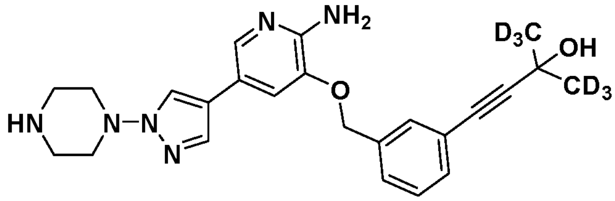

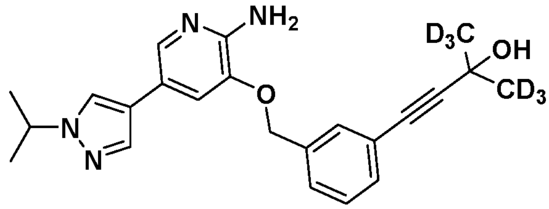

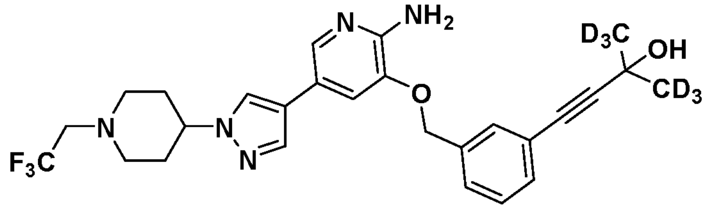

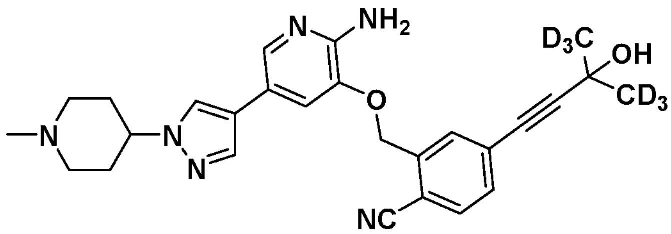

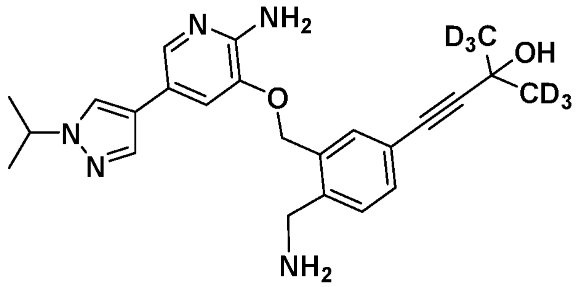

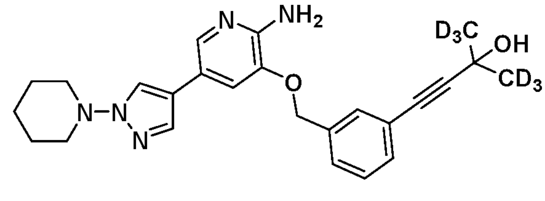

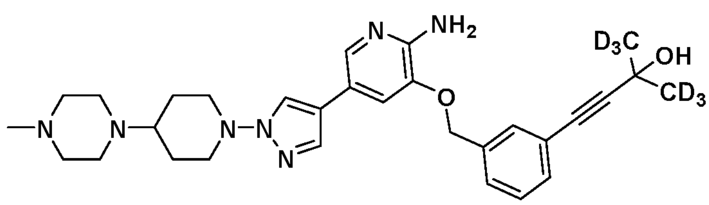

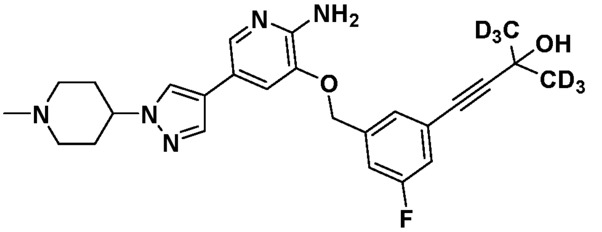

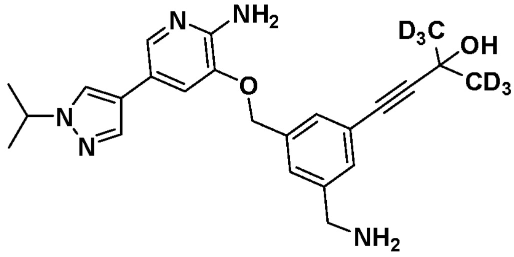

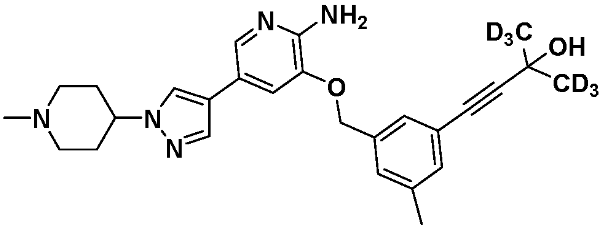

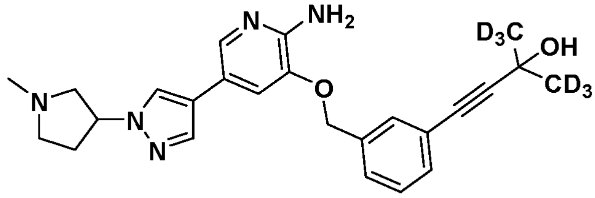

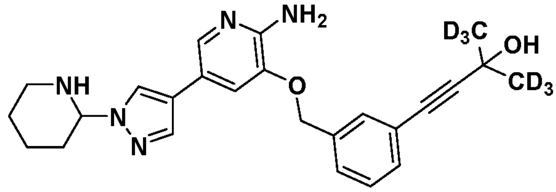

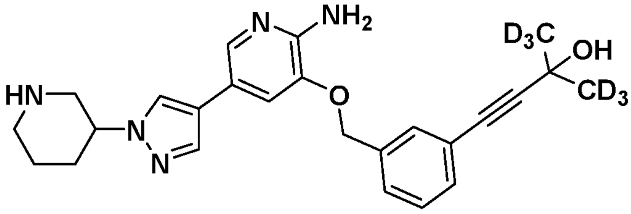

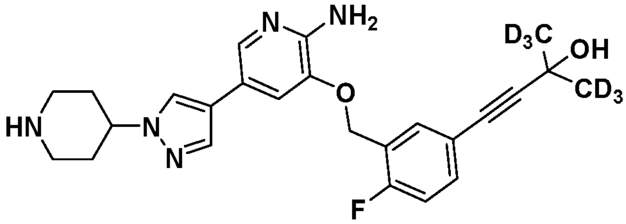

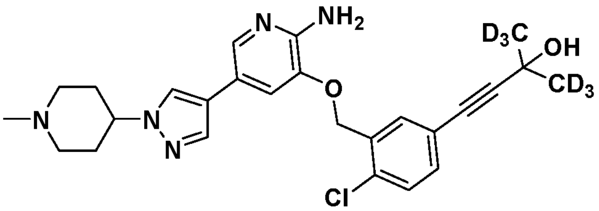

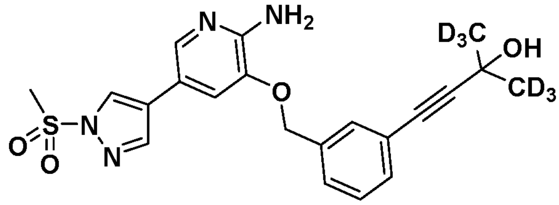

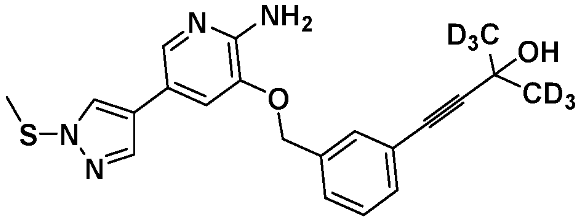

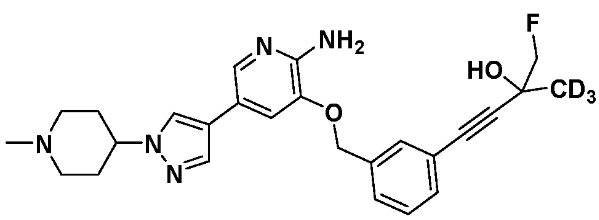

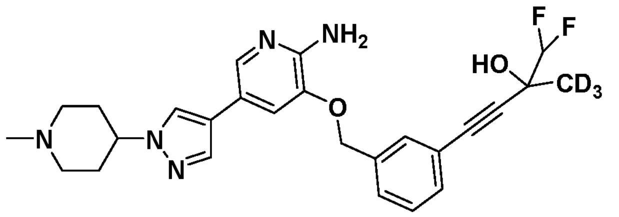

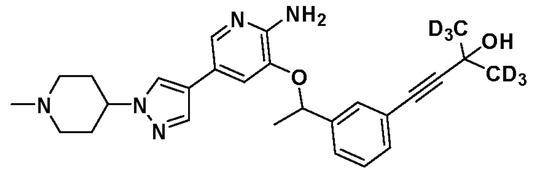

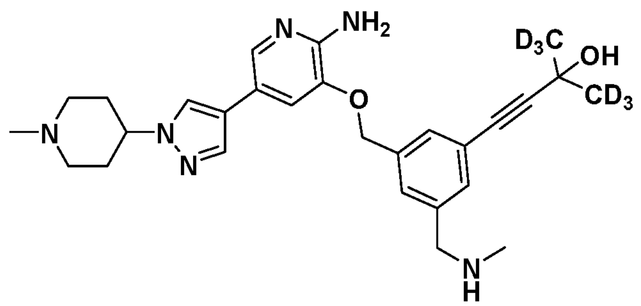

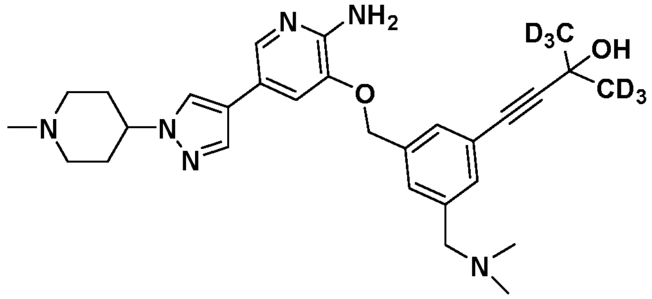

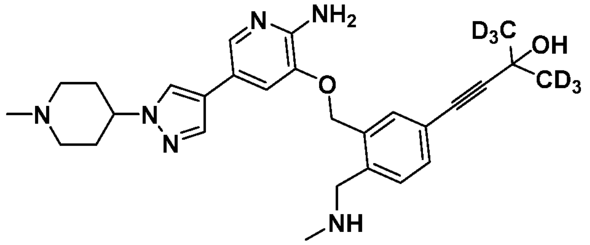

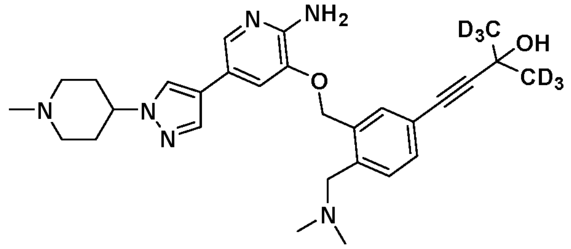

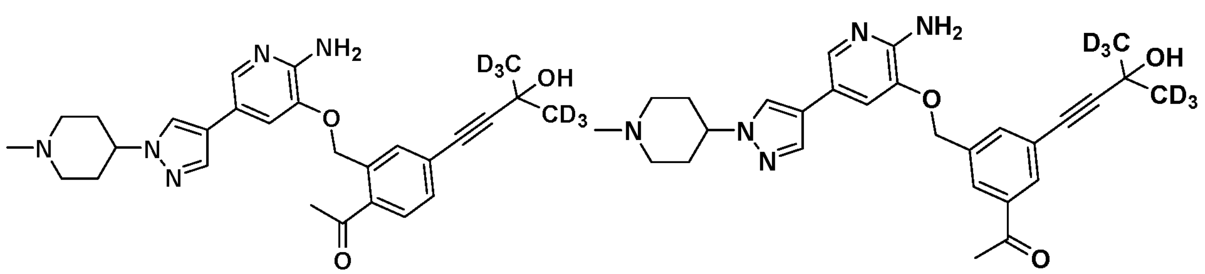

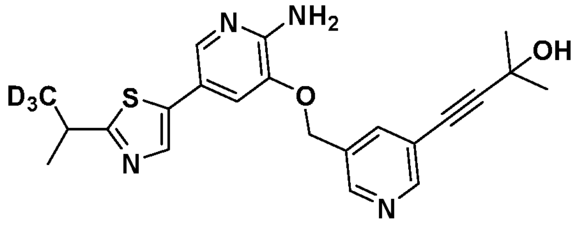

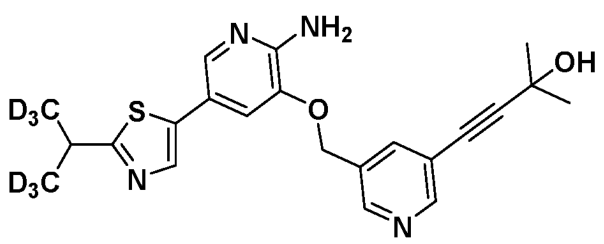

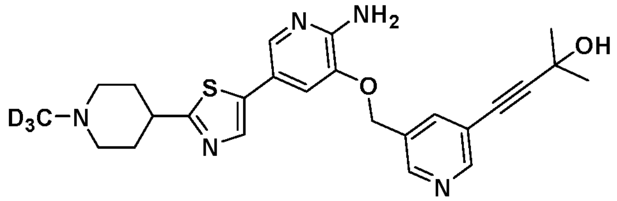

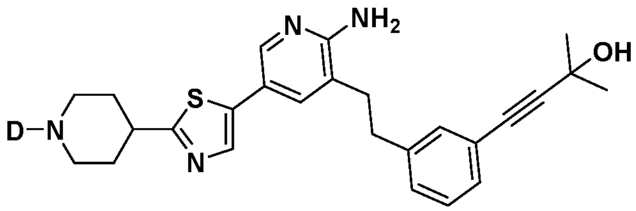

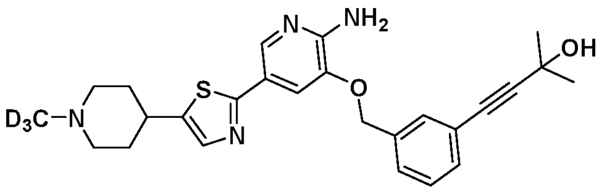

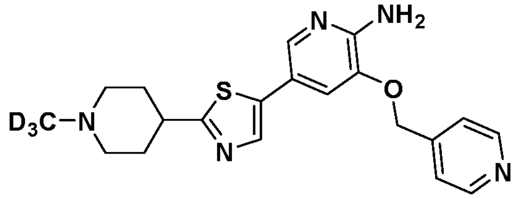

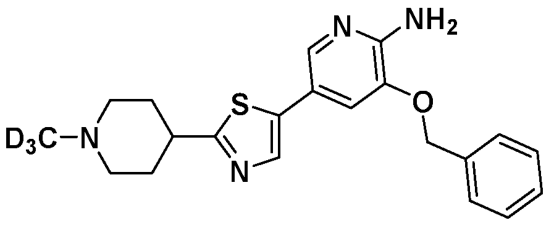

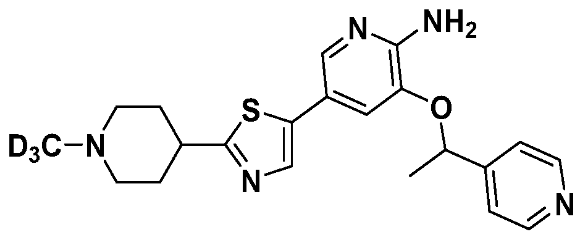

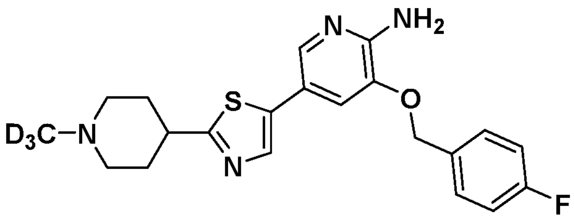

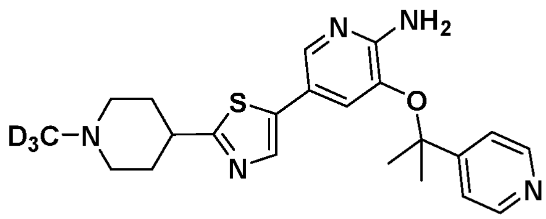

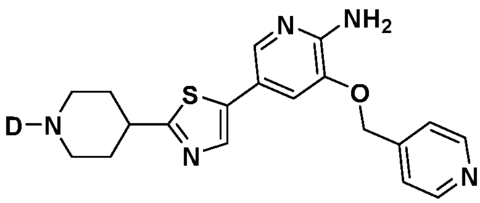

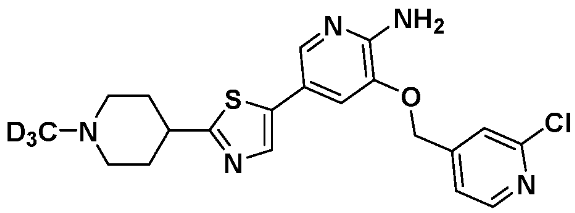

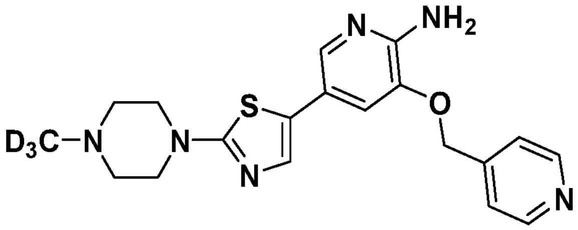

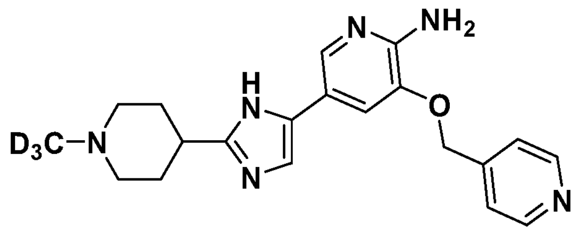

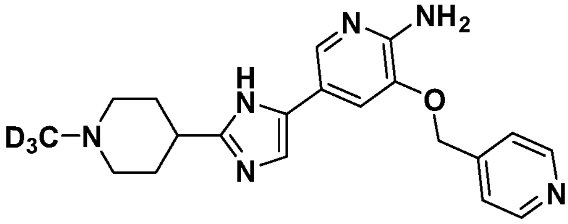

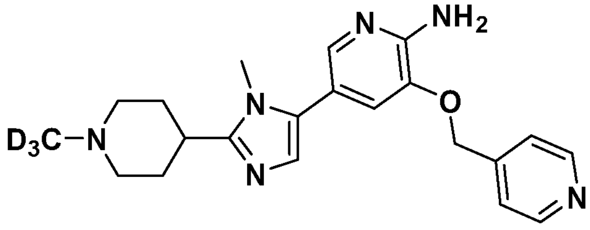

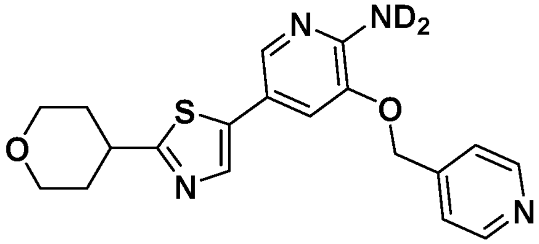

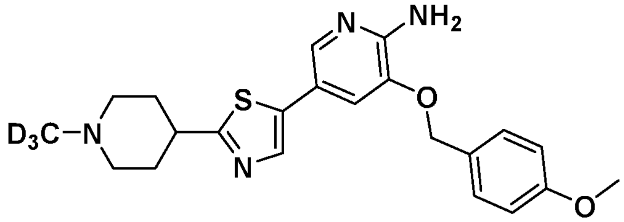

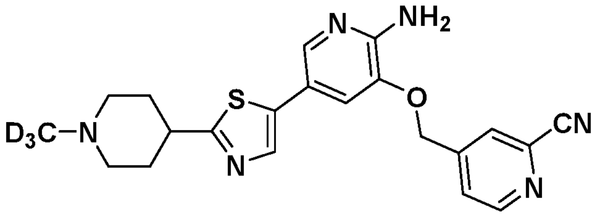

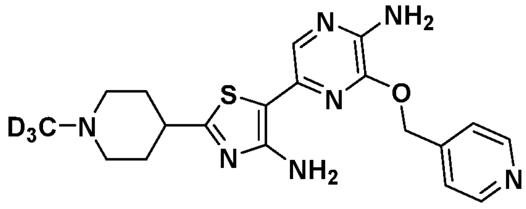

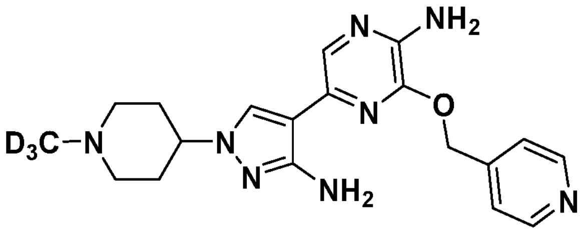

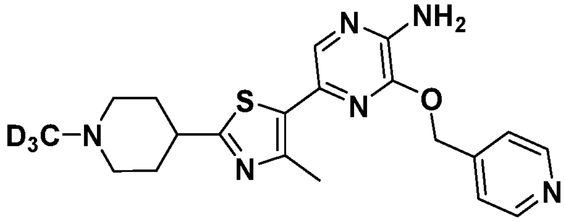

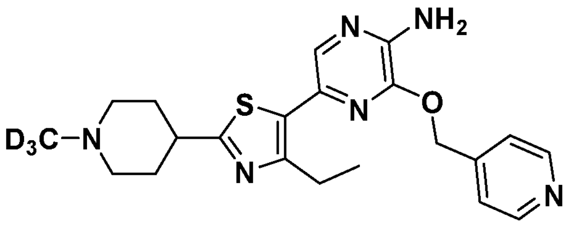

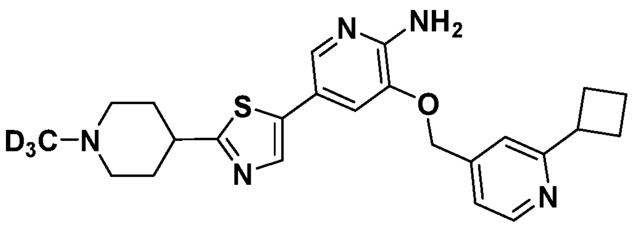

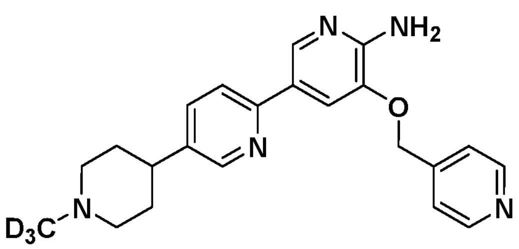

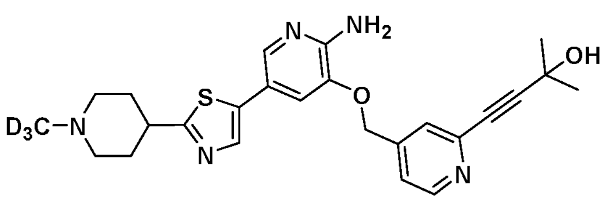

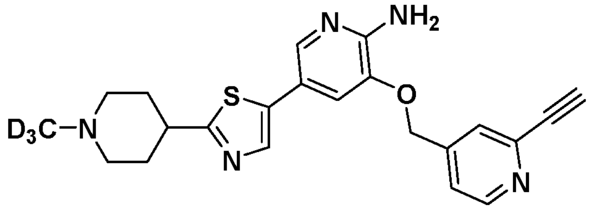

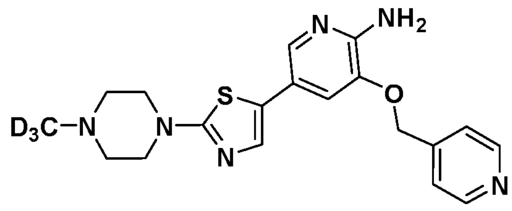

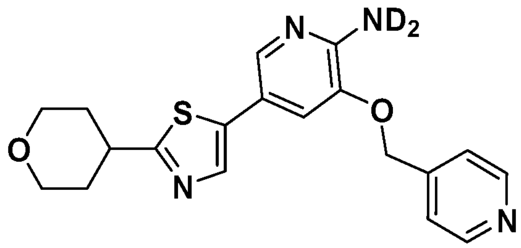

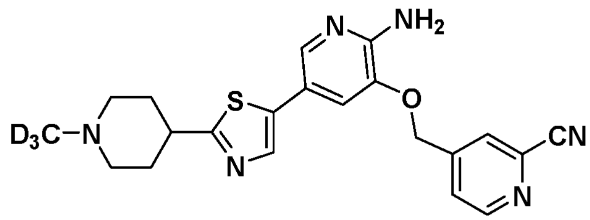

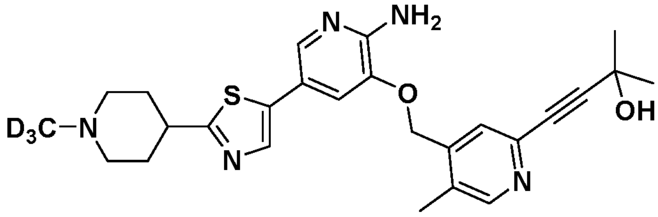

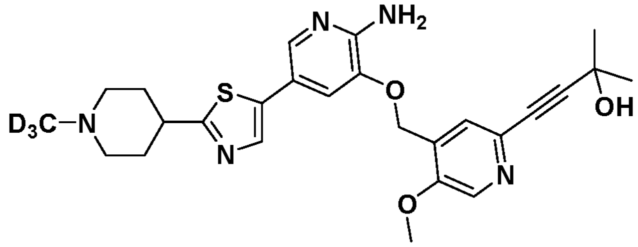

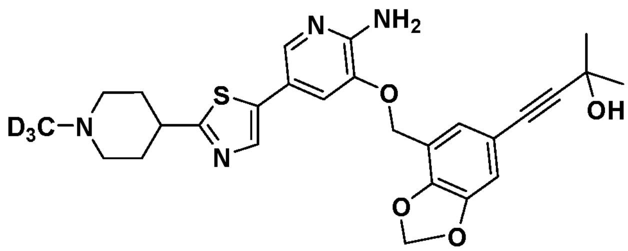

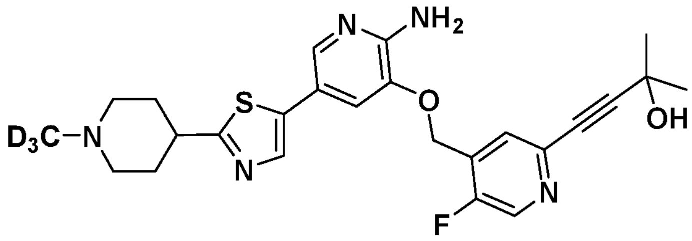

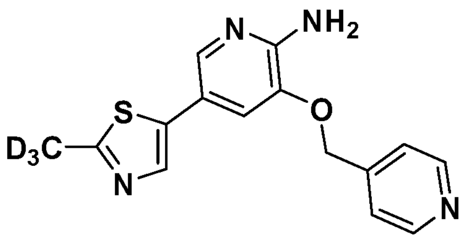

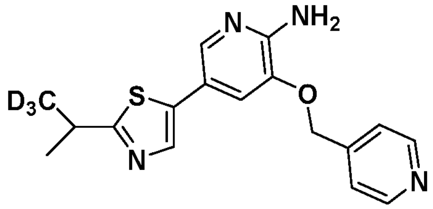

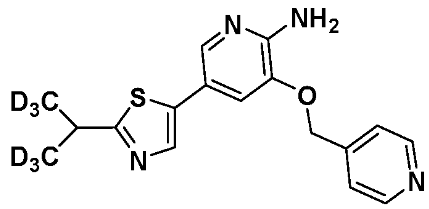

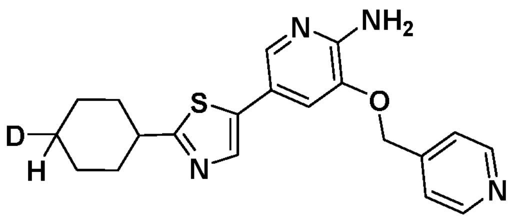

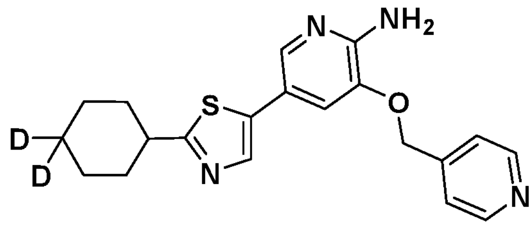

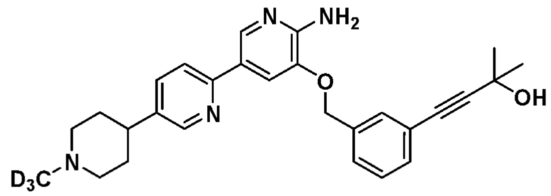

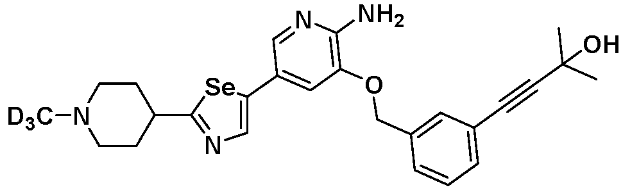

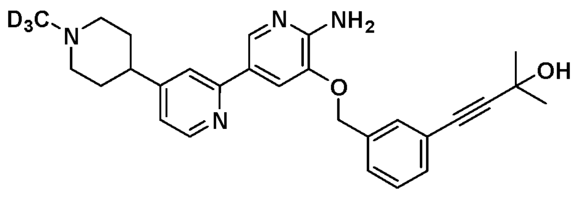

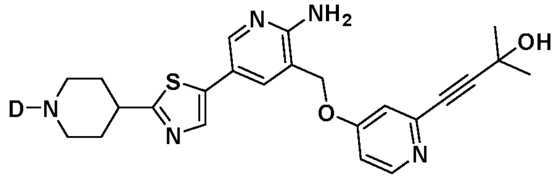

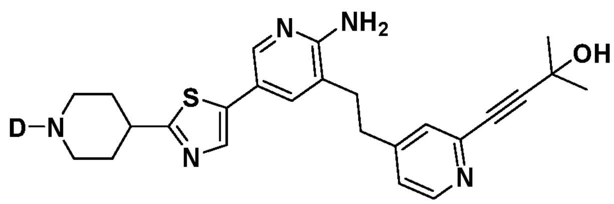

,

and

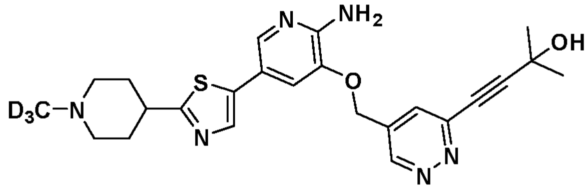

.

**17.** A crystalline form of the compound according to any one of claims 1-16, or pharmaceutically acceptable salts, stereoisomers, esters, prodrugs and solvates of the compound represented by formula I.

**18.** The crystalline form according to claim 17, wherein the crystalline form is a crystalline form of 4-(3-(((2-amino-5-(1-(1-trideuteriomethylpiperidin-4-yl)-1H-pyrazol-4-yl)pyridin-3-yl)oxy)meth yl)phenyl)-2-methylbut-3-yn-2-ol, which has an XRPD pattern with characteristic peaks at at least three of the positions with 2θ values of 13.1° ± 0.2°, 16.3° ± 0.2°, 17.5° ± 0.2° and 23.8° ± 0.2°.

**19.** The crystalline form according to claim 18, wherein the XRPD pattern of the crystalline form has further characteristic

peaks at at least three of the positions with 2θ values of 8.1° ± 0.2°, 12.2° ± 0.2°, 15.3° ± 0.2°, 18.0° ± 0.2°, 19.3° ± 0.2°, 19.5° ± 0.2°, 21.3° ± 0.2° and 21.6° ± 0.2°.

**20.** The crystalline form according to claim 18, wherein the crystalline form has an XRPD pattern substantially as shown in FIG. 1.

**21.** The crystalline form according to claim 17, wherein the crystalline form is a crystalline form of 4-(3-(((2-amino-5-(1-(1-trideuteriomethylpiperidin-4-yl)-1*H*-pyrazol-4-yl)pyridin-3-yl)oxy)meth yl)phenyl)-2-methylbut-3-yn-2-ol, which has an XRPD pattern with characteristic peaks at at least three of the positions with 2θ values of 5.7° ± 0.2°, 11.3° ± 0.2°, 22.7° ± 0.2° and 23.5° ± 0.2°.

**22.** The crystalline form according to claim 21, wherein the XRPD pattern of the crystalline form has further characteristic peaks at at least three of the positions with 2θ values of 7.1° ± 0.2°, 8.8° ± 0.2°, 14.1° ± 0.2°, 17.0° ± 0.2°, 18.0° ± 0.2° and 18.8° ± 0.2°.

**23.** The crystalline form according to claim 21, wherein the crystalline form has an XRPD pattern substantially as shown in FIG. 6.

**24.** The crystalline form according to claim 17, wherein the crystalline form is a crystalline form of 4-(3-(((2-amino-5-(1-(1-trideuteriomethylpiperidin-4-yl)-1*H*-pyrazol-4-yl)pyridin-3-yl)oxy)meth yl)phenyl)-2-methylbut-3-yn-2-ol hydrochloride, which has an XRPD pattern with characteristic peaks at at least three of the positions at 2θ values of 13.0° ± 0.2°, 16.3° ± 0.2°, 17.5° ± 0.2°, 19.4° ± 0.2° and 23.8° ± 0.2°.

**25.** The crystalline form according to claim 24, wherein the XRPD pattern of the crystalline form has further characteristic peaks at at least three of the positions with 2θ values of 8.1° ± 0.2°, 12.1° ± 0.2°, 15.3° ± 0.2°, 18.0° ± 0.2° and 21.4° ± 0.2°.

**26.** The crystalline form according to claim 24, wherein the crystalline form has an XRPD pattern substantially as shown in FIG. 11.

**27.** A pharmaceutical composition comprising the compound or pharmaceutically acceptable salts, stereoisomers, esters, prodrugs and solvates of the compound represented by formula I according to any one of claims 1-16, or the crystalline form according to any one of claims 17-26, and pharmaceutically acceptable excipients.

**28.** Use of the compound or pharmaceutically acceptable salts, stereoisomers, esters, prodrugs and solvates of the compound represented by formula I according to any one of claims 1-16, or the crystalline form according to any one of claims 17-26, or the pharmaceutical composition according to claim 23, in preparation of drugs for preventing and/or treating tumors.

**29.** The use according to claim 28, wherein the tumors are selected from lymphoma, blastoma, medulloblastoma, retinoblastoma, sarcoma, liposarcoma, synovial cell sarcoma, neuroendocrine tumor, carcinoid tumor, gastrinoma, islet cell carcinoma, mesothelioma, schwannoma, acoustic neuroma, meningioma, adenocarcinoma, melanoma, leukemia or lymphoid malignancies, squamous cell carcinoma, epithelial squamous cell carcinoma, lung cancer, small cell lung cancer, non-small cell lung cancer, lung adenocarcinoma, lung squamous cell carcinoma, peritoneal cancer, hepatocellular cancer, gastric cancer, intestinal cancer, pancreatic cancer, glioblastoma, cervical cancer, ovarian cancer, liver cancer, bladder cancer, liver cancer, breast cancer, metastatic breast cancer, colon cancer, rectal cancer, colorectal cancer, uterine cancer, salivary gland cancer, kidney cancer, prostate cancer, vulval cancer, thyroid cancer, liver cancer, anal cancer, penile cancer, merkel cell cancer, esophageal cancer, biliary tract cancer, head and neck cancer and hematologic malignancies.

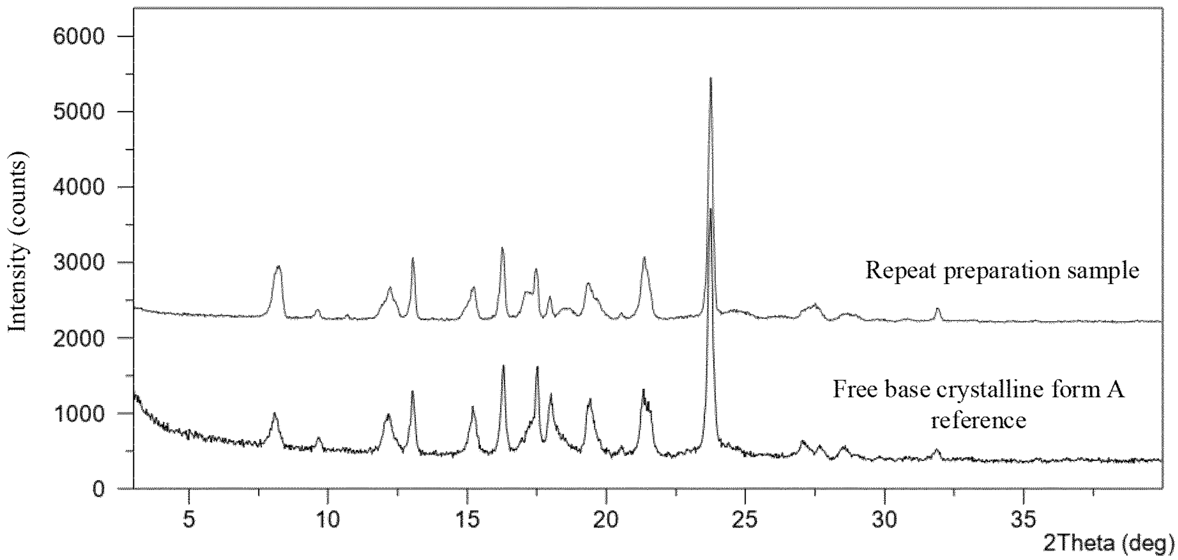
6000
5000
4000
3000
2000
1000
0
Intensity (counts)
Repeat preparation sample
Free base crystalline form A reference
5
10
15
20
25
30
35
2Theta (deg)

FIG. 1

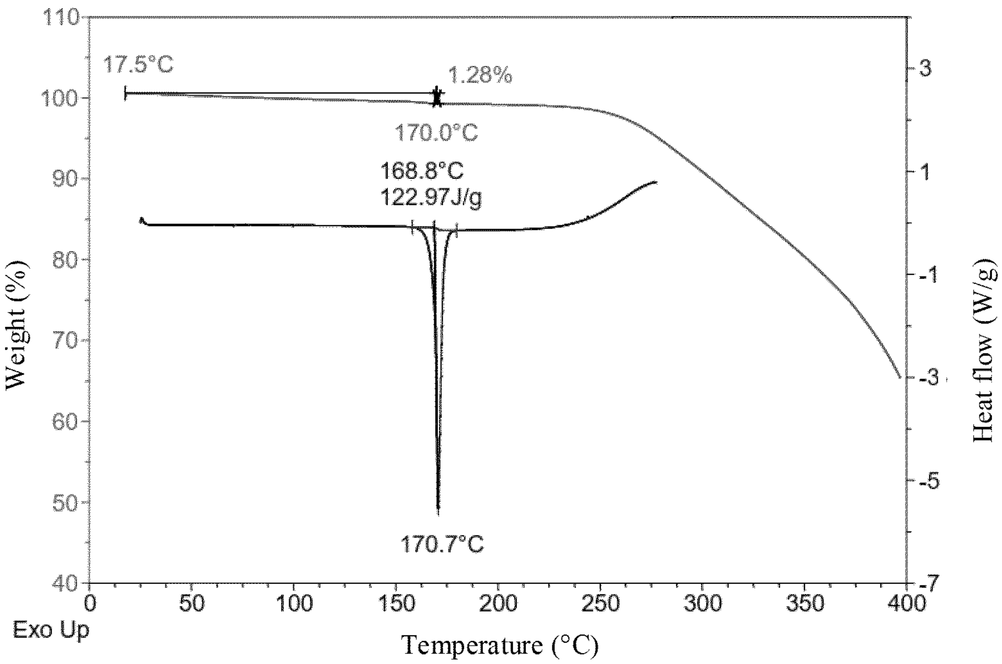
17.5°C
1.28%
170.0°C
168.8°C
122.97J/g
170.7°C
Weight (%)
Heat flow (W/g)
Exo Up
Temperature (°C)

FIG. 2

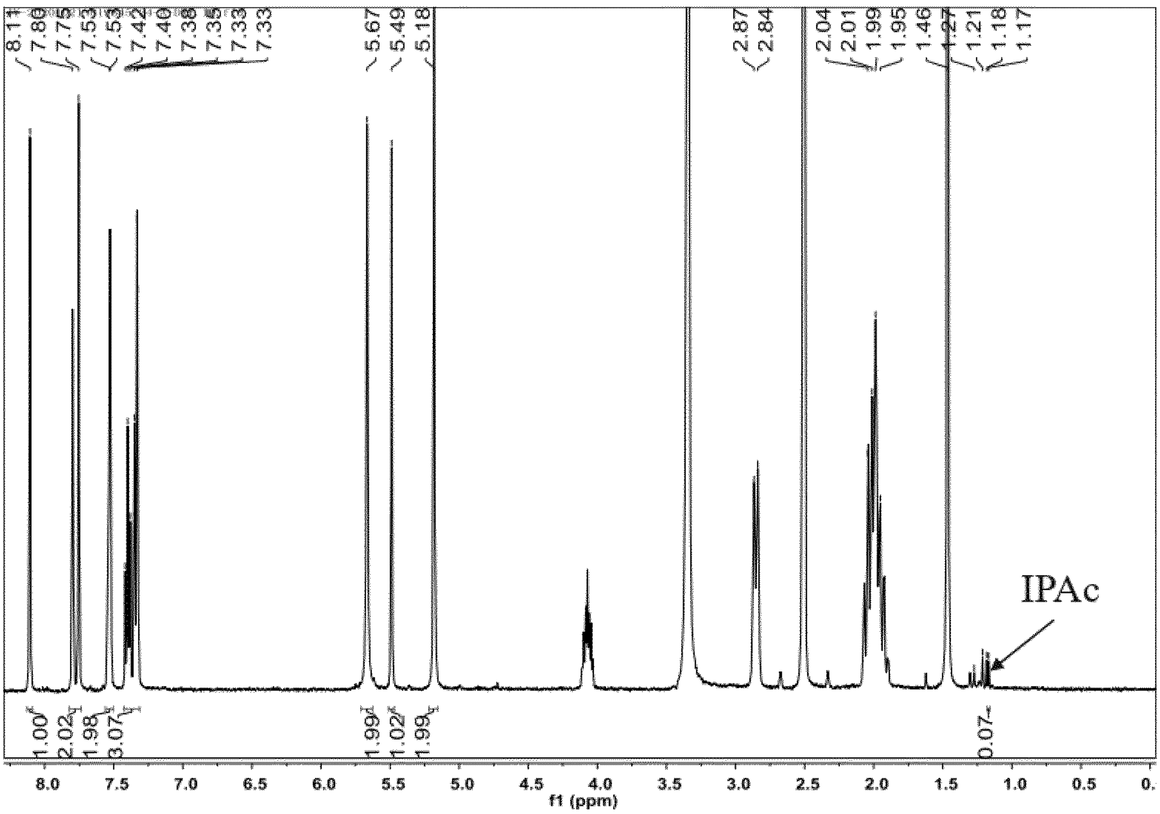
8.11
7.80
7.75
7.53
7.53
7.42
7.40
7.38
7.35
7.33
7.33
5.67
5.49
5.18
2.87
2.84
2.04
2.01
1.99
1.95
1.46
1.27
1.21
1.18
1.17
IPAc
1.00
2.02
1.98
3.07
1.99
1.02
1.99
0.07
f1 (ppm)

FIG. 3

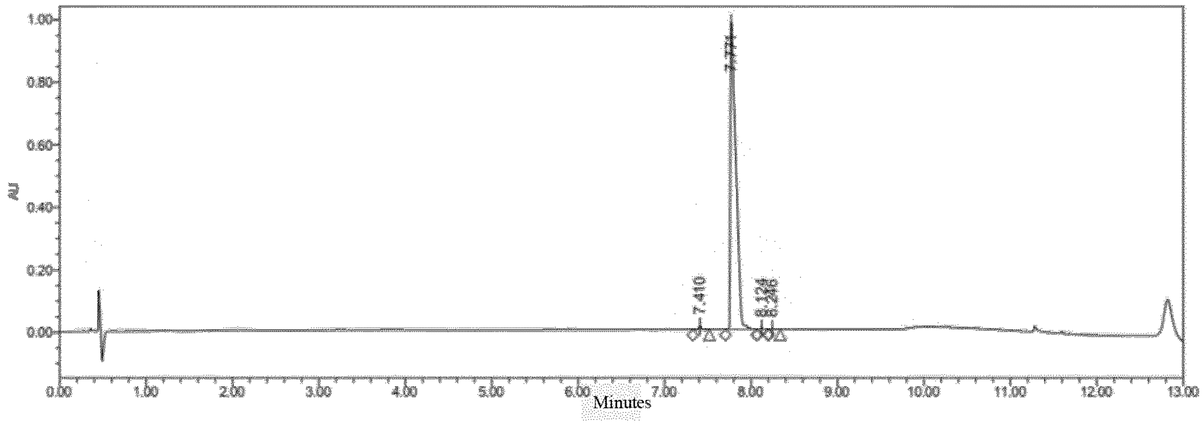


FIG. 4

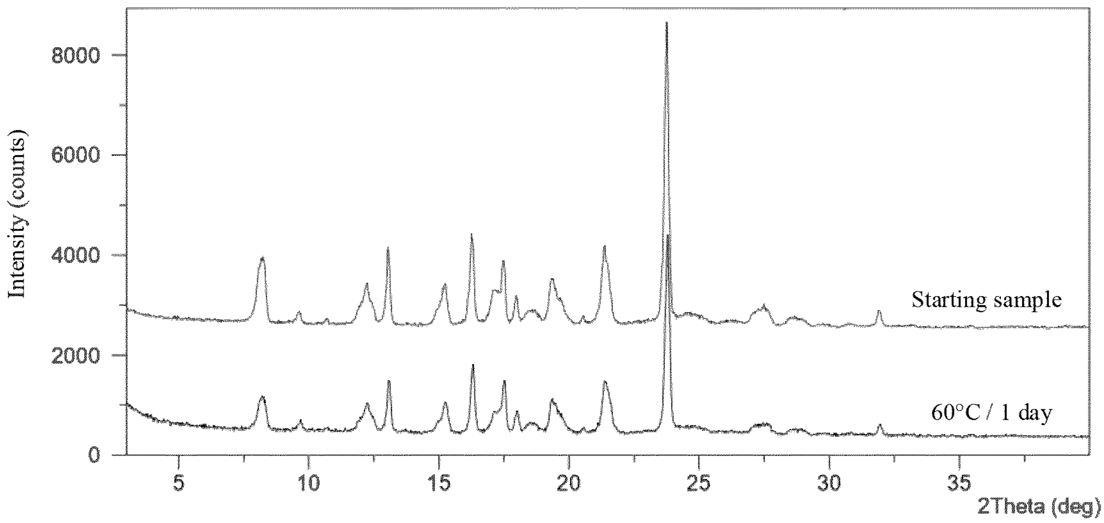


FIG. 5

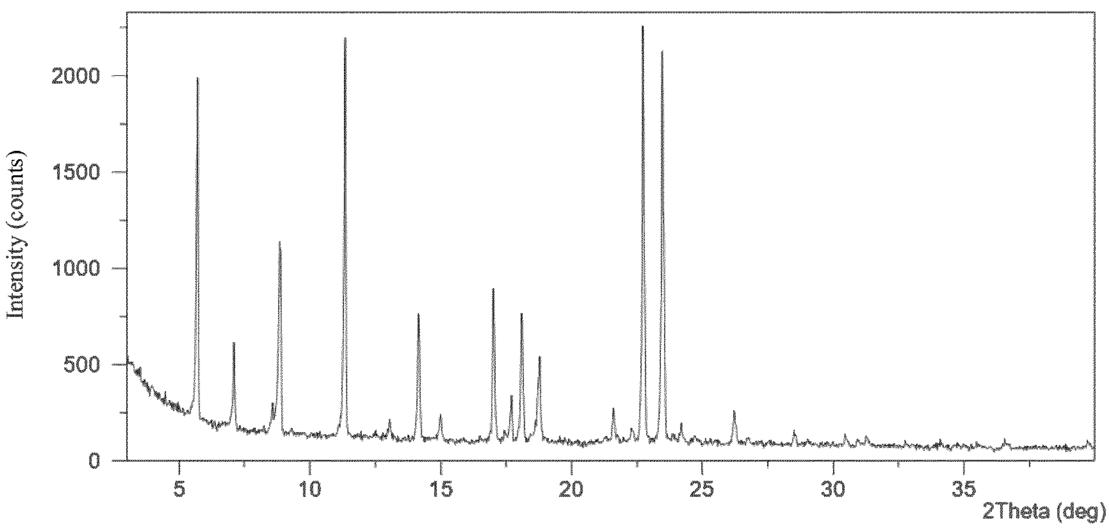


FIG. 6

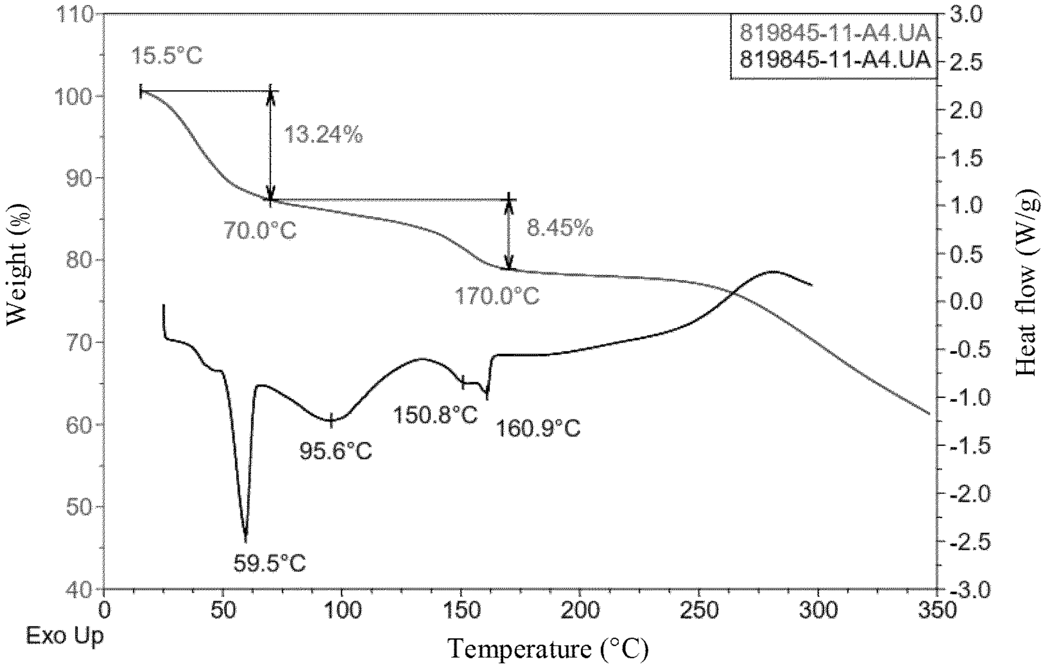

819845-11-A4.UA
819845-11-A4.UA
15.5°C
13.24%
70.0°C
8.45%
170.0°C
95.6°C
150.8°C
160.9°C
59.5°C
Weight (%)
Heat flow (W/g)
Temperature (°C)
Exo Up


FIG. 7

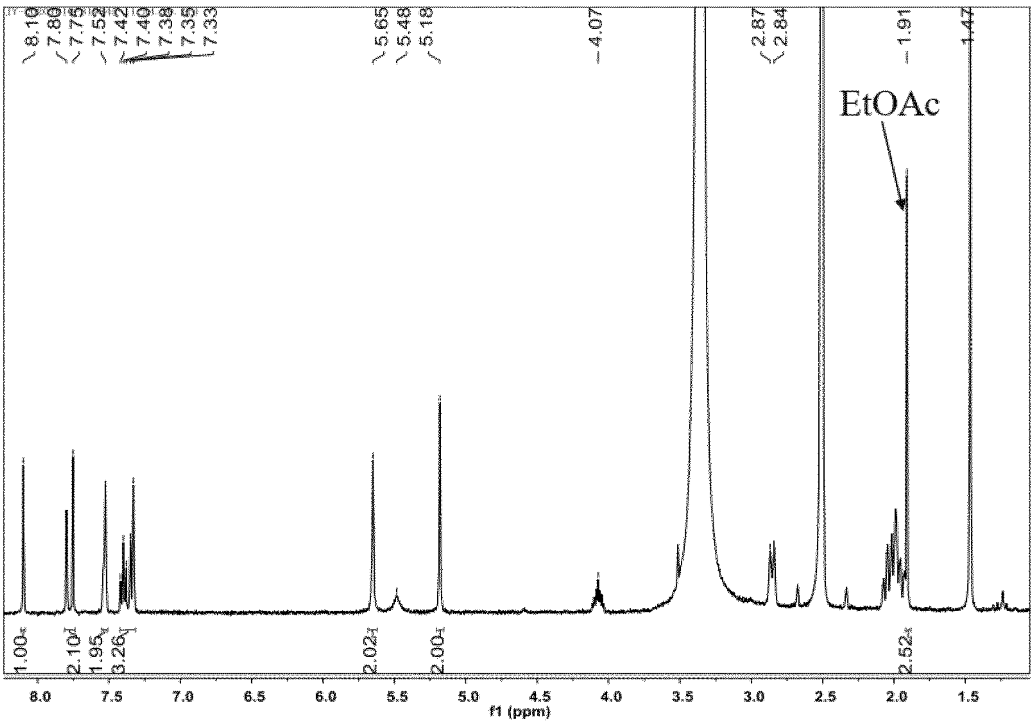

8.10
7.80
7.75
7.52
7.42
7.40
7.38
7.35
7.33
5.65
5.48
5.18
4.07
2.87
2.84
1.91
1.47
EtOAc
1.00
2.10
1.95
3.26
2.02
2.00
2.52
f1 (ppm)


FIG. 8

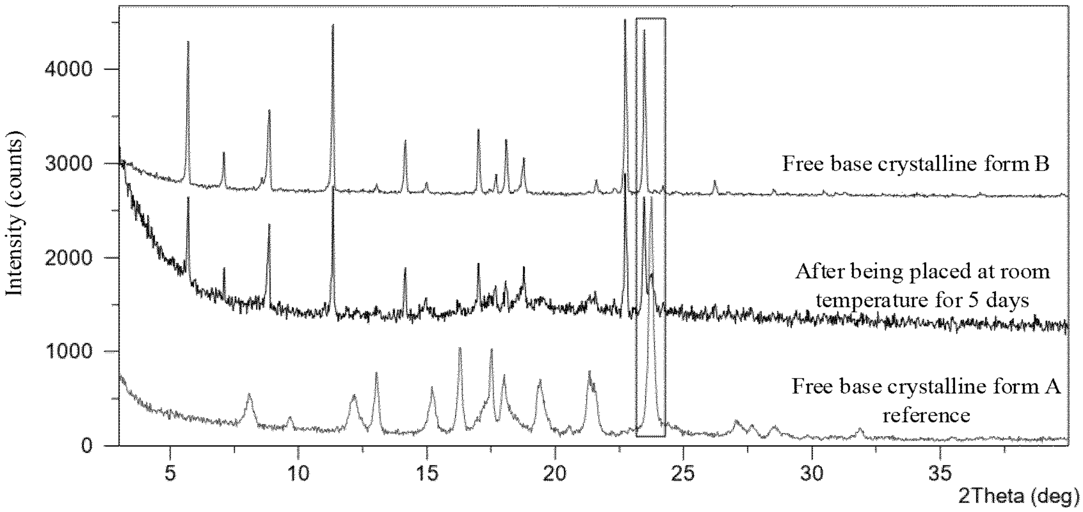
Intensity (counts)
4000
3000
2000
1000
0
5
10
15
20
25
30
35
2Theta (deg)
Free base crystalline form B
After being placed at room temperature for 5 days
Free base crystalline form A reference

FIG. 9

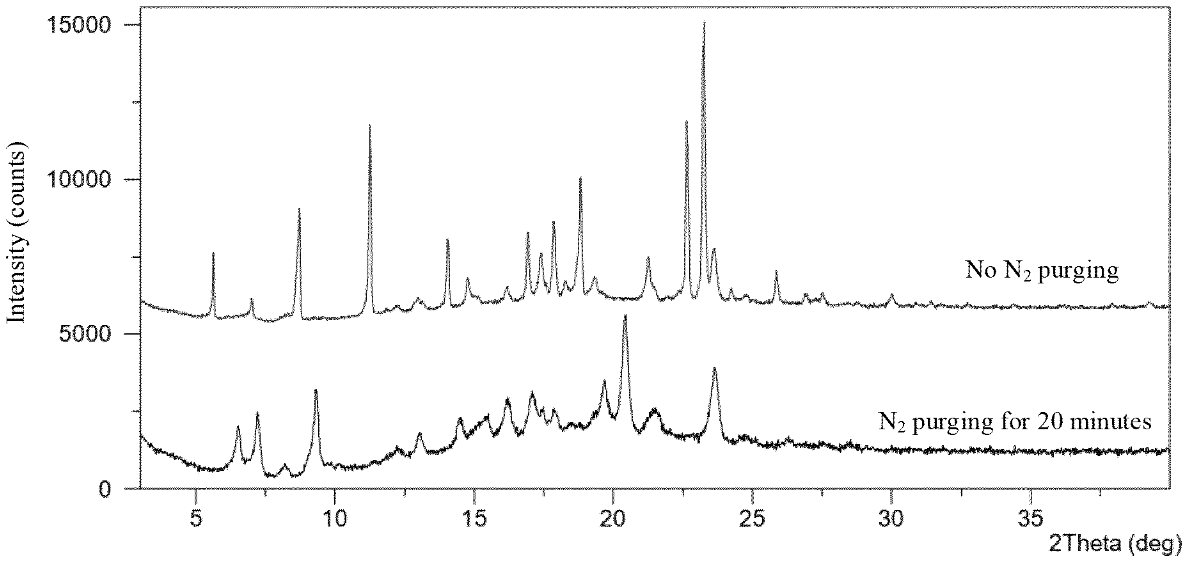
Intensity (counts)
15000
10000
5000
0
5
10
15
20
25
30
35
2Theta (deg)
No $N_2$ purging
$N_2$ purging for 20 minutes

FIG. 10

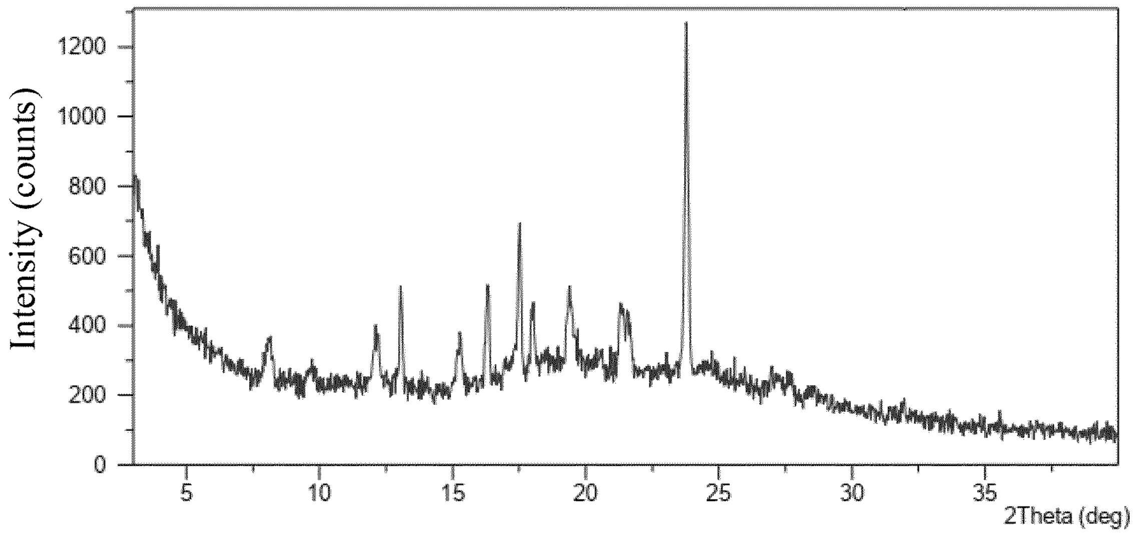

Intensity (counts)
0
200
400
600
800
1000
1200
5
10
15
20
25
30
35
2Theta (deg)


FIG. 11

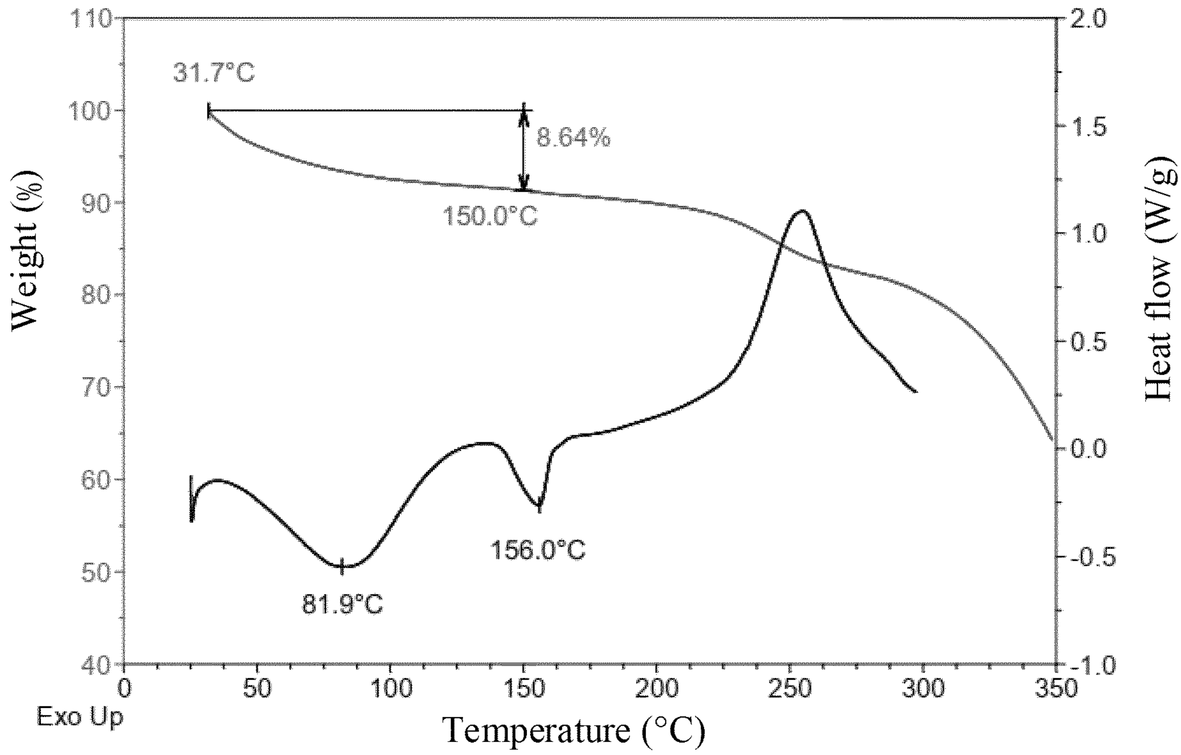

31.7°C
8.64%
150.0°C
156.0°C
81.9°C
Weight (%)
Heat flow (W/g)
110
100
90
80
70
60
50
40
2.0
1.5
1.0
0.5
0.0
-0.5
-1.0
0
50
100
150
200
250
300
350
Exo Up
Temperature (°C)


FIG. 12

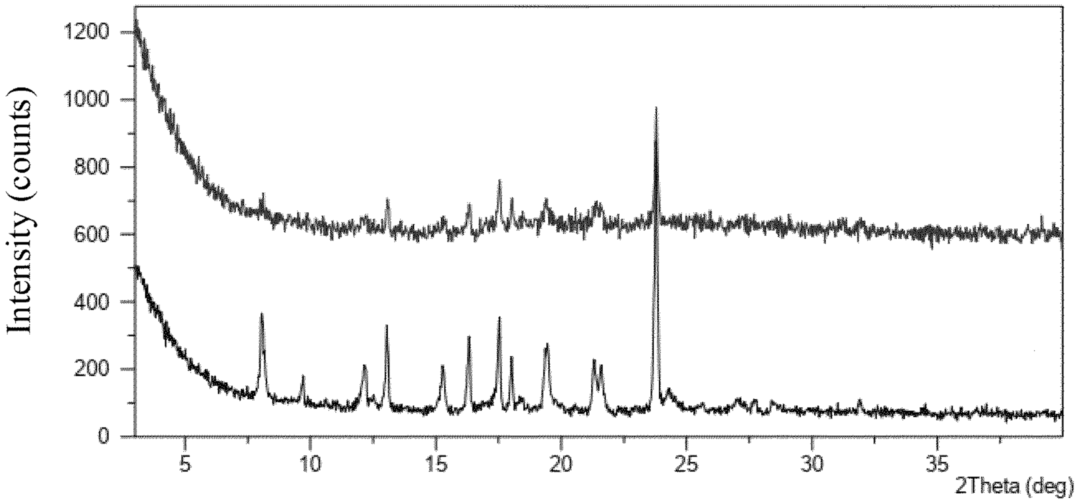
Intensity (counts)
1200
1000
800
600
400
200
0
5
10
15
20
25
30
35
2Theta (deg)

FIG. 13

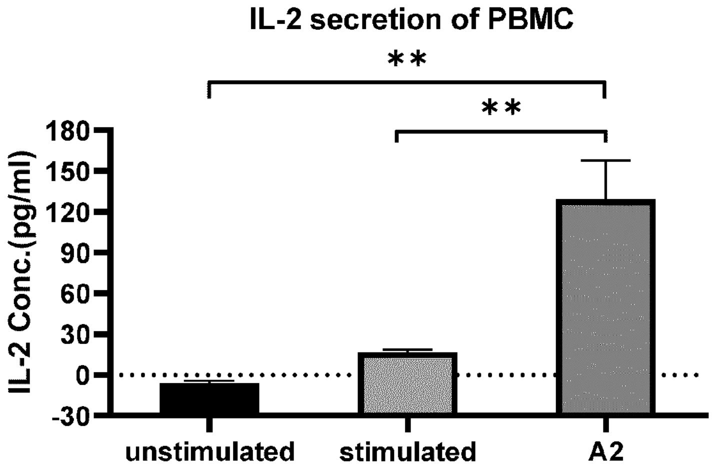
IL-2 secretion of PBMC
**
**
IL-2 Conc.(pg/ml)
180
150
120
90
60
30
0
-30
unstimulated
stimulated
A2

FIG. 14

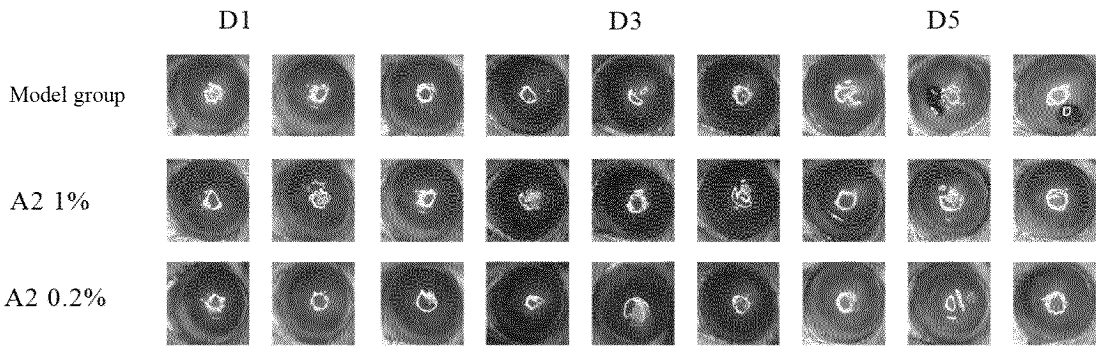
D1
D3
D5
Model group
A2 1%
A2 0.2%

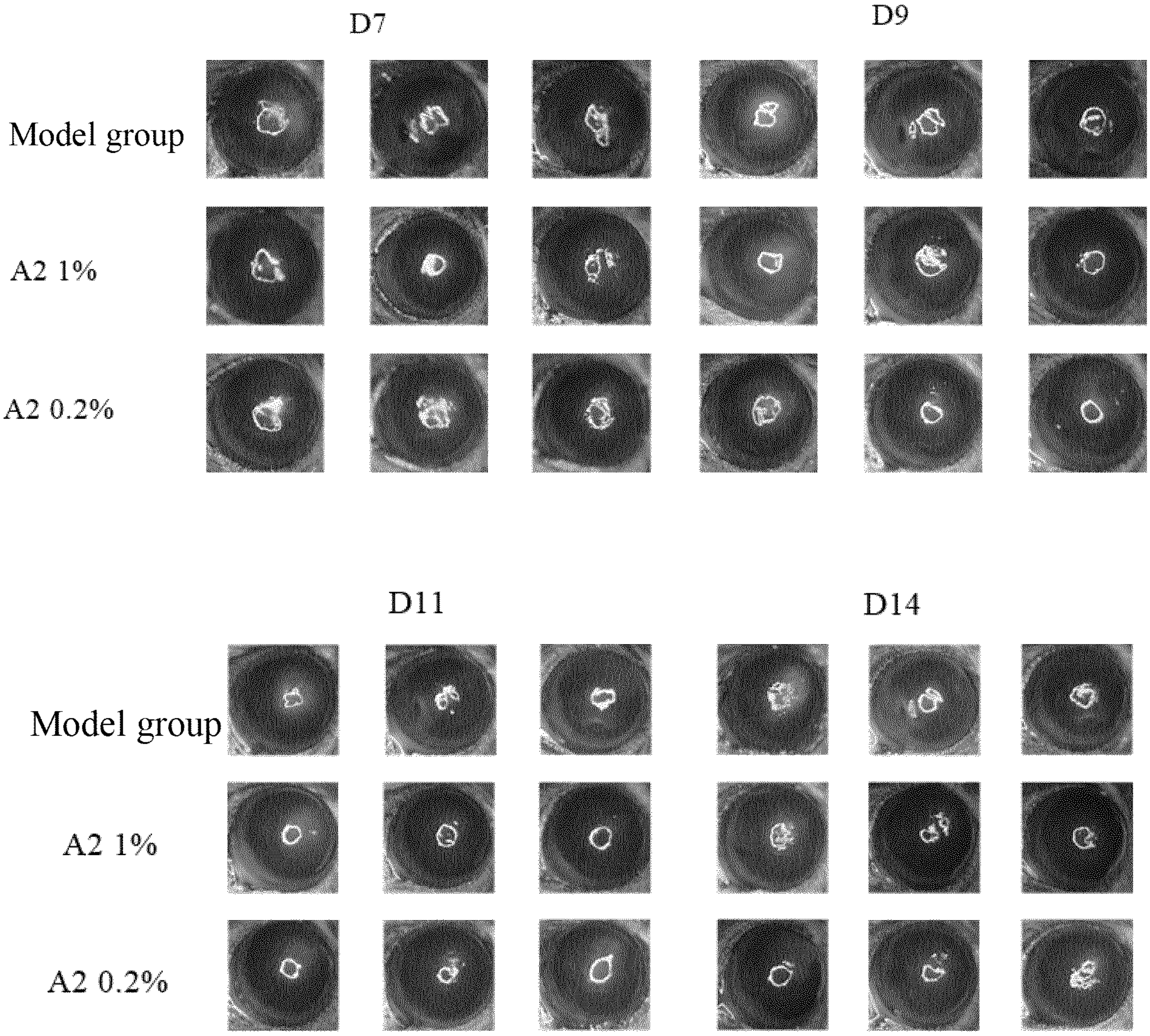


FIG. 15

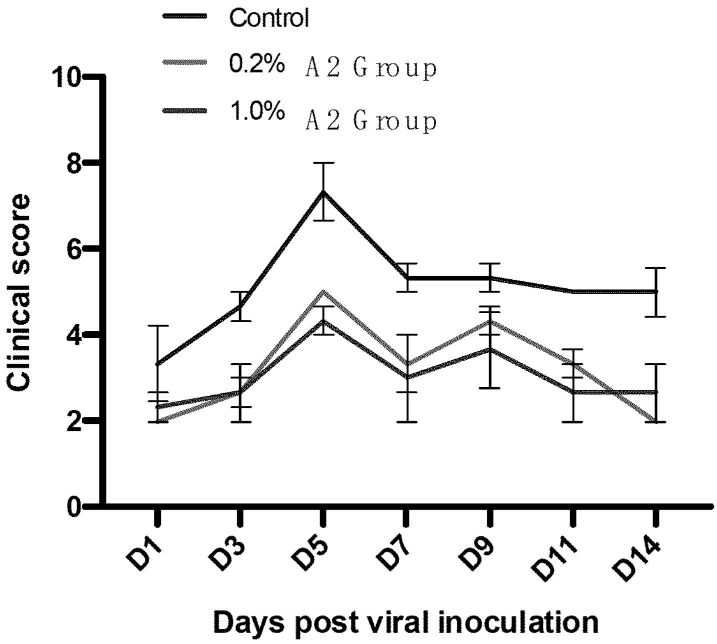
Control
0.2% A2 Group
1.0% A2 Group
10
8
6
4
2
0
Clinical score
D1
D3
D5
D7
D9
D11
D14
Days post viral inoculation

FIG. 16

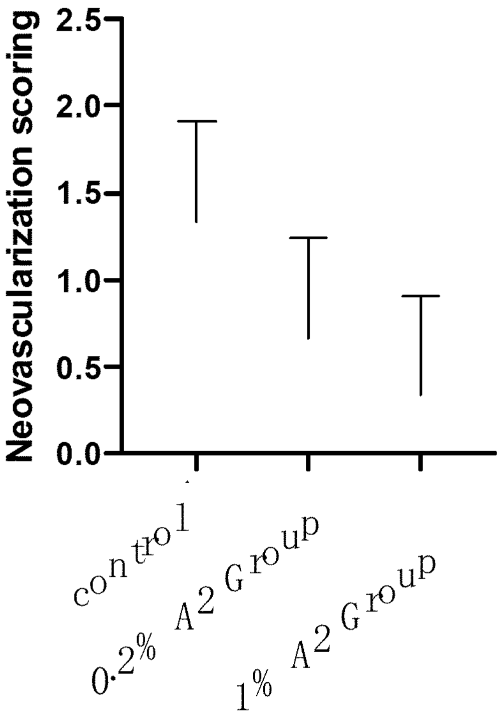
2.5
2.0
1.5
1.0
0.5
0.0
Neovascularization scoring
control
0.2% A2 Group
1% A2 Group

FIG. 17

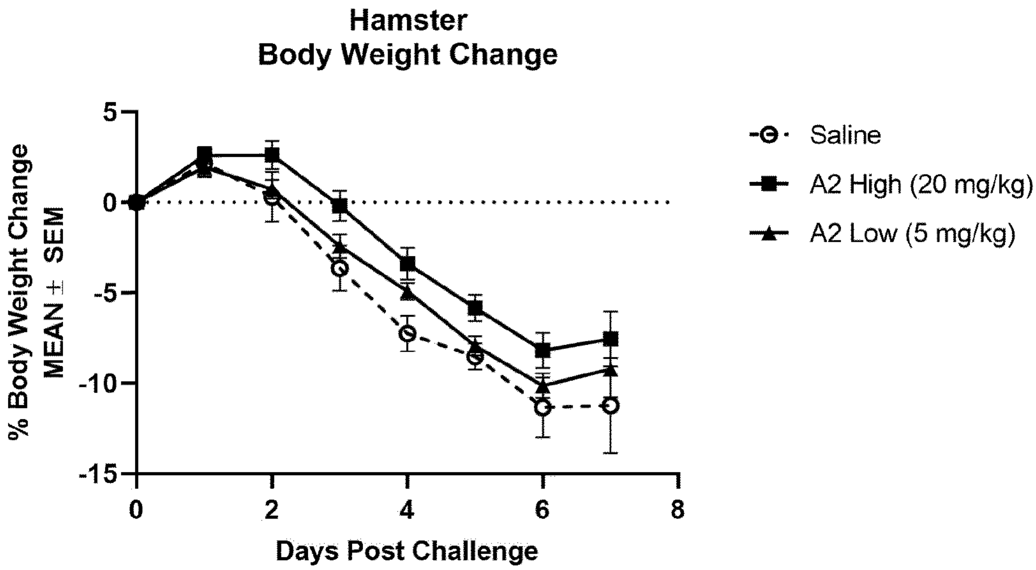
Hamster
Body Weight Change
% Body Weight Change
MEAN ± SEM
5
0
-5
-10
-15
0
2
4
6
8
Days Post Challenge
Saline
A2 High (20 mg/kg)
A2 Low (5 mg/kg)

FIG. 18

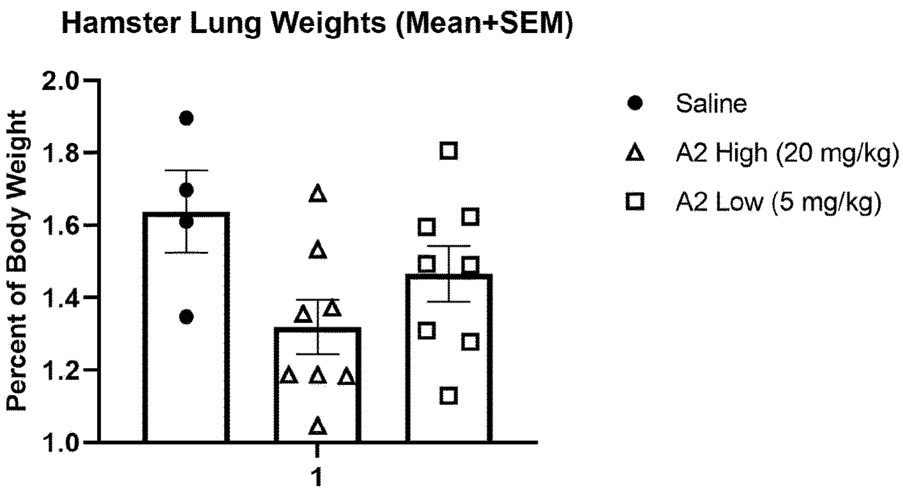
Hamster Lung Weights (Mean+SEM)
Percent of Body Weight
2.0
1.8
1.6
1.4
1.2
1.0
1
Saline
A2 High (20 mg/kg)
A2 Low (5 mg/kg)

FIG. 19

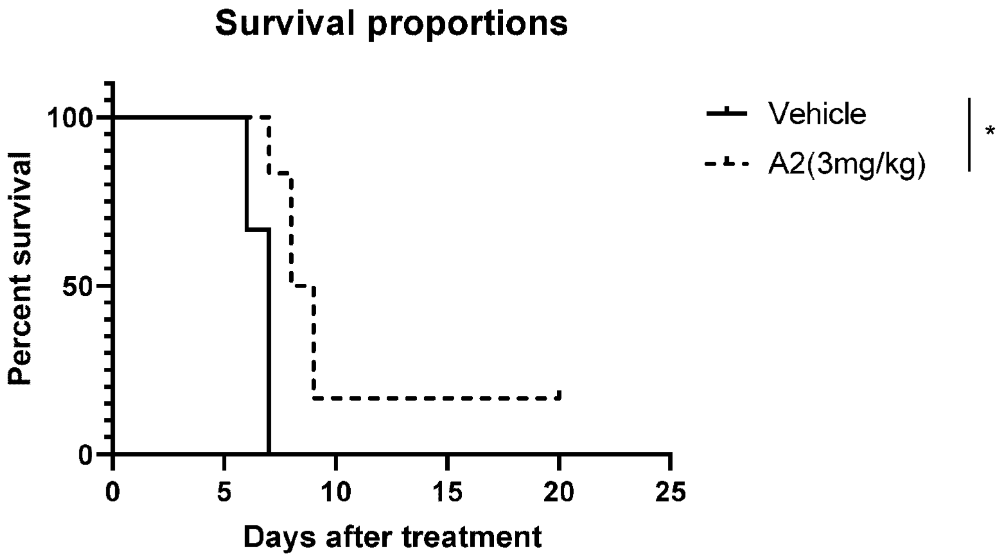
Survival proportions
Percent survival
100
50
0
0
5
10
15
20
25
Days after treatment
Vehicle
A2(3mg/kg)
*

FIG. 20

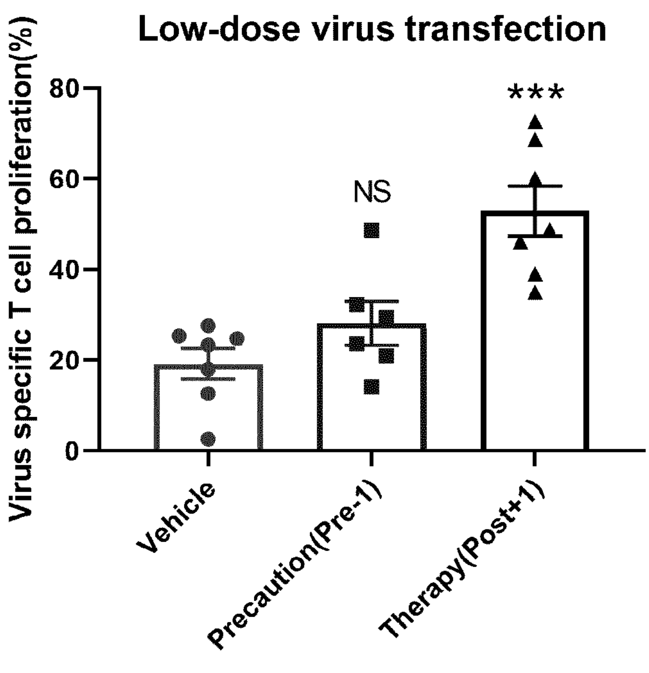
Low-dose virus transfection
Virus specific T cell proliferation(%)
80
60
40
20
0
NS
***
Vehicle
Precaution(Pre-1)
Therapy(Post+1)

FIG. 21

## INTERNATIONAL SEARCH REPORT

International application No.
**PCT/CN2023/084751**

### A. CLASSIFICATION OF SUBJECT MATTER

A61K 31/4545(2006.01)i; A61K 31/4439(2006.01)i; A61K 31/496(2006.01)i; A61K 31/551(2006.01)i; C07D 417/14(2006.01)i; C07D 417/04(2006.01)i; C07D 401/14(2006.01)i; C07D 401/04(2006.01)i; C07D 421/14(2006.01)i; A61P 31/00(2006.01)i; A61P 31/04(2006.01)i; A61P 31/10(2006.01)i; A61P 31/12(2006.01)i; A61P 31/14(2006.01)i; A61P 31/20(2006.01)i; A61P 33/00(2006.01)i; A61P 35/00(2006.01)i; A61P 35/02(2006.01)i

According to International Patent Classification (IPC) or to both national classification and IPC

### B. FIELDS SEARCHED

Minimum documentation searched (classification system followed by classification symbols)

IPC: A61K, A61P, C07D

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CJFD, CNKI, PUBMED, REGISTRY, CAPLUS: HPK1激酶, 病原体, 病毒, 感染, 癌症, 肿瘤, 白血病, HPK1 kinase, pathogen, virus, infection, cancer, tumor, leukemia, 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| PX | CN 114767676 A (ZHUHAI YUFAN BIOTECHNOLOGIES CO., LTD.) 22 July 2022 (2022-07-22)<br>claims 1-29 | 1-29 |
| PX | CN 114437058 A (ZHUHAI YUFAN BIOTECHNOLOGIES CO., LTD.) 06 May 2022 (2022-05-06)<br>claims 1-26, and description, paragraphs [0001], [0186] and [0195]-[0197] | 1-29 |
| X | WO 2021254118 A1 (ZHUHAI YUFAN BIOTECHNOLOGIES CO., LTD.) 23 December 2021 (2021-12-23)<br>claims 1-15, description, page 18, lines 15-17, page 21, line 14-page 22 | 1-29 |
| X | CN 110396087 A (ZHUHAI YUFAN BIOTECHNOLOGIES CO., LTD.) 01 November 2019 (2019-11-01)<br>abstract, and claims 1-7 and 9-14 | 27-29 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 June 2023** | **30 June 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/084751**

**C. DOCUMENTS CONSIDERED TO BE RELEVANT**

| Category* | Citation of document, with indication, where appropriate, of the relevant passages | Relevant to claim No. |
|---|---|---|
| X | WO 2019196937 A1 (EAST CHINA UNIVERSITY OF SCIENCE AND TECHNOLOGY) 17 October 2019 (2019-10-17)<br>description, page 6, lines 2-30, embodiments 1-6, 8-13, 15-21, 24, 30, 32, 34-36, 40-42, 44-54 and 56 | 1-15, 27-29 |
| X | CN 101023064 A (PFIZER INC.) 22 August 2007 (2007-08-22)<br>claims 11-15, and description, page 10, paragraph 3, page 31, last paragraph-page 32, embodiments 6, 8 and 10-19 | 27-29 |

Form PCT/ISA/210 (second sheet) (July 2022)

**INTERNATIONAL SEARCH REPORT**
**Information on patent family members**

International application No.
**PCT/CN2023/084751**

| Patent document cited in search report | | | Publication date (day/month/year) | Patent family member(s) | | | Publication date (day/month/year) |
|---|---|---|---|---|---|---|---|
| CN | 114767676 | A | 22 July 2022 | None | | | |
| CN | 114437058 | A | 06 May 2022 | WO | 2022089225 | A1 | 05 May 2022 |
| WO | 2021254118 | A1 | 23 December 2021 | EP | 4154881 | A1 | 29 March 2023 |
| | | | | KR | 20230026387 | A | 24 February 2023 |
| | | | | AU | 2021290594 | A1 | 02 February 2023 |
| | | | | BR | 112022025714 | A2 | 03 January 2023 |
| | | | | CA | 3182850 | A1 | 23 December 2021 |
| | | | | CO | 2022018508 | A2 | 30 December 2022 |
| CN | 110396087 | A | 01 November 2019 | PL | 3781563 | T3 | 03 October 2022 |
| | | | | JP | 2021519827 | A | 12 August 2021 |
| | | | | JP | 7212142 | B2 | 24 January 2023 |
| | | | | IL | 278036 | A | 30 November 2020 |
| | | | | CA | 3097949 | A1 | 31 October 2019 |
| | | | | AU | 2019260159 | A1 | 26 November 2020 |
| | | | | ES | 2927346 | T3 | 04 November 2022 |
| | | | | KR | 20210005668 | A | 14 January 2021 |
| | | | | US | 2021276994 | A1 | 09 September 2021 |
| | | | | EP | 3781563 | A1 | 24 February 2021 |
| | | | | EP | 3781563 | A4 | 29 June 2022 |
| | | | | BR | 112020021862 | A2 | 26 January 2021 |
| WO | 2019196937 | A1 | 17 October 2019 | US | 2021179598 | A1 | 17 June 2021 |
| | | | | KR | 20210023814 | A | 04 March 2021 |
| | | | | EP | 3782993 | A1 | 24 February 2021 |
| | | | | EP | 3782993 | A4 | 08 June 2022 |
| | | | | JP | 2021521214 | A | 26 August 2021 |
| CN | 101023064 | A | 22 August 2007 | AP | 2007003888 | A0 | 28 February 2007 |
| | | | | AP | 200703888 | A0 | 07 March 2012 |
| | | | | HK | 1105414 | A1 | 15 February 2008 |
| | | | | CA | 2578066 | A1 | 02 March 2006 |
| | | | | CA | 2578066 | E | 11 October 2011 |
| | | | | US | 2006046991 | A1 | 02 March 2006 |
| | | | | US | 7858643 | B2 | 28 December 2010 |
| | | | | US | 2012263706 | A1 | 18 October 2012 |
| | | | | US | 8785632 | B2 | 22 July 2014 |
| | | | | CR | 8860 | A | 24 July 2007 |
| | | | | SI | 1786785 | T1 | 30 July 2010 |
| | | | | EA | 200700352 | A1 | 31 August 2007 |
| | | | | EA | 013678 | B1 | 30 June 2010 |
| | | | | NZ | 552866 | A | 25 June 2010 |
| | | | | WO | 2006021884 | A2 | 02 March 2006 |
| | | | | WO | 2006021884 | A3 | 22 June 2006 |
| | | | | ECSP | 077276 | A | 29 March 2007 |
| | | | | TNSN | 07070 | A1 | 02 June 2008 |
| | | | | NO | 2013008 | I1 | 29 April 2013 |
| | | | | NO | 2013008 | I2 | 21 October 2013 |
| | | | | EP | 1786785 | A2 | 23 May 2007 |
| | | | | EP | 1786785 | B1 | 07 April 2010 |
| | | | | EP | 1786785 | B9 | 22 May 2013 |
| | | | | CY | 1110044 | T1 | 14 January 2015 |

Form PCT/ISA/210 (patent family annex) (July 2022)

**INTERNATIONAL SEARCH REPORT**
**Information on patent family members**

International application No.
**PCT/CN2023/084751**

| Patent document cited in search report | Publication date (day/month/year) | Patent family member(s) | | | Publication date (day/month/year) |
|---|---|---|---|---|---|
| | | UY | 29081 | A1 | 31 March 2006 |
| | | MA | 28828 | B1 | 01 August 2007 |
| | | AP | 200703888 | D0 | 28 February 2007 |
| | | AU | 2005276135 | A1 | 02 March 2006 |
| | | AU | 2005276135 | B2 | 28 April 2011 |
| | | MX | 2007002312 | A | 16 April 2007 |
| | | PA | 8643301 | A1 | 03 July 2006 |
| | | AR | 050788 | A1 | 22 November 2006 |
| | | DE | 602005020465 | D1 | 20 May 2010 |
| | | KR | 20070038562 | A | 10 April 2007 |
| | | KR | 100859891 | B1 | 23 September 2008 |
| | | GEP | 20104906 | B | 25 February 2010 |
| | | RS | 51362 | B | 28 February 2011 |
| | | JP | 2008510790 | A | 10 April 2008 |
| | | JP | 4242911 | B2 | 25 March 2009 |
| | | TW | 200621715 | A | 01 July 2006 |
| | | TWI | 358406 | B | 21 February 2012 |
| | | PE | 20060501 | A1 | 28 June 2006 |
| | | NO | 20071290 | L | 29 May 2007 |
| | | NO | 333231 | B1 | 15 April 2013 |
| | | ZA | 200700127 | B | 27 August 2008 |
| | | NI | 200700057 | A | 07 March 2008 |
| | | DK | 1786785 | T3 | 31 May 2010 |
| | | PL | 1786785 | T3 | 31 August 2010 |
| | | HRP | 20100298 | T1 | 30 June 2010 |
| | | LU | 92155 | I2 | 22 April 2013 |
| | | LU | 92155 | I9 | 04 January 2019 |
| | | US | 2014288086 | A1 | 25 September 2014 |
| | | NL | 1029799 | A1 | 28 February 2006 |
| | | NL | 1029799 | C2 | 17 October 2006 |
| | | US | 2010324061 | A1 | 23 December 2010 |
| | | BR | 122020017756 | B1 | 15 February 2022 |
| | | HN | 2005000476 | A | 07 December 2009 |
| | | BRPI | 0513915 | A | 20 May 2008 |
| | | IL | 181384 | A0 | 04 July 2007 |
| | | IL | 181384 | A | 30 August 2012 |
| | | MY | 145177 | A | 30 December 2011 |
| | | PT | 1786785 | E | 21 May 2010 |
| | | GT | 200500225 | A | 21 March 2006 |
| | | UA | 87153 | C2 | 25 June 2009 |
| | | AT | 463486 | T | 15 April 2010 |
| | | ME | 01788 | B | 28 February 2011 |
| | | ES | 2341351 | T3 | 18 June 2010 |
| | | ES | 2341351 | T9 | 23 July 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 113861188 A
- CN 113316576 A
- WO 2021213317 A1
- CN 110396087 A


### Non-patent literature cited in the description


- **GRIFONI A** ; **WEISKOPF D** ; **RAMIREZ S I** ; **MATEUS J** ; **DAN J M** ; **MODERBACHER C R** ; **RAWLINGS S A** ; **SUTHERLAND A** ; **PREMKUMAR L** ; **JADI R S**. Targets of T Cell Responses to SARS-CoV-2 Coronavirus in Humans with COVID-19 Disease and Unexposed Individuals[J. *Cell*, 2020, vol. 181 (7), 1489-1501
- **LIAO W** ; **LIN J-X** ; **LEONARD W J.** IL-2 Family Cytokines: New Insights into the Complex Roles of IL-2 as a Broad Regulator of T helper Cell Differentiation[J. *Current Opinion in Immunology*, 2011, vol. 23 (5), 598-604
- **CHANNAPPANAVAR R** ; **ZHAO J** ; **PERLMAN S**. T cell-mediated immune response to respiratory coronaviruses[J].. *Immunologic Research*, 2014, vol. 59 (1), 118-128
- **WADA, E et al.** *Seikagaku*, 1994, vol. 66, 15
- **GANNES, LZ et al.** *Comp Biochem Physiol Mol Integr Physiol*, 1998, vol. 119, 725